(19) Europäisches Patentamt
European Patent Office
Office européen des brevets

(11) EP 4 722 217 A1

(12) **EUROPEAN PATENT APPLICATION**
published in accordance with Art. 153(4) EPC

(43) Date of publication:
**08.04.2026 Bulletin 2026/15**

(21) Application number: **24830933.8**

(22) Date of filing: **28.06.2024**

(51) International Patent Classification (IPC):
***C07D 487/08** (2006.01)* ***C07D 519/00** (2006.01)*
***A61P 35/00** (2006.01)* ***A61P 35/02** (2006.01)*
***A61K 31/517** (2006.01)* ***A61K 31/519** (2006.01)*

(52) Cooperative Patent Classification (CPC):
**A61K 31/517; A61K 31/519; A61P 35/00; A61P 35/02; C07D 487/08; C07D 519/00**

(86) International application number:
**PCT/CN2024/102179**

(87) International publication number:
**WO 2025/002302 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.06.2023 CN 202310772640**

(71) Applicant: **Sunshine Lake Pharma Co., Ltd.**
**Dongguan, Guangdong 523000 (CN)**

(72) Inventors:
- **LIU, Haiwang**
  **Dongguan, Guangdong 523871 (CN)**
- **XIE, Hongming**
  **Dongguan, Guangdong 523871 (CN)**
- **FENG, Xihui**
  **Dongguan, Guangdong 523871 (CN)**
- **LUO, Guolin**
  **Dongguan, Guangdong 523871 (CN)**
- **ZHANG, Yingjun**
  **Dongguan, Guangdong 523871 (CN)**

(74) Representative: **Becker Kurig & Partner Patentanwälte mbB**
**Bavariastraße 7**
**80336 München (DE)**

# (54) PYRIMIDO-PYRIDINE COMPOUND, PHARMACEUTICAL COMPOSITION THEREOF AND USE THEREOF

(57) A pyrimido-pyridine compound, a pharmaceutical composition thereof and the use thereof. The compound and the pharmaceutical composition thereof as KRAS G12D inhibitors can be used for preparing a drug for preventing or treating KRAS G12D-related diseases, and can be particularly used for preparing a drug for preventing or treating cancers.

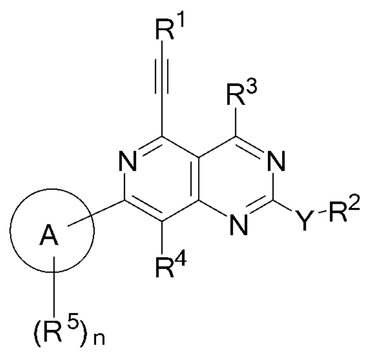

(I)

EP 4 722 217 A1

## Description

### FIELD OF THE INVENTION

The present invention belongs to the field of medicine, and specifically relates to a new class of pyrimidopyridine compounds as KRAS G12D inhibitors, pharmaceutical compositions thereof, and the use of such compounds and pharmaceutical compositions thereof in the preparation of drugs for preventing or treating KRAS G12D-related diseases.

### BACKGROUND ART

The RAS gene is one of the most commonly mutated genes in cancer (20%-25%). Currently known members of the RAS gene family include KRAS, NRAS and HRAS, among which KRAS mutation is the most common, accounting for approximately 85%. The mutation rate of KRAS is highest in pancreatic ductal adenocarcinoma (PDAC), reaching 97%, followed by colorectal cancer, multiple myeloma and lung cancer, at 52%, 42% and 32%, respectively. The most common KRAS gene mutation is point mutation, and common mutation forms include KRAS G12D mutation (41%), KRAS G12V (28%), and KRAS G12C (14%) mutations. RAS gene mutations are often associated with poor prognosis in cancer. KRAS can be transiently activated by upstream growth factors or tyrosine kinases (such as EGFR). The activated KRAS can activate downstream pathways, including the PI3K-AKT-mTOR signaling pathway that controls cell generation and the RAS-RAF-MEK-ERK signaling pathway that controls cell proliferation. This also lays a biological foundation for the combination of many targets.

In recent years, some progress has been made in drug development using the allosteric sites of the KRAS G12C mutant. For example, in 2013, a research group reported the discovery of a small molecule inhibitor of KRAS G12C (Nature, 2013, 503, 548-551); however, there has been little research on KRAS G12D. Compared with KRAS G12C mutation, the proportion of other mutation types such as KRAS G12D and KRAS G12V is higher in pancreatic cancer. Therefore, inhibiting KRAS G12D mutation is a potentially effective approach for treating pancreatic cancer. Among them, Mirati announced a series of KRAS G12D inhibitors in patent application WO20211041671, which showed specificity for KRAS G12D mutants and had anti-pancreatic cancer activity.

Currently, the development of compounds that target and inhibit KRAS G12D mutations to treat KRAS G12D-related diseases remains highly attractive and urgently needed.

### SUMMARY

The present invention provides a compound, or a pharmaceutical composition thereof, which can be used as a KRAS inhibitor, particularly a KRAS G12D inhibitor. The present invention also relates to the use of the compound or pharmaceutical composition thereof for preparing a medicament for treating diseases and/or disorders, especially cancer, by inhibiting KRAS activity.

The compounds of the present invention are a class of non-covalent KRAS G12D specific inhibitors that can effectively bind to KRAS G12D-GTP and inhibit the phosphorylation of ERK downstream of KRAS G12D.

In one aspect, the present invention provides a compound having Formula (I) or a stereoisomer, a tautomer, an N-oxide, a solvate, a metabolite, a pharmaceutically acceptable salt or a prodrug thereof,

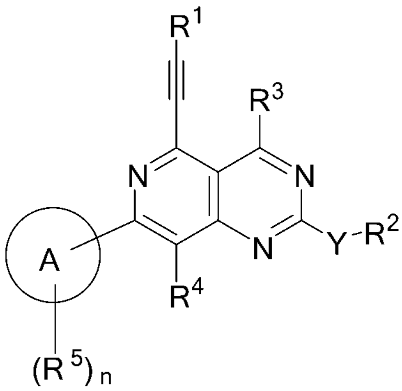

(I),

wherein,

$R^1$ is -H, -D, -CN, $-NH_2$, -C(=O)H, -C(=O)OH, $-C(=O)OR^{6a}$, $-C(=O)NR^6R^7$, $-NR^6C(=O)R^7$, - $NR^{6a}C(=O)NR^6R^7$,

$-C(=O)R^{6a}$, $-NR^6S(=O)_2R^7$, $-S(=O)_2NR^6R^7$, $-NR^{6a}S(=O)_2NR^6R^7$, $-NR^6R^7$, $-C_{1-6}$ alkyl-$NR^6C(=O)R^7$, $-C_{1-6}$ alkyl-$NR^6R^7$, $-C_{1-6}$ alkyl-$C(=O)OR^6$, $-C_{1-6}$ alkyl-$C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl or 3-12 membered heterocyclyl; wherein each of the $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl and 3-12 membered heterocyclyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$ and $C_{1-6}$ alkyl;

Y is bond, O or S;

$R^2$ is

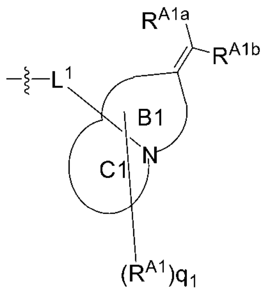 ,

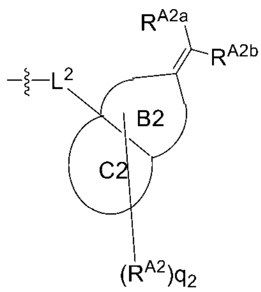 ,

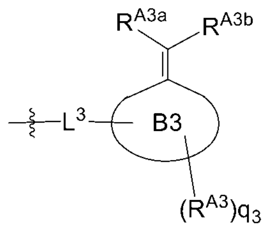 ,

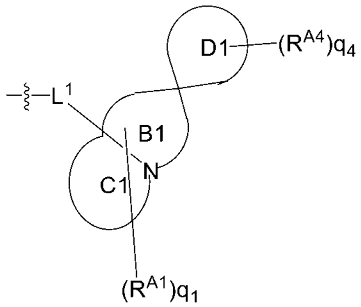 ,

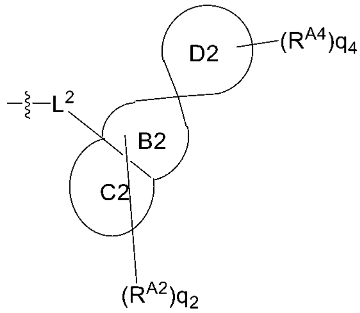 or

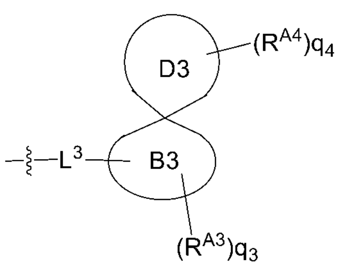 ;

each $L^1$, $L^2$ and $L^3$ is independently $C_{1-6}$ alkylene or $C_{1-6}$ heteroalkylene, wherein the $C_{1-6}$ alkylene and $C_{1-6}$ heteroalkylene are each independently optionally substituted with 1, 2, 3 or 4 $R^a$;

$R^a$ is -D, -OH, -F, -Cl, -Br, -I, -CN, $-NH_2$, -C(=O)H, -C(=O)OH, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{3-6}$ cycloalkyl or 3-6 membered heterocyclyl; or two $R^a$ attached to the same carbon atom together form $C_{3-6}$ cycloalkyl;

each Ring B1 and ring C1 is independently 3-7 membered monocyclic heterocyclyl;

each Ring B2, ring B3 and ring C2 is independently $C_{3-7}$ monocyclic cycloalkyl or 3-7 membered monocyclic heterocyclyl;

each of Ring D1, ring D2 and ring D3 is independently $C_{3-7}$ monocyclic cycloalkyl or 3-7 membered monocyclic heterocyclyl;

each $R^{A1}$, $R^{A2}$, $R^{A3}$ and $R^{A4}$ is independently -H, -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl;

or, two $R^{A1}$ attached to the same carbon atom together form

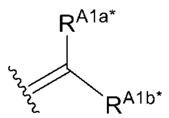 ;

or, two $R^{A1}$ together with the same carbon atom to which they are attached, form $C_{3-6}$ cycloalkyl or 3-6 membered

heterocyclyl, wherein each of the $C_{3-6}$ cycloalkyl and 3-6 membered heterocyclyl is independently optionally substituted with 1, 2, 3 or 4 $R^{8a}$; or, two $R^{A1}$ attached to two adjacent atoms together with the two adjacent atoms to which they are attached, form $C_{3-6}$ cycloalkyl or a 3-6 membered heterocyclyl, wherein each of the $C_{3-6}$ cycloalkyl and 3-6 membered heterocyclyl is independently optionally substituted with 1, 2, 3 or 4 $R^{8a*}$;
or, two $R^{A2}$ attached to the same carbon atom together form

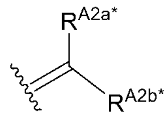
;

or, two $R^{A2}$ together with the same carbon atom to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{9a}$; or, two $R^{A2}$ attached to two adjacent atoms together with the two adjacent atoms to which they are attached, form $C_{3-6}$ cycloalkyl or a 3-6 membered heterocyclyl, wherein each of the $C_{3-6}$ cycloalkyl and 3-6 membered heterocyclyl is independently optionally substituted with 1, 2, 3 or 4 $R^{9a*}$;
or, two $R^{A3}$ attached to the same carbon atom together form

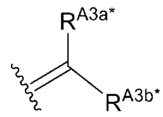
;

or, two $R^{A3}$ together with the same carbon atom to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{10a}$; or, two $R^{A3}$ attached to two adjacent atoms together with the two adjacent atoms to which they are attached, form $C_{3-6}$ cycloalkyl or a 3-6 membered heterocyclyl, wherein each of the $C_{3-6}$ cycloalkyl and 3-6 membered heterocyclyl is independently optionally substituted with 1, 2, 3 or 4 $R^{10a*}$;
each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$C_{1-6}$ alkyl-$NR^6C(=O)R^7$, -$C_{1-6}$ alkyl-$NR^6R^7$, -$C_{1-6}$ alkyl-$C(=O)OR^6$, -$C_{1-6}$ alkyl-$C(=O)NR^6R^7$, $C_{1-6}$ alkyl, $C_{1-6}$ haloalkyl, $C_{1-6}$ cyanoalkyl, $C_{1-6}$ hydroxyalkyl, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{6-12}$ aryl, 5-12 membered heteroaryl, $C_{3-12}$ cycloalkyl or 3-12 membered heterocyclyl;
each $R^{8a}$, $R^{8a*}$, $R^{9a}$, $R^{9a*}$, $R^{10a}$ and $R^{10a*}$ is independently -D, -OH, -F, -Cl, -Br, -I, -CN, -$NH_2$, -C(=O)OH, - $C(=O)NH_2$, oxo, $C_{1-6}$ alkyl, $C_{1-6}$ haloalkyl, $C_{1-6}$ alkoxy, $C_{1-6}$ hydroxyalkyl, $C_{6-10}$ aryl, 5-10 membered heteroaryl, -$C(=O)OC_{1-6}$ alkyl, -$C(=O)NHC_{1-6}$ alkyl or -$C(=O)N(C_{1-6}$ alkyl$)_2$;
$R^3$ is

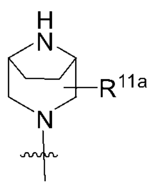
,
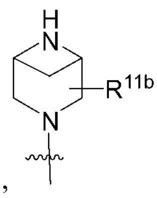
,
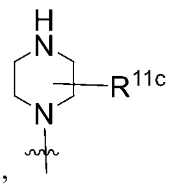
or
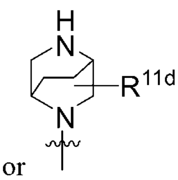
;

each $R^{11}$a, $R^{11b}$, $R^{11c}$ and $R^{11d}$ is independently -H, -D, -OH, -SH, -F, -Cl, -Br, -I, -CN, -C(=O)H, -$C(=O)OR^{6a}$, -C(=O) $NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ haloalkoxy or 5-6 membered heteroaryl;
$R^4$ is -H, -D, -OH, -SH, -F, -Cl, -Br, -I, -CN, methyl, ethyl, n-propyl, i-propyl, n-butyl or $C_{1-4}$ haloalkyl;
Ring A is $C_{6-12}$ aryl or 5-12 membered heteroaryl;
each $R^5$ is independently -D, -OH, -F, -Cl, -Br, -I, -CN, -SH, -$CH_2C(=O)NR^{6b}R^{7b}$, -$C(=O)R^{6c}$, -$C(=O)OR^{6c}$, -C(=O) $NR^{6b}R^{7b}$, -$NR^{6b}C(=O)R^{7b}$, -$NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein each of the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl is independently optionally substituted with 1, 2, 3 or 4 substituents selected from -D, -OH, -F, -Cl, -Br, -I, -CN, $-NH_2$, -C(=O)H, -C(=O)OH, $C_{1-6}$ alkyl, $C_{1-6}$ alkoxy, $C_{3-6}$ cycloalkyl and 3-6 membered heterocyclyl;

each $R^6$, $R^7$, $R^{6b}$, $R^{6b}$, $R^{6c}$, $R^{7b}$, $R^{6d}$ and $R^{7d}$ is independently -H, -D or $C_{1-6}$ alkyl, wherein the $C_{1-6}$ alkyl is independently optionally substituted with 1, 2, 3 or 4 substituents selected from -D, -OH, -F, -Cl, -Br, -I, -CN, -C(=O)H, -C(=O)OH, $-NH_2$, $C_{1-6}$ alkoxy, $C_{6-12}$ aryl, $C_{3-6}$ cycloalkyl and 3-6 membered heterocyclyl;

n is 0, 1, 2, 3, 4, 5, 6 or 7; and

each $q_1$, $q_2$ and $q_3$ is independently 0, 1, 2, 3, 4, 5 or 6;

each $q_4$ is independently 0, 1, 2, 3 or 4;

wherein, the compound of formula (I) is not

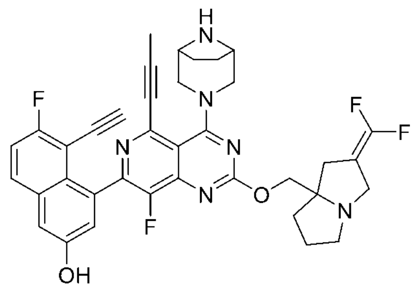

and

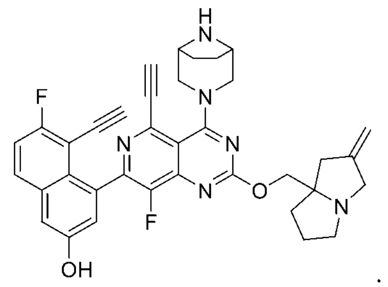

.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, $-NR^{6a}S(=O)_2NR^6R^7$, $-NR^6R^7$, $-C_{1-4}$ alkyl-$NR^6C(=O)R^7$, $-C_{1-4}$ alkyl-$NR^6R^7$, $-C_{1-4}$ alkyl-$C(=O)OR^6$, $-C_{1-4}$ alkyl-$C(=O)NR^6R^7$, $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{1-a}$ alkoxy $C_{1-4}$ alkyl, ($C_{3-6}$ cycloalkyl)-$C_{1-4}$ alkyl, ($C_{7-12}$ cycloalkyl)-$C_{l-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl or 3-6 membered heterocyclyl; wherein each of the $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{1-4}$ alkoxy $C_{1-a}$ alkyl, ($C_{3-6}$ cycloalkyl)-$C_{l-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-$C_{1-4}$ alkyl )-O-$C_{1-4}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl and 3-6 membered heterocyclyl is independently optionally substituted with 1, 2, 3 or 4 substituents selected from D, - OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$ and $C_{1-4}$ alkyl; wherein, $R^6$, $R^7$, $R^{6a}$, $R^{6e}$ and $R^{7e}$ are each as defined herein.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, $-NR^{6a}S(=O)_2NR^6R^7$, $-NR^6R^7$, $-CH_2NR^6C(=O)R^7$, $-CH_2NR^6R^7$, $-(CH_2)_2NR^6R^7$, $-CH_2C(=O)OR^6$, $-(CH_2)_2C(=O)OR^6$, $-(CH_2)_3C(=O)OR^6$, $-CH_2C(=O)NR^6R^7$, $-(CH_2)_2C(=O)NR^6R^7$, $-(CH_2)_3C(=O)NR^6R^7$, $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-CH_2CN$, $-(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, $-CH(OH)CH_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCH_3$, $-OCH_2CH_3$, $-O(CH_2)_2CH_3$, $-O(CH_2)_3CH_3$, $-OC(CH_3)_3$, $-OCH(CH_3)_2$, $-CH_2OCH_3$, $-(CH_2)_2OCH_3$, $-(CH_2)_2OCH_2CH_3$, $-CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-cyclopropyl, $-CH_2$-cyclobutyl, $-(CH_2)_2$-cyclobutyl, $-CH_2$-cyclopentyl, $-(CH_2)_2$cyclopentyl, $-CH_2$-azetidinyl, $-CH_2$-pyrrolidinyl, $-CH_2$-piperidyl, $-CH_2$-morpholinyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, $-CH_2O$-spiro[2.3]hexyl, $-CH_2O$-spiro[3.3]heptanyl, $-CH_2SH$, $-(CH_2)_2SH$,

phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl or pyrrolidinyl; wherein each of the $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-CH_2CN$, $-(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, $-CH(OH)CH_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCH_3$, $-OCH_2CH_3$, $-O(CH_2)_2CH_3$, $-O(CH_2)_3CH_3$, $-OC(CH_3)_3$, $-OCH(CH_3)_2$, $-CH_2OCH_3$, $-(CH_2)_2OCH_3$, $-(CH_2)_2OCH_2CH_3$, $-CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-cyclopropyl, $-CH_2$-cyclobutyl, $-(CH_2)_2$-cyclobutyl, $-CH_2$-cyclopentyl, $-(CH_2)_2$cyclopentyl, $-CH_2$-azetidinyl, $-CH_2$-pyrrolidinyl, $-CH_2$-piperidyl, $-CH_2$-morpholinyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, $-CH_2O$-spiro[2.3]hexyl, $-CH_2O$-spiro[3.3]heptanyl, -phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl and pyrrolidinyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$, methyl, ethyl, n-propyl, isopropyl, n-butyl, isobutyl or tert-butyl; wherein, $R^6$, $R^7$, $R^{6a}$, $R^{6e}$ and $R^{7e}$ are each as defined herein.

In some embodiments, $R^{A1}$, $R^{A2}$, $R^{A3}$ and $R^{A4}$ are each independently -H, -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{l-4}$ alkyl, $C_{3-6}$ cycloalkyl or 3-6 membered heterocyclyl; or, $R^{A1}$, $R^{A2}$, $R^{A3}$ and $R^{A4}$ are each independently -H, -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-OCH_3$, $-OCH_2CH_3$, $-OCH_2CH_3$, $-OC(CH_3)_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCF_3$, $-OCHF_2$, $-CH_2$-phenyl, $-CH_2$-pyridyl, $-CH_2$-pyrrolidinyl, $-CH_2$-piperidinyl, $-CH_2$-cyclopropyl, $-CH_2$-cyclopentyl, cyclopropyl, cyclobutyl, cyclopentyl, cyclohexyl, oxiranyl, oxetanyl, tetrahydropyranyl, morpholinyl, azetidinyl or pyrrolidinyl; wherein, $R^{6d}$ and $R^{7d}$ are each as defined herein;

or, two $R^{A1}$ attached to the same carbon atom together form

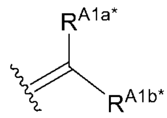

;

or, two $R^{A1}$ together with the same carbon atom to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl ais independently optionally substituted with 1, 2, 3 or 4 $R^{8a}$; or, two $R^{A1}$ attached to two adjacent atoms together with the two adjacent atoms to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{8a*}$;

or, two $R^{A2}$ attached to the same carbon atom together form

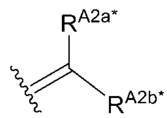

;

or, two $R^{A2}$ together with the same carbon atom to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{9a}$; or, two $R^{A2}$ attached to two adjacent atoms together with the two adjacent atoms to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{9a*}$;
or, two $R^{A3}$ attached to the same carbon atom together form

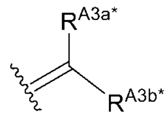
;

or, two $R^{A3}$ together with the same carbon atom to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{10a}$; or, two $R^{A3}$ attached to two adjacent atoms together with the two adjacent atoms to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{10a*}$;
each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$C_{1-4}$ alkyl-$NR^6C(=O)R^7$, -$C_{1-4}$ alkyl-$NR^6R^7$, -$C_{1-4}$ alkyl-$C(=O)OR^6$, -$C_{1-4}$ alkyl-$C(=O)NR^6R^7$, $C_{1-4}$ alkyl, $C_{1-4}$ haloalkyl, $C_{1-4}$ cyanoalkyl, $C_{1-4}$ hydroxyalkyl, $C_{1-4}$ alkoxy $C_{1-4}$ alkyl, ($C_{3-6}$ cycloalkyl)-$C_{1-4}$ alkyl, (3-6 membered heterocyclyl)-$C_{1-4}$ alkyl, ($C_{3-6}$ cycloalkyl)-O-$C_{1-4}$ alkyl, (3-6 membered heterocyclyl)-O-$C_{1-4}$ alkyl, phenyl, 5-6 membered heteroaryl, $C_{3-6}$ cycloalkyl or 3-6 membered heterocyclyl; wherein, $R^6$ and Rare each as defined herein;
each $R^{8a}$, $R^{8a*}$, $R^{9a}$, $R^{9a*}$, $R^{102}$ and $R^{10a*}$ is independently -D, -OH, -F, -Cl, -Br, -I, -CN, -$NH_2$, -C(=O)OH, - C(=O)$NH_2$, oxo, $C_{1-4}$ alkyl, $C_{1-4}$ haloalkyl, $C_{1-4}$ alkoxy, $C_{1-4}$ hydroxyalkyl, phenyl, 5-6 membered heteroaryl, - C(=O)O$C_{1-4}$ alkyl, -C(=O)NH$C_{1-4}$ alkyl or -C(=O)N($C_{1-4}$ alkyl)$_2$.

In some embodiments, each $R^{A1a}$ , $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$CH_2NR^6C(=O)R^7$, -$CH_2NR^6R^7$, -$CH_2C(=O)OR^6$, -$(CH_2)_2C(=O)OR^6$, -$(CH_2)_3C(=O)OR^6$, -$CH_2C(=O)NR^6R^7$, $(CH_2)_2C(=O)NR^6R^7$, -$(CH_2)_3C(=O)NR^6R^7$, -$CH_3$, -$CH_2CH_3$, -$(CH_2)_2CH_3$, -$(CH_2)_3CH_3$, -$C(CH_3)_3$, -$CH(CH_3)_2$, -$CH_2CH(CH_3)_2$, -$(CH_2)_2CHF_2$, -$(CH_2)_2CF(CF_3)_2$, -$CF_3$, -$CHF_2$, -$CH_2F$, - $(CH_2)_2F$, -$(CH_2)_2Cl$, -$CH_2CF_3$, -$CH_2CN$, -$(CH_2)_2CN$, -$(CH_2)_3CN$, -$CH(CH_3)CN$, -$C(CH_3)_2CN$, -$CH_2OH$, - $(CH_2)_2OH$, -$(CH_2)_3OH$, -$CH(OH)CH_3$, -$CH_2OCH_3$, -$(CH_2)_2OCH_3$, -$(CH_2)_2OCH_2CH_3$, -$CH_2OCH_2CH_3$, - $CH_2OC(CH_3)_3$, -$CH_2$-cyclopropyl, -$CH_2$-cyclobutyl, -$CH_2$-cyclopentyl, -$CH_2$-cyclohexyl, -$(CH_2)_2$-cyclopentyl, -$(CH_2)_3$-cyclopentyl, -$(CH_2)_2$-cyclohexyl, -$CH_2$-cyclopentyl, -$CH_2$-azetidinyl, -$CH_2$-oxetanyl, -$CH_2$-tetrahydrofuranyl, -$CH_2$-morpholinyl, -$CH_2O$-cyclopropyl, -$CH_2O$-cyclobutyl, -$(CH_2)_2O$-cyclobutyl, -$CH_2O$-cyclopentyl, -$(CH_2)_2O$-cyclopentyl, -$CH_2O$-azetidinyl, -$CH_2O$-oxetanyl, -$CH_2O$-tetrahydrofuranyl, -$CH_2O$-morpholinyl, phenyl, pyridinyl, pyrimidinyl, cyclopropyl, cyclobutyl, cyclopentyl, cyclohexyl, oxiranyl, oxetanyl, tetrahydropyranyl, morpholinyl, azetidinyl or pyrrolidinyl; wherein, $R^6$ and $R^7$ are each as defined herein;
each $R^{8a}$, $R^{8a*}$, $R^{9a}$, $R^{9a*}$, $R^{10a}$ and $R^{10a*}$ is independently -D, -OH, -F, -Cl, -Br, -I, -CN, -$NH_2$, -C(=O)OH, - C(=O)$NH_2$, oxo, -$CH_3$, -$CH_2CH_3$, -$(CH_2)_2CH_3$, -$(CH_2)_3CH_3$, -$C(CH_3)_3$, -$CH(CH_3)_2$, -$CH_2CH(CH_3)_2$, - $(CH_2)_2CHF_2$, -$(CH_2)_2CF(CF_3)_2$, -$CF_3$, -$CHF_2$, -$CH_2F$, -$(CH_2)_2F$, -$(CH_2)_2Cl$, -$CH_2CF_3$, -$OCH_3$, -$OCH_2CH_3$, - $OCH_2CH_3$, -$OC(CH_3)_3$, -$CH_2OH$, -$(CH_2)_2OH$, -$(CH_2)_3OH$, phenyl, pyridinyl, pyrimidinyl, -C(=O)$OCH_3$, - C(=O)$NHCH_3$ or -C(=O)$N(CH_3)_2$.
In some embodiments, ring A is one of the following sub-structures,

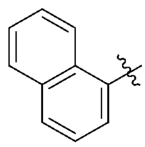
,
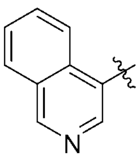
,
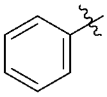
,
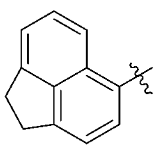
,
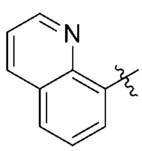
,
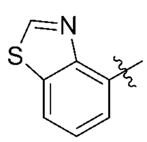
,
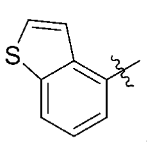
,
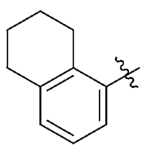
,

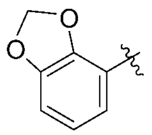, 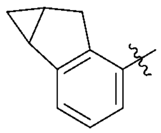, 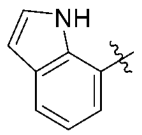, 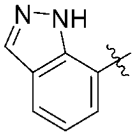, 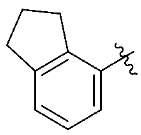, 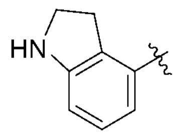, 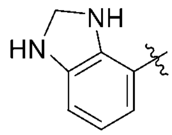,

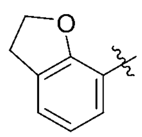, 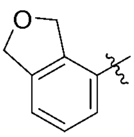, 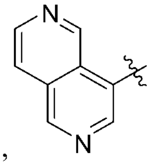, 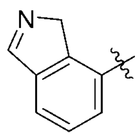, 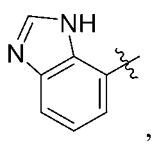, 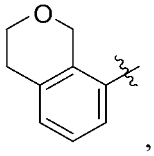, 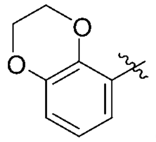 or 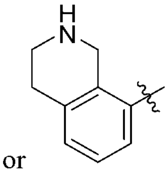;

each $R^5$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{76}$, $-NR^{6b}C^{r}-OIR^{7b}$ $-NR^{66}R^{7b}$ $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-12 membered aryl, 5-12 membered heteroaryl, $C_{3-6}$ cycloalkyl or 3-6 membered heterocyclyl, wherein each of the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-10 membered heteroaryl, $C_{3-6}$ cycloalkyl and 3-6 membered heterocyclyl is independently optionally substituted with 1, 2, 3 or 4 substituents selected from -D, - OH, -F, -Cl, -Br, -I, -CN, $-NH_2$, -C(=O)H, -C(=O)OH, $C_{1-4}$ alkyl, $C_{1-a}$ alkoxy, $C_{3-6}$ cycloalkyl and 3-6 membered heterocyclyl; wherein, $R^{6b}$, $R^{7b}$ and $R^{6c}$ are each as defined herein.

In some embodiments, each $R^5$ is independently -D, -OH, -F, -Cl, -Br, -I, -CN, -SH, $-CH_2C(=O)NR^{6b}R^{7b}$, - $C(=O)R^{6c}$, $-C(=O)OR^{6e}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, $-C=CCH_3$, - $CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, - $(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CF_3$, $-CH_2CF_3$, $-CHF_2$, - $CH_2F$, $-(CH_2)_2Cl$, -CH=CHF, -CH=CHCl, $-CH=CHCH_2F$, $-C=CCH_2F$, $-C\equiv C(CH_2)_2F$, $-C\equiv CF$, $-OCF_3$, $-OCHF_2$, - $OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein each of the $-CH_3$, $-CH_2CH_3$, $-(CH_2)_2CH_3$, $-(CH_2)_3CH_3$, - $C(CH_3)_3$, $-CH(CH_3)_2$, $-SCH_3$, $-SCH_2CH_3$, $-CH=CH_2$, $-CH=CHCH_3$, $-CH_2CH=CH_2$, $-C\equiv CH$, $-C\equiv CCH_3$, - $CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, - $(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CH_2CF_3$, $-CHF_2$, $-CH_2F$, - $(CH_2)_2Cl$, -CH=CHF, -CH=CHCl, $-CH=CHCH_2F$, $-C\equiv CCH_2F$, $-C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl is independently optionally substituted with 1, 2, 3 or 4 substituents selected from -D, -OH, -F, -Cl, -Br, -I, -CN, - $NH_2$, -C(=O)H, -C(=O)OH, methyl, ethyl, n-propyl, isopropyl, methoxy, ethoxy, n-propoxy, i-propoxy, cyclopropyl, cyclobutyl, cyclopentyl, cyclohexyl, pyrrolidinyl, oxazolidinyl, tetrahydrofuranyl, piperidyl and piperazinyl; wherein, $R^{6b}$, $R^{7b}$ and $R^{6c}$ are as defined herein.

In some embodiments, each $R^6$, $R^7$, $R^{6a}$, $R^{6b}$, $R^{6c}$, $R^{7b}$, $R^{6d}$, $R^{7d}$, $R^{6e}$ and $R^{7e}$ is independently -H, -D, methyl, ethyl, n-propyl, isopropyl, n-butyl, isobutyl or tert-butyl, wherein each of the methyl, ethyl, n-propyl, isopropyl, n-butyl, isobutyl or tert-butyl is independently optionally substituted with 1, 2, 3 or 4 substituents selected from -D, -OH, -F, -Cl, -Br, -I, -CN, C(=O)H, -C(=O)OH, $-NH_2$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl.

In some embodiments, $R^2$ is

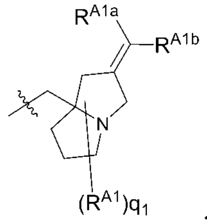, 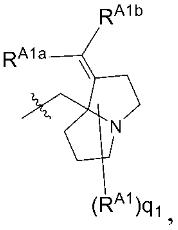, 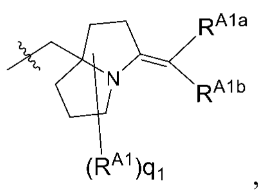, 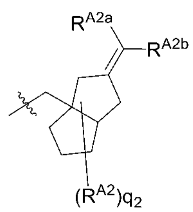,

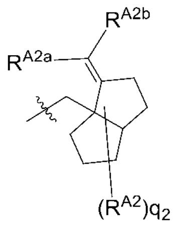 or 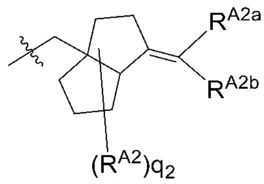 ,

wherein, $R^{A1}a$, $R^{A1b}$, $R^{A2a}$, $R^{A2b}$, $R^{A1}$, $R^{A2}$, $q_1$ and $q_2$ are each as defined herein.

In some embodiments, $R^2$ is

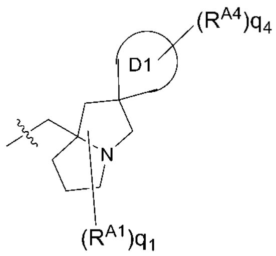 , 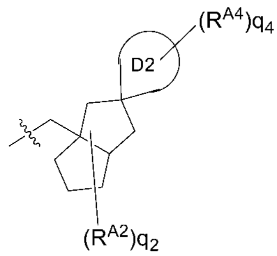

or

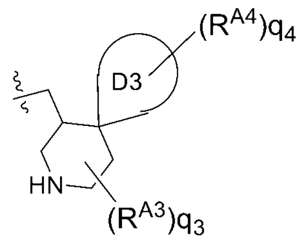 ,

wherein, each of ring D1, ring D2 and ring D3is independently cyclopropyl, cyclobutyl, cyclopentyl, cyclohexyl, oxiranyl, oxetanyl, azetidinyl, pyrrolidinyl, oxazolidinyl, tetrahydrofuranyl, piperidinyl and piperazinyl, wherein, $R^{A1}$, $R^{A2}$, $R^{A3}$, $R^{A4}$, Q1, $q_2$, $q_3$ and $q_4$ are each as defined herein.

In some embodiments, $R^2$ is

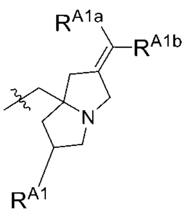 , 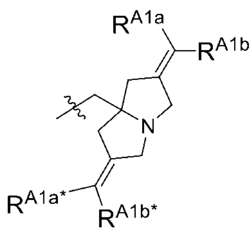 , 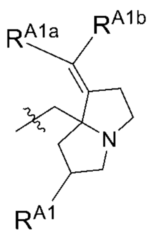 , 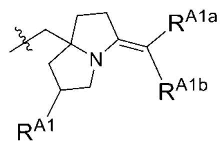 ,

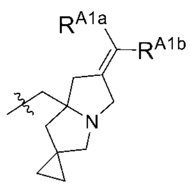 , 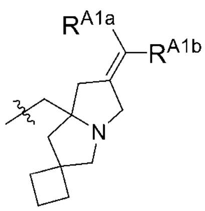 , 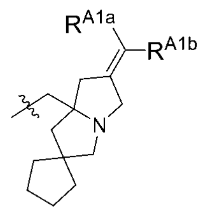 , 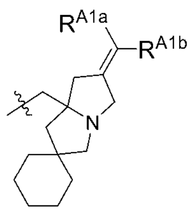 , 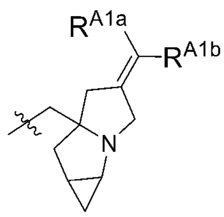 ,

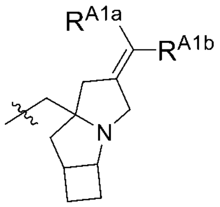, 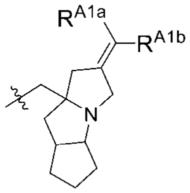, 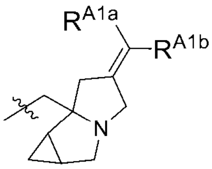, 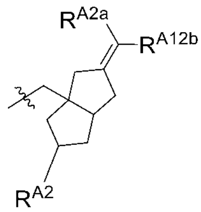, 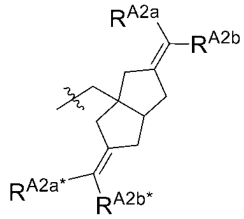,

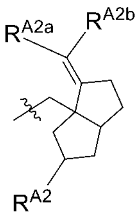, 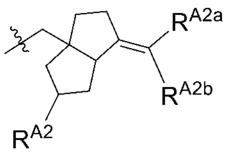, 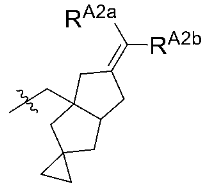 or 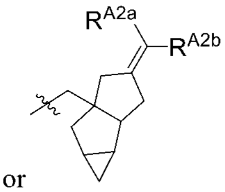,

wherein, $R^{A1a}$, $R^{A1b}$, $R^{A2a}$, $R^{A2b}$, $R^{A1}$, $R^{A2}$, $R^{A1a*}$ and $R^{A1b*}$ are each as defined herein.

In some embodiments, $R^2$ is

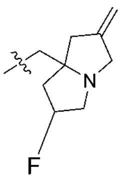, 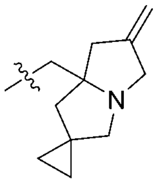, 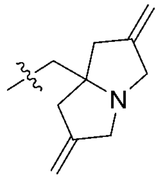, 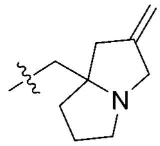, 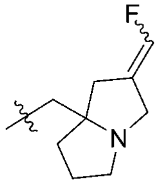,

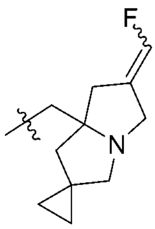, 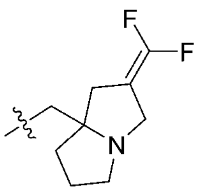, 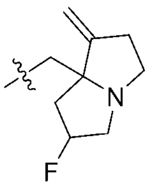, 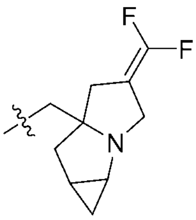, 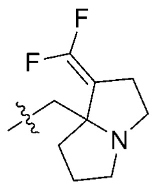, 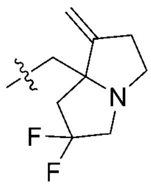,

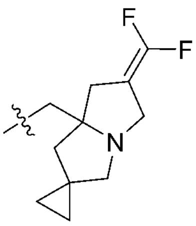, 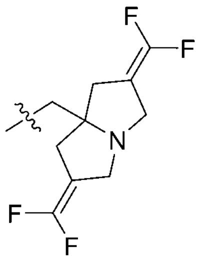, 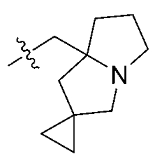 or 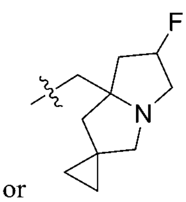.

In some embodiments, the compound of the present invention is a compound of Formula (I-1), (I-2) or (I-3), or a stereoisomer, tautomer, nitrogen oxide, solvate, metabolite, pharmaceutically acceptable salt, or prodrug thereof,

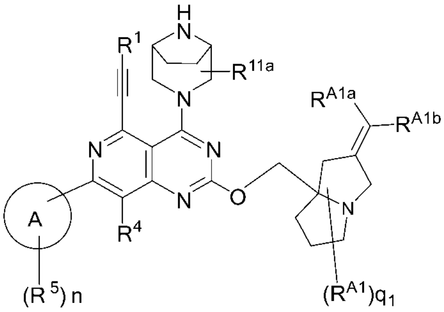
(I-1),
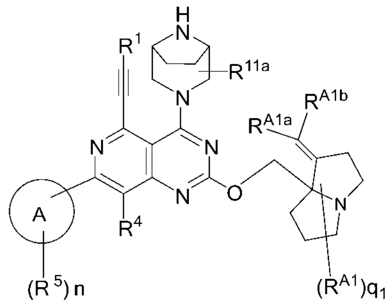
(I-2)

or

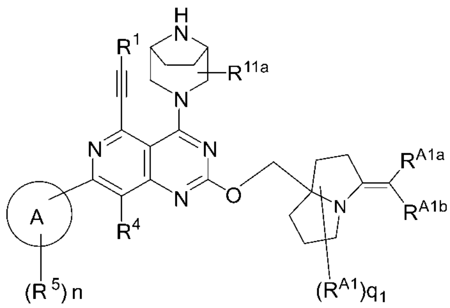
(I-3),

wherein, the $R^1$, $R^4$, $R^5$, $R^{A1}$, $R^{A1a}$, $R^{A1b}$, $R^{11a}$, n and $q_1$ are each as defined herein.

In some embodiments, the compound of the present invention is a compound of Formula (I-1a), (I-1b) or (I-1c), or a stereoisomer, tautomer, nitrogen oxide, solvate, metabolite, pharmaceutically acceptable salt, or prodrug thereof,

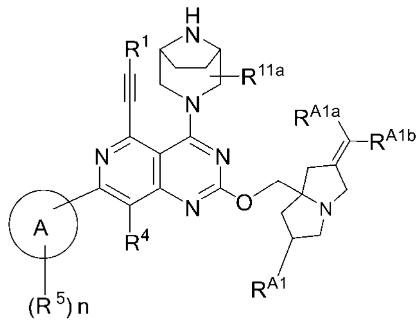
(I-1a),
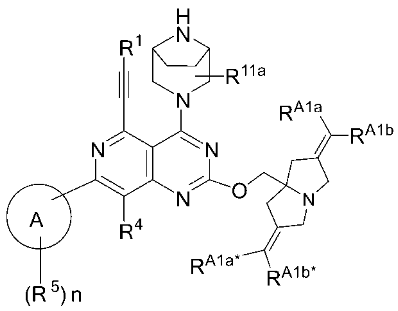
(I-1b)

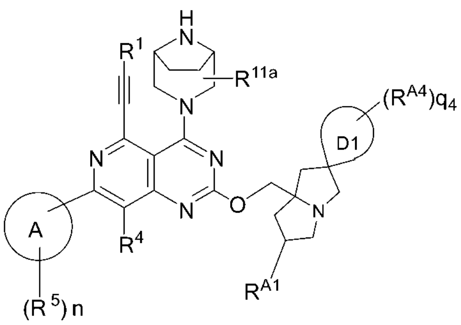
(I-1c),

wherein, the $R^1$, $R^4$, $R^5$, $R^{A1}$, $R^{A1a}$, $R^{A1b}$, $R^{A1a*}$, $R^{A1b*}$, $R^{11a}$, $R^{A4}$, $q_4$ and n are each as defined herein.

In other aspect, provided herein is a pharmaceutical composition comprising the compound disclosed herein.

In some embodiments, the pharmaceutical compositions of the present invention further comprise pharmaceutically acceptable adjuvant.

In some embodiments, the adjuvants of the present invention include, but are not limited to, carriers, excipients, diluents, vehicles, or combinations thereof. In some embodiments, the pharmaceutical composition may be in a liquid,

solid, semi-solid, gel, or spray dosage form.

In other aspect, the present invention provides a use of the pharmaceutical composition of the present invention in the preparation of a medicament for preventing, treating, or alleviating a disease associated with KRAS G12D.

In some embodiments, the KRAS G12D-related disease described herein is cancer.

In some embodiments, the cancer described herein is non-small cell lung cancer, small cell lung cancer, colorectal cancer, rectal cancer, colon cancer, small intestine cancer, pancreatic cancer, uterine cancer, gastric cancer, esophageal cancer, prostate cancer, ovarian cancer, breast cancer, leukemia, melanoma, lymphoma or neuroma.

In other aspect, the present invention further provides a method for preventing or treating a disease associated with KRAS G12D, comprising administering to a patient a therapeutically effective amount of the compound according to the present invention or a pharmaceutical composition thereof.

In other aspect, the present invention relates to a method for the preparation, isolation, and purification of the compound of Formula (I), (I-1), (I-2), (I-3), (I-1a) or (I-1b).

Unless otherwise stated, all stereoisomers, tautomers, N-oxides, hydrates, solvates, metabolites, and pharmaceutically acceptable salts and prodrugs of the compounds disclosed herein are within the scope of the invention.

The phrase "pharmaceutically acceptable" refers to that the substance or composition must be compatible chemically and/or toxicologically, with the other ingredients comprising a formulation, and/or the mammal being treated therewith.

The salt of the compounds disclosed herein also include salts of the compounds which are not necessarily pharmaceutically acceptable salts, and which may be useful as intermediates for preparing and/or purifying compounds of Formula (I), (I-1), (I-2), (I-3), (I-1a) or (I-1b) or salts for separating enantiomers of compounds of Formula (I), (I-1), (I-2), (I-3), (I-1a) or (I-1b).

The foregoing merely summarizes certain aspects disclosed herein and is not intended to be limiting in nature. These aspects and other aspects and embodiments are described more fully below.

## DETAILED DESCRIPTION OF THE INVENTION

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, "patient" refers to a human (including adults and children) or other animal. In one embodiment, "patient" refers to a human.

The term "comprise" is an open expression, it means comprising the contents disclosed herein, but don't exclude other contents.

"Stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, etc. Unless otherwise indicated, all stereoisomers or mixtures of stereoisomers of the structural formulas described herein are encompassed within the scope of the present invention. Additionally, unless otherwise stated, structures depicted herein are also meant to include compounds that differ only in the presence of one or more isotopically enriched atoms.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry

of Organic Compounds", John Wiley & Sons, Inc., New York, 1994.

Any resulting mixtures of stereoisomers can be separated on the basis of the physicochemical differences of the constituents, into the pure or substantially pure geometric isomers, enantiomers, diastereomers, for example, by chromatography and/or fractional crystallization. Cis and trans isomers are diastereomer.

The term "tautomer" or "tautomeric form" refers to structural isomers of different energies which are interconvertible via a low energy barrier. Where tautomerization is possible (e.g. in solution), a chemical equilibrium of tautomers can be reached. For example, proton tautomers (also known as prototropic tautomers) include interconversions via migration of a proton, such as keto-enol and imine-enamine isomerizations. Valence tautomers include interconversions by reorganization of some of the bonding electrons. A specific example of keto-enol tautomerization is the interconversion of pentane-2,4-dione and 4-hydroxypent-3-en-2-one tautomers. Another example of tautomerization is phenol-keto tautomerization. The specific example of phenol-keto tautomerisms is pyridin-4-ol and pyridin-4(1H)-one tautomerism. Unless otherwise stated, all tautomeric forms of the compounds disclosed herein are within the scope of the invention.

As described herein, compounds disclosed herein may independently optionally be substituted with one or more substituents, such as are illustrated generally below, or as exemplified by particular classes, subclasses, and species of the invention. It should be understood that the terms "independently optionally substituted with..." and "optionally substituted with..." are interchangeably used with the term "substituted or unsubstituted." In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

Furthermore, what need to be explained is that the phrase "each...is independently" and "each of...and...is independently", unless otherwise stated, should be broadly understood. The specific options expressed by the same symbol are independent of each other in different groups; or the specific options expressed by the same symbol are independent of each other in same groups.

At various places in the present specification, substituents of compounds disclosed herein are disclosed in groups or in ranges. It is specifically intended that the invention include each and every individual subcombination of the members of such groups and ranges. For example, the term "$C_{1-6}$ alkyl" is specifically intended to individually disclose methyl, ethyl, $C_3$ alkyl, $C_4$ alkyl, $C_5$ alkyl and $C_6$ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "alkyl" refers to a saturated linear or branched-chain monovalent hydrocarbon group of 1-20 carbon atoms, wherein the alkyl group is optionally substituted with one or more substituents described herein. In some embodiments, the alkyl group contains 1-6 carbon atoms, referring to $C_{1-6}$ alkyl; in still other embodiments, the alkyl group contains 1-4 carbon atoms, referring to $C_{1-4}$ alkyl; in yet other embodiments, the alkyl group contains 1-3 carbon atoms, referring to $C_{1-3}$ alkyl. Examples of alkyl groups include, but are not limited to, methyl (Me, $-CH_3$), ethyl (Et, $-CH_2CH_3$), *n*-propyl (*n*-Pr, $-CH_2CH_2CH_3$), isopropyl (*i*-Pr, $-CH(CH_3)_2$), *n*-butyl (*n*-Bu, $-CH_2CH_2CH_2CH_3$), isobutyl (*i*-Bu, $-CH_2CH(CH_3)_2$), ***sec***-butyl (***s***-Bu, $-CH(CH_3)CH_2CH_3$), ***tert***-butyl (*t*-Bu, $-C(CH_3)_3$), *n*-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), *n*-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), *n*-heptyl, *n*-octyl, and the like.

The term "alkylene" refers to a saturated divalent hydrocarbon group derived from a straight or branched chain saturated hydrocarbon by the removal of two hydrogen atoms. In some embodiments, the alkylene group contains 1-6 carbon atoms, referring to $C_{1-6}$ alkylene; in still other embodiments, the alkylene group contains 1-4 carbon atoms, referring to $C_{1-4}$ alkylene; in yet other embodiments, the alkylene group contains 1-3 carbon atoms, referring to $C_{1-3}$ alkylene; in yet other embodiments, the alkylene group contains 1-2 carbon atoms, referring to $C_{1-2}$ alkylene. Some non-limiting examples of the alkylene group include : $-CH_2-$, $-CH_2CH_2-$, $-CH(CH_3)CH_2-$, and the like.

The term "alkenyl" refers to linear or branched-chain monovalent hydrocarbon radical of 2 to 12 carbon atoms with at least one site of unsaturation, i.e., a carbon-carbon, $sp^2$ double bond, wherein the alkenyl radical may be optionally substituted independently with one or more substituents described herein, and includes radicals having "*cis*" and " *trans"* orientations, or alternatively, "*E*" and "*Z*" orientations. In some embodiments, the alkenyl contains 2-6 carbon atoms, denoted as $C_{2-6}$ alkenyl. In other embodiments, the alkenyl contains 2-4 carbon atoms, denoted as $C_{2-4}$ alkenyl. Some non-limiting examples of the alkenyl group include ethenyl or vinyl ($-CH=CH_2$), allyl ($-CH_2CH=CH_2$), 1-propenyl (i.e.,

propenyl, $-CH=CH-CH_3$), and the like.

The term "alkynyl" refers to a linear or branched monovalent hydrocarbon radical of 2 to 12 carbon atoms with at least one site of unsaturation, i.e., a carbon-carbon, sp triple bond, wherein the alkynyl radical may be optionally substituted independently with one or more substituents described herein. In some embodiments, the alkynyl contains 2 to 6 carbon atoms, denoted as $C_{2-6}$ alkynyl. In other embodiments, the alkynyl contains 2 to 4 carbon atoms, denoted as $C_{2-4}$ alkynyl. Examples of alkynyl include, but are not limited to, ethynyl ($-C\equiv CH$), propargyl ($-CH_2C\equiv CH$), 1-propynyl ($-C\equiv C-CH_3$), and the like.

The term "cyanoalkyl" refers to an alkyl group substitued with one or more cyano groups, wherein the cyano group and the alkyl group are as defined herein. In some embodiments, "cyanoalkyl" refers to an alkyl group substitued with one cyano group. In some embodiments, "cyanoalkyl" is $C_{1-6}$ cyanoalkyl, that is, $C_{1-6}$ alkyl group substituted with one or more cyano groups. In some preferred embodiments, the $C_{1-6}$ cyanoalkyl is a $C_{1-6}$ alkyl group substituted with a single cyano group. In other embodiments, "cyanoalkyl" is $C_{1-4}$ cyanoalkyl, that is, $C_{1-4}$ alkyl group substituted with one or more cyano groups. Examples of cyanoalkyl include, but are not limited to, $-CH_2CN$, $-CH_2CH_2CH_2CH_2CN$, $-CH_2CH_2CN$, $-CH_2CH(CN)CH_2CH_2CN$, - $CH_2CH(CN)CH_2CH(CH_3)CN$, and the like.

The term "hydroxyalkyl" refers to an alkyl group substitued with one or more hydroxyl groups, wherein the alkyl group and the hydroxyl group are as defined herein. In some embodiments, hydroxyalkyl refers to an alkyl group substitued with 1, 2, 3 or 4 hydroxyl groups. In some embodiments, hydroxyalkyl refers to an alkyl group substitued with one or two hydroxyl groups. In some embodiments, hydroxyalkyl refers to $C_{1-6}$ hydroxyalkyl, that is, $C_{1-6}$ alkyl group substituted with one or more hydroxyl groups. In some embodiments, hydroxyalkyl refers to $C_{1-4}$ hydroxyalkyl. In some embodiments, hydroxyalkyl refers to $C_{1-3}$ hydroxyalkyl. Examples of hydroxyalkyl include, but are not limited to, $-CH_2OH$, $-CH_2CH_2CH_2CH_2OH$, $-CH_2CH_2OH$, - $CH_2CH(OH)CH_2CH_2OH$, $-CH_2CH(OH)CH_2CH(CH_3)OH$, and the like.

The term "haloalkyl" refers to an alkyl group substituted with one or more halogen atoms, wherein the alkyl group and halogen atoms are as defined herein. In some embodiments, the haloalkyl is $C_{1-6}$ haloalkyl, referring to a $C_{1-6}$ alkyl group substituted with one or more halogen atoms; in other embodiments, the haloalkyl is $C_{1-4}$ haloalkyl, referring to a $C_{1-4}$ alkyl group substituted with one or more halogen atoms; in yet other embodiments, the haloalkyl is $C_{1-3}$ haloalkyl, referring to a $C_{1-3}$ alkyl group substituted with one or more halogen atoms. Some non-limiting examples of such group include monofluoromethyl, difluoromethyl, trifluoromethyl, 1-fluoroethyl, 2-fluoroethyl, 1,2-difluoroethyl, 1,1-difluoroethyl, 2,2-difluoroethyl, monochloromethyl, dichloromethyl, trichloromethyl, 2-chloroethyl, 1-chloroethyl, 1,2-dichloroethyl, 1,1-dichloroethyl, 2,2-dichloroethyl, 1,1-dibromoethyl, and the like.

The term "haloalkenyl" refers to an alkenyl group substituted with one or more halogen atoms, wherein the alkenyl group is as defined herein. In some embodiments, the haloalkenyl is $C_{2-6}$ haloalkenyl, referring to a $C_{2-6}$ alkenyl group substituted with one or more halogen atoms; in other embodiments, the haloalkenyl is $C_{2-4}$ haloalkenyl, referring to a $C_{2-4}$ alkenyl group substituted with one or more halogen atoms. Some non-limiting examples of such group include 1-chloroethenyl ($-CCl=CH_2$), 2-fluoroethenyl ($-CH=CHF$), 1-fluoroallyl ($-CHFCH=CH_2$), 3-fluoropropenyl (i.e., $-CH=CH-CH_2F$), 3,3-difluoropropenyl (i.e., $-CH=CH-CHF_2$).

The term "haloalkynyl" refers to an alkynyl group substituted with one or more halogen atoms, wherein the alkynyl group is as defined herein. In some embodiments, the haloalkynyl is $C_{2-6}$ haloalkynyl, referring to a $C_{2-6}$ alkynyl group substituted with one or more halogen atoms; in other embodiments, the haloalkynyl is $C_{2-4}$ haloalkynyl, referring to a $C_{2-4}$ alkynyl group substituted with one or more halogen atoms. Examples of such groups include, but are not limited to, 1-chloroethynyl ($-C\equiv CCl$), 1-chloropropargyl ($-CHClC\equiv CH$), 3-chloropropynyl ($-C\equiv C-CH_2Cl$), and the like.

The term "hydroxyalkynyl" refers to an alkynyl group substituted with one or more hydroxy groups, wherein hydroxy and alkynyl are as defined herein. In some embodiments, the hydroxyalkynyl is $C_{2-6}$ hydroxyalkynyl, referring to a $C_{2-6}$ alkynyl group substituted with one or more hydroxyl; in other embodiments, the hydroxyalkynyl is $C_{2-4}$ hydroxyalkynyl, referring to a $C_{2-4}$ alkynyl group substituted with one or more hydroxyl. Such examples include, but are not limited to, 3-hydroxypropynyl ($-C\equiv C-CH_2OH$), 4-hydroxybutynyl ($-C\equiv C-(CH_2)_2OH$), and the like.

The term "alkoxyalkyl" refers to an alkyl group substitued with one alkoxy group, wherein the alkoxy group and the alkyl group are as defined herein. In some embodiments, alkoxyalkyl refers to $C_{1-6}$ alkoxy $C_{1-6}$ alkyl; in other embodiments, alkoxyalkyl refers to $C_{1-4}$ alkoxy $C_{1-4}$ alkyl; in still some embodiments, the alkoxyalkyl refers to $C_{1-4}$ alkoxy $C_{1-3}$ alkyl; In some embodiments, alkoxyalkyl refers to $C_{1-3}$ alkoxy $C_{1-3}$ alkyl. Examples of alkoxy groups include, but are not limited to: methoxymethyl, ethoxymethyl, n-propoxymethyl, isopropoxymethyl, 1-methoxyethyl, 1-methoxy-*n*-propyl, 1-methoxyisopropyl, 1-ethoxyethyl, 1-ethoxy-*n*-propyl, 1-ethoxyisopropyl, 1-(*n*-propoxy)ethyl, 1-(isopropoxy)ethyl, 1-(*n*-propoxy)-*n*-propyl, 1-(*n*-propoxy)isopropyl, 1-(isopropoxy)-*n*-propyl, 1-(isopropoxy)isopropyl, and the like.

The term "mercaptoalkyl" refers to an alkyl group substitued with one or more mercapto groups, wherein the alkyl group is as defined herein. In some embodiments, mercaptoalkyl refers to $C_{1-6}$ mercaptoalkyl, that is, $C_{1-6}$ alkyl group substituted with one or more mercapto groups; preferably, $C_{1-6}$ mercaptoalkyl is a $C_{1-6}$ alkyl substituted with one mercapto group. In other embodiments, mercaptoalkyl refers to $C_{1-4}$ mercaptoalkyl. In other embodiments, mercaptoalkyl refers to $C_{1-3}$ mercaptoalkyl. Examples of mercaptoalkyl groups include, but are not limited to, mercaptomethyl ($-CH_2SH$), 2-mercaptoethyl ($-(CH_2)_2SH$), 3-mercaptopropyl ($-(CH_2)_3SH$), 2,3-dimercaptopropyl ($-CH_2CH(SH)CH_2(SH)$), and the like.

The term "alkoxy" means an alkyl group attached to the rest of the molecule via an oxygen atom, wherein the alkyl group is as defined herein. Unless otherwise specified, the alkoxy group contains 1-12 carbon atoms. In some embodiments, the alkoxy group contains 1-6 carbon atoms, referring to $C_{1-6}$ alkoxy; in still other embodiments, the alkoxy group contains 1-4 carbon atoms, referring to $C_{1-4}$ alkoxy; in yet other embodiments, the alkoxy group contains 1-3 carbon atoms, referring to $C_{1-3}$ alkoxy. The alkoxy group may be optionally substituted with one or more substituents disclosed herein. Some non-limiting examples of the alkoxy group include, but are not limited to, methoxy (MeO, $-OCH_3$), ethoxy (EtO, $-OCH_2CH_3$), 1-propoxy (*n*-PrO, *n*-propoxy, $-OCH_2CH_2CH_3$), 2-propoxy (*i*-PrO, *i*-propoxy, $-OCH(CH_3)_2$), 1-butoxy (*n*-BuO, *n*-butoxy, $-OCH_2CH_2CH_2CH_3$), 2-methyl-1-propoxy (*i*-BuO, *i*-butoxy, $-OCH_2CH(CH_3)_2$), 2-butoxy (*s*-BuO, *s*-butoxy, $-OCH(CH_3)CH_2CH_3$), 2-methyl-2-propoxy (*t*-BuO, t-butoxy, $-OC(CH_3)_3$), 1-pentoxy (*n*-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-1-butoxy ($-OCH_2CH_2CH(CH_3)_2$), 2-methyl-1-butoxy ($-OCH_2CH(CH_3)CH_2CH_3$), and the like.

The term "haloalkoxy" refers to an alkoxy group substituted with one or more halogen atoms, wherein the alkoxy group and halogen atoms are as defined herein. In some embodiments, haloalkoxy refers to a haloalkoxy group containing 1 to 6 carbon atoms, i.e., $C_{1-6}$ haloalkoxy; in other embodiments, haloalkoxy refers to a haloalkoxy group containing 1 to 4 carbon atoms, i.e., $C_{1-4}$ haloalkoxy; in yet other embodiments, haloalkoxy refers to a haloalkoxy group containing 1 to 3 carbon atoms, i.e., $C_{1-3}$ haloalkoxy. Some non-limiting examples of the haloalkoxy group include trifluoromethoxy ($-OCF_3$), monofluoromethoxy ($-OCH_2F$), 2-fluoroethoxy ($-OCH_2CH_2F$), and the like.

The term "cycloalkyl" refers to a monovalent saturated monocyclic or bicyclic carbon ring system containing 3 to 12 carbon atoms, wherein a $-CH_2-$ group in the carbon ring may be optionally replaced by - C(=O)- (or -(CO)-). In one embodiment, the cycloalkyl group contains 3-10 carbon atoms, i.e., $C_{3-10}$ cycloalkyl; in another embodiment, the cycloalkyl group contains 3-7 carbon atoms, i.e., $C_{3-7}$ cycloalkyl; in another embodiment, the cycloalkyl group contains 3-6 carbon atoms, i.e., $C_{3-6}$ cycloalkyl; in another embodiment, the cycloalkyl group contains 3-5 carbon atoms, i.e., $C_{3-5}$ cycloalkyl. In another embodiment, the cycloalkyl group is a monocyclic cycloalkyl group containing 3 to 7 carbon atoms, ie, a $C_{3-7}$ monocyclic cycloalkyl group. Examples of cycloalkyl include, but are not limited to, cyclopropyl, cyclobutyl, cyclopentyl, cyclohexyl, octahydro-1*H*-indenyl, octahydro-bicyclo[3.3.0]octanyl, etc. Examples of carbocycles where a $-CH_2-$ group is replaced by -C(=O)- include, but are not limited to, cyclopentanone and cyclobutanone.

The term "heterocycle" or "heterocyclyl" refers to a saturated or partially unsaturated monocyclic, bicyclic, or tricyclic system containing 3 to 12 ring atoms, wherein at least one ring atom is selected from nitrogen, sulfur, and oxygen atoms; and wherein the heterocycle or heterocyclyl is non-aromatic and contains no aromatic rings. When a heterocycle is attached to the rest of the molecule via a single attachment point, it is referred to as a monovalent heterocyclyl group. Unless otherwise specified, the heterocyclyl group may be carbon or nitrogen linked, and a $-CH_2-$ group can be optionally replaced by a -C(=O)- group. The sulfur can be optionally oxygenized to *S*-oxide. The nitrogen can be optionally oxygenized to *N*-oxide. In some embodiments, the heterocycle or heterocyclyl consists of 3-10 atoms, referring to a 3-10 membered heterocycle or a 3-10 membered heterocyclyl; in other embodiments, the heterocycle or heterocyclyl consists of 3-9 atoms, referring to a 3-9 membered heterocycle or a 3-9 membered heterocyclyl; in other embodiments, the heterocycle or heterocyclyl consists of 5-9 atoms, referring to a 5-9 membered heterocycle or a 5-9 membered heterocyclyl; in other embodiments, the heterocycle or heterocyclyl consists of 3-6 atoms, referring to a 3-6 membered heterocycle or a 3-6 membered heterocyclyl; in other embodiments, the heterocycle or heterocyclyl consists of 5-6 atoms, referring to a 5-6 membered heterocycle or a 5-6 membered heterocyclyl. In other embodiments, the heterocycle or heterocyclyl is a monocyclic ring consisting of 3-7 atoms, representing a 3-7 membered monocyclic heterocycle or a 3-7 membered monocyclic heterocyclyl. Examples of the heterocycle include, but are not limited to: oxirane, aziridine, azetidine, oxetane, pyrrolidine, tetrahydrofuran, tetrahydrothiophene, thiazolidine, pyrazolidine, pyrazoline, oxazolidine, imidazolidine, piperidine, piperazine, morpholine, 3,8-diazabicyclo[3.2.1]octane, 3,6-diazabicyclo[3.1.1]heptane, and 2,5-diazabicyclo[2.2.2]octane. Examples of the heterocyclyl group include, but are not limited to: oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidinyl, piperazinyl, or morpholinyl, etc.

The term "aryl" refers to monocyclic, bicyclic and tricyclic carbocyclic ring systems having a total of 6-14 ring members, or 6-12 ring members, or 6-10 ring members, wherein at least one ring in the system is aromatic, wherein each ring in the system contains 3 to 7 ring members. In some embodiments, the aryl group contains 6 to 12 ring atoms, referring to $C_{6-12}$ aryl or 6-12-membered aryl. In some embodiments, the aryl group contains 6 to 10 ring atoms, referring to $C_{6-10}$ aryl or 6-10-membered aryl. Examples of the aryl group may include, but are not limited to: phenyl, naphthyl, 1,2,3,4-tetrahydronaphthalenyl, and anthryl.

The term "heteroaryl" or "heteroaromatic ring" refers to a monovalent monocyclic, bicyclic, or tricyclic system containing 5 to 14 ring atoms, or 5 to 12 ring atoms, or 5 to 10 ring atoms, or 5 to 6 ring atoms, wherein at least one ring is aromatic and at least one ring contains one or more ring heteroatoms selected from nitrogen, oxygen, and sulfur. The heteroaryl group is generally, but not necessarily bonded to the parent molecule through an aromatic ring of the heteroaryl group. When a $-CH_2-$ group is present in the heteroaryl group, the $-CH_2-$ group may be optionally replaced by -C(=O)-.

Unless otherwise specified, the heteroaryl group may be attached to the rest of the molecule (e.g., the core structure in a general formula) through any chemically feasible atom, which may be a carbon or nitrogen atom. The term "heteroaryl" may be used interchangeably with "heteroaromatic ring" or "heteroaromatic compound". In some embodiments, the heteroaryl is a heteroaromatic group containing 5-12 ring atoms, referring to a 5-12 membered heteroaryl; in other embodiments, the heteroaryl is a heteroaromatic group containing 5-10 ring atoms, referring to a 5-10 membered heteroaryl; in yet other embodiments, the heteroaryl is a heteroaromatic group containing 5- 6 ring atoms, referring to a 5-6 membered heteroaryl. Examples of heteroaryl include, but are not limited to: furyl, imidazolyl, isoxazolyl, oxazolyl, pyrrolyl, pyrazolyl, pyridyl, pyrimidinyl, pyridazinyl, pyrazinyl, thienyl, thiazolyl, triazolyl, tetrazolyl, benzopyridyl, benzimidazolyl, benzopyrazolyl, benzopyrrolidyl, etc.

The term "alkylthio" refers to an alkyl group attached to the rest of the molecule through a sulfur atom, wherein the alkyl group is as defined herein. In some embodiments, the alkylthio is $C_{1-6}$ alkylthio, referring to an alkylthio group containing 1 to 6 carbon atoms; in other embodiments, the alkylthio is $C_{1-4}$ alkylthio, referring to an alkylthio group containing 1 to 4 carbon atoms; in yet other embodiments, the alkylthio is $C_{1-3}$ alkylthio, referring to an alkylthio group containing 1 to 3 carbon atoms. Examples of alkylthio include, but are not limited to: methylthio ($-SCH_3$), ethylthio ($-SCH_2CH_3$), etc.

The term "haloalkylthio" refers to an alkylthio group substituted with one or more halogen atoms, wherein the alkylthio group is as defined herein. In some embodiments, the haloalkylthio is $C_{1-6}$ haloalkylthio, referring to a $C_{1-6}$ alkylthio substituted with one or more halogens; in other embodiments, the haloalkylthio is $C_{1-4}$ haloalkylthio, referring to a $C_{1-4}$ alkylthio substituted with one or more halogens; in yet other embodiments, the haloalkylthio is $C_{1-3}$ haloalkylthio, referring to a $C_{1-3}$ alkylthio substituted with one or more halogens. Examples of haloalkylthio include, but are not limited to: trifluoromethylthio ($-SCF_3$), 2,2,2-trifluoroethylthio ($-SCH_2CF_3$), monofluoromethylthio ($-SCH_2F$), etc.

The term "arylalkyl" refers to an alkyl group substituted with one aryl group, wherein the aryl and alkyl groups are as defined herein. In some embodiments, the arylalkyl is ($C_{6-10}$ aryl)-$C_{1-6}$ alkyl or (6-10 membered aryl)-$C_{1-6}$ alkyl; in other embodiments, the arylalkyl is ($C_{6-10}$ aryl)-$C_{1-4}$ alkyl or (6-10 membered aryl)-$C_{1-4}$ alkyl; in yet other embodiments, the arylalkyl is ($C_{6-10}$ aryl)-$C_{1-3}$ alkyl or (6-10 membered aryl)-$C_{1-3}$ alkyl; in other embodiments, the arylalkyl is phenyl $C_{1-6}$ alkyl; in other embodiments, the arylalkyl is phenyl $C_{1-4}$ alkyl; in yet other embodiments, the arylalkyl is phenyl $C_{1-3}$ alkyl. Examples of arylalkyl include, but are not limited to: benzyl, phenethyl, naphthylmethyl, etc.

The term "heteroarylalkyl" refers to an alkyl group substituted with one heteroaryl group, wherein the heteroaryl and alkyl groups are as defined herein. In some embodiments, the heteroarylalkyl is (5-12-membered heteroaryl)-$C_{1-6}$ alkyl; in other embodiments, the heteroarylalkyl is (5-12-membered heteroaryl)-$C_{1-4}$ alkyl; in yet other embodiments, the heteroarylalkyl is (5-12-membered heteroaryl)-$C_{1-3}$ alkyl; in other embodiments, the heteroarylalkyl is (5-6-membered heteroaryl)-$C_{1-6}$ alkyl; in other embodiments, the heteroarylalkyl is (5-6-membered heteroaryl)-$C_{1-4}$ alkyl; in yet other embodiments, the heteroarylalkyl is (5-6-membered heteroaryl)-$C_{1-3}$ alkyl. Examples of heteroarylalkyl include, but are not limited to: pyrimidinylmethyl, pyridinylmethyl, pyridinylethyl, pyrazolylmethyl, etc.

The term "heterocyclylalkyl" refers to an alkyl group substituted with one heterocyclyl group, wherein the heterocyclyl and alkyl groups are specifically as defined herein. In some embodiments, the heterocyclylalkyl is (3-6 membered heterocyclyl)-$C_{1-6}$ alkyl; in other embodiments, the heterocyclylalkyl is (3-6 membered heterocyclyl)-$C_{1-4}$ alkyl; in other embodiments, the heterocyclylalkyl is (3-6 membered heterocyclyl)-$C_{1-3}$ alkyl. Examples of heterocyclylalkyl include, but are not limited to: piperidinylmethyl, piperidinylethyl, pyrrolidinylmethyl, etc.

The term "cycloalkylalkyl" refers to an alkyl group substituted with one cycloalkyl group. In some embodiments, the cycloalkylalkyl is $C_{3-6}$ cycloalkyl $C_{1-6}$ alkyl; in other embodiments, the cycloalkylalkyl is $C_{3-6}$ cycloalkyl $C_{1-4}$ alkyl; in yet other embodiments, the cycloalkylalkyl is $C_{3-6}$ cycloalkyl $C_{1-3}$ alkyl. Examples of cycloalkylalkyl include, but are not limited to: cyclopropylmethyl, cyclopentylmethyl, cyclohexylmethyl, cyclohexylethyl, etc.

The term "halogen" refers to F (fluoro), Cl (chloro), Br (bromo), or I (iodo).

The term "oxo" refers to =O.

The term "cyano" refers to -CN or $-C\equiv N$.

The term "mercapto" refers to -SH.

The term "hydroxy" refers to -OH.

The term "carboxy" refers to -C(=O)OH.

The term "amino" refers to $-NH_2$.

The term "composed of j-k atoms" or "j-k membered" refers to a cyclic group comprising j to k ring atoms, wherein the ring atoms include carbon atoms and/or heteroatoms such as O, N, S, or P; j and k each independently represent any non-zero natural number with $k > j$; and "j-k" includes j, k, and every natural number therebetween. For example: "composed of 3-8 atoms" or "3-8 membered", "composed of 3-6 atoms" or "3-6 membered", "composed of 5-10 atoms" or "5-10 membered", or "composed of 5-6 atoms" or "5-6 membered" indicates a cyclic group comprising 3-8 (i.e., 3,4,5,6,7,8), 3-6 (i.e., 3,4,5,6), 5-10 (i.e., 5,6,7,8,9,10), or 5-6 (i.e., 5,6) ring atoms, respectively, wherein the ring atoms include carbon atoms and/or heteroatoms such as O, N, S, or P.

As described herein, a ring system formed by substituent $(R^{A1})q_1$ attached to the central ring via a single bond

indicates that q $R^{A1}$ substituents may substitute at any substitutable position or chemically feasible location on said ring. For example, formula a represents a hexahydro-1*H*-pyrrolopyrrole ring which may be substituted by $q_1$ $R^{A1}$ at any substitutable position or any reasonable position on the ring. When $q_1$ is greater than 1, each $R^8$ may be independently selected from the same or different substituent groups.

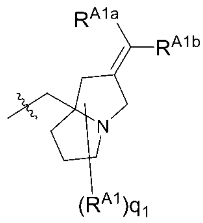

Formula a

In the present invention, the "〰" in

" 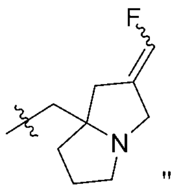 "

and

" 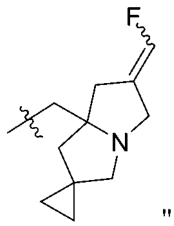 "

respectively indicate that the alkenyl group can be in *cis, trans* or a mixture thereof.

The term "prodrug" refers to a compound that is transformed *in vivo* into a compound of Formula (I), (I-1), (I-2), (I-3), (I-1a) or (I-1b). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salts" refers to organic or inorganic salts of a compound disclosed herein. Pharmaceutically acceptable salts are well known in the art, for example: S. M. Berge et al., describe pharmaceutically acceptable salts in detail in J. Pharmaceutical Sciences, 1977, 66: 1-19, which is incorporated herein by reference. Pharmaceutically acceptable salts formed with non-toxic acids include, but are not limited to, inorganic acid salts formed by reaction with amino groups. This invention also envisions the quaternization of any basic nitrogen-containing groups of the compounds disclosed herein. Water or oil soluble or dispersable products may be obtained by such quaternization. Further pharmaceutically acceptable salts include, when appropriate, nontoxic ammonium, quaternary ammonium, and amine cations formed using counterions.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid, ethanolamine or mixtures thereof. The term "hydrate"

refers to the complex where the solvent molecule is water.

The term "hydrate" can be used when said solvent is water. In one embodiment, one solvent molecule is associated with one molecule of the compounds disclosed herein, such as a hydrate; in another embodiment, more than one solvent molecule may be associated with one molecule of the compounds disclosed herein, such as a dihydrate; in still another embodiment, less than one solvent molecule may be associated with one molecule of the compounds disclosed herein, such as a hemihydrate. Furthermore, all the solvates of the invention retain the biological effectiveness of the non-hydrate form of the compounds disclosed herein.

As used herein, the term "treat", "treating" or "treatment" of any disease or disorder refers in some embodiments, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

The term "preventing" or "prevention" refers to a reduction in risk of acquiring a disease or disorder (i.e., causing at least one of the clinical symptoms of the disease not to develop in a subject that may be exposed to or predisposed to the disease but does not yet experience or display symptoms of the disease).

The term "therapeutically effective amount" refers to an amount of a compound sufficient to elicit a therapeutic effect when administered to a subject for treating a disease. The therapeutically effective amount may vary depending on the compound, the disease and its severity, as well as the condition, age, body weight, and sex of the subject to be treated.

Unless otherwise specified, all suitable isotopic variations, stereoisomers, tautomers, solvates, metabolites, pharmaceutically acceptable salts, and prodrugs of the compounds of the present invention are encompassed within the scope of the invention.

In the structures disclosed herein, when the stereochemistry of any specific chiral atom is unspecified, all stereoisomers of such structure are considered part of the invention and are included as compounds disclosed herein. When stereochemistry is indicated by a solid wedge or dashed line denoting a specific configuration, the stereoisomer of the structure is thereby explicitly defined.

Nitrogen oxides of the compounds of the invention are also included within the scope of the invention. Nitrogen oxides of the compounds of the invention can be prepared by oxidation of the corresponding nitrogen-containing basic substance using a conventional oxidizing agent (e.g., hydrogen peroxide) at elevated temperatures in the presence of an acid such as acetic acid, or by reaction with a peracid in a suitable solvent, such as peracetic acid in methylene chloride, ethyl acetate or methyl acetate, or with 3-chloroperoxybenzoic acid in chloroform or methylene chloride.

The compound of formula (I), (I-1), (I-2), (I-3), (I-1a) or (I-1b) may exist in the form of a salt.

Any formula given herein is also intended to represent isotopically unenriched forms as well as isotopically enriched forms of the compounds. Any formula given herein is also intended to represent isotopically unenriched forms as well as isotopically enriched forms of the compounds. Examples of isotopes that can be incorporated into compounds of the invention include isotopes of hydrogen, carbon, nitrogen, oxygen, phosphorus, sulfur, fluorine, chlorine, and iodine, such as $^{2}H$, $^{3}H$, $^{11}C$, $^{13}C$, $^{14}C$, $^{15}N$, $^{17}O$, $^{18}O$, $^{18}F$, 31p, $^{32}P$, $^{35}S$, $^{36}Cl$, $^{125}I$, respectively.

## DESCRIPTION OF COMPOUNDS OF THE INVENTION

The present invention provides a compound, or a pharmaceutical composition thereof, useful as a KRAS inhibitor, particularly a KRAS G12D inhibitor. The invention further relates to the use of said compound or pharmaceutical composition in the manufacture of a medicament for treating diseases and/or disorders by inhibiting KRAS activity.

The superior properties of the compounds of the present invention, such as half-life, clearance rate, selectivity, bioavailability, chemical stability, metabolic stability, membrane permeability, and solubility may lead to reduced side effects, broadened therapeutic index, or improved tolerability.

In one aspect, the present invention provides a compound having Formula (I) or a stereoisomer, a tautomer, an N-oxide, a solvate, a metabolite, a pharmaceutically acceptable salt or a prodrug thereof,

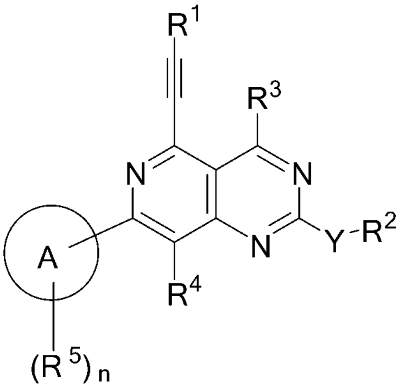

(I),

wherein, the ring A, $R^1$, Y, $R^2$, $R^3$, $R^4$, $R^5$ and n are each as defined herein.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, $-NR^{6a}S(=O)_2NR^6R^7$, $-NR^6R^7$, $-C_{1-6}$ alkyl-$NR^6C(=O)R^7$, $-C_{1-6}$ alkyl-$NR^6R^7$, $-C_{1-6}$ alkyl-$C(=O)OR^6$, $-C_{1-6}$ alkyl-$C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl or 3-12 membered heterocyclyl; wherein each of the $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl and 3-12 membered heterocyclyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$ and $C_{1-6}$ alkyl; wherein, $R^6$, $R^7$, $R^{6a}$, $R^{6e}$ and $R^{7e}$ are each as defined herein.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, $-NR^{6a}S(=O)_2NR^6R^7$, $-NR^6R^7$, $-C_{1-4}$ alkyl-$NR^6C(=O)R^7$, $-C_{1-4}$ alkyl-$NR^6R^7$, $-C_{1-4}$ alkyl-$C(=O)OR^6$, $-C_{1-4}$ alkyl-$C(=O)NR^6R^7$, $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl or 3-6 membered heterocyclyl; wherein each of the $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl and 3-6 membered heterocyclyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$ and $C_{1-4}$ alkyl; wherein, $R^6$, $R^7$, $R^{6a}$, $R^{6e}$ and $R^{7e}$ are each as defined herein.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, $-NR^{6a}S(=O)_2NR^6R^7$, $-NR^6R^7$, $-CH_2NR^6C(=O)R^7$, $-CH_2NR^6R^7$, $-(CH_2)_2NR^6R^7$, $-CH_2C(=O)OR^6$, $-(CH_2)_2C(=O)OR^6$, $-(CH_2)_3C(=O)OR^6$, $-CH_2C(=O)NR^6R^7$, $-(CH_2)_2C(=O)NR^6R^7$, $-(CH_2)_3C(=O)NR^6R^7$, $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-CH_2CN$, $-(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, $-CH(OH)CH_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCH_3$, $-OCH_2CH_3$, $-O(CH_2)_2CH_3$, $-O(CH_2)_3CH_3$, $-OC(CH_3)_3$, $-OCH(CH_3)_2$, $-CH_2OCH_3$, $-(CH_2)_2OCH_3$, $-(CH_2)_2OCH_2CH_3$, $-CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-cyclopropyl, $-CH_2$-cyclobutyl, $-(CH_2)_2$-cyclobutyl, $-CH_2$-cyclopentyl, $-(CH_2)_2$cyclopentyl, $-CH_2$-azetidinyl, $-CH_2$-pyrrolidinyl, $-CH_2$-piperidyl, $-CH_2$-morpholinyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, -CHzO-pyrrolidinyl, $-CH_2O$-spiro[2.3]hexyl, $-CH_2O$-spiro[3.3]heptanyl, $-CH_2SH$, $-(CH_2)_2SH$, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl or pyrrolidinyl; wherein each of the $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-CH_2CN$, $-(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, $-CH(OH)CH_3$, $-(CH_2)_2CHF_2$, $-(CH_2)_2CF(CF_3)_2$, $-CHF_2$, $-CH_2F$, $-(CH_2)_2F$, $-(CH_2)_2Cl$, $-CH_2CF_3$, $-OCH_3$, $-OCH_2CH_3$, $-O(CH_2)_2CH_3$, $-O(CH_2)_3CH_3$, $-OC(CH_3)_3$, $-OCH(CH_3)_2$, $-CH_2OCH_3$, $-(CH_2)_2OCH_3$,

$-(CH_2)_2OCH_2CH_3$, $-CH_2OCH_2CH_3$, - $CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-cyclopropyl, $-CH_2$-cyclobutyl, $-(CH_2)_2$-cyclobutyl, $-CH_2$-cyclopentyl, $-(CH_2)_2$cyclopentyl, $-CH_2$-azetidinyl, $-CH_2$-pyrrolidinyl, $-CH_2$-piperidyl, $-CH_2$-morpholinyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, - $CH_2O$-cyclopentyl, $-(CH_2)_2O$-cyclopentyl, $-CH_2O$-azetidinyl, $-CH_2O$-oxetanyl, $-CH_2O$-tetrahydrofuranyl, - $CH_2O$-spiro[2.3]hexyl, $-CH_2O$-spiro[3.3]heptanyl,phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl and pyrrolidinyl is independently optionally substituted with 1, 2, 3 or 4 substituents selected from D, -OH, -F, -Cl, -Br, -I, CN, - $C(=O)OR^{6e}$, $-NR^{6e}R^{7e}$, $-C(=O)NR^{6e}R^{7e}$, $-NR^{6e}C(=O)R^{7e}$, methyl, ethyl, n-propyl, isopropyl, n-butyl, isobutyl or tert-butyl; wherein, $R^6$, $R^7$, $R^{6a}$, $R^{6e}$ and $R^{7e}$ are each as defined herein.

In some embodiments, Y is bond, O or S.

In some embodiments, $R^2$ is

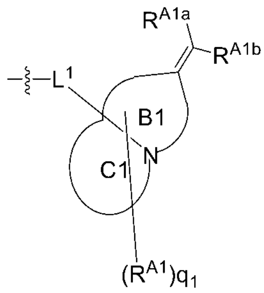 , 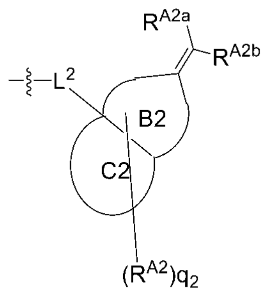 , 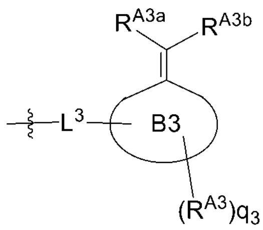 ,

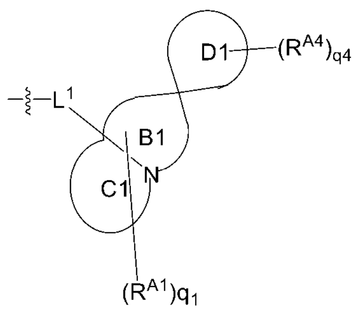 , 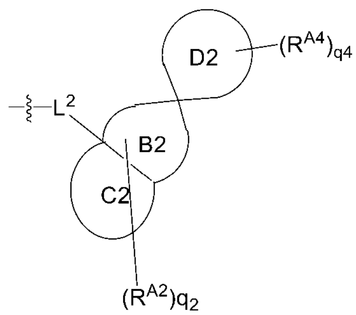 , 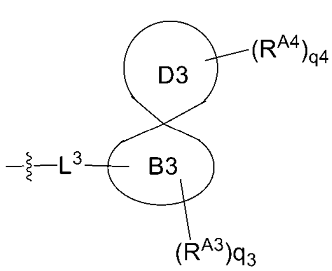 ,

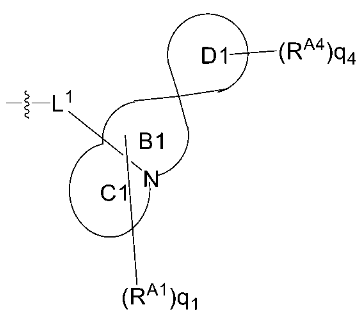 , 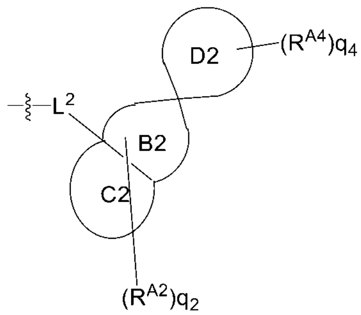 or 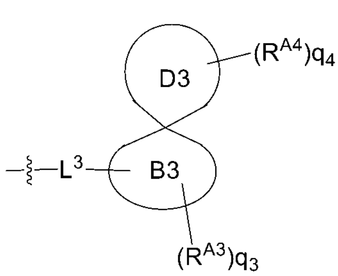 ,

wherein, ring B1, ring C1, ring B2, ring C2, ring B3, ring D1, ring D2, ring D3, $L^1$, $L^2$, $L^3$, $R^{A1}$, $R^{A2}$, $R^{A3}$, $R^{A4}$, $R^{A1a}$, $R^{A1b}$, $R^{A2a}$, $R^{A1b}$, $R^{A3a}$, $R^{A3b}$, q1, q2, q3 and q4 are each as defined herein.

In some embodiments, each $L^1$, $L^2$ and $L^3$ is independently $C_{1-6}$ alkylene or $C_{1-6}$ heteroalkylene, wherein each of the $C_{1-6}$ alkylene and $C_{1-6}$ heteroalkylene is independently optionally substituted with 1, 2, 3 or 4 $R^a$.

In some preferred embodiments, each $L^1$, $L^2$ and $L^3$ is independently $-CH_2-$, $-(CH_2)_2-$, $-(CH_2)_3-$ or $-(CH_2)_4-$, wherein the $-CH_2-$, $-(CH_2)_2-$, $-(CH_2)_3-$ and $-(CH_2)_4-$ independently optionally substituted with 1, 2, 3 or 4 $R^a$.

In some more preferred embodiments, each $L^1$, $L^2$ and $L^3$ is independently $-CH_2-$.

In some embodiments, $R^a$ is -D, -OH, -F, -Cl, -Br, -I, -CN, $-NH_2$, -C(=O)H, -C(=O)OH, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{3-6}$ cycloalkyl or 3-6 membered heterocyclyl; or two $R^a$ attached to the same carbon atom together form $C_{3-6}$ cycloalkyl;

In some embodiments, $R^a$ is -D, -OH, -F, -Cl, -Br, -I, -CN, $-NH_2$, -C(=O)H, -C(=O)OH, $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{3-6}$ cycloalkyl or 3-6 membered heterocyclyl; or two $R^a$ attached to the same

carbon atom together form $C_{3-6}$ cycloalkyl;

In some embodiments, each Ring B1 and ring C1 is independently 3-7 membered monocyclic heterocyclyl.

In some embodiments, each Ring B2, ring B3 and ring C2 is independently $C_{3-7}$ monocyclic cycloalkyl or 3-7 membered monocyclic heterocyclyl.

each Ring D1, ring D2 and ring D3 is independently $C_{3-7}$ monocyclic cycloalkyl or 3-7 membered monocyclic heterocyclyl.

In some embodiments, each $R^{A1}$, $R^{A2}$, $R^{A3}$ and $R^{A4}$ is independently -H, -D, -OH, -F, -Cl, -Br, -I, -CN, - $NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl; wherein, $R^{6d}$ and $R^{7d}$ are each as defined herein.

In some embodiments, each $R^{A1}$, $R^{A2}$, $R^{A3}$ and $R^{A4}$ is independently -H, -D, -OH, -F, -Cl, -Br, -I, -CN, - $NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-6}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl or 3-6 membered heterocyclyl; wherein, $R^{6d}$ and $R^{7d}$ are each as defined herein.

In some embodiments, each $R^{A1}$, $R^{A2}$, $R^{A3}$ and $R^{A4}$ is independently -H, -D, -OH, -F, -Cl, -Br, -I, -CN, - $NR^{6d}R^{7d}$, $-C(=O)NR^{6d}R^{7d}$, $-CH_2NR^{6d}R^{7d}$, $-CH_2OC(=O)NR^{6d}R^{7d}$, $-CH_3$, $-CH_2CH_3$, $-(CH_2)_2CH_3$, $-(CH_2)_3CH_3$, - $C(CH_3)_3$, $-CH(CH_3)_2$, $-CH_2CH(CH_3)_2$, $-OCH_3$, $-OCH_2CH_3$, $-OCH_2CH_3$, $-OC(CH_3)_3$, $-(CH_2)_2CHF_2$, - $(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCF_3$, $-OCHF_2$, $-CH_2$-phenyl, $-CH_2$-pyridyl, $-CH_2$-pyrrolidinyl, $-CH_2$-piperidinyl, $-CH_2$-cyclopropyl, $-CH_2$-cyclopentyl, cyclopropyl, cyclobutyl, cyclopentyl, cyclohexyl, oxiranyl, oxetanyl, tetrahydropyranyl, morpholinyl, azetidinyl and pyrrolidinyl; wherein, $R^{6d}$ and $R^{7d}$ are each as defined herein.

In some embodiments, two $R^{A1}$ attached to the same carbon atom together form

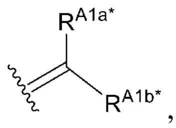

wherein, the $R^{A1a*}$ and $R^{A1b*}$ are each as defined herein.

In some embodiments, two $R^{A1}$ together with the same carbon atom to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3, or 4 $R^{8a}$ , wherein, the $R^{8a}$ is as defined herein.

In some preferred embodiments, two $R^{A1}$ together with the same carbon atom to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{8a}$,wherein the $R^{8a}$ is as defined herein.

In some embodiments, two $R^{A1}$ attached to two adjacent atoms together with the two adjacent atoms to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3, or 4 $R^{8a*}$, wherein, the $R^{8a*}$ is as defined herein.

In some preferred embodiments, two $R^{A1}$ attached to the two adjacent atoms together with the two adjacent atoms to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{8a*}$,wherein the $R^{8a*}$ is as defined herein.

In some embodiments, two $R^{A2}$ attached to the same carbon atom together form

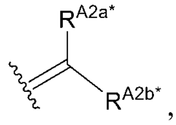

wherein, the $R^{A2a*}$ and $R^{A2b*}$ are each as defined herein.

In some embodiments, two $R^{A2}$ together with the same carbon atom to which they are attached, form a $C_{3-6}$ cycloalkyl or 3-6 membered heterocyclyl, wherein each of the $C_{3-6}$ cycloalkyl and 3-6 membered heterocyclyl is independently optionally substituted with 1, 2, 3, or 4 $R^{9a}$ , wherein, the $R^{9a}$ is as defined herein.

In some preferred embodiments, two $R^{A2}$ together with the same carbon atom to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{9a}$, wherein the $R^{9a}$ is as defined herein.

In some embodiments, two $R^{A2}$ attached to two adjacent atoms together with the two adjacent atoms to which they are attached, form a $C_{3-6}$ cycloalkyl or 3-6 membered heterocyclyl, wherein each of the $C_{3-6}$ cycloalkyl and 3-6 membered heterocyclyl is independently optionally substituted with 1, 2, 3, or 4 $R^{9a*}$, wherein, the $R^{9a*}$ is as defined herein.

In some preferred embodiments, two $R^{A2}$ attached to the two adjacent atoms together with the two adjacent atoms to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{9a*}$,wherein the $R^{9a*}$ is as defined herein.

In some embodiments, two $R^{A3}$ attached to the same carbon atom together form

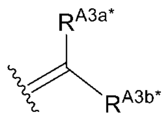
,

wherein, the $R^{A3a*}$ and $R^{A3b*}$ are each as defined herein.

In some embodiments, two $R^{A3}$ together with the same carbon atom to which they are attached, form $C_{3-6}$ cycloalkyl or 3-6 membered heterocyclyl, wherein the $C_{3-6}$ cycloalkyl and 3-6 membered heterocyclyl are each independently optionally substituted with 1, 2, 3, or 4 $R^{10a}$ , wherein, the $R^{10a}$ is as defined herein.

In some preferred embodiments, two $R^{A3}$ together with the same carbon atom to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{10a}$,wherein the $R^{10a}$ is as defined herein.

In some embodiments, two $R^{A3}$ attached to two adjacent atoms together with the two adjacent atoms to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3, or 4 $R^{10a*}$, wherein, the $R^{10a*}$ is as defined herein.

Two $R^{A3}$ attached to the two adjacent atoms together with the two adjacent atoms to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{10a*}$,wherein the $R^{10a*}$ is as defined herein.

In some embodiments, each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$C_{1-6}$ alkyl-$NR^6C(=O)R^7$, -$C_{1-6}$ alkyl-$NR^6R^7$, -$C_{1-6}$ alkyl-$C(=O)OR^6$, -$C_{1-6}$ alkyl-$C(=O)NR^6R^7$, $C_{1-6}$ alkyl, $C_{1-6}$ haloalkyl, $C_{1-6}$ cyanoalkyl, $C_{1-6}$ hydroxyalkyl, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{6-12}$ aryl, 5-12 membered heteroaryl, $C_{3-12}$ cycloalkyl or 3-12 membered heterocyclyl; wherein, $R^6$ and $R^7$ are each as defined herein.

In some embodiments, each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$C_{1-4}$ alkyl-$NR^6C(=O)R^7$, -$C_{1-4}$ alkyl-$NR^6R^7$, -$C_{1-4}$ alkyl-$C(=O)OR^6$, -$C_{1-4}$ alkyl-$C(=O)NR^6R^7$, $C_{1-4}$ alkyl, $C_{1-4}$ haloalkyl, $C_{1-4}$ cyanoalkyl, $C_{1-4}$ hydroxyalkyl, $C_{1-4}$ alkoxy $C_{1-4}$ alkyl, ($C_{3-6}$ cycloalkyl)-$C_{1-4}$ alkyl, (3-6 membered heterocyclyl)-$C_{1-4}$ alkyl, ($C_{3-6}$ cycloalkyl)-O-$C_{1-4}$ alkyl, (3-6 membered heterocyclyl)-O-$C_{1-4}$ alkyl, phenyl, 5-6 membered heteroaryl, $C_{3-6}$ cycloalkyl or 3-6 membered heterocyclyl; wherein, $R^6$ and $R^7$ are each as defined

herein;

In some embodiments, each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, $-CH_2NR^6C(=O)R^7$, $-CH_2NR^6R^7$, $-CH_2C(=O)OR^6$, $-(CH_2)_2C(=O)OR^6$, $-(CH_2)_3C(=O)OR^6$, $-CH_2C(=O)NR^6R^7$, $(CH_2)_2C(=O)NR^6R^7$, $-(CH_2)_3C(=O)NR^6R^7$, $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-(CH_2)_2CHF_2$, $-(CH_2)_2CF(CF_3)_2$, $-CF_3$, $-CHF_2$, $-CH_2F$, - $(CH_2)_2F$, $-(CH_2)_2Cl$, $-CH_2CF_3$, $-CH_2CN$, $-(CH_2)_2CN$, $-(CH_2)_3CN$, $-CH(CH_3)CN$, $-C(CH_3)_2CN$, $-CH_2OH$, - $(CH_2)_2OH$, $-(CH_2)_3OH$, $-CH(OH)CH_3$, $-CH_2OCH_3$, $-(CH_2)_2OCH_3$, $-(CH_2)_2OCH_2CH_3$, $-CH_2OCH_2CH_3$, - $CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-cyclopentyl, $-CH_2$-azetidinyl, $-CH_2$-oxetanyl, $-CH_2$-tetrahydrofuranyl, $-CH_2$-morpholinyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, $-CH_2O$-morpholinyl, phenyl, pyridinyl, pyrimidinyl, cyclopropyl, cyclobutyl, cyclopentyl, cyclohexyl, oxiranyl, oxetanyl, tetrahydropyranyl, morpholinyl, azetidinyl or pyrrolidinyl; wherein, $R^6$ and $R^7$ are each as defined herein.

In some embodiments, each $R^{8a}$, $R^{8a*}$, $R^{9a}$, $R^{9a*}$, $R^{10a}$ and $R^{10a*}$ is independently -D, -OH, -F, -Cl, -Br, -I, - CN, $-NH_2$, -C(=O)OH, $-C(=O)NH_2$, oxo, $C_{1-6}$ alkyl, $C_{1-6}$ haloalkyl, $C_{1-6}$ alkoxy, $C_{1-6}$ hydroxyalkyl, $C_{6-10}$ aryl, 5-10 membered heteroaryl, $-C(=O)OC_{1-6}$ alkyl, $-C(=O)NHC_{1-6}$ alkyl or $-C(=O)N(C_{1-6}\text{ alkyl})_2$.

In some embodiments, each $R^{8a}$, $R^{8a*}$, $R^{9a}$, $R^{9a*}$, $R^{10a}$ and $R^{10a*}$ is independently -D, -OH, -F, -Cl, -Br, -I, - CN, $-NH_2$, -C(=O)OH, $-C(=O)NH_2$, oxo, $C_{1-4}$ alkyl, $C_{1-4}$ haloalkyl, $C_{1-4}$ alkoxy, $C_{1-4}$ hydroxyalkyl, phenyl, 5-6 membered heteroaryl, $-C(=O)OC_{1-4}$ alkyl, $-C(=O)NHC_{1-4}$ alkyl or $-C(=O)N(C_{1-4}\text{ alkyl})_2$.

In some embodiments, each $R^{8a}$, $R^{8a*}$, $R^{9a}$, $R^{9a*}$, $R^{10a}$ and $R^{10a*}$ is independently-D, -OH, -F, -Cl, -Br, -I, - CN, $-NH_2$, -C(=O)OH, $-C(=O)NH_2$, oxo, $-CH_3$, $-CH_2CH_3$, $-(CH_2)_2CH_3$, $-(CH_2)_3CH_3$, $-C(CH_3)_3$, $-CH(CH_3)_2$, - $CH_2CH(CH_3)_2$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCH_3$, - $OCH_2CH_3$, $-OCH_2CH_3$, $-OC(CH_3)_3$, $-CH_2OH$, $-(CH_2)_2OH$, $-(CH_2)_3OH$, phenyl, pyridinyl, pyrimidinyl, - $C(=O)OCH_3$, $-C(=O)NHCH_3$ or -C(=O) $N(CH_3)_2$.

In some embodiments, $R^2$ is

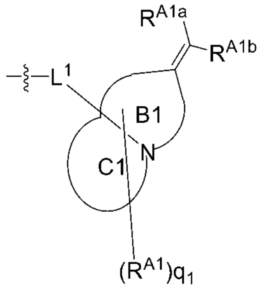

,

wherein, ring B1, ring C1, $L^1$, $R^{A1}$, $R^{A1a}$, $R^{A1b}$, $R^{A2a}$, $R^{A2b}$ and $q_1$ are each as defined herein.

In some embodiments, $R^2$ is

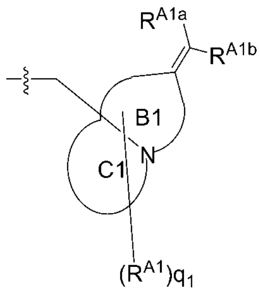

,

wherein, ring B1, ring C1, $R^{A1}$, $R^{A1a}$, $R^{A1b}$, $R^{A2a}$, $R^{A2b}$ and $q_1$ are each as defined herein.

In some embodiments,

$R^3$ is

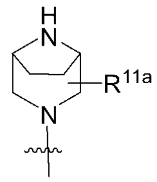, 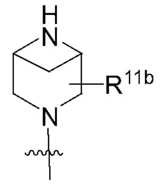, 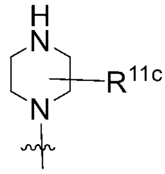, 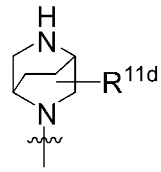,

wherein, $R^{11a}$, $R^{11b}$, $R^{11c}$ and $R^{11d}$ are each as defined herein.

In some embodiments, each $R^{11a}$, $R^{11b}$, $R^{11c}$ and $R^{11d}$ is independently -H, -D, -OH, -SH, -F, -Cl, -Br, -I, - CN, -C(=O)H, -C(=O)OR$^{6a}$, -C(=O)NR$^6$R$^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ haloalkoxy or 5-6 membered heteroaryl; wherein, $R^6$, $R^7$ and $R^{6a}$ are each as defined herein.

In some embodiments, $R^{11a}$, $R^{11b}$, $R^{11c}$ and $R^{11d}$ are each independently -H, -D, -OH, -SH, -F, -Cl, -Br, -I, - CN, -C(=O)H, -C(=O)OCH$_3$, -C(=O)NH$_2$, -CH$_3$, -CH$_2$CH$_3$, -(CH$_2$)$_2$CH$_3$, -(CH$_2$)$_4$CH$_3$, -CH(CH$_3$)$_2$, - CH$_2$CH(CH$_3$)$_2$, -C(CH$_3$)$_3$, -CH$_2$OH, -(CH$_2$)$_2$OH, -CH$_2$CN, -(CH$_2$)$_2$CN, -CF$_3$, -CHF$_2$, -CH$_2$F, -OCF$_3$, -OCH$_3$ or - OCH$_2$CH$_3$.

In some embodiments, $R^4$ is -H, -D, -OH, -SH, -F, -Cl, -Br, -I, -CN, methyl, ethyl, *n*-propyl, *i*-propyl, *n*-butyl or $C_{1-4}$ haloalkyl.

In some embodiments, ring A is $C_{6-12}$ aryl or 5-12 membered heteroaryl.

In some embodiments, ring A is one of the following sub-structures,

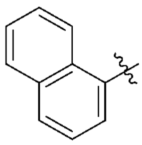, 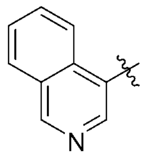, 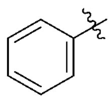, 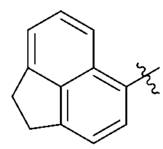, 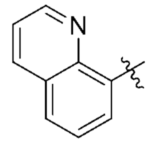, 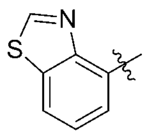, 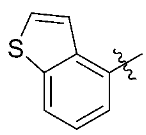,

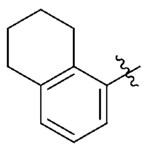, 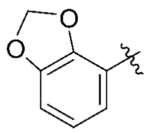, 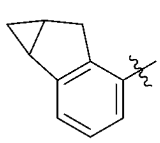, 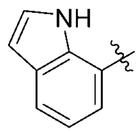, 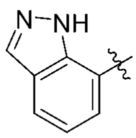, 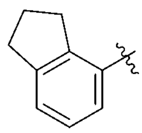, 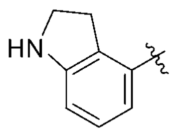,

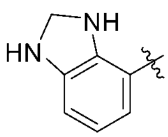, 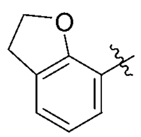, 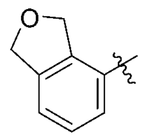, 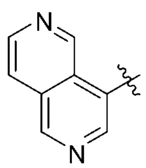, 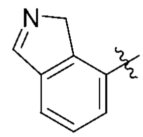, 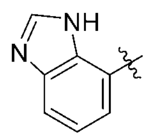, 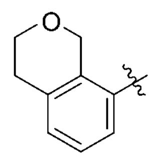,

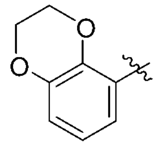 or 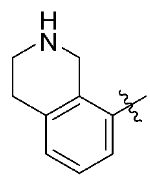.

In some embodiments, each $R^5$ is independently -D, -OH, -F, -Cl, -Br, -I, -CN, -SH, -CH$_2$C(=O)NR$^{6b}$R$^{7b}$, - C(=O) R$^{6c}$, -C(=O)OR$^{6c}$, -C(=O)NR$^{6b}$R$^{7b}$, -NR$^{6b}$C(=O)R$^{7b}$, -NR$^{6b}$R$^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein each of the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl is independently optionally substituted with 1, 2, 3 or 4 substituents selected from -D, -OH, -F, -Cl, -Br, -I, -CN, -NH$_2$, -C(=O)H, -C(=O) OH, $C_{1-6}$ alkyl, $C_{1-6}$ alkoxy, $C_{3-6}$ cycloalkyl or 3-6 membered heterocyclyl, wherein, $R^{6b}$, $R^{7b}$ and $R^{6c}$ are each as defined herein.

In some embodiments, each $R^5$ is independently -D, -OH, -F, -Cl, -Br, -I, -CN, -SH, -CH$_2$C(=O)NR$^{6b}$R$^{7b}$, - C(=O) R$^{6c}$, -C(=O)OR$^{6c}$, -C(=O)NR$^{6b}$R$^{7b}$, -NR$^{6b}$C(=O)R$^{7b}$, -NR$^{6b}$R$^{7b}$, $C_{1-4}$ alkyl, $C_{1-4}$ alkylthio, $C_{2-4}$ alkenyl, $C_{2-4}$ alkynyl, $C_{2-4}$

hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-12 membered aryl, 5-12 membered heteroaryl, $C_{3-6}$ cycloalkyl or 3-6 membered heterocyclyl, wherein each of the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-10 membered heteroaryl, $C_{3-6}$ cycloalkyl and 3-6 membered heterocyclyl is independently optionally substituted with 1, 2, 3 or 4 substituents selected from -D, - OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl or3-6 membered heterocyclyl; wherein, $R^{6b}$, $R^{7b}$ and $R^{6c}$ are each as defined herein.

In some embodiments, each $R^5$ is independently -D, -OH, -F, -Cl, -Br, -I, -CN, -SH, $-CH_2C(=O)NR^{6b}R^{7b}$, - C(=O) $R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C≡CH, $-C≡CCH_3$, - $CH_2C≡CH$, $-C≡CCH_2OH$, $-C≡C(CH_2)_2OH$, $-OCH_3$, $-OCH_2CH_3$, $-O(CH_2)_2CH_3$, $-CH_2CN$, $-(CH_2)_2CN$, - $(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CF_3$, $-CH_2CF_3$, $-CHF_2$, - $CH_2F$, $-(CH_2)_2Cl$, -CH=CHF, -CH=CHCl, $-CH=CHCH_2F$, $-C≡CCH_2F$, $-C≡C(CH_2)_2F$, -C≡CF, $-OCF_3$, $-OCHF_2$, - $OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein each of the $-CH_3$, $-CH_2CH_3$, $-(CH_2)_2CH_3$, $-(CH_2)_3CH_3$, - $C(CH_3)_3$, $-CH(CH_3)_2$, $-SCH_3$, $-SCH_2CH_3$, $-CH=CH_2$, $-CH=CHCH_3$, $-CH_2CH=CH_2$, -C≡CH, $-C≡CCH_3$, - $CH_2C≡CH$, $-C≡CCH_2OH$, $-C≡C(CH_2)_2OH$, $-OCH_3$, $-OCH_2CH_3$, $-O(CH_2)_2CH_3$, $-CH_2CN$, $-(CH_2)_2CN$, - $(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CH_2CF_3$, $-CHF_2$, $-CH_2F$, - $(CH_2)_2Cl$, -CH=CHF, -CH=CHCl, $-CH=CHCH_2F$, $-C≡CCH_2F$, $-C≡C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl is independently optionally substituted with 1, 2, 3 or 4 substituents selected from -D, -OH, -F, -Cl, -Br, -I, -CN, - $NH_2$, -C(=O)H, -C(=O)OH, methyl, ethyl, n-propyl, isopropyl, methoxy, ethoxy, n-propoxy, i-propoxy, cyclopropyl, cyclobutyl, cyclopentyl, cyclohexyl, pyrrolidinyl, oxazolidinyl, tetrahydrofuranyl, piperidyl and piperazinyl; wherein, $R^{6b}$, $R^{7b}$ and $R^{6c}$ are each as defined herein.

In some embodiments, each $R^6$, $R^7$, $R^{6b}$, $R^{6b}$, $R^{6c}$, $R^{7b}$, $R^{6d}$ and $R^{7d}$ is independently -H, -D or $C_{1-6}$ alkyl, wherein the $C_{1-6}$ alkyl is independently optionally substituted with 1, 2, 3 or 4 substituents selected from -D, - OH, -F, -Cl, -Br, -I, -CN, -C(=O)H, -C(=O)OH, $-NH_2$, $C_{1-6}$ alkoxy, $C_{6-12}$ aryl, $C_{3-6}$ cycloalkyl and 3-6 membered heterocyclyl.

In some embodiments, each $R^6$, $R^7$, $R^{6a}$, $R^{6b}$, $R^{6c}$, $R^{7b}$, $R^{6d}$, $R^{7d}$, $R^{6e}$ and $R^{7e}$ is independently -H, -D, methyl, ethyl, n-propyl, isopropyl, n-butyl, isobutyl or tert-butyl, wherein each of the methyl, ethyl, n-propyl, isopropyl, n-butyl, isobutyl and tert-butyl is independently optionally substituted with 1, 2, 3 or 4 substituents selected from -D, -OH, -F, -Cl, -Br, -I, -CN, C(=O)H, -C(=O)OH, $-NH_2$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl.

In some embodiments,

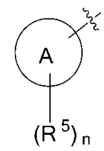

is the structure:

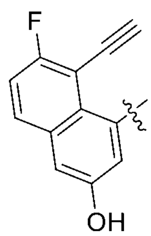 , 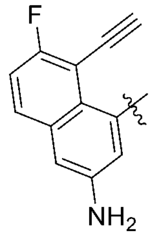 , 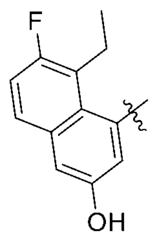 ,

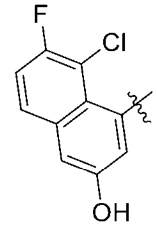, 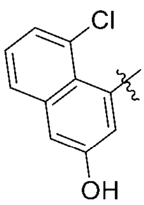, 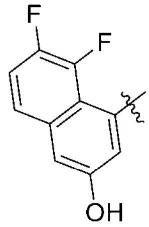, 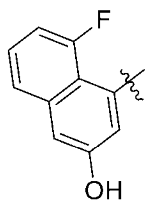, 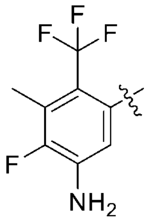, 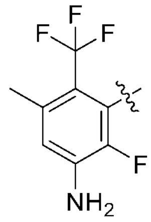, 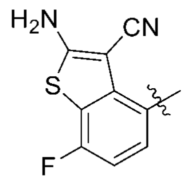,

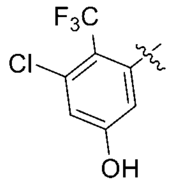 or 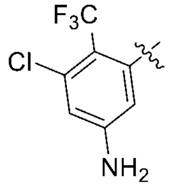.

In some embodiments, n is 0, 1, 2, 3, 4, 5, 6 or 7.

In some embodiments, each $q_1$, $q_2$ and $q_3$ is independently 0, 1, 2, 3, 4, 5 or 6.

In some embodiments, each $q_4$ is independently 0, 1, 2, 3 or 4.

In some embodiments, $R^2$ is

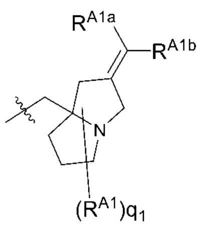, 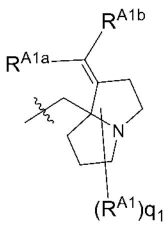, 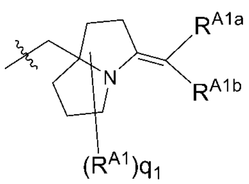, 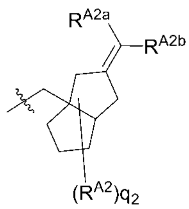,

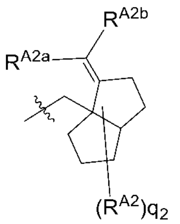 or 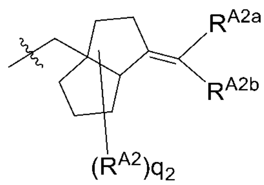,

wherein, $R^{A1a}$, $R^{A1b}$, $R^{A2a}$, $R^{A2b}$, $R^{A1}$, $R^{A2}$, $q_1$ and $q_2$ are each as defined herein.

In some embodiments, $R^2$ is

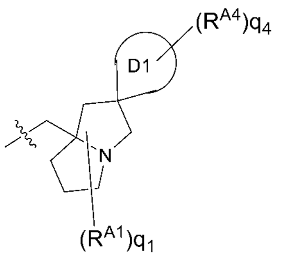, 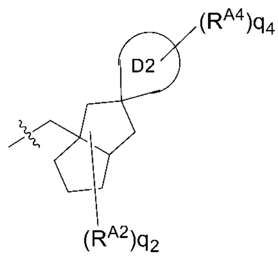

or

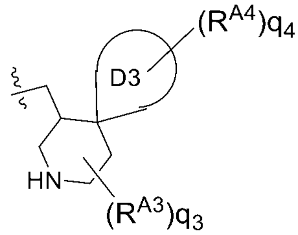,

wherein, each of ring D1, ring D2 and ring D3 is independently cyclopropyl, cyclobutyl, cyclopentyl, cyclohexyl, oxiranyl, oxetanyl, azetidinyl, pyrrolidinyl, oxazolidinyl, tetrahydrofuranyl, piperidinyl and piperazinyl, wherein, $R^{A1}$, $R^{A2}$, $R^{A3}$, $R^{A4}$, $q_1$, $q_2$, $q_3$ and $q_4$ are as defined herein.

In some embodiments, $R^2$ is

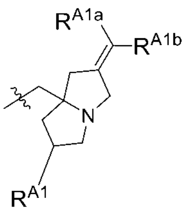, 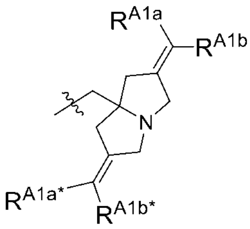, 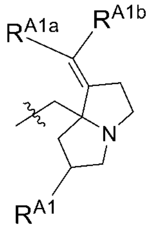, 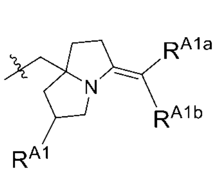,

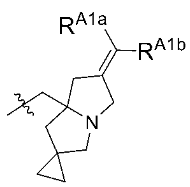, 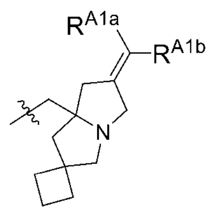, 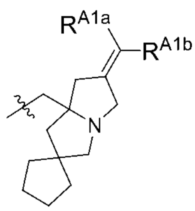, 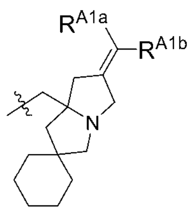, 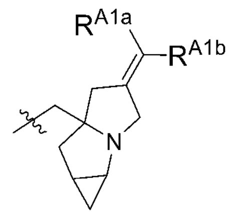,

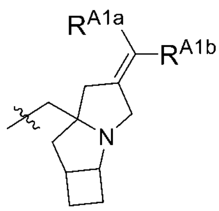, 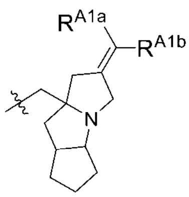, 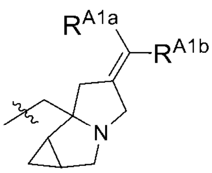, 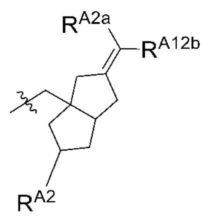, 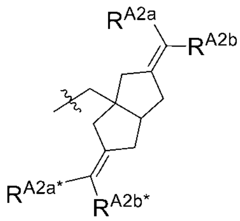,

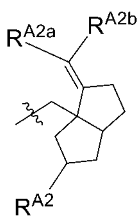, 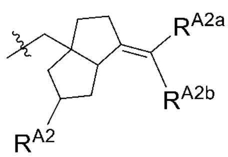, 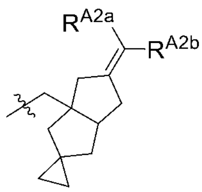 or 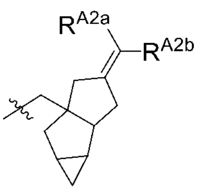,

wherein, $R^{A1a}$, $R^{A1b}$, $R^{A2a}$, $R^{A2b}$, $R^{A1}$, $R^{A2}$, $R^{A1a*}$ and $R^{A1b*}$ are as defined herein.

In some embodiments, $R^2$ is

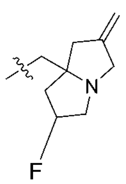, 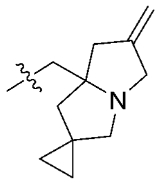, 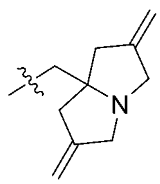, 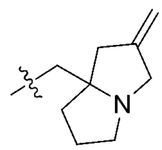, 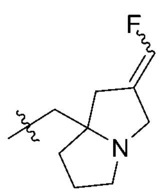,

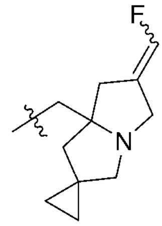, 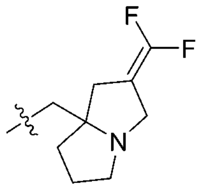, 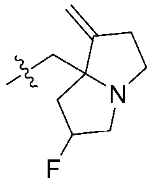, 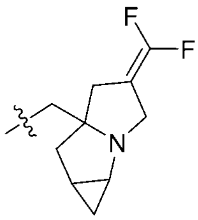, 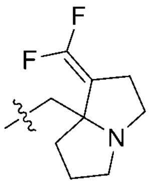, 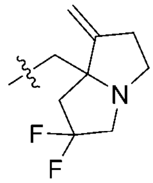,

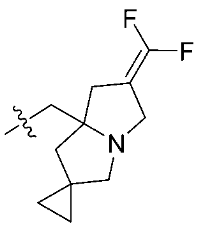, 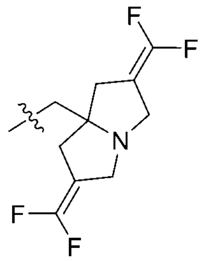, 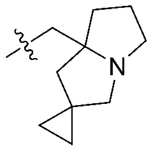 or 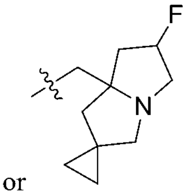.

In some embodiments, $R^2$ is

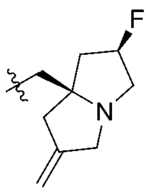, 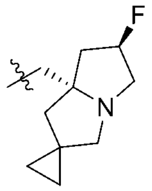, 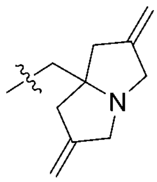, 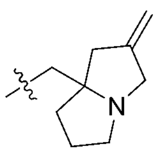, 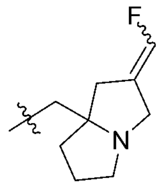,

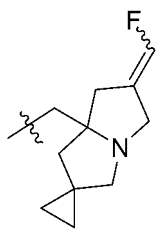, 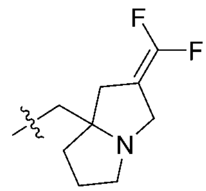 or 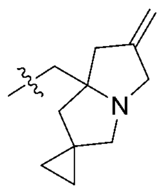.

In some embodiments, the compound of the present invention is a compound of Formula (I-1), or a stereoisomer, tautomer, nitrogen oxide, solvate, metabolite, pharmaceutically acceptable salt, or prodrug thereof,

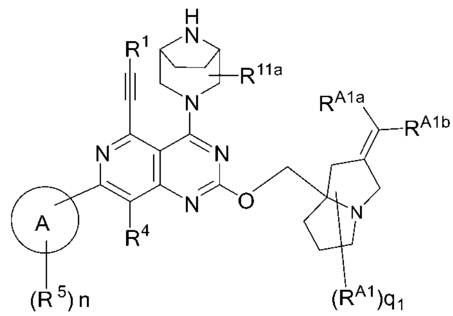 (I-1),

wherein, the $R^1$, $R^4$, $R^5$, $R^{A1}$, $R^{A1a}$, $R^{A1b}$, $R^{11a}$, n and $q_1$ are each as defined herein.

In some embodiments, the compound of the present invention is a compound of Formula (I-2), or a stereoisomer, tautomer, nitrogen oxide, solvate, metabolite, pharmaceutically acceptable salt, or prodrug thereof,

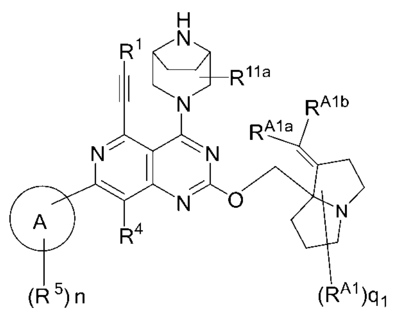

(I-2),

wherein, the $R^1$, $R^4$, $R^5$, $R^{A1}$, $R^{A1a}$, $R^{A1b}$, $R^{11a}$, n and $q_1$ are each as defined herein.

In some embodiments, the compound of the present invention is a compound of Formula (I-3), or a stereoisomer, tautomer, nitrogen oxide, solvate, metabolite, pharmaceutically acceptable salt, or prodrug thereof,

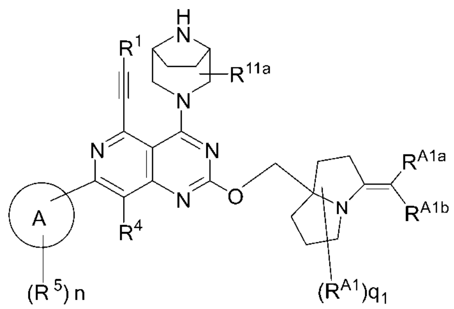

(I-3),

wherein, the $R^1$, $R^4$, $R^5$, $R^{A1}$, $R^{A1a}$, $R^{A1b}$, $R^{11a}$, n and $q_1$ are each as defined herein.

In some embodiments, the compound of the present invention is a compound of Formula (I-1a), or a stereoisomer, tautomer, nitrogen oxide, solvate, metabolite, pharmaceutically acceptable salt, or prodrug thereof,

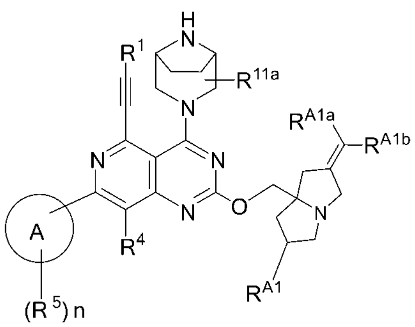

(I-1a),

wherein, the $R^1$, $R^4$, $R^5$, $R^{A1}$, $R^{A1a}$, $R^{A1b}$, $R^{11a}$ and n are each as defined herein.

In some embodiments, the compound of the present invention is a compound of Formula (I-1b), or a stereoisomer, tautomer, nitrogen oxide, solvate, metabolite, pharmaceutically acceptable salt, or prodrug thereof,

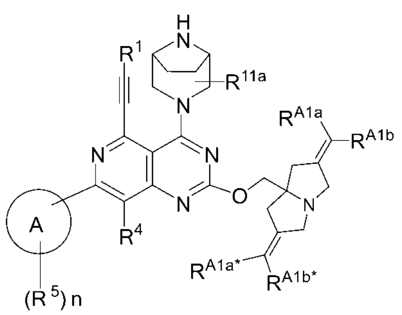

(I- 1b),

wherein, the $R^1$, $R^4$, $R^5$, $R^{A1a}$, $R^{A1b}$, $R^{A1a*}$, $R^{A1b*}$, $R^{11a}$ and n are each as defined herein.

In some embodiments, the compound of the present invention is a compound of Formula (I-1c), or a stereoisomer, tautomer, nitrogen oxide, solvate, metabolite, pharmaceutically acceptable salt, or prodrug thereof,

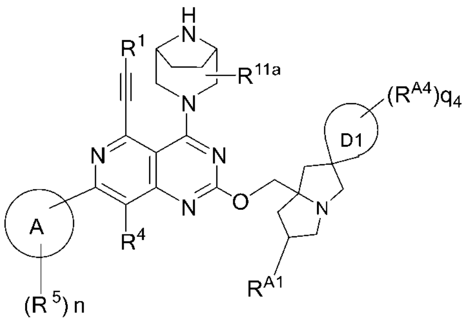

(I- 1c),

wherein, the $R^1$, $R^4$, $R^5$, $R^{A1}$, $R^{A1a}$, $R^{A1b}$, $R^{A1a*}$, $R^{A1b*}$, $R^{11a}$, $R^{A4}$, $q_4$ and n are each as defined herein.

In some embodiments, the compound of the present invention is a compound of Formula (IB), or a stereoisomer, tautomer, nitrogen oxide, solvate, metabolite, pharmaceutically acceptable salt, or prodrug thereof,

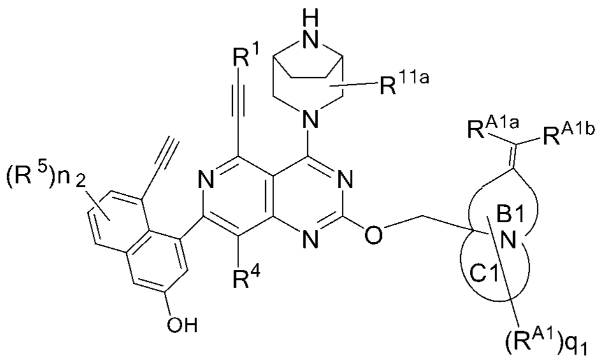

(IB),

wherein, $n_2$ is 1, 2, 3, 4 or 5; the $R^1$, $R^4$, $R^5$, $R^{A1a}$, $R^{A1b}$, $R^{A1}$ and $q_1$ are each as defined herein.

In some embodiments, the compound of the present invention is a compound of Formula (IC), or a stereoisomer, tautomer, nitrogen oxide, solvate, metabolite, pharmaceutically acceptable salt, or prodrug thereof,

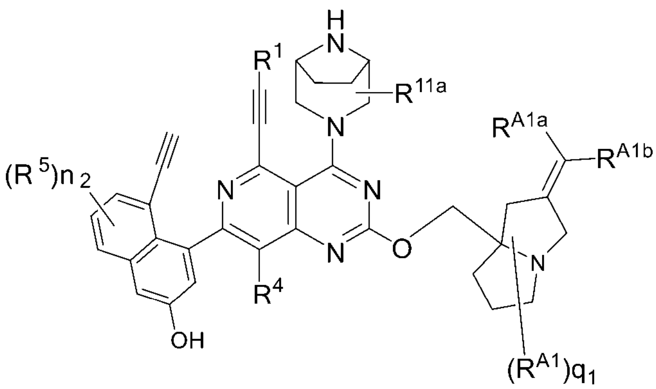

(IC)

(IC),

wherein, $n_2$ is 1, 2, 3, 4 or 5; the $R^1$, $R^4$, $R^5$, $R^{A1a}$, $R^{A1b}$, $R^{A1}$ and $q_1$ are each as defined herein.

In some embodiments, the compounds described herein have the following structure, or a stereoisomer, tautomer, N-oxide, solvate, metabolite, pharmaceutically acceptable salt, or prodrug thereof,

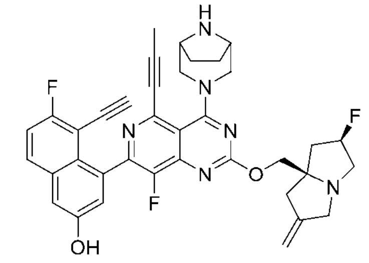
1,
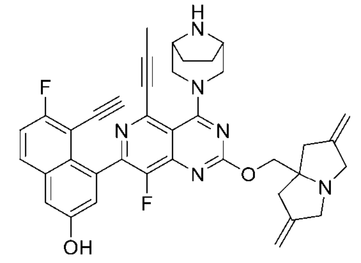
2,
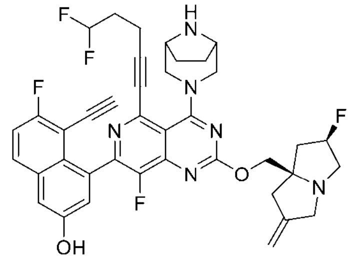
3,
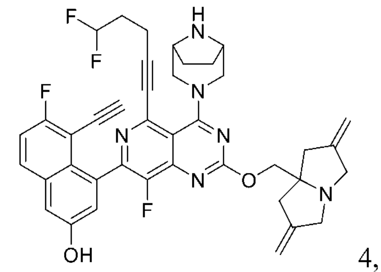
4,
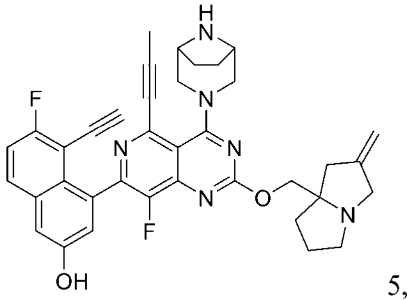
5,
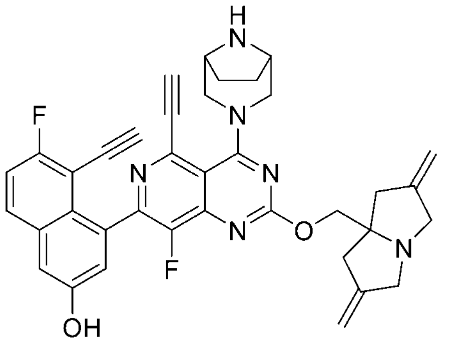
6,
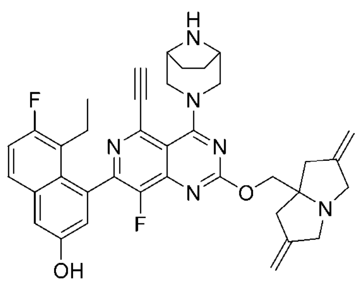
7,
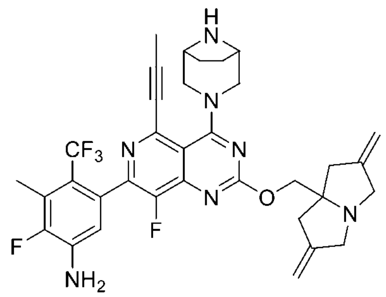
8,
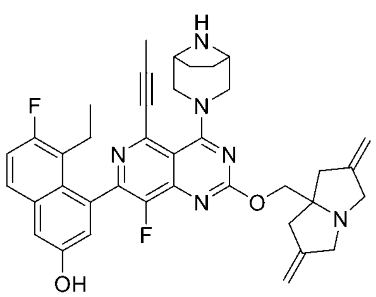
9,
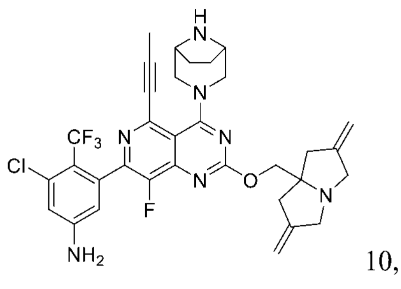
10,
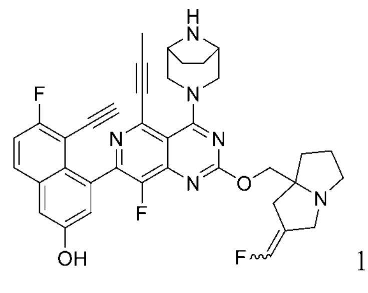
11,
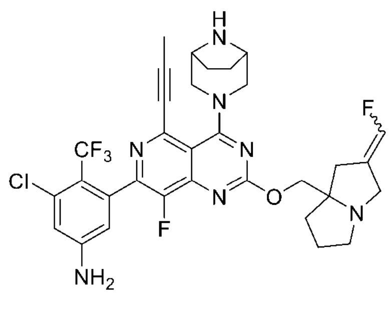
12,
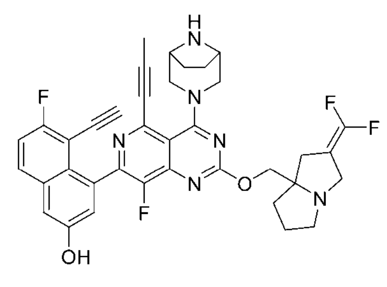
13,
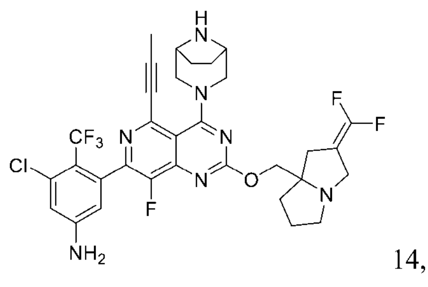
14,
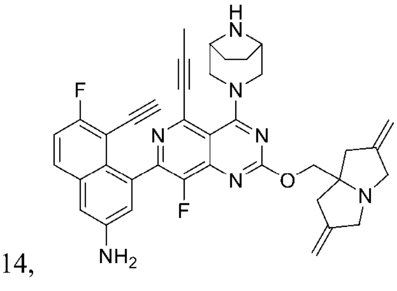
15,

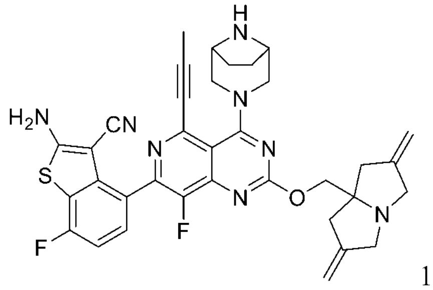
16,
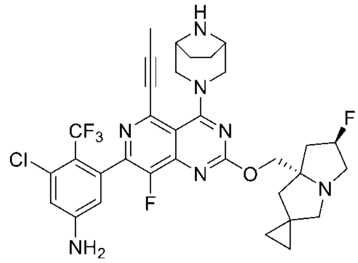
17,
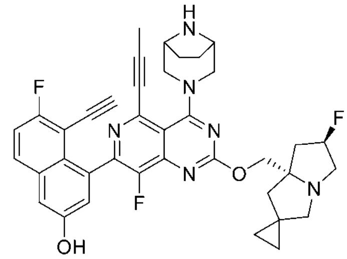
18,

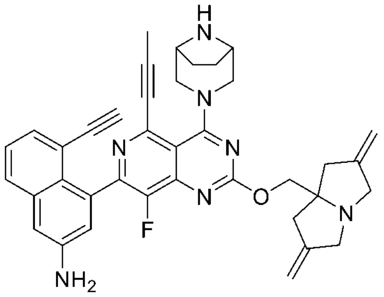
19,
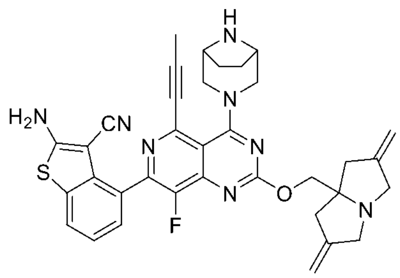
20,
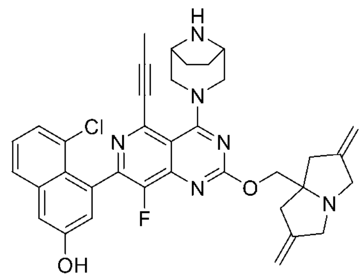

21,
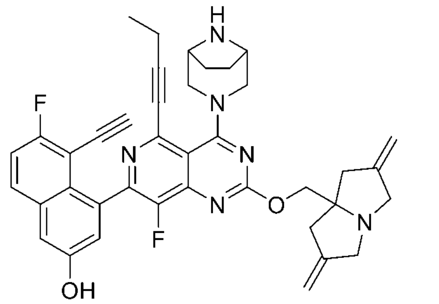
22,
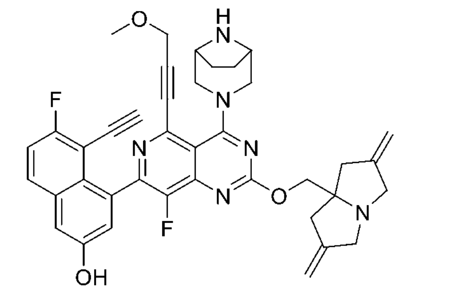
23,

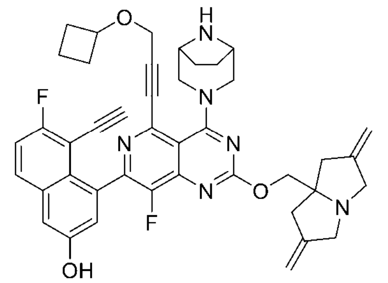
24,
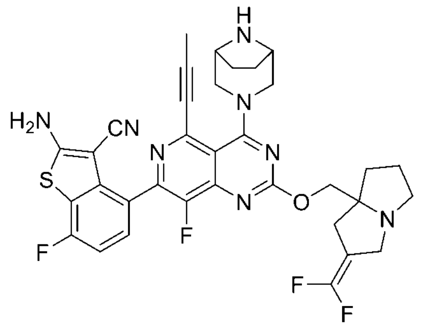
25,

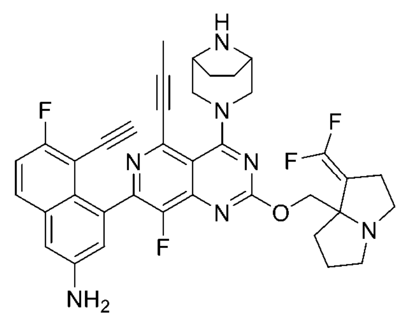
26,
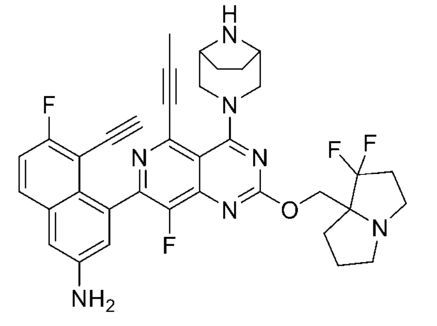
27,

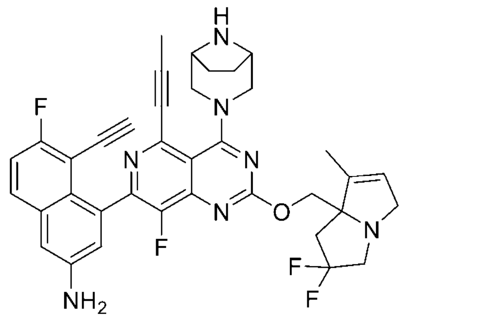

28,

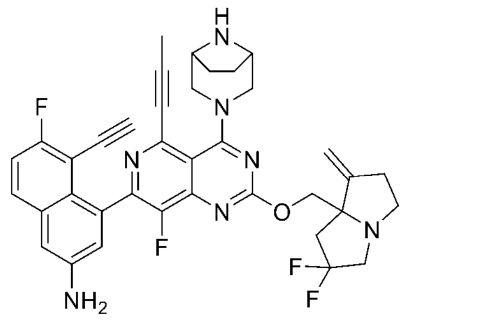

29,

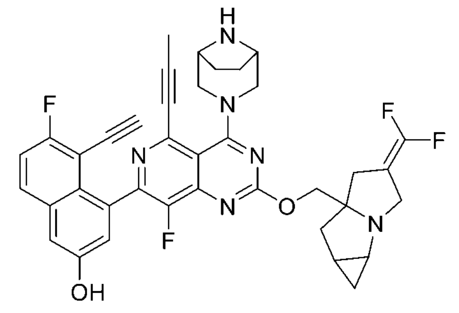

30,

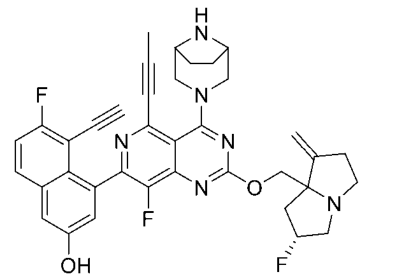

31,

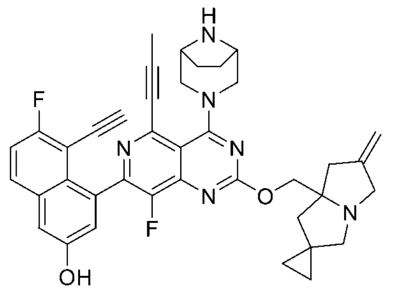

32,

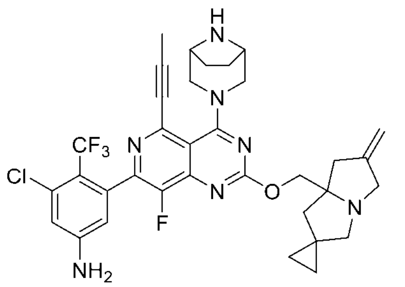

33,

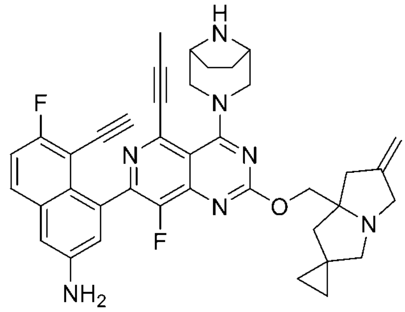

34,

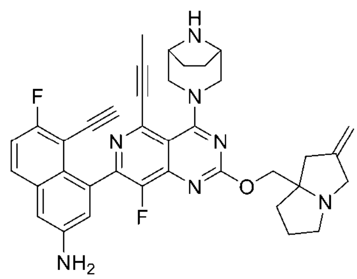

35,

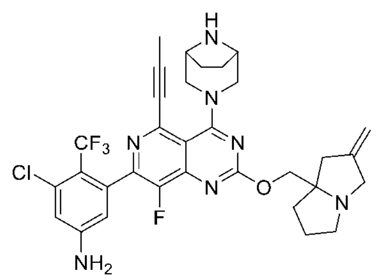

36,

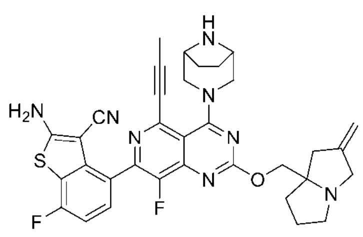

37,

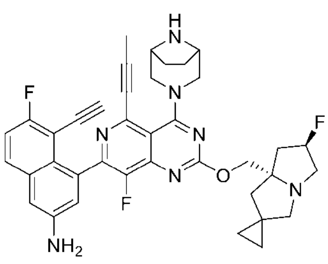

38,

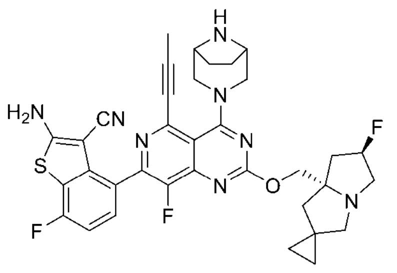

39, 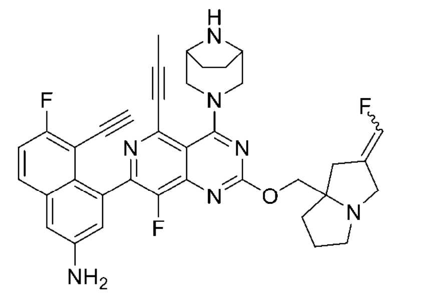 40, 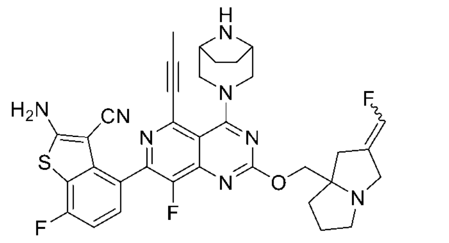 41

or

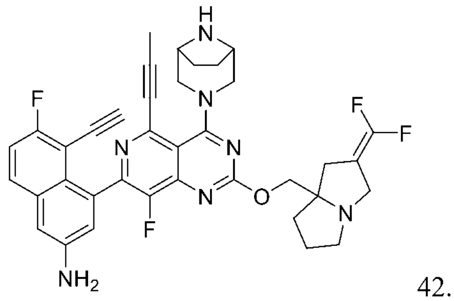

42.

In other aspect, provided herein is a pharmaceutical composition comprising the compound disclosed herein.

In some embodiments, the pharmaceutical compositions of the present invention further comprise pharmaceutically acceptable adjuvant.

In some embodiments, the adjuvants of the present invention include, but are not limited to, carriers, excipients, diluents, vehicles, or combinations thereof. In some embodiments, the pharmaceutical composition may be in a liquid, solid, semi-solid, gel, or spray dosage form.

In other aspect, the present invention provides a use of the pharmaceutical composition of the present invention in the preparation of a medicament for preventing, treating, or alleviating a disease associated with KRAS G12D.

In some embodiments, the KRAS G12D-related disease described herein is cancer.

In some embodiments, the compound of the present invention or pharmaceutical composition thereof is effective for preventing, treating, or alleviating cancer conditions in patients including, but not limited to: sarcoma (angiosarcoma, fibrosarcoma, rhabdomyosarcoma, liposarcoma), myxoma, rhabdomyosarcoma, fibroma, lipoma and teratoma; lung cancer: bronchial cancer (squamous cell, undifferentiated small cell, undifferentiated large cell, adenocarcinoma), non-small cell lung cancer, small cell lung cancer, alveolar (bronchiolar) cancer, bronchial adenoma, sarcoma, lymphoma, chondroma, hamartoma, mesothelioma; gastrointestinal cancer: esophagus (squamous cell carcinoma, Cancers of the stomach (ductal adenocarcinoma, insulinoma, glucagonoma, gastrinoma, carcinoid tumor, vipoma), small intestine (adenocarcinoma, lymphoma, carcinoid tumor, Kaposi's sarcoma, leiomyoma, hemangioma, lipoma, neurofibroma, fibroma), colorectal cancer; genitourinary tract cancers: kidney (adenocarcinoma, Wilms' tumor (Nephroblastoma), lymphoma, leukemia), bladder and urethra cancers (squamous cell carcinoma, Transitional cell carcinoma, adenocarcinoma), prostate (adenocarcinoma, sarcoma), testicular (seminoma, teratoma, embryonal carcinoma, teratocarcinoma, choriocarcinoma, sarcoma, stromal cell carcinoma, fibroma, fibroadenoma, adenomatous tumor, lipoma) cancer; liver cancer: liver cancer (hepatocellular carcinoma), bile duct cancer, hepatoblastoma, angiosarcoma, hepatocellular adenoma, hemangioma; bile duct cancer: gallbladder cancer, ampullary carcinoma, bile duct cancer; bone cancer: osteosarcoma, fibrosarcoma, malignant fibrous histiocytoma, chondrosarcoma, Ewing's sarcoma, malignant lymphoma Cancers of the nervous system: neuroma, skull (osteomas, hemangiomas, granulomas, xanthomas, osteitis deformans), meningeal cancers (meningiomas, meningeal sarcomas, gliomas), brain cancers (astrocytomas, medulloblastomas, gliomas, ependymomas, germ cell tumors (pinealomas), glioblastoma multiforme , oligodendroglioma, schwannoma, retinoblastoma, congenital tumors), spinal neurofibroma, meningioma, glioma, sarcoma); gynecological cancers: uterus (endometrial carcinoma (serous cystadenocarcinoma, mucinous cystadenocarcinoma, unclassified carcinoma), granulosa cell tumor, interstitial cell tumor, dysgerminoma, malignant teratoma), vulva (squamous cell carcinoma, intraepithelial carcinoma, adenocarcinoma), fibrosarcoma, melanoma), vaginal cancer (clear cell carcinoma, squamous cell carcinoma, grape-like sarcoma) Cancers of the ovary (embryonal rhabdomyosarcoma), fallopian tube (carcinoma), ovarian, and breast; Hematologic cancers: Leukemias (myeloid leukemias (acute and chronic), acute lymphocytic leukemia, chronic lymphocytic leukemia, myeloproliferative leukemia), multiple myeloma, and myelodysplastic syndromes), Hodgkin's disease, and non-Hodgkin's lymphomas (malignant lymphomas); Skin cancers: melanoma, basal cell

carcinoma, squamous cell carcinoma, Kaposi's sarcoma, nevus dysplasia, lipoma, hemangioma, dermatofibroma, keloid, and psoriasis; and Adrenal gland cancer: neuroblastoma.

In some embodiments, the cancer described herein is non-small cell lung cancer, small cell lung cancer, colorectal cancer, rectal cancer, colon cancer, small intestine cancer, pancreatic cancer, uterine cancer, gastric cancer, esophageal cancer, prostate cancer, ovarian cancer, breast cancer, leukemia, melanoma, lymphoma or neuroma.

In other aspect, the present invention further provides a method for preventing or treating a disease associated with KRAS G12D, comprising administering to a patient a therapeutically effective amount of the compound according to the present invention or a pharmaceutical composition thereof.

In other aspect, the present invention relates to a method for the preparation, isolation, and purification of the compound of Formula (I), (I-1), (I-2), (I-3), (I-1a), (I-1b), (IB) or (IC).

Unless otherwise stated, all stereoisomers, tautomers, N-oxides, hydrates, solvates, metabolites, and pharmaceutically acceptable salts and prodrugs of the compounds disclosed herein are within the scope of the invention.

The term "pharmaceutically acceptable" refers to that the substance or composition must be compatible chemically and/or toxicologically,

with the other ingredients comprising a formulation, and/or the mammal being treated therewith.

The salt of the compounds disclosed herein also include salts of the compounds which are not necessarily pharmaceutically acceptable salts, and which may be useful as intermediates for preparing and/or purifying compounds of Formula (I), (I-1), (I-2), (I-3), (I-1a), (I-1b), (IB) or (IC) or salts for separating enantiomers of compounds of Formula (I), (I-1), (I-2), (I-3), (I-1a), (I-1b), (IB) or (IC).

**Pharmaceutical compositions, formulations, administration and uses of the compounds of the present invention**

Characteristics of the pharmaceutical composition of the present invention include a compound having the structure of Formula (I), (I-1), (I-2), (I-3), (I-1a), (I-1b), (IB) or (IC), a compound disclosed herein, or an embodiment compound, and a pharmaceutically acceptable carrier. The amount of the compound in the pharmaceutical composition of the present invention is effective to treat or alleviate a KRAS G12D-mediated disease in a patient.

It will also be appreciated that certain of the compounds disclosed herein can exist in free form for treatment, or where appropriate, as a pharmaceutically acceptable derivative thereof. Some non-limiting examples of the pharmaceutically acceptable derivative include pharmaceutically acceptable prodrugs, salts, esters, salts of such esters, or any other adducts or derivatives which upon administration to a patient in need is capable of providing, directly or indirectly, a compound as otherwise described herein, or a metabolite or residue thereof.

As described herein, the pharmaceutical composition of the present invention further comprises a pharmaceutically acceptable excipient, including, but not limited to, any solvent, diluent, or other liquid vehicle; dispersing or suspending agent; surfactant; tonicity agent; thickening agent; emulsifier; preservative; solid binder; or lubricant; and the like, suitable for a particular target dosage form. As described in the following: In Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, based on a synthesis of the literature herein, it is demonstrated that various excipients are applicable in the formulation of pharmaceutically acceptable compositions through industry-standard preparation methods. Except where conventional excipients are incompatible with the compound of the present invention-such as instances producing any adverse biological effects or detrimental interactions with other components of the pharmaceutical composition-their use remains encompassed within the scope of the present invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants.

In preparing the pharmaceutical composition provided by the present invention, the active ingredient is typically

mixed with an excipient, diluted by the excipient, or encapsulated within a carrier in the form of, for example, a capsule, sachet, paper, or other container. When employed as a diluent, the excipient may be a solid, semi-solid, or liquid material that functions as a vehicle, carrier, or medium for the active ingredient. Suitable carriers include, but are not limited to, magnesium carbonate, magnesium stearate, talc, sugar, lactose, pectin, dextrin, starch, gelatin, tragacanth, methylcellulose, sodium carboxymethylcellulose, low-melting waxes, cocoa butter, and the like. Accordingly, the compositions may be formulated as tablets, pills, powders, lozenges, sachets, cachets, elixirs, suspensions, emulsions, solutions, syrups, aerosols (in solid form or in a liquid medium), ointments, soft and hard gelatin capsules, suppositories, sterile injectable solutions, and sterile packaged powders. In one embodiment, the composition is formulated for oral administration. In one embodiment, the composition is formulated as a tablet or capsule.

The compound or the pharmaceutical composition of the present invention may be administered in the form of oral dosage forms such as tablets, capsules each of which includes a sustained-release or time-release formula, pills, powders, granules, elixirs, tinctures, suspensions, syrups and emulsifiers. They may also be administered intravenously (bolus or infusion), intraperitoneally, subcutaneously or intramuscularly, all dosage forms used are well-known to one of ordinary skill in the pharmaceutical arts. They can be administered alone, but will generally be administered together with a single pharmaceutical carrier based on the chosen mode of administration and standard pharmaceutical practice.

The compound or the pharmaceutical composition of the present invention may be administered intranasally, for topical use with a suitable intranasal vehicle, or transdermally by the use of a transdermal patch. When administered in the form of a transdermal delivery system, the dosage administered throughout the dosage is continuous rather than intermittent.

The compound or the pharmaceutical composition of the invention may also be administered in the form of liposomal delivery systems, such as small, monolayer, large, single-layered vesicles and multilamellar vesicles. Liposomes can be formed by different phospholipids, such as cholesterol, stearylamine, or phosphatidylcholine.

The compound or the pharmaceutical composition of the present invention are also conjugated to soluble polymers that serve as targeted drug carriers. Such polymers may encompass polyvinylpyrrolidone, pyran copolymer, polyhydroxypropylmethacrylamide-phenol, polyhydroxyethylaspartamide phenol, or polyethylene oxide-polylysine substituted with palmitoyl residues. Moreover, the compounds of the invention can be coupled to a class of biodegradable polymers, used to complete controlled release of drugs. For example, polylactic acid, polyglycolic acid, copolymers of polylactic acid and polyglycolic acid, polyepsilon caprolactone, polyhydroxybutyric acid, polyorthoesters, polyacetals, polydihydropyrans, polycyanoacrylates, and crosslinked or amphipathic blocking copolymers of hydrogels.

The dosage regimen of the compound or the pharmaceutical composition of this invention will vary with known factors such as the pharmacokinetic profile of the particular agent and its mode and route of administration; the race, age, sex, health status, medical condition and weight of the recipient; The nature and extent of symptoms; the type of concurrent treatment; the frequency of treatment; the route of administration; the renal and hepatic function of the patient; and the effect desired. A physician or veterinarian can make a decision and prescribe an effective amount of medication to prevent, counteract or prevent the development of cancer.

In accordance with the general guidelines, the dosage of each active ingredient used is in the range of about 0.001 to 1000 mg/kg of body weight, preferably from about 0.01 to 100 mg/kg of body weight, in order to achieve the indicated effect between. The compounds of the present invention may be administered once daily, or they may be administered in two, three or four times daily.

Each unit dose of dosage form (pharmaceutical composition) suitable for administration may contain from about 1 mg to about 100 mg of active ingredient. In these pharmaceutical compositions, the weight of the active ingredient will generally be about 0.5-95% of the total weight of the composition.

The compounds and compositions described herein may be administered alone or in combination with other compounds or other therapeutic agents. The compounds or compositions of the present invention may be administered concurrently or sequentially with other therapeutic agents via the same or different routes of administration. The compounds of the present invention may be included together with other therapeutic agents in a single formulation or in separate formulations.

When the compounds of the present invention are administered together with other therapeutic agents, the amount of each component in a typical daily dose and typical dosage form may generally be reduced relative to the usual dose administered alone, taking into account the additive or synergistic effects of the therapeutic agents when administered in combination.

The compound or pharmaceutically acceptable salt or hydrate thereof or pharmaceutical composition thereof of the present invention can be effectively used for preventing, treating or alleviating KRAS-mediated diseases, especially KRAS G12D-mediated diseases, particularly cancer, in patients.

In some embodiments, the compound of the present invention or pharmaceutical composition thereof is effective for preventing, treating, or alleviating cancer conditions in patients including, but not limited to: sarcoma (angiosarcoma, fibrosarcoma, rhabdomyosarcoma, liposarcoma), myxoma, rhabdomyosarcoma, fibroma, lipoma and teratoma; lung cancer: bronchial cancer (squamous cell, undifferentiated small cell, undifferentiated large cell, adenocarcinoma), non-small cell lung cancer, small cell lung cancer, alveolar (bronchiolar) cancer, bronchial adenoma, sarcoma, lymphoma, chondroma, hamartoma, mesothelioma; gastrointestinal cancer: esophagus (squamous cell carcinoma, Cancers of the stomach (ductal adenocarcinoma, insulinoma, glucagonoma, gastrinoma, carcinoid tumor, vipoma), small intestine (adenocarcinoma, lymphoma, carcinoid tumor, Kaposi's sarcoma, leiomyoma, hemangioma, lipoma, neurofibroma, fibroma), colorectal cancer; genitourinary tract cancers: kidney (adenocarcinoma, Wilms' tumor (Nephroblastoma), lymphoma, leukemia), bladder and urethra cancers (squamous cell carcinoma, Transitional cell carcinoma, adenocarcinoma), prostate (adenocarcinoma, sarcoma), testicular (seminoma, teratoma, embryonal carcinoma, teratocarcinoma, choriocarcinoma, sarcoma, stromal cell carcinoma, fibroma, fibroadenoma, adenomatous tumor, lipoma) cancer; liver cancer: liver cancer (hepatocellular carcinoma), bile duct cancer, hepatoblastoma, angiosarcoma, hepatocellular adenoma, hemangioma; bile duct cancer: gallbladder cancer, ampullary carcinoma, bile duct cancer; bone cancer: osteosarcoma, fibrosarcoma, malignant fibrous histiocytoma, chondrosarcoma, Ewing's sarcoma, malignant lymphoma Cancers of the nervous system: neuroma, skull (osteomas, hemangiomas, granulomas, xanthomas, osteitis deformans), meningeal cancers (meningiomas, meningeal sarcomas, gliomas), brain cancers (astrocytomas, medulloblastomas, gliomas, ependymomas, germ cell tumors (pinealomas), glioblastoma multiforme , oligodendroglioma, schwannoma, retinoblastoma, congenital tumors), spinal neurofibroma, meningioma, glioma, sarcoma); gynecological cancers: uterus (endometrial carcinoma (serous cystadenocarcinoma, mucinous cystadenocarcinoma, unclassified carcinoma), granulosa cell tumor, interstitial cell tumor, dysgerminoma, malignant teratoma), vulva (squamous cell carcinoma, intraepithelial carcinoma, adenocarcinoma), fibrosarcoma, melanoma), vaginal cancer (clear cell carcinoma, squamous cell carcinoma, grape-like sarcoma) Cancers of the ovary (embryonal rhabdomyosarcoma), fallopian tube (carcinoma), ovarian, and breast; Hematologic cancers: Leukemias (myeloid leukemias (acute and chronic), acute lymphocytic leukemia, chronic lymphocytic leukemia, myeloproliferative leukemia), multiple myeloma, and myelodysplastic syndromes), Hodgkin's disease, and non-Hodgkin's lymphomas (malignant lymphomas); Skin cancers: melanoma, basal cell carcinoma, squamous cell carcinoma, Kaposi's sarcoma, nevus dysplasia, lipoma, hemangioma, dermatofibroma, keloid, and psoriasis; and Adrenal gland cancer: neuroblastoma.

In particular, the compound of the present invention or pharmaceutical composition thereof is effective for preventing, treating, or alleviating cancer conditions in patients including non-small cell lung cancer, small cell lung cancer, colorectal cancer, rectal cancer, colon cancer, small intestine cancer, pancreatic cancer, uterine cancer, gastric cancer, esophageal cancer, prostate cancer, ovarian cancer, breast cancer, leukemia, melanoma, lymphoma or neuroma.

## GENERAL SYNTHETIC PROCEDURES

To describe the present invention, the invention will be further illustrated by the following examples with respect to its technical solution. The following examples are provided solely to illustrate specific embodiments of the present invention, enabling those skilled in the art to understand the invention, but are not to be construed as limiting the scope of protection thereof. In the specific embodiments of the present invention, technical means or methods not otherwise specified are conventional technical means or methods in the art.

Unless further defined, the definitions of substituents are as described herein in the present invention. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius (°C), room temperature in the examples refers to 15 °C - 30 °C; in some examples, room temperature is 20 °C - 30 °C. Reagents were purchased from commercial suppliers such as Aldrich Chemical Company, Arco Chemical Company and Alfa Chemical Company, and were used without further purification. Unless otherwise specified, general reagents were purchased from Shantou XiLong Chemical Factory, Guangdong Guanghua Reagent Chemical Factory Co. Ltd., Guangzhou Reagent Chemical Factory, Tianjin YuYu Fine Chemical Ltd., Tianjin Fuchen Chemical Reagent Factory, Wuhan Xinhua Yuan Technology Development Co., Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous CH2Cl2 and CHCl3 were obtained by refluxing the solvent with CaH2. EtOAc, PE, hexane, DMAC and DMF were treated with anhydrous $Na_2SO_4$ prior use.

The reactions set forth below were done generally under a positive pressure of nitrogen or argon or with a drying tube (unless otherwise stated) in anhydrous solvents, and the reaction flasks were typically fitted with rubber septa for the

introduction of substrates and reagents via syringe. Glassware was oven dried and/or heat dried. Glassware was oven dried and/or heat dried.

Column chromatography was conducted using a silica gel column. Silica gel (300-400 mesh) was purchased from Qingdao Ocean Chemical Factory.

The $^1H$ NMR spectra were recorded using a Bruker 400 MHz, 600 MHz or 599 MHz nuclear magnetic resonance spectrometer. $^1H$ NMR spectra were obtained by using $CDCl_3$, DMSO-$d_6$, $CD_3OD$ or acetone-$d_6$ solutions (reported in ppm), with TMS (0 ppm) or chloroform (7.26 ppm) as the reference standard. When peak multiplicities are reported, the following abbreviations are used: s (singlet), d (doublet), t (triplet), q (quartet), m (multiplet), br (broadened), br s (broadened singlet, br.s), dd (doublet of doublets), dt (doublet of triplets), qt (quartet of triplets). Coupling constant *J*, when given, was reported in Hertz (Hz).

Low-resolution mass spectrometry (MS) data were acquired using an Agilent 6120 quadrupole HPLC-MS system (column: Zorbax SB-C18, 2.1 × 30 mm, 3.5 μm, 6 min, flow rate: 0.6 mL/min). The mobile phases consisted of a combination of A (0.1 % formic acid in $CH_3CN$) and B (0.1 % formic acid in $H_2O$) in gradient mode (5% to 95%), and an ESI source was used, the peak of HPLC was recorded with UV-Vis detection at 210/254 nm.

Pure compounds were purified using either an Agilent 1260 preparative HPLC system or a Calesep pump 250 preparative HPLC system (column: NOVASEP 50/80 mm DAC) with UV detection at 210 nm/254 nm.

The following abbreviations or English words are used throughout this invention:

| | | | |
|---|---|---|---|
| EtOAc, EA | ethyl acetate | DIPEA | *N,N*- diisopropylethylamine |
| $PdCl_2(PPh_3)_2$, | $Pd(PPh_3)_2Cl_2$ Bis(triphenylphosphine)palladium(II) chloride | $POCl_3$ | Phosphorus oxychloride |
| TEA, $Et_3N$ | triethylamine | toluene | toluene |
| PE | Petroleum ether | OMOM | Methoxymethyloxy, $-OCH_2OCH_3$ |
| THF | tetrahydrofuran | TIPS | Triisopropylsilyl |
| DMSO | dimethylsulfoxide | $K_3PO_4 \cdot 7H_2O$ | Potassium phosphate heptahydrate |
| DMSO-$d_6$ | Dimethyl sulfoxide-$d_6$ | ice-bath | ice-bath |
| DCM | dichloromethane | $C_SF$ | Caesium fluoride |
| TFA, $CF_3COOH$ | Trifluoroacetic acid dioxane, 1,4-dioxane | DMF | *N*,*N*-dimethylformamide |
| | | Pre-TLC | Preparative thin layer chromatography |
| $CS_2CO_3$ | cesium carbonate | h | hour, hours |
| HCl/dioxane | A soultion of hydrogen chloride in 1,4-dioxane | min | minute, minutes |
| MeCN, | ACN acetonitrile | NaH | sodium hydride |
| PTSA | *P*-toluenesulfonic acid | °C | degrees Celsius |
| $Na_2SO_3$ | sodium sulfate | g | gram |
| $NaHCO_3$ | sodium bicarbonate | mmol | millimole |
| MeOH | methanol | mL | milliliter |
| $NH_3$ | Ammonia | mg | milligram |
| $NH_3$/MeOH, methanol$NH_3$.MeOH | a solution of Ammonia in | rt | room temperature |
| M, mol/L | Mole/liter | HTRF | homogeneous time-resolved fluorescence |
| $NH_2$Boc | *tert-Butyl* carbamate | TMS | Trimethylsilyl group |
| THF/$H_2O$ | A mixture of tetrahydrofuran and water | TMSOTf | Trimethylsilyl trifluoromethanesulfonate |
| Boc | *tert*-butoxycarbonyl | TMPMgCl-LiCl | 2,2,6,6-Tetramethylpiperidinylmagnesium chloride lithium chloride complex |
| $(Boc)_2O$ | Di-tert-butyl dicarbonate | $K_3PO_4$ | Tripotassium Orthophosphate |
| CuI | cuprous iodide | TBAF | Tetrabutylammonium fluoride |

(continued)

| | | | |
|---|---|---|---|
| Xphos Pd G3 | Methanesulfonato(2-dicyclohexylphosphino-2',4',6'-tri-i-propyl-1,1'-biphenyl)(2'-amino-1,1'-biphenyl-2-yl)palladium(II) | HRMS | High-resolution mass spectrometry |
| DAST | diethylaminosulphur trifluoride | LiHMDS | Lithium bis(trimethylsilyl)amide |
| $LiAlH_4$ | Lithium aluminum hydride | PMB | 4-methoxy-benzyl |
| PMBCl | 4-Methoxybenzyl chloride | NIS | *N*-iodosuccinimide |
| $Pd(dppf)Cl_2 \cdot CH_2Cl_2$ | | $B_2Pin_2$ | Bis(pinacolato)diborane |
| $Pd(dppf)Cl_2$ | 1,1'-bis(diphenylphosphino)ferrocene-palladium(II)dichloride dichloromethane complex | DIBAL-H | diisobutyl aluminium hydride |
| HMPT | Tris(dimethylamino)phosphine | cataCXium A Pd G3 | Mesylate[(di(l-adamantyl)-n-butylphosphine)-2-(2'-amino-1,1'-biphenyl)]palladium(II) |

Scheme 1

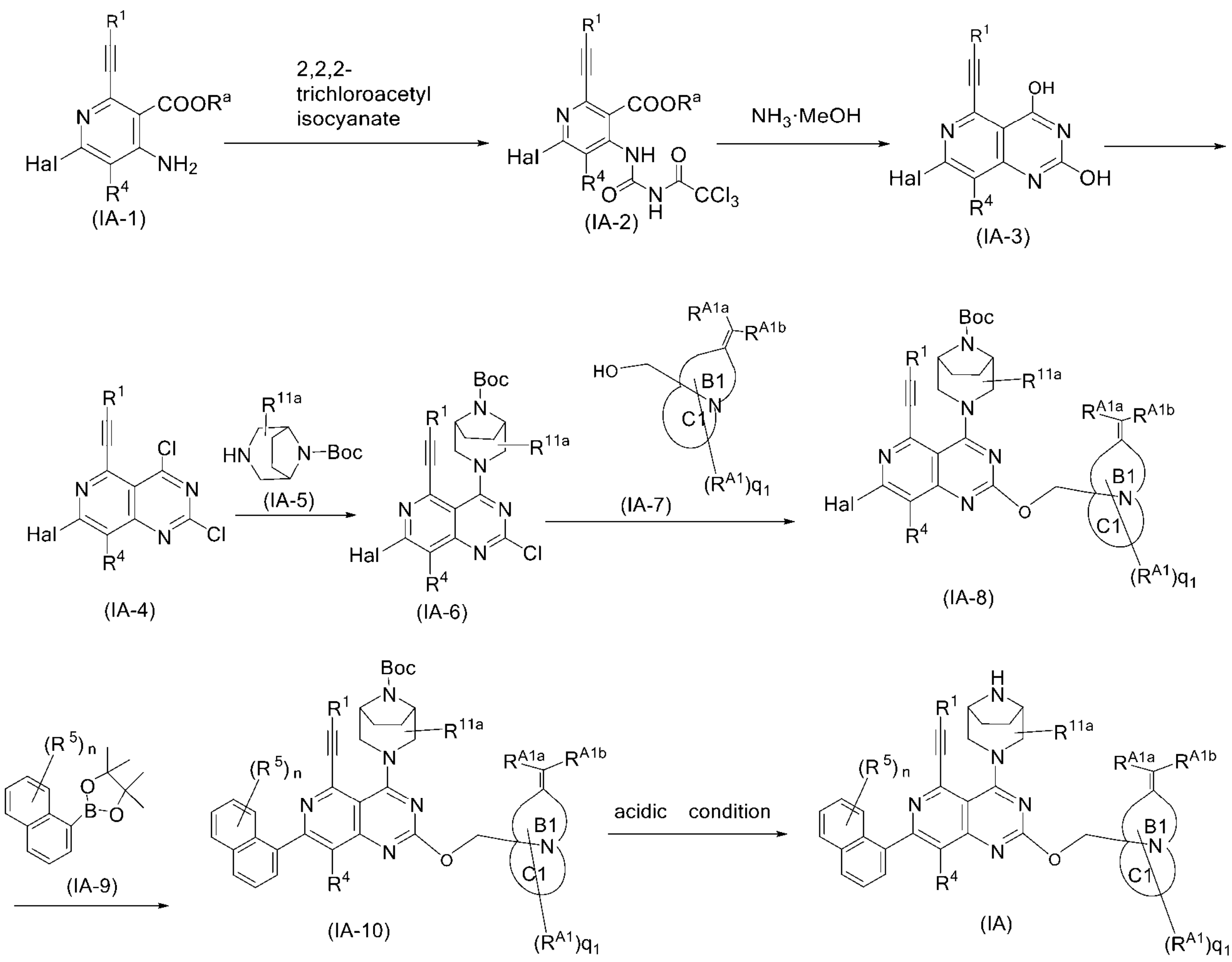


Compound (IA) can be synthesized with reference to the method described in Scheme 1. wherein, ring B1, ring C1, $R^1$, $R^4$, $R^5$, $R^{11a}$, $R^{A1a}$, $R^{A1b}$, $R^{A1}$, $q_1$ and n are each as defined herein; Hal is halogen, preferably Cl or Br; $R^a$ is a $C_{1-4}$ alkyl, preferably methyl or ethyl. Compound (IA-1) reacts with 2,2,2-trichloroacetyl isocyanate in a suitable solvent (such as tetrahydrofuran) to obtain compound (IA-2); compound (IA-2) reacts in a solution of ammonia in methanol to obtain compound (IA-3); compound (IA-3) reacts with $POCl_3$ under suitable conditions (such as heating, in the presence of DIPEA) to obtain compound (IA-4); compound (IA-4) reacts with compound (IA-5) under suitable conditions (such as in the presence of DIPEA) to obtain compound (IA-6); compound (IA-6) reacts with compound (IA-7) under suitable conditions

(such as low temperature, in the presence of NaH) to obtain compound (IA-8); compound (IA-8) reacts with compound (IA-9) in the presence of a suitable catalyst (such as XPhos Pd G3) to obtain compound (IA-10); compound (IA-10) reacts under acidic conditions (such as TMSOTf) to obtain compound (IA).

Scheme 2

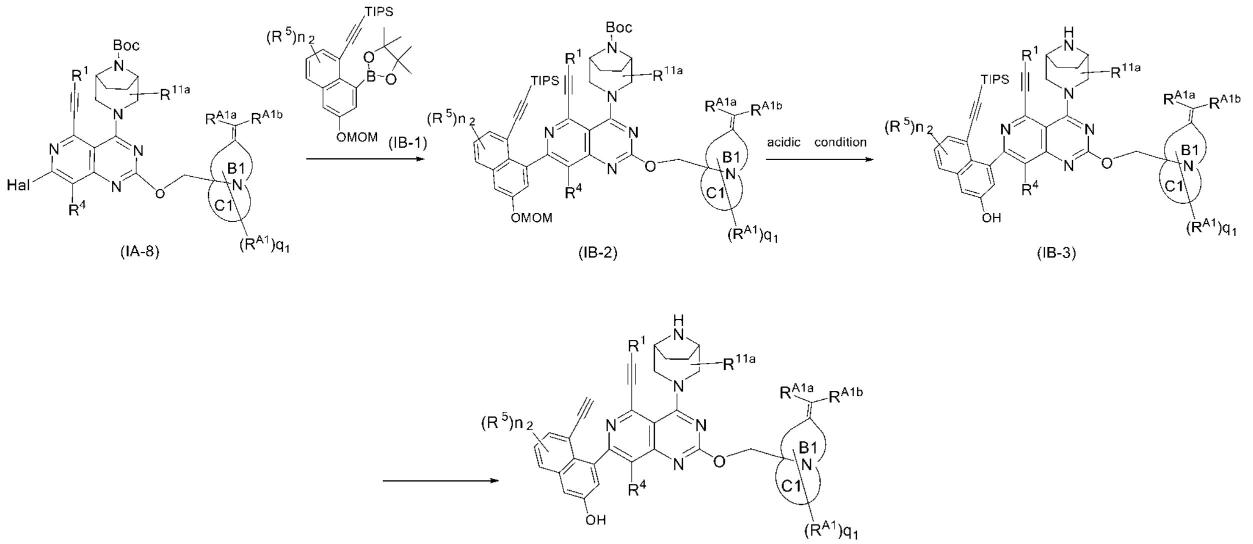


(IA-8) (IB-2) (IB-3)

(IB)

Compound (IB) can be synthesized with reference to the method described in Scheme 2. wherein, ring C1, $R^1$, $R^4$, $R^5$, $R^{11a}$, $R^{A1a}$, $R^{A1b}$, $R^{A1}$ and $q_1$ are each as defined herein; $n_2$ is an natural number ranging from 1 to 5, preferably 1 or 2; Hal is halogen, preferably Cl or Br. Compound (IA-8) reacts with compound (IB-1) in the presence of a suitable catalyst (e.g., XPhos Pd G3) to obtain compound (IB-2); compound (IB-2) reacts under acidic conditions (e.g., TMSOTf) to obtain compound (IB-3); compound (IB-3) removes the TIPS group under suitable conditions (e.g., under the presence of CsF in DMF solvent) to obtain compound (IB).

Scheme 3

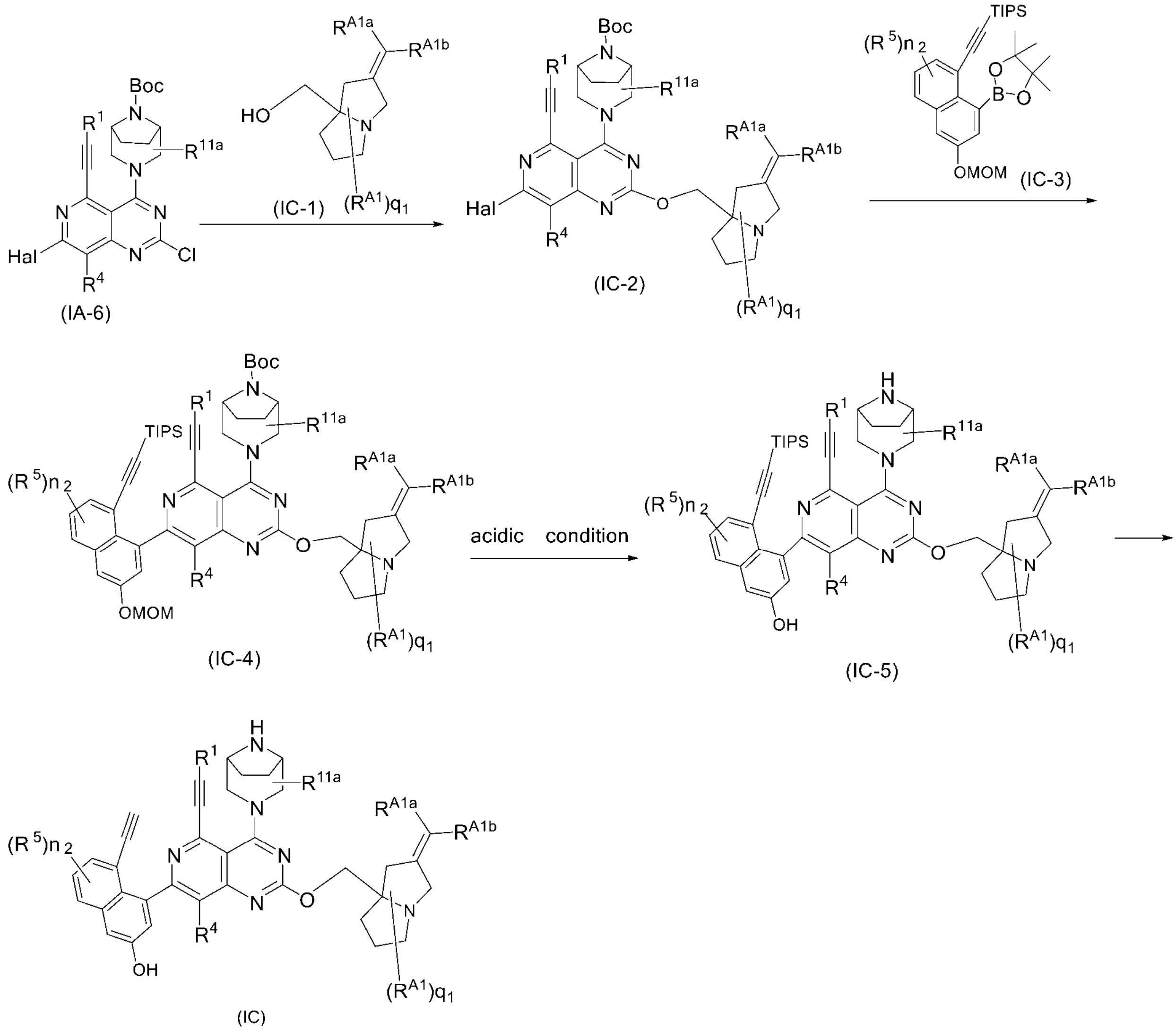


Compound (IC) can be synthesized with reference to the method described in Scheme 3. wherein $R^1$, $R^4$, $R^5$, $R^{11a}$, $R^{A1a}$, $R^{A1b}$, $R^{A1}$ and $q_1$ are each as defined herein; $n_2$ is an integer from 1 to 5, preferably 1 or 2; Hal is a halogen, preferably Cl or Br. Compound (IA-6) reacts with compound (IC-1) under suitable conditions (e.g., low temperature, under the action of NaH) to obtain compound (IC-2); compound (IC-2) reacts with compound (IC-3) in the presence of a suitable catalyst (e.g., XPhos Pd G3) to obtain compound (IC-4); compound (IC-4) reacts under acidic conditions (e.g., TMSOTf) to obtain compound (IC-5); compound (IC-5) removes the TIPS group under suitable conditions (e.g., under the presence of CsF in DMF solvent) to obtain compound (IC).

Scheme 4

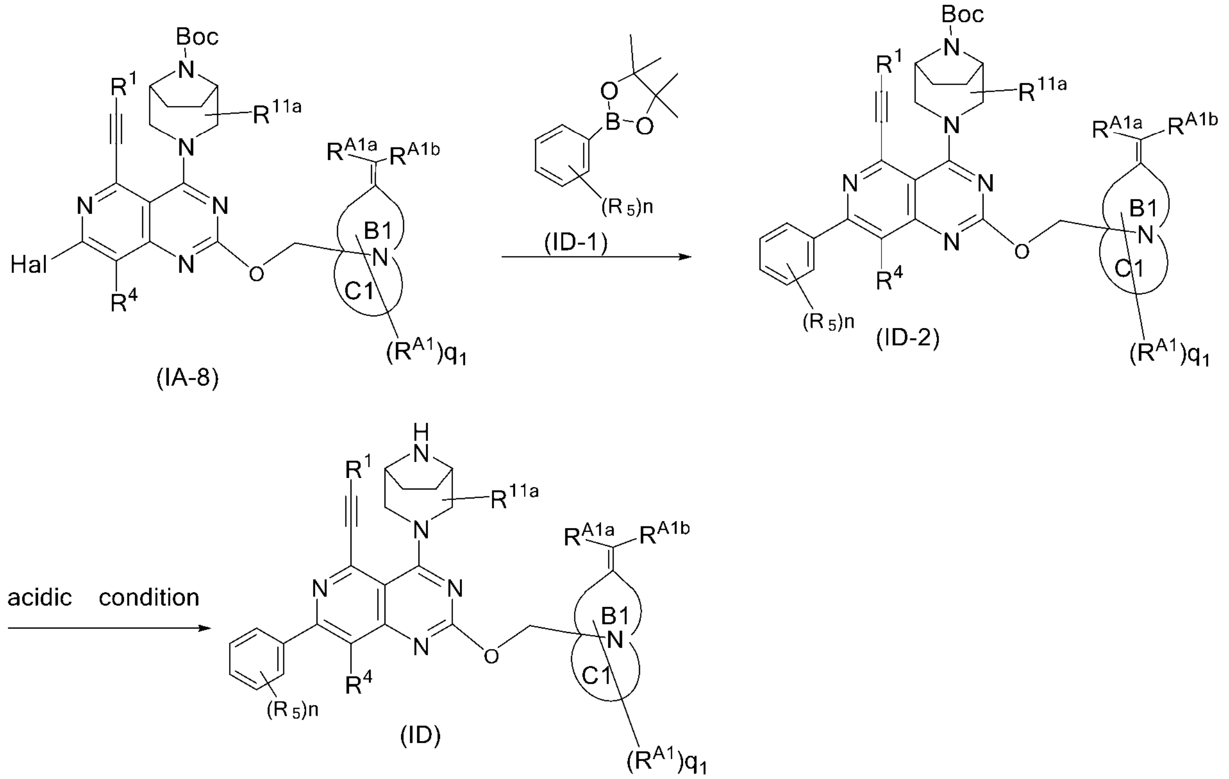

(IA-8) (ID-2) (ID)

Compound (ID) can be synthesized with reference to the method described in Scheme 4. wherein ring B1, ring C1, $R^1$, $R^4$, $R^5$, $R^{11a}$, $R^{A1a}$, $R^{A1b}$, $R^{A1}$, $q_1$ and n are each as defined herein; Hal is a halogen, preferably Cl or Br. Compound (IA-8) reacts with compound (ID-1) in the presence of a suitable catalyst (e.g., XPhos Pd G3) to obtain compound (ID-2); compound (ID-2) reacts under acidic conditions (e.g., TMSOTf) to obtain compound (ID).

The compounds, pharmaceutical compositions, and uses provided by the present invention will be further illustrated below with reference to the examples.

## Example

### Synthesis of Intermediate Compound M1

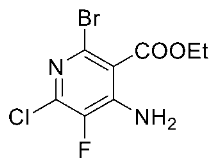

**M1**

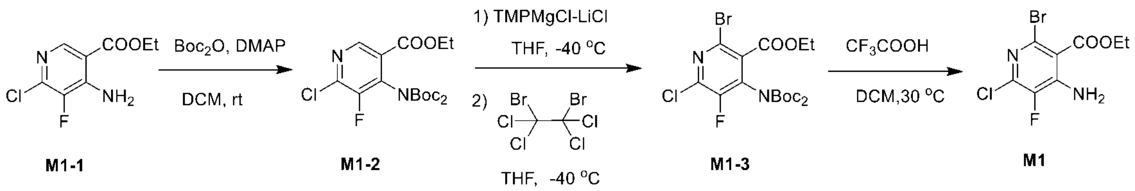

**M1-1** **M1-2** **M1-3** **M1**

Step 1: Synthesis of Compound **M1-2**

Into a 1000 mL single-necked flask were added compound **M1-1** (10 g, 45.74 mmol), DMAP (1.12 g, 9.15 mmol)

and DCM (200 mL). The mixture was stirred at room temperature, and di-tert-butyl dicarbonate (21.96 g, 100.63 mmol) was added dropwise. After complete addition, the mixture was stirred at room temperature for 18 h. The reaction was stopped, imidazole (3.11 g, 45.74 mmol) was added into the mixture. After stirring for 0.5 h, the mixture was washed with saturated ammonium chloride solution (100 mL × 3). The mixture were separated, and the organic phase was further washed with saturated sodium chloride solution (100 mL × 2). The combined organic phases were dried over anhydrous sodium sulfate and concentrated to obtain 17.4 g of yellow solid **M1-2** with a yield of 90.8%, which was used directly in the subsequent step. $^1$H NMR (599 MHz, $CDCl_3$): δ 8.79 (s, 1H), 4.37 (q, *J* = 7.1 Hz, 2H), 1.41 (s, 18H), 1.38 (t, *J* = 7.1 Hz, 3H).

Step 2: Synthesis of Compound **M1-3**

Into a 500 mL single-necked flask were added compound **M1-2** (10 g, 23.88 mmol), and under nitrogen protection, anhydrous THF (100 mL) was added, the mixture was stirred and cooled to -40 °C, and a TMPMgCl·LiCl solution in tetrahydrofuran (35.82 mL, 35.82 mmol, 1 M) was added dropwise at -40 °C. After stirring at -40 °C for 4 h, a solution of 1,2-dibromotetrachloroethane (9.33 g, 28.66 mmol) in THF (30 mL) was added dropwise. Stirring continued at -40 °C for an additional 4 h. The reaction was stopped, the mixture was quenched with saturated ammonium chloride solution (100 mL), and extracted with ethyl acetate (EA, 100 mL × 2). The combined organic phases were dried over anhydrous sodium sulfate and concentrated. The residue was purified by column chromatography using PE/DCM (v/v = 100/1-1/1) as eluent to obtain 8.5 g of a yellow solid **M1-3** with a yield of 71.5%. $^1$H NMR (599 MHz, $CDCl_3$): δ 4.40 (q, *J* = 7.2 Hz, 2H), 1.42 (s, 18H), 1.37 (t, *J*= 7.2 Hz, 3H).

Step 3: Synthesis of Compound **M1**

To a 250 mL single-necked flask were added compound **M1-3** (5 g, 10.05 mmol), DCM (50 mL) and TFA (14.97 mL, 200.1 mmol). The mixture was stirred at 30 °C for 5 h. The reaction was stopped, and saturated sodium carbonate was added to adjust the pH to neutral. The mixture was then extracted with DCM (30 mL × 3). The combined organic phases were dried over anhydrous sodium sulfate and concentrated to obtain 2.9 g of a pale brown solid **M1** with a yield of 97%. $^1$H NMR (599 MHz, $CDCl_3$): δ 6.07 (s, 2H), 4.41 (q, *J* = 7.1 Hz, 2H), 1.41 *(t, J*= 7.1 Hz, 3H); LC-MS (ESI, pos. ion) m/z: 297.1 $[M+H]^+$.

**Synthesis of Intermediate Compound M2**

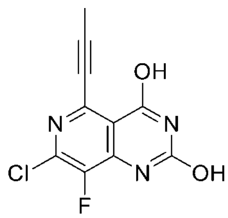

**M2**

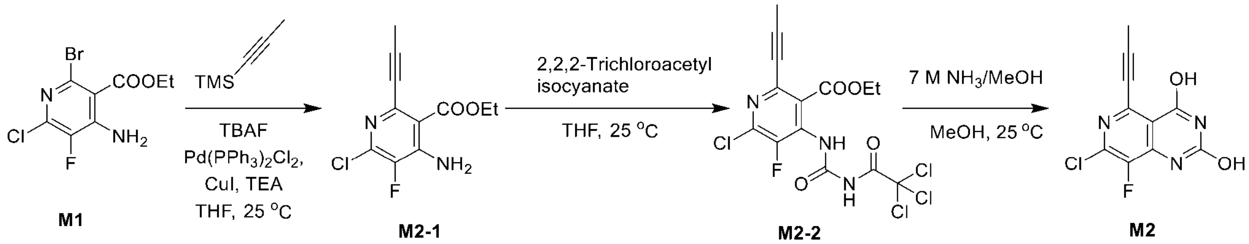


M1 M2-1 M2-2 M2

Step 1: Synthesis of Compound **M2-1**

Into a 50 mL two-necked flask were added compound **M1** (1.0 g, 3.36 mmol), $Pd(PPh_3)_2Cl_2$ (0.472 g, 0.67 mmol) and CuI (0.128 g, 0.67 mmol). The mixture was purged with nitrogen three times. Then, 1-(trimethylsilyl)propyne (0.566 g, 5.04 mmol), TEA (2.33 mL, 16.8 mmol) and anhydrous THF (10 mL) were added. The mixture was purged with nitrogen three times again. TBAF solution in tetrahydrofuran (5.04 mL, 5.04 mmol, 1.0 M) was added dropwise under stirring. After

completion of the addition, the mixture was stirred at room temperature (25 °C) for 6 h. The reaction was stopped and filtered through a celite pad. The filtrate was concentrated and purified by column chromatography using PE/DCM (v/v = 100/1-2/1) as eluent to obtain 0.523 g of a yellow solid **M2-1** with a yield of 60.6%. LC-MS (ESI, pos. ion) m/z: 257.3 $[M+H]^+$.

Step 2: Synthesis of Compound **M2-2**

Into a 50 mL single-necked flask were added compound **M2-1** (0.52 g, 2.03 mmol) and anhydrous THF (6 mL). The mixture was stirred to dissolve compound **M2-1** under a nitrogen atmosphere. Then, 2,2,2-trichloroacetyl isocyanate (0.497 g, 2.64 mmol) was added. The resulting mixture was stirred at room temperature (25 °C) for 1 h. The reaction was stopped and concentrated. The residue was used directly in the next step. The yield was calculated as 100%. LC-MS (ESI, pos. ion) m/z: 445.9 $[M+H]^+$.

Step 3: Synthesis of Compound **M2**

Into the compound **M2-2** (0.885 g, 1.99 mmol) obtained in the previous step was added methanol (6 mL), and the mixture was stirred to dissolve compound **M2-2.** Then, a solution of ammonia in methanol (2.84 mL, 19.99 mmol, 7 M) was added. The resulting mixture was stirred at room temperature 25 °C for 2 h. The reaction was stopped and concentrated. The crude material was triturated with MTBE (10 mL) for 0.5 h and filtered to give 0.48 g of a white solid **M2.** The combined yield for step 2 and step 3 was 95%. LC-MS (ESI, pos. ion) m/z: 254.1 $[M+H]^+$.

**Synthesis of Intermediate Compound M3**

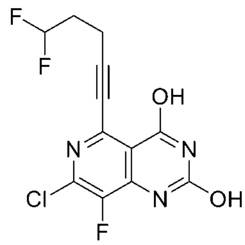

**M3**

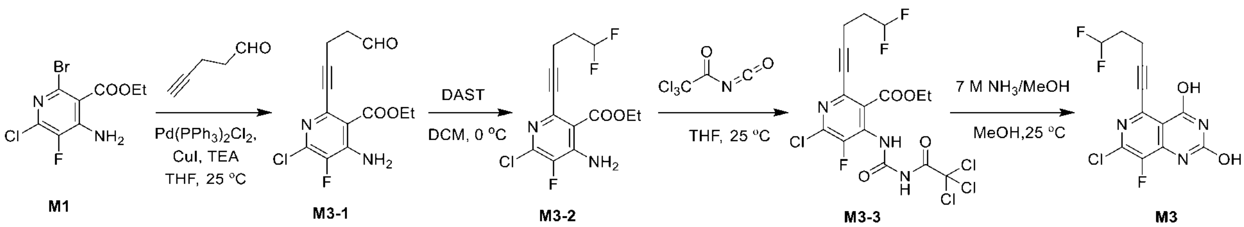


Step 1: Synthesis of Compound **M3-1**

Into a 500 mL single-necked flask were added compound **M1** (10 g, 33.61 mmol), 4-formyl-1-butyne (3.78 g, 43.69 mmol), $Pd(PPh_3)_2Cl_2$ (2.36 g, 3.36 mmol), CuI(0.64 g, 3.36 mmol), TEA (10.20 g, 100.83 mmol) and anhydrous THF (200 mL). The mixture was purged with nitrogen three times. The mixture was stirred at room temperature 25 °C for 4 h. The reaction was stopped and filtered through a celite pad. The filtrate was dried over anhydrous sodium sulfate and concentrated. The residue was purified by column chromatography using PE/EA (v/v = 5/1-3/1) as eluent to obtain 4.74 g of a yellow solid **M3-1** with a yield of 47.2%. LC-MS (ESI, pos. ion) m/z: 299.30 $[M+H]^+$; $^1H$ NMR (400 MHz, $CDCl_3$) $\delta$ 9.83 (d, *J*= 1.0 Hz, 1H), 6.26 (s, 2H), 4.41 (q, *J* = 7.1 Hz, 2H), 2.84 - 2.73 (m, 4H), 1.42 (t, *J* = 7.1 Hz, 3H).

Step 2: Synthesis of Compound **M3-2**

Into a 250 mL single-necked flask were added compound **M3-1** (4.74 g, 15.87 mmol) and dichloromethane (90 mL). Diethylaminosulfur trifluoride (DAST, 12.79 g, 79.35 mmol) was added dropwise at 0 °C. The mixture was stirred at 0 °C for 2 h and quenched by addition of saturated sodium bicarbonate (100 mL). The layers were separated, and the

aqueous phase was extracted with dichloromethane (100 mL). The combined organic phases were dried over anhydrous sodium sulfate, concentrated, and purified by column chromatography using PE/EA (v/v = 5/1) as eluent to obtain 2.12 g of a yellow solid **M3-2** with a yield of 41.7%. LC-MS (ESI, pos. ion) m/z: 321.1 $[M+H]^+$; $^1H$ NMR (400 MHz, $CDCl_3$) δ 6.27 (s, 2H), 6.00 (tt, *J* = 56.4, 4.5 Hz, 1H), 4.41 (q, *J* = 7.1 Hz, 2H), 2.63 (t, *J* = 7.3 Hz, 2H), 2.16 (ttd, *J* = 16.7, 7.3, 4.4 Hz, 2H), 1.41 (t, *J* = 7.1 Hz, 3H).

Step 3: Synthesis of Compound **M3-3**

Into a 50 mL single-necked flask were added compound **M3-2** (2.1 g, 6.55 mmol) and anhydrous THF (40 mL). The mixture was stirred to dissolve compound **M3-2.** Then, 2,2,2-trichloroacetyl isocyanate (1.95 g, 9.82 mmol) was slowly added. The resulting mixture was stirred at room temperature (25 °C) for 40 min. The reaction was stopped and concentrated to obtain a yellow solid. The residue was used directly in the next step. The yield was calculated as 100%. LC-MS (ESI, pos. ion) m/z: 508.0 $[M+H]^+$.

Step 4: Synthesis of Compound **M3**

Into the compound **M3-3** obtained in the previous step was added methanol (45 mL), and the mixture was stirred to dissolve compound **M3-3.** Then, a solution of ammonia in methanol (4.67 mL, 32.7 mmol, 7 M) was added. The resulting mixture was stirred at room temperature 25 °C for 2.5 h. The reaction was stopped and concentrated. The crude material was triturated with MTBE (15 mL) for 0.5 h, filtered, and dried to obtain 1.98 g of a yellow solid **M3.** The combined yield for step 3 and step 4 was 95.2%. LC-MS (ESI, pos. ion) m/z: 318.1 $[M+H]^+$.

**Synthesis of Intermediate Compound M4**

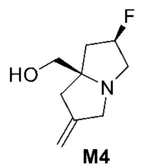

**M4**

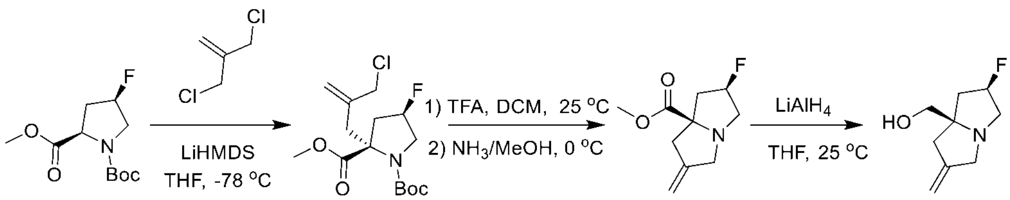


**M4-1** **M4-2** **M4-3** **M4**

Step 1: Synthesis of Compound **M4-2**

Into a 500 mL single-necked flask were added **M4-1** (10 g, 40.44 mmol) and anhydrous THF (100 mL), The mixture was purged with nitrogen three times, the temperature was lowered to -78 °C, and LiHMDS (80.88 mL, 80.88 mmol, 1 M THF solution) was added dropwise. The mixture was stirred at -78 °C for 15 min. 3-Chloro-2-chloromethylpropene (7.58 g, 60.66 mmol) was added at -78 °C. The reaction solution was transferred to room temperature (25 °C) and stirred for 15 h. The temperature was lowered to 0 °C, the pH was adjusted to 7 with 1 M hydrochloric acid, water (200 mL) was added, and the mixture was extracted with EA (100 mL). The layers were separated, and EA (100 mL) and saturated aqueous sodium chloride solution (100 mL) were added to the aqueous phase. The layers were separated, the organic phases were combined, and the organic phases were washed with saturated aqueous sodium chloride solution (150 mL). The organic phase was dried over anhydrous sodium sulfate and purified by column chromatography using PE/EA (v/v = 9/1) as eluent to obtain 7.5 g of yellow liquid **M4-2** with a yield of 55.2%.

Step 2: Synthesis of Compound **M4-3**

Into a 250 mL single-necked flask were added **M4-2** (7.5 g, 22.33 mmol) and dichloromethane (70 mL). The

temperature was cooled to 0 °C, and trifluoroacetic acid (30.7 g, 269.2 mmol) was added dropwise. The mixture was transferred to room temperature (25 °C) and stirred for 4 h. The reaction was stopped and concentrated under reduced pressure at 45 °C. Methanol (30 mL) was added at 0 °C. The pH was adjusted to 8 with a soution of ammonia in methanol (7 M) at 0 °C. The mxiture was evaporated to dryness and purified by column chromatography using PE/EA (v/v = 1/1) as eluent to obtain 4.1 g of a yellow liquid **M4-3** with a yield of 92.1% . LC-MS (ESI, pos. ion) m/z: 200.2 $[M+H]^+$.

Step 3: Synthesis of Compound **M4**

Into a 250 mL single-necked flask were added $LiAlH_4$ (1.56 g, 41.16 mmol) and anhydrous THF (40 mL) at room temperature. The mixture was purged with nitrogen three times. A solution of **M4-3** (4.1 g, 20.58 mmol) in THF (40 mL) was added dropwise at -10 °C. After the addition was complete, the mixture was stirred for 15 minutes. The mixture was then transferred to room temperature (25 °C) for 2 h. After the reaction of the raw materials was complete, the mixture was transferred to 0 °C, sodium sulfate decahydrate (3.32 g, 10.29 mmol) was added to the mixture, and the resulting mixture was returned to room temperature with stirring for 1 h to quench the reaction. After quenching, the reaction mixture was filtered through a celite pad, and the filtrate was dried to obtain 2.15 g of a yellow oily product **M4** with a yield of 61%. LC-MS (ESI, pos. ion) m/z: 172.2 $[M+H]^+$.

**Synthesis of Intermediate Compound M5**

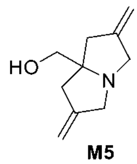

**M5**

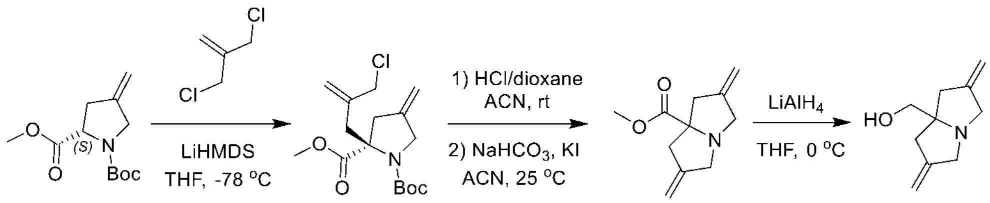


**M5-1** **M5-2** **M5-3** **M5**

Step 1: Synthesis of Compound **M5-2**

Into a 500 mL single-necked flask were added **M5-1** (5 g, 20.72 mmol) and anhydrous THF (30 mL). The mixture was purged with nitrogen three times and the temperature was lowered to -78 °C. A solution of LiHMDS in tetrahydrofuran (41.44 mL, 41.44 mmol) was added dropwise. The mixture was stirred at -78 °C for 60 min. 3-Chloro-2-chloromethylpropene (3.84 mL, 41.44 mmol) was slowly added dropwise at -78 °C. The mixture was stirred at -78 °C for 3 h. Stirring was stopped and the mixture was transferred to room temperature. Saturated ammonium chloride solution (100 mL) was added to quench the mixture. The layers were separated and the aqueous phase was extracted with EA (80 mL × 2). The organic phases were combined, dried over anhydrous sodium sulfate, and purified by column chromatography using PE/EA (v/v = 20/1) as the eluent to obtain 6.25 g of light yellow liquid **M5-2** with a yield of 91.4%.

Step 2: Synthesis of Compound **M5-3**

Into a 500 mL single-necked flask were added **M5-2** (6.25 g, 18.95 mmol) and acetonitrile (50 mL). The temperature was cooled to 0 °C and stirred for 5 minutes. A solution of hydrochloric acid in 1,4-dioxane (47.73 mL, 189.5 mmol, 4 M) was added dropwise. The mixture was transferred to 25 °C and stirred for 1 h. The reaction was stopped. The reaction solution was concentrated and spin-dried to dryness. Acetonitrile (50 mL) was added for dissolution. Sodium bicarbonate (7.77 g, 92.5 mmol) and potassium iodide (0.31 g, 1.85 mmol) were then added. The reaction solution was stirred at room temperature for 1.5 h. Stirring was stopped, the reaction solution was filtered, washed with acetonitrile (50 mL), and the filtrate was concentrated. The product was purified by column chromatography using PE/EA (v/v = 4/1-3/2) as the eluent to obtain 3.05 g of yellow liquid **M5-3** with a yield of 85.31%. LC-MS (ESI, pos. ion) m/z: 194.2 $[M+H]^+$.

Step 3: Synthesis of Compound **M5**

Into a 250 mL single-necked flask was added $LiAlH_4$ (1.2 g, 31.56 mmol) at room temperature. The mixture was purged with nitrogen three times. Anhydrous THF (31 mL) was added at -10 °C and stirred for 5 minutes. A solution of **M5-3** (3.05 g, 15.78 mmol) in THF (20 mL) was slowly added dropwise. After the addition was complete, the temperature was turned to 0 °C and the reaction was allowed to proceed for 1 h. After the reaction of the raw materials was complete, sodium sulfate decahydrate (2.54 g, 7.89 mmol) was slowly added to quench the reaction. After quenching, the reaction mixture was filtered through celite pad, and the filtrate was dried to obtain 1.59 g of a yellow oily product **M5** with a yield of 60.8%. LC-MS (ESI, pos. ion) m/z: 166.2 $[M+H]^+$.

**Synthesis of Intermediate Compound M8**

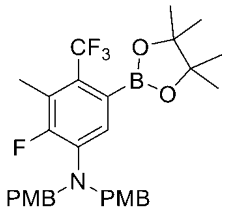

**M8**

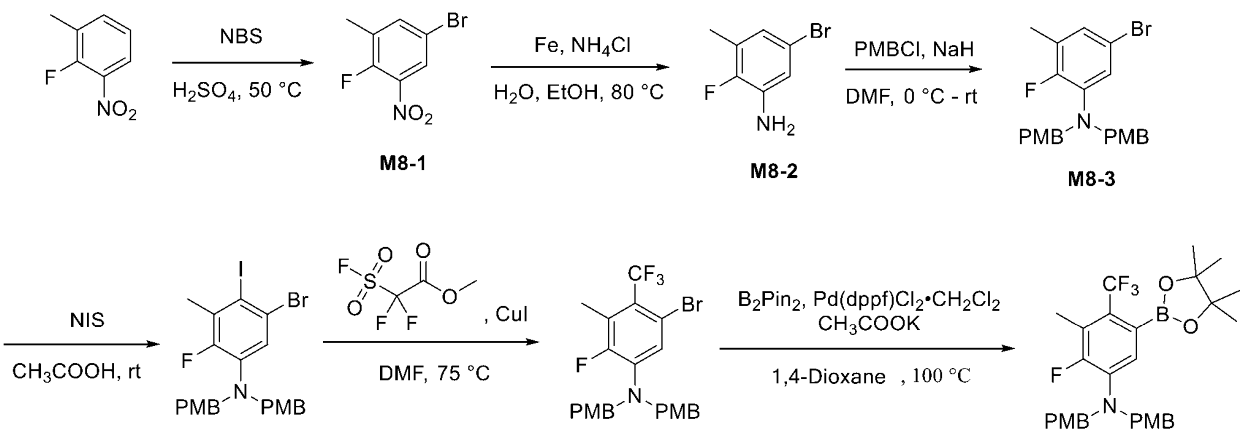


M8-1 M8-2 M8-3 M8-4 M8-5 M8

Step 1: Synthesis of Compound **M8-1**

Into a 250 mL single-necked flask were added 2-fluoro-3-nitrotoluene (10 g, 64.46 mmol), concentrated sulfuric acid (60 mL) and NBS (13.19 g, 74.13 mmol), and the mixture was stirred at 50 °C for 2 h. The reaction was stopped and the reaction solution was poured into ice water and extracted with EA (100 mL). The organic phase was dried over anhydrous sodium sulfate and spin-dried. It was then purified by column chromatography using PE/EA (v/v = 10/1) as the eluent to obtain 14 g of an orange-red oil compound **M8-1** with a yield of 90.15%.

Step 2: Synthesis of Compound **M8-2**

Into a 500 mL single-necked flask were added **M8-1** (16 g, 68.37 mmol), ethanol (100 mL), iron powder (19.09 g, 341.85 mmol) and ammonium chloride (56.98 mL, 341.85 mmol, 6 M aqueous solution). The mixture was heated to 80 °C and stirred for 2 h. The reaction solution was filtered and the filtrate was concentrated. The residue was diluted with ethyl acetate (500 mL) and washed with water (50 mL). The organic phase was separated and dried over anhydrous sodium sulfate. The product was purified by column chromatography using PE/EA (v/v = 20/1-5/1) as the eluent to obtain 10 g of a yellow oil compound **M8-2** with a yield of 71.68%. LC-MS (ESI, pos. ion) m/z: 204.0 $[M+H]^+$.

Step 3: Synthesis of Compound **M8-3**

Into a 250 mL three-necked flask were added **M8-2** (10 g, 49.01 mmol) and anhydrous DMF (60 mL). The mixture was cooled to 0°C and stirred. NaH (5.88 g, 147.03 mmol, mass fraction 60%) was added, and the mixture was transferred to room temperature and stirred for 20 min. 4-Methoxybenzyl chloride (15.35 g, 98.02 mmol) was added to the reaction solution, and the resulting mixture was stirred at room temperature for 1 h. The reaction was quenched by adding saturated ammonium chloride solution (40 mL), the mixturew was diluted with water (40 mL), and extracted with ethyl acetate (100 mL). The organic phase was concentrated, dried over anhydrous sodium sulfate, and purified by column chromatography using PE/EA (v/v = 20/1) as the eluent to obtain 18.4 g of a white solid compound **M8-3** with a yield of 84.5%. LC-MS (ESI, pos. ion) m/z: 444.1 $[M+H]^+$.

Step 4: Synthesis of Compound **M8-4**

Into a 50 mL two-necked flask were added **M8-3** (7 g, 15.75 mmol) and acetic acid (20 mL) under nitrogen protection and stirred for 2 minutes. Subsequently, NIS (5.32 g, 23.63 mmol) was added and stirred at room temperature for 15 h. The reaction was quenched by adding saturated sodium thiosulfate solution (10 mL). The product was extracted with EA (20 mL), dried over anhydrous sodium sulfate, and purified by column chromatography using PE/EA (v/v = 10/1) as the eluent to obtain 8.7 g of a colorless oil **M8-4** with a yield of 96.8%. LC-MS (ESI, pos. ion) m/z:570.0 $[M+H]^+$.

Step 5: Synthesis of Compound **M8-5**

Into a 50 mL single-necked flask were added **M8-4** (8.7 g, 15.26 mmol), methyl fluorosulfonyldifluoroacetate (27.85 g, 144.97 mmol), cuprous iodide (29.06 g, 152.6 mmol) and DMF (90 mL). The atmosphere was replaced with nitrogen three times and the mixture was heated to 75 °C and stirred for 15 h. The reaction was stopped and filtered after returning to room temperature. EA (60 mL) was added to the mixture and the organic phase was washed with saturated brine (20 mL × 3). The organic phase was dried over anhydrous sodium sulfate, spin-dried, and purified by column chromatography using PE/EA (v/v = 10/1) as the eluent to obtain 2 g of the orange oily compound **M8-5** with a yield of 25.6%.

Step 6: Synthesis of Compound **M8**

Into a 50 mL single-necked flask were added **M8-5** (2 g, 1.95 mmol), bis(pinacolato)diborane (1.49 g, 5.85 mmol), $Pd(dppf)Cl_2 \cdot CH_2Cl_2$ (0.16 g, 0.20 mmol), potassium acetate (0.58 g, 5.95 mmol) and 1,4-dioxane (20 mL). After the mixture was purged with nitrogen three times, the mixture was heated to 100 °C and stirred for 3 h. The silica gel sample was then spin-dried and purified by column chromatography using PE/EA (v/v = 10/1) as the eluent to obtain 0.93 g of a white solid compound **M8** with a yield of 85.2%. LC-MS (ESI, pos. ion) m/z:560.3 $[M+H]^+$.

**Synthesis of Intermediate Compound M9**

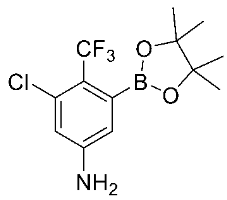

**M9**

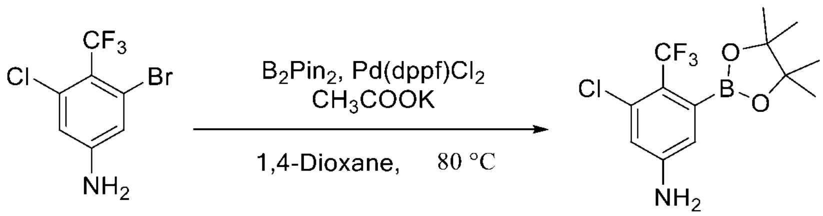


**M9**

Step 1: Synthesis of Compound **M9**

Into a 100 mL single-necked flask were added 3-bromo-5-chloro-4-(trifluoromethyl)aniline (5 g, 18.22 mmol, purchased from Beijing LeYan Technology), bis(pinacolato)diborane (5.55 g, 21.86 mmol), Pd(dppf)$Cl_2$· (1.19 g, 1.82 mmol), potassium acetate (5.36 g, 54.66 mmol) and 1,4-dioxane (200 mL). After the mixture was purged with nitrogen three times, the mixture was heated to 80 °C and stirred for 18 h to terminate the reaction. The reaction solution was filtered through celite pad and concentrated. The crude product was purified by column chromatography using PE/EA (v/v = 4/1-1/1) as the eluent to obtain 2.535 g of a white solid compound **M9** with a yield of 43.3%. LC-MS (ESI, pos. ion) m/z:322.0 $[M+H]^+$; $^1H$ NMR (400 MHz, $CDCl_3$) δ 6.73 (d, *J* = 1.9 Hz, 1H), 6.55 (d, *J* = 1.9 Hz, 1H), 3.97 (s, 2H), 1.35 (s, 12H). $^{19}F$ NMR (376 MHz, $CD_3Cl_3$) δ -56.70 (3F).

**Example 1: Synthesis of Compound 1**

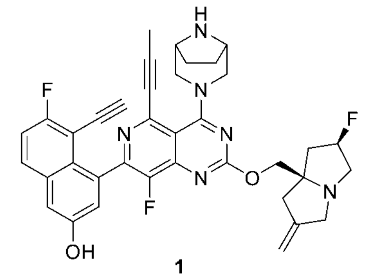

**1**

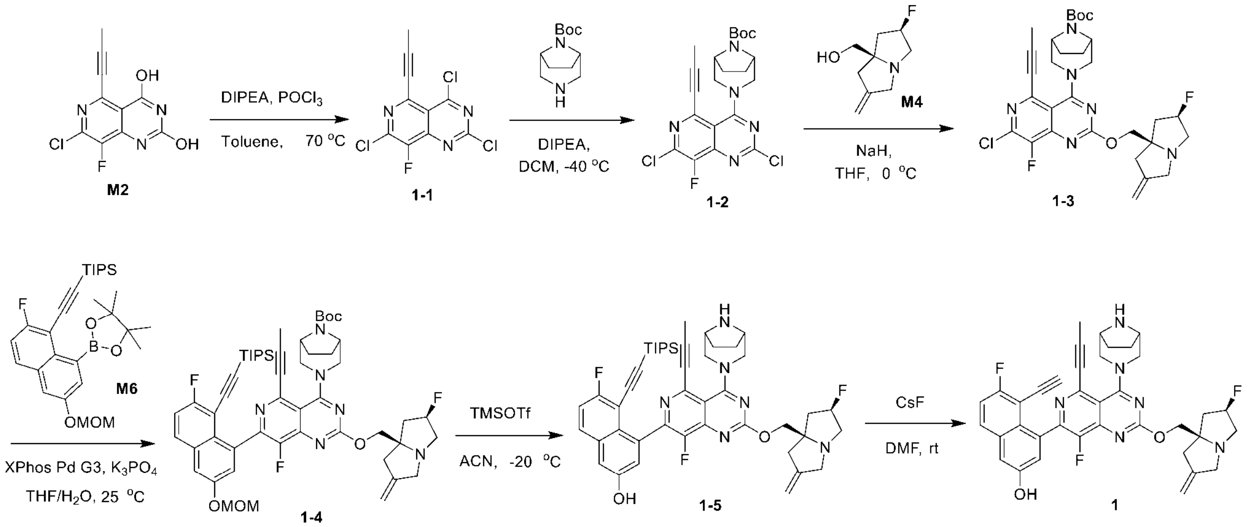


Step 1: Synthesis of Compound **1-1**

Into a 100 mL single-necked flask were added compound **M2** (2.00 g, 7.89 mmol) and anhydrous toluene (20 mL). Then, phosphoryl chloride (2.16 mL, 23.67 mmol) and DIPEA (6.52 mL, 39.45 mmol) were added. The mixture was heated to 70 °C and stirred for 1 h. The reaction was stopped and the mixture was cooled. The mixture was concentrated and used

directly in the next step. The yield was calculated as 100%.

Step 2: Synthesis of Compound **1- 2**

Into the compound **1-1** obtained in the previous step was added anhydrous dichloromethane (20 mL). The mixture was purged with nitrogen three times and stirred to dissolve. The mixture was cooled to -40 °C and stirred for 5 minutes. DIPEA (6.51 mL, 39.4 mmol) and 8-BOC-3,8-diazabicyclo[3.2.1]octane (1.67 g, 7.88 mmol) were added sequentially. After stirring at -40 °C for 0.5 h, the reaction was stopped and quenched with saturated ammonium chloride solution (50 mL). The mixture was warmed to room temperature and extracted with DCM (50 mL × 2). The organic phase was separated, dried over anhydrous sodium sulfate, and concentrated. The residue was purified by column chromatography using PE/EA (v/v = 9/1) as eluent to obtain 787 mg of a yellow solid compound **1-2.** The combined yield for step 1 and step 2 was 21.4%. LC-MS (ESI, pos. ion) m/z: 466.2 $[M+H]^+$.

Step 3: Synthesis of Compound **1- 3**

Into a 100 mL two-necked flask was added sodium hydride (0.13 g, 3.28 mmol, 60%). The mixture was purged with nitrogen three times, and anhydrous THF (8 mL) was added. The mixture was stirred at 0 °C for 5 minutes. A solution of **M4** (0.28 g, 1.64 mmol) in THF (2 mL) was slowly added dropwise. The mixture was stirred at 0 °C for 1 h. A solution of compound **1-2** (383 mg, 0.82 mmol) in THF (5 mL) was slowly added dropwise. The mixture was stirred at 0 °C for 2.5 h. The reaction was stopped. The reaction mixture was diluted with EA (15 mL) and washed with saturated ammonium chloride solution (15 mL), separated, and the aqueous phase was extracted with EA (10 mL × 2). The combined organic phases were dried over anhydrous sodium sulfate and concentrated. The residue was purified by column chromatography using DCM/MeOH (v/v = 50/1) as eluent to obtain 312 mg of a yellow solid compound **1-3** with a yield of 63.2%. LC-MS (ESI, pos. ion) m/z: 601.3 $[M+H]^+$.

Step 4: Synthesis of Compound **1- 4**

Into a 25 mL two-necked flask were added compound **1-3** (200 mg, 0.33 mmol, 1 equiv.), **M6** (0.25 g, 0.49 mmol), XPhos Pd G3 (56 mg, 0.066 mmol), $K_3PO_4 \cdot 7H_2O$ (280 mg, 0.83 mmol) and THF/$H_2O$ (v/v = 4.8 mL/1.6 mL). The mixture was purged with nitrogen three times and stirred at room temperature 25 °C for 13 h. The reaction was monitored by TLC, indicating that half of the starting material **1-3** remained. Additional portions of **M6** (0.25 g, 0.49 mmol, 1.5 equiv.), XPhos Pd G3 (56 mg, 0.066 mmol, 0.2 equiv.) and $K_3PO_4 \cdot 7H_2O$ (280 mg, 0.83 mmol, 2.5 equiv) were added. The mixture was purged with nitrogen three times and stirred for 1.5 h. The reaction remained incomplete, and the addition step was repeated until compound **1-3** was substantially consumed. Saturated ammonium chloride solution (20 mL) was added. The mixture was extracted with EA (20 mL × 2). The combined organic phases were dried over anhydrous sodium sulfate and concentrated. The residue was purified by column chromatography using DCM/MeOH (v/v = 50/1) as eluent to obtain 296 mg of a red solid compound **1-4** with a yield of 93.5%. LC-MS (ESI, pos. ion) m/z: 951.4 $[M+H]^+$.

Step 5: Synthesis of Compound **1- 5**

Into a 50 mL single-necked flask were added compound **1-4** (296 mg, 0.31 mmol) and acetonitrile (7 mL). The mixture was cooled to -20 °C and stirred for 5 minutes. TMSOTf (0.14 mL, 0.78 mmol) was added dropwise. After stirring for 60 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 9/1) as eluent to obtain 82 mg of a yellow solid compound **1-5** with a yield of 32.6%, LC-MS (ESI, pos. ion) m/z: 807.5 $[M+H]^+$.

Step 6: Synthesis of Compound **1**

Into a 25 mL single-necked flask were added **1-5** (82 mg, 0.10 mmol), cesium fluoride (230 mg, 1.5 mmol) and DMF (0.5 mL). The mixture was purged with nitrogen three times and stirred at room temperature for 5 h. The reaction was stopped, and water (10 mL) was added, precipitating a solid. Filtration afforded the crude solid. The crude product was purified by column chromatography using DCM/MeOH (v/v = 8/1) as eluent to obtain 36 mg of a yellow solid compound **1** with a yield of 54.4%. LC-MS (ESI, pos. ion) m/z: 651.5 $[M+H]^+$; HRMS (ESI): 651.2709 $[M+H]^+$; $^1$H NMR (400 MHz, $CD_3OD$) δ 7.86 (dd, *J* = 9.1, 5.7 Hz, 1H), 7.37 - 7.27 (m, 2H), 7.20 (s, 1H), 5.33 (d, *J* = 53.3 Hz, 1H), 5.02 (s, 2H), 4.71 - 4.20 (m, 5H), 4.08 - 3.68 (m, 5H), 3.52 - 3.36 (m, 1H), 3.05 - 2.80 (m, 2H), 2.61 - 2.38 (m, 2H), 2.19 (s, 3H), 2.25 - 2.05 (m, 1H), 1.96 - 1.59 (m, 4H). $^{19}$F NMR (376 MHz, $CD_3OD$) δ -111.52 (1F), -139.92 (1F), -174.11 (1F).

**Example 2: Synthesis of Compound 2**

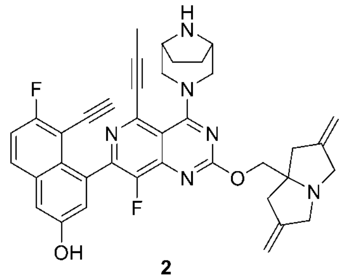

2

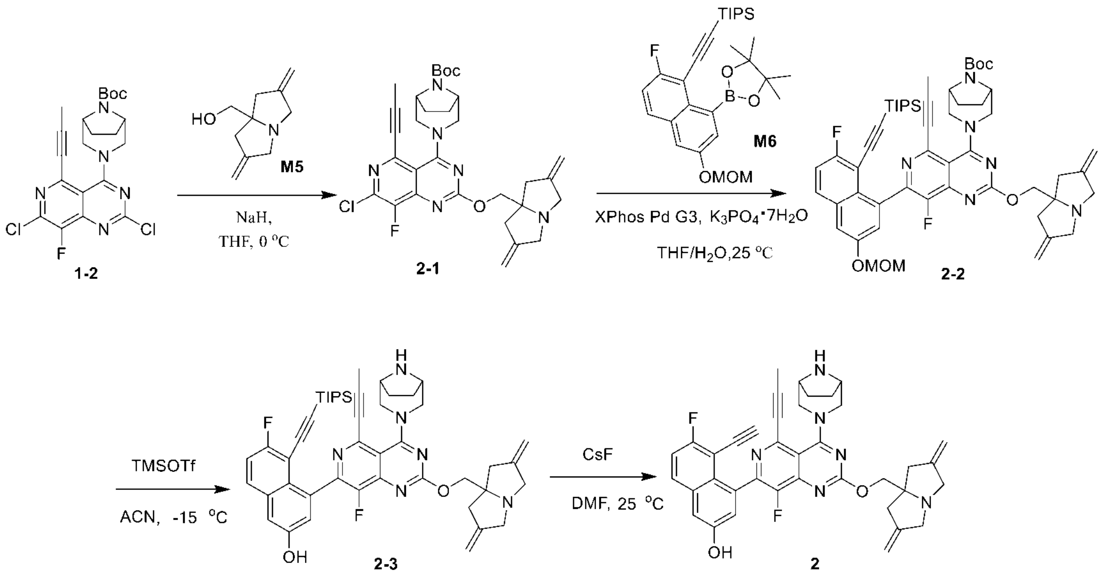


1-2 2-1 2-2 2-3 2

Step 1: Synthesis of Compound **2-1**

Into a 100 mL two-necked flask was added sodium hydride (0.13 g, 3.28 mmol, 60% purity). The mixture was purged with nitrogen, and anhydrous THF (7 mL) was added. The mixture was stirred at 0 °C for 5 minutes. A solution of **M5** (0.24 g, 1.48 mmol) in THF (2 mL) was slowly added dropwise. The mixture was stirred at 0 °C for 1 h. A solution of compound **1-2** (383 mg, 0.82 mmol) in THF (5 mL) was slowly added dropwise. The mixture was stirred at 0 °C for 3 h, then the stirring was stopped, and a saturated ammonium chloride solution (30 mL) was added to the mixture to quench the reaction. The layers were separated, and the aqueous phase was extracted with EA (20 mL × 2). The organic phases were combined, dried over anhydrous sodium sulfate, concentrated, and purified by column chromatography using DCM/MeOH (v/v = 50/1) as the eluent to obtain 271 mg of yellow solid compound **2-1** with a yield of 55.4%. LC-MS (ESI, pos. ion) m/z: 595.5 $[M+H]^+$.

Step 2: Synthesis of Compound **2-2**

Into a 25 mL two-necked flask were added compound **2-1** (200 mg, 0.34 mmol, 1.0 eq), **M6** (0.26 g, 0.51 mmol), XPhos Pd G3 (58 mg, 0.068 mmol), $K_3PO_4 \cdot 7H_2O$ (290 mg, 0.85 mmol) and $THF/H_2O$ (v/v = 5.1 mL/1.7 mL). The mixture was purged with nitrogen three times and stirred at room temperature 25 °C for 1 h. The reaction was monitored by TLC, indicating that half of the starting material **2-1** remained. Additional portions **of M6** (0.26 g, 0.51 mmol, 1.5 equiv.), XPhos Pd G3 (58 mg, 0.068 mmol, 0.2 equiv.) and $K_3PO_4$-$7H_2O$ (290 mg, 0.85 mmol, 2.5 equiv) were added. The mixture was purged with nitrogen three times and stirred for 0.5 h. The reaction remained incomplete, and the addition step was repeated until compound **2-1** was substantially consumed. Saturated ammonium chloride solution (20 mL) was added. The mixture was extracted with EA (20 mL × 2). The combined organic phases were dried over anhydrous sodium sulfate and concentrated. The residue was purified by column chromatography using DCM/MeOH (v/v = 30/1) as eluent to obtain 227 mg of a red solid compound **2-2** with a yield of 71.5%. LC-MS (ESI, pos. ion) m/z: 473.5 $[M+2H]^{2+}$.

Step 3: Synthesis of Compound **2-3**

Into a 50 mL single-necked flask were added compound **2-2** (227 mg, 0.24 mmol) and acetonitrile (6 mL). The mixture was cooled to -15 °C and stirred for 5 minutes. TMSOTf (0.12 mL, 0.65 mmol) was added dropwise. After stirring for 30 minutes, the reaction was stopped and quenched with saturated aqueous sodium bicarbonate solution (20 mL). The mixture was extracted with DCM (15 mL × 2). The combined organic phases were dried over anhydrous sodium sulfate and concentrated. The residue was purified by column chromatography using DCM/MeOH (v/v = 9/1) as eluent to obtain 110 mg of a yellow solid compound **2-3** with a yield of 57.2%, LC-MS (ESI, pos. ion) m/z: 801.4 $[M+H]^+$.

Step 4: Synthesis of Compound **2**

Into a 25 mL single-necked flask were added **1-5** (110 mg, 0.14 mmol), cesium fluoride (320 mg, 2.1 mmol) and DMF (0.8 mL). The mixture was purged with nitrogen three times and stirred at room temperature 25 °C for 17.5 h. The reaction was stopped, and water (15 mL) was added, precipitating a solid. Filtration afforded the crude solid. The crude product was purified by thin-layer chromatography using DCM/MeOH (v/v = 8/1) as eluent to obtain 67 mg of a yellow solid compound 2 with a yield of 75.7%. LC-MS (ESI, pos. ion) m/z: 645.5 $[M+H]^+$; HRMS (ESI): 645.2802 $[M+H]^+$; $^1$H NMR (400 MHz, $CD_3OD$): δ 7.86 (dd, *J= 9.1,* 5.7 Hz, 1H), 7.38 - 7.27 (m, 2H), 7.24 - 7.16 (m, 1H), 5.13 - 5.00 (m, 4H), 4.64 - 4.27 (m, 4H), 4.09 - 3.72 (m, 6H), 3.54 - 3.39 (m, 2H), 3.33 (s, 1H), 2.93 - 2.76 (m, 2H), 2.71 - 2.56 (m, 2H), 2.20 (s, 3H), 1.99 - 1.62 (m, 4H). $^{19}$F NMR (376 MHz, $CD_3OD$) δ -111.51 (1F), -139.85 (1F).

**Example 3: Synthesis of Compound 3**

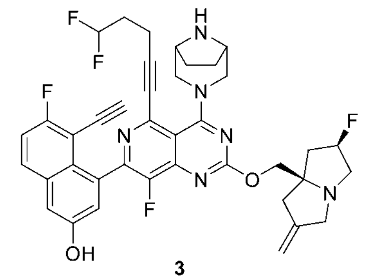

3

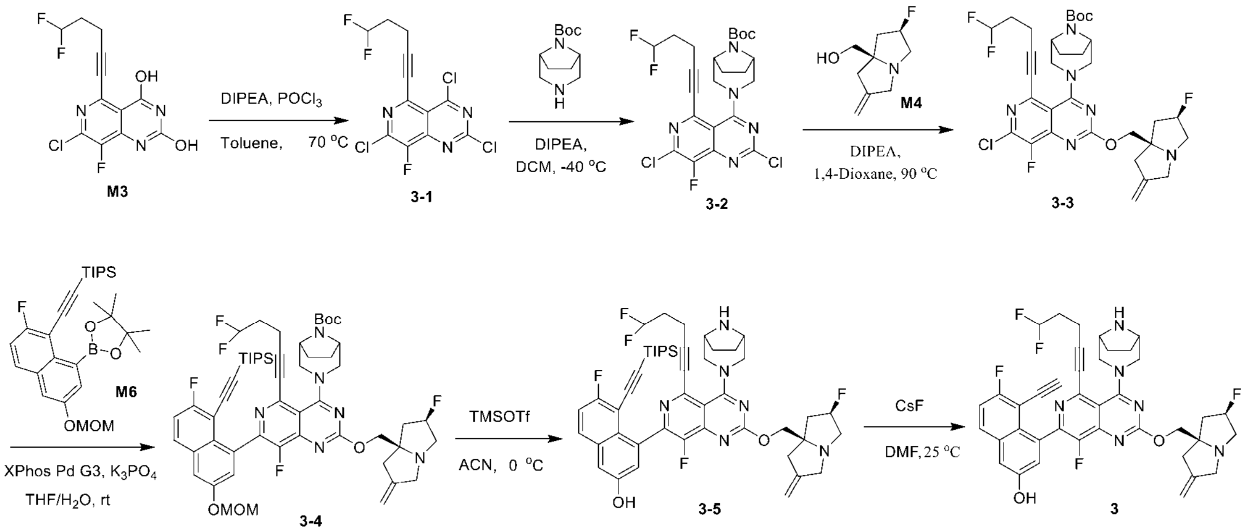


Step 1: Synthesis of Compound **3-1**

Into a 100 mL single-necked flask were added compound **M3** (1.88 g, 5.92 mmol) and anhydrous toluene (38 mL). Then, phosphoryl chloride (3.63 g, 23.68 mmol) and DIPEA (3.06 g, 23.68 mmol) were added. The mixture was heated to 70 °C and stirred for 1.5 h. The reaction was stopped and the mixture was cooled. The mixture was concentrated and used

directly in the next step. The yield was calculated as 100%.

Step 2: Synthesis of Compound **3-2**

Into the compound **3-1** obtained in the previous step was added anhydrous dichloromethane (40 mL). The mixture was purged with nitrogen three times and stirred to dissolve. The mixture was cooled to -40 °C and stirred for 5 minutes. DIPEA (2.28 g, 17.67 mmol) and a solution of8-BOC-3,8-diazabicyclo[3.2.1]octane (1.25 g, 5.89 mmol) in DCM (10 mL) were added sequentially. After stirring at -40 °C for 0.5 h, the reaction was stopped and quenched with saturated ammonium chloride solution (50 mL). The mixture was warmed to room temperature and extracted with DCM (30 mL × 2). The organic phase was separated, dried over anhydrous sodium sulfate, and concentrated. The residue was purified by column chromatography using PE/EA (v/v = 10/1-2/1) as eluent to obtain 2.01 g of a yellow solid compound 3-2. The combined yield for step 1 and step 2 was 64.3%. LC-MS (ESI, pos. ion) m/z: 531.5 $[M+H]^+$.

Step 3: Synthesis of Compound **3-3**

Into a 100 mL two-necked flask were added compound **3-2** (1.65 g, 3.105 mmol), **M4** (798 mg, 4.65 mmol), DIPEA (2.56 mL, 15.52 mmol) and anhydrous 1,4-dioxane (18 mL). The mixture was purged with nitrogen three times, the temperature was raised to 90 °C and stirred for 12 h. Stirring was stopped, the mixture was concentrated, and purified by column chromatography using DCM/MeOH (v/v = 100/1-30/1) as the eluent to obtain 450 mg of a yellow oil compound **3-3** with a yield of 21.8%. LC-MS (ESI, pos. ion) m/z: 666.0 $[M+H]^+$.

Step 4: Synthesis of Compound **3-4**

Into a 25 mL two-necked flask were added compound **3-3** (450 mg, 0.68 mmol), **M6** (0.697 g, 1.36 mmol), Xphos Pd G3 (172.7 mg, 0.20 mmol), $K_3PO_4$-$7H_2O$ (1.15 g, 3.40 mmol) and THF/$H_2O$ (v/v = 10 mL/2 mL). The mixture was purged with nitrogen three times. The mixture was heated to 30 °C and stirred for 6 h. The reaction was stopped and added saturated ammonium chloride solution (20 mL). The mixture was extracted with DCM (50 mL). The organic phases were dried over anhydrous sodium sulfate and concentrated. The residue was purified by column chromatography using DCM/MeOH (v/v = 100/1-30/1) as eluent to obtain 323 mg of a tan oil compound **3-4** with a yield of 47.0%. LC-MS (ESI, pos. ion) m/z: 508.4 $[M+2H]^{2+}$.

Step 5: Synthesis of Compound **3-5**

Into a 50 mL single-necked flask were added compound **3-4** (270 mg, 0.27 mmol) and acetonitrile (10 mL). The mixture was cooled to 0 °C and stirred for 5 minutes. TMSOTf (0.24 mL, 1.35 mmol) was added dropwise. After stirring for 60 minutes at 0 °C, the reaction was stopped and quenched with saturated aqueous sodium bicarbonate solution (20 mL). The mixture was extracted with DCM (30 mL × 3). The combined organic phases were dried over anhydrous sodium sulfate and concentrated. The residue was purified by thin-layer chromatography using DCM/MeOH (v/v = 10/1) as eluent to obtain 65 mg of a yellow solid compound **3-5** with a yield of 28.1%, LC-MS (ESI, pos. ion) m/z: 871.4 $[M+H]^+$.

Step 6: Synthesis of Compound **3**

Into a 25 mL single-necked flask were added compound **3-5** (80 mg, 0.092 mmol), cesium fluoride (140 mg, 0.92 mmol) and DMF (2 mL). The mixture was purged with nitrogen three times and stirred at room temperature 25 °C for 4 h. The reaction was stopped, and water (8 mL) was added, precipitating a solid. Filtration afforded the crude solid. The crude product was purified by thin-layer chromatography using DCM/MeOH/TEA (v/v = 10/1/0.1) as eluent to obtain 27 mg of a brown solid compound **3** with a yield of 41.1%, LC-MS (ESI, pos. ion) m/z: 715.2 $[M+H]^+$; HRMS (ESI): 715.2836 $[M+H]^+$; $^1H$ NMR (400 MHz, $CD_3OD$): δ 7.86 (dd, *J*= 9.1, 5.7 Hz, 1H), 7.38 - 7.29 (m, 2H), 7.21 (s, 1H), 6.05 (tt, *J* = 56.4, 4.3 Hz, 1H), 5.34 (d, *J= 53.2* Hz, 1H), 5.02 (s, 2H), 4.72 - 4.26 (m, 4H), 4.05 - 3.75 (m, 5H), 3.50 - 3.39 (m, 1H), 3.34 (s, 1H), 3.03 - 2.84 (m, 2H), 2.77 (t, *J* = 7.4 Hz, 2H), 2.60 - 2.39 (m, 2H), 2.29 - 2.04 (m, 4H), 1.95 - 1.64 (m, 4H). $^{19}F$ NMR (376 MHz, $CD_3OD$): δ -111.44 (1F), -119.49 (2F), -139.27 (1F), -174.25 (1F).

**Example 4: Synthesis of Compound 4**

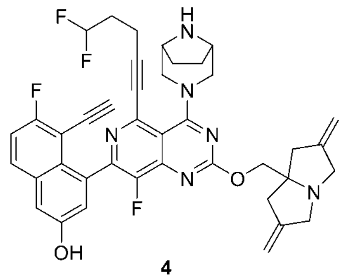

4

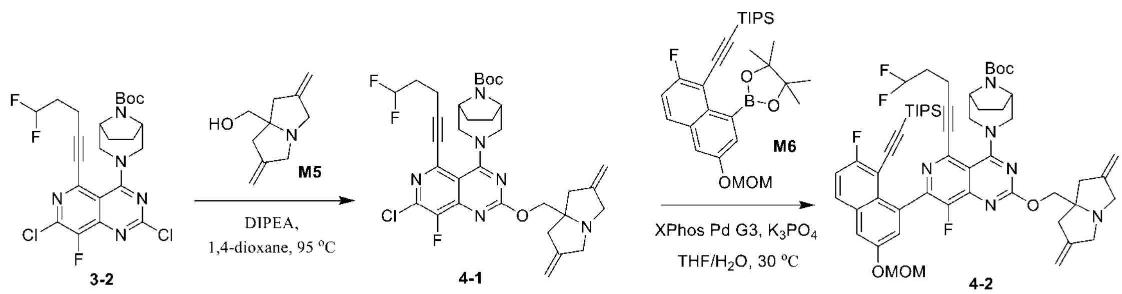


3-2 4-1 4-2

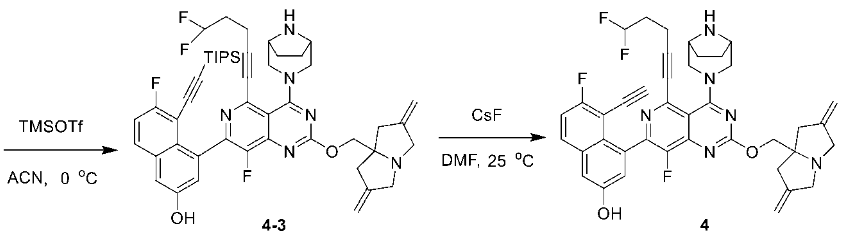


4-3 4

Step 1: Synthesis of Compound **4-1**

Into a 50 mL two-necked flask were added compound **3-2** (450 mg, 0.85 mmol), **M5** (280.9 mg, 1.7 mmol), DIPEA (0.70 mL, 4.25 mmol) and anhydrous 1,4-dioxane (6 mL). The mixture was purged with nitrogen three times, the temperature was raised to 95 °C and stirred for 12 h. Stirring was stopped, the mixture was concentrated, and purified by column chromatography using DCM/MeOH (v/v = 100/1-30/1) as the eluent to obtain 220 mg of a yellow oil compound **4-1** with a yield of 39.3%. LC-MS (ESI, pos. ion) m/z: 659.3 $[M+H]^+$.

Step 2: Synthesis of Compound **4- 2**

Into a 25 mL two-necked flask were added compound **4-1** (220 mg, 0.33 mmol), **M6** (338 mg, 0.66 mmol), Xphos Pd G3 (139.7 mg, 0.17 mmol), $K_3PO_4 \cdot 7H_2O$ (0.56 g, 1.65 mmol) and $THF/H_2O$ (v/v = 5 mL/1 mL). The mixture was purged with nitrogen three times. The mixture was heated to 30 °C and stirred for 6 h. The reaction was stopped and added saturated ammonium chloride solution (20 mL). The mixture was extracted with EA (30 mL × 3). The combined organic phases were dried over anhydrous sodium sulfate and concentrated. The residue was purified by column chromatography using DCM/MeOH (v/v = 100/1-30/1) as eluent to obtain 200 mg of a tan oil compound **4-2** with a yield of 59.4%. LC-MS (ESI, pos. ion) m/z: 1009.5 $[M+H]^+$.

Step 3: Synthesis of Compound **4-3**

Into a 50 mL single-necked flask were added compound **4-2** (180 mg, 0.18 mmol) and acetonitrile (6 mL). The mixture was cooled to 0 °C and stirred for 5 minutes. TMSOTf (0.16 mL, 0.90 mmol) was added dropwise. After stirring for 30 minutes at 0 °C, the reaction was stopped and quenched with saturated aqueous sodium bicarbonate solution (20 mL). The mixture was extracted with DCM (30 mL × 3). The combined organic phases were dried over anhydrous sodium sulfate and concentrated to obtain 140 mg of a yellow solid compound **4-3** with a yield of 90.7%, LC-MS (ESI, pos. ion) m/z: 865.4 $[M+H]^+$.

Step 4: Synthesis of Compound **4**

Into a 25 mL single-necked flask were added compound **4-3** (140 mg, 0.16 mmol), cesium fluoride (240 mg, 1.6 mmol) and DMF (2 mL). The mixture was purged with nitrogen three times and stirred at room temperature for 5 h. The reaction was stopped, and water (10 mL) was added, precipitating a solid. Filtration afforded the crude solid. The crude product was purified by thin-layer chromatography using DCM/MeOH/TEA (v/v = 100/10/0.5) as eluent to obtain 35 mg of a brown solid compound **4** with a yield of 30.5%, LC-MS (ESI, pos. ion) m/z: 709.3 $[M+H]^+$; HRMS (ESI): 709.2906 $[M+H]^+$; $^1H$ NMR (400 MHz, $CD_3OD$) δ 7.87 (dd, *J* = 9.1, 5.7 Hz, 1H), 7.42 - 7.27 (m, 2H), 7.22 (s, 1H), 6.05 (tt, *J* = 56.4, 4.3 Hz, 1H), 5.13 - 5.01 (m, 4H), 4.69 - 4.28 (m, 4H), 4.09 - 3.78 (m, 6H), 3.54 - 3.43 (m, 2H), 3.34 (s, 1H), 2.91 - 2.81 (m, 2H), 2.77 (t, *J* = 7.4 Hz, 2H), 2.69 - 2.57 (m, 2H), 2.30 - 2.13 (m, 2H), 1.97 - 1.63 (m, 4H). $^{19}F$ NMR (376 MHz, $CD_3OD$): δ -111.43 (1F), -119.46 (2F), -139.26 (1F).

**Example 5: Synthesis of Compound 5**

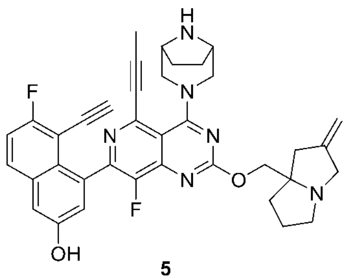

5

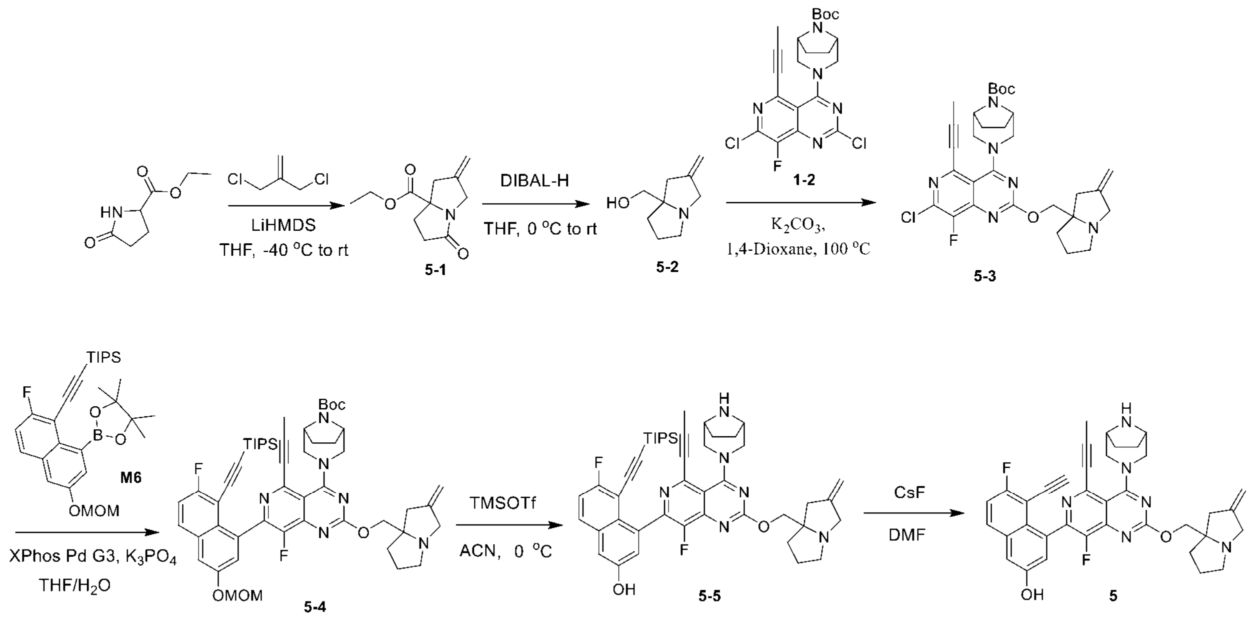


5-1 5-2 1-2 5-3 M6 5-4 5-5 5

Step 1: Synthesis of Compound **5-1**

At room temperature, ethyl 5-oxopyrrolidine-2-carboxylate (10 g, 63.63 mmol), 3-chloro-2-chloromethylpropene (11.93 g, 95.45 mmol) and anhydrous THF (20 mL) were added to a 250 mL two-necked flask. The mixture was purged with nitrogen three times, and the temperature was lowered to -40 °C. LiHMDS (100 mL, 100 mmol, 1.0 M THF solution) was added dropwise. After the addition was complete, the reaction was incubated at -40 °C for 30 min and then moved to room temperature and stirred for 16 h. The reaction was stopped and quenched with saturated ammonium chloride (50 mL). The product was extracted with EA (50 mL × 2). The organic phases were combined and washed twice with saturated brine (50 mL × 2). The organic phases were dried over anhydrous sodium sulfate, concentrated, and purified by column chromatography using PE/DCM (v/v = 0/100) as the eluent to obtain 5.3 g of a yellow oil compound 5-1 with a yield of 40%. LC-MS (ESI, pos. ion) m/z: 210.1 $[M+H]^+$.

Step 2: Synthesis of Compound **5- 2**

At room temperature, **5-1** (1.3 g, 5.30 mmol) was added to a 250 mL single-necked flask. The temperature was lowered to 0 °C, and a solution of diisobutylaluminum hydride in toluene (1.5 M, 67.55 mL, 101.32 mmol) was added dropwise. The mixture was stirred at room temperature overnight. The reaction solution was added to 250 mL of ice water with stirring and then stirred for 15 min. The mixture was filtered through celite pad and the filter cake was rinsed with ethyl acetate (200 mL). The filtrate was separated and the organic phase was dried over anhydrous sodium sulfate and concentrated. The product was purified by column chromatography using DCM/MeOH (v/v = 4/1) as the eluent to obtain 1.2 g of a yellow solid compound **5-2** with a yield of 31%. LC-MS (ESI, pos. ion) m/z: 154.2 $[M+H]^+$.

Step 3: Synthesis of Compound **5-3**

1-2 (1 g, 2.14 mmol), **5-2** (0.66 g, 4.28 mmol), potassium carbonate (1.18 g, 8.56 mmol) and 1,4-dioxane (15 mL) were added to a 250 mL single-necked flask at room temperature. The mixture was purged with nitrogen three times, and the temperature was heated to 100 °C and refluxed for 16 h. The reaction was stopped and filtered. The filter cake was washed with EA (60 mL). The combined filtrates were added with saturated ammonium chloride (60 mL), and the extracts were separated. The aqueous phase was extracted with EA (50 mL). The organic phases were combined, dried over anhydrous sodium sulfate, concentrated, and purified by column chromatography using DCM/MeOH (v/v = 100/0-20/1) as the eluent to obtain 0.43 g of dark solid compound 5-3 with a yield of 34%. LC-MS (ESI, pos. ion) m/z: 583.3 $[M+H]^+$.

Step 4: Synthesis of Compound **5- 4**

Into a 50 mL single-necked flask were added compound 5-3 (430 mg, 0.74 mmol), **M6** (0.57 g, 1.11 mmol), $K_3PO_4$-$7H_2O$ (880 mg, 2.59 mmol) and THF/$H_2O$ (10 mL/1 mL). The mixture was purged with nitrogen three times, and Xphos Pd G3 (190 mg, 0.22 mmol) was added portionwise. The mixture was stirred at 35 °C for 5 h. The reaction was stopped and to the mixture was added saturated ammonium chloride solution (20 mL). The mixture was extracted with EA (20 mL × 2). The combined organic phases were dried over anhydrous sodium sulfate and concentrated. The residue was purified by column chromatography using DCM/MeOH (v/v = 20/1) as eluent to obtain 320 mg of a yellow solid compound **5-4** with a yield of 46%. LC-MS (ESI, pos. ion) m/z: 933.3 $[M+H]^+$.

Step 5: Synthesis of Compound **5-5**

Into a 50 mL single-necked flask were added compound **5-4** (320 mg, 0.34 mmol) and acetonitrile (3 mL). The mixture was cooled to 0 °C and stirred for 5 minutes. TMSOTf (0.23g, 1.02 mmol) was added dropwise and stirred for 60 minutes, the reaction was stopped and quenched with saturated aqueous sodium bicarbonate solution (20 mL). The mixture was extracted with EA (10 mL × 2). The combined organic phases were dried over anhydrous sodium sulfate and concentrated. The residue was purified by thin-layer chromatography using DCM/MeOH (v/v = 8/1) as eluent to obtain 270 mg of a yellow solid compound 5-5 with a yield of 99%, LC-MS (ESI, pos. ion) m/z: 789.3 $[M+H]^+$.

Step 6: Synthesis of Compound **5**

Into a 25 mL single-necked flask were added **5-5** (110 mg, 0.34 mmol), cesium fluoride (260 mg, 1.7 mmol) and DMF (2 mL). The mixture was purged with nitrogen three times and stirred at room temperature 25 °C for 16 h. The reaction was stopped, and water (80 mL) was added, precipitating a solid. Filtration afforded the crude solid. The crude product was purified by thin-layer chromatography using DCM/MeOH/TEA (v/v = 7/1/0.05) as eluent to obtain 28 mg of a yellow solid compound **5** with a yield of 13%. LC-MS (ESI, pos. ion) m/z: 633.3 $[M+H]^+$; $^1H$ NMR (400 MHz, $CD_3OD$) δ 7.92 - 7.82 (m, 1H), 7.40 - 7.29 (m, 2H), 7.27 - 7.19 (m, 1H), 5.20 (s, 2H), 4.66 - 4.47 (m, 4H), 4.22 - 4.10 (m, 1H), 3.91 - 3.66 (m, 4H), 3.62 - 3.48 (m, 1H), 3.13 - 3.02 (m, 1H), 3.01 - 2.91 (m, 1H), 2.76 - 2.62 (m, 1H), 2.38 - 2.26 (m, 2H), 2.20 (s, 3H), 2.18 - 1.95 (m, 4H), 1.94 - 1.71 (m, 4H). $^{19}F$ NMR (376 MHz, $CD_3OD$) δ -111.48 (1F), -140.34 (1F).

**Example 6: Synthesis of Compound 6**

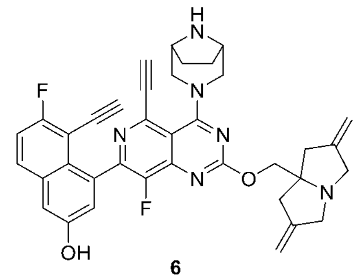

6

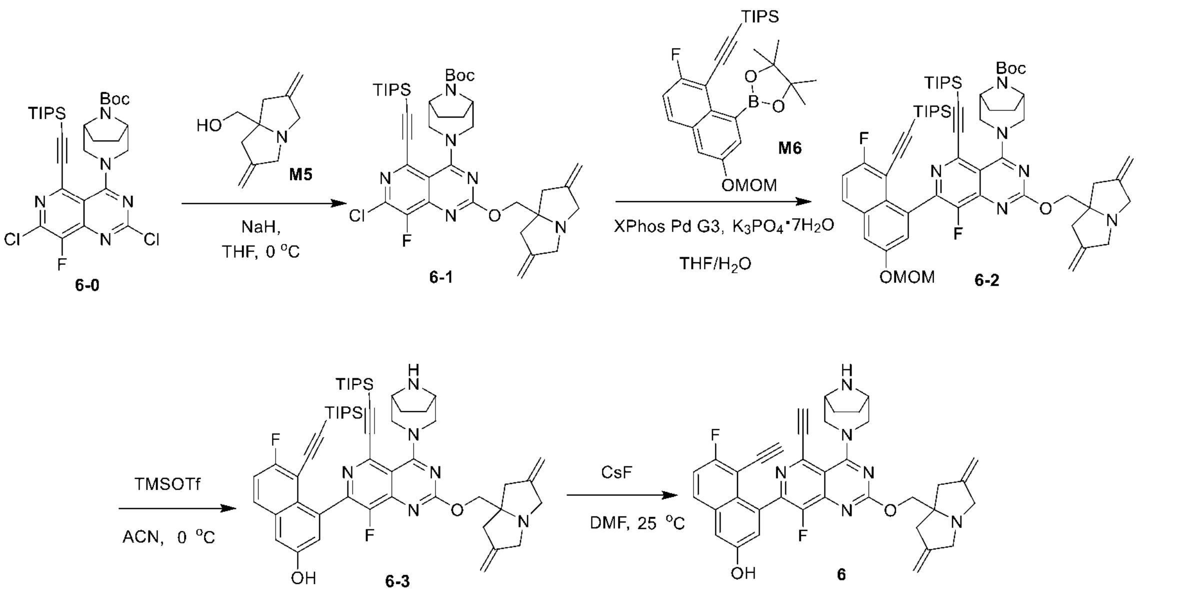


6-0 6-1 6-2 6-3 6

Step 1: Synthesis of Compound **6-1**

Into a 100 mL two-necked flask was added sodium hydride (0.13 g, 3.28 mmol, 60% purity). The mixture was purged with nitrogen three times, and anhydrous THF (5 mL) was added. The mixture was stirred at 0 °C for 5 minutes. A solution of **M5** (0.27 g, 1.64 mmol) in THF (1.5 mL) was slowly added dropwise. The mixture was stirred at 0 °C for 1 h. A solution of compound **6-0** (500 mg, 0.82 mmol, the synthesis method of compound **6-0** was synthesized according to the synthesis method of **M8-5** on page 56 of patent WO2023061463) in THF (2 mL) was slowly added dropwise. The mixture was stirred at 0 °C for 3 h. The reaction was stopped by stirring, and a saturated ammonium chloride solution (50 mL) was added to quench the reaction. The layers were separated, and the aqueous phase was extracted with EA (20 mL × 2). The organic phases were combined, dried over anhydrous sodium sulfate, concentrated, and purified by column chromatography using DCM/MeOH (v/v = 50/1) as the eluent to obtain 220 mg of yellow solid compound **6-1** with a yield of 36.3%. LC-MS (ESI, pos. ion) m/z: 737.4 $[M+H]^+$.

Step 2: Synthesis of Compound **6- 2**

Into a 25 mL two-necked flask were added compound **6-1** (140 mg, 0.19 mmol, 1.0 eq), **M6** (0.19 g, 0.38 mmol, purchased from Shanghai Lingkai Pharmaceutical), Xphos Pd G3 (32 mg, 0.038 mmol), $K_3PO_4$-$7H_2O$ (160 mg, 0.47 mmol) and THF/$H_2O$ (v/v = 3 mL/1 mL). The mixture was purged with nitrogen three times and stirred at room temperature 25 °C for 2 h. Additional XPhos Pd G3 (0.2 equiv.) was added, the mixture was purged with nitrogen three times and stirred for 1 h. The reaction was still not complete, and the step of adding materials was repeated until the reaction of raw material **6-1** was basically complete. Saturated ammonium chloride solution (20 mL) was added and extracted with DCM (20 mL × 2). The organic phases were combined and dried over anhydrous sodium sulfate, concentrated, and purified by column chromatography using DCM/MeOH (v/v = 50/1) as the eluent to obtain 188 mg of red solid compound **6-2** with a yield of 91%. LC-MS (ESI, pos. ion) m/z: 544.5 $[M+2H]^{2+}$.

Step 3: Synthesis of Compound **6-3**

Into a 25 mL single-necked flask were added compound **6-2** (188 mg, 0.17 mmol) and acetonitrile (4 mL). The

mixture was cooled to 0 °C and stirred for 5 minutes. TMSOTf (0.092 mL, 0.51 mmol) was added dropwise and stirred for 30 minutes, the reaction was stopped and quenched with saturated aqueous sodium bicarbonate solution (15 mL). The mixture was extracted with DCM (15 mL × 3). The combined organic phases were dried over anhydrous sodium sulfate and concentrated. The residue was purified by thin-layer chromatography using DCM/MeOH (v/v = 10/1) as eluent to obtain 68 mg of a yellow solid compound **6-3** with a yield of 42%. LC-MS (ESI, pos. ion) m/z: 943.9 $[M+H]^+$.

Step 4: Synthesis of Compound **6**

Into a 25 mL single-necked flask were added **6-5** (68 mg, 0.072 mmol), cesium fluoride (160 mg, 1.08 mmol) and DMF (0.6 mL). The mixture was purged with nitrogen three times and stirred at room temperature for 3 h. The reaction was stopped, and water (14 mL) was added, precipitating a solid. Filtration afforded the crude solid. The crude product was purified by thin-layer chromatography using DCM/MeOH (v/v = 8/1) as eluent to obtain 8 mg of a yellow solid compound **6** with a yield of 18%. LC-MS (ESI, pos. ion) m/z: 631.3 $[M+H]^+$; HRMS (ESI): 631.2658 $[M+H]^+$; $^1H$ NMR (400 MHz, $CD_3OD$) δ 7.88 (dd, *J* = 9.1, 5.7 Hz, 1H), 7.42 - 7.30 (m, 2H), 7.29 - 7.18 (m, 1H), 5.12 - 4.98 (m, 4H), 4.74 - 4.46 (m, 3H), 4.42 - 4.24 (m, 3H), 3.88 - 3.75 (m, 3H), 3.73 - 3.61 (m, 2H), 3.60 - 3.47 (m, 1H), 3.43 - 3.35 (m, 2H), 2.89 - 2.73 (m, 2H), 2.68 - 2.54 (m, 2H), 1.84 - 1.56 (m, 4H). $^{19}F$ NMR (376 MHz, $CD_3OD$) δ -111.47 (1F), -138.25 (1F).

**Example 7: Synthesis of Compound 7**

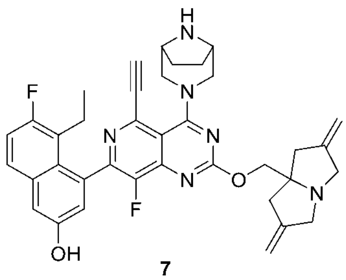

7

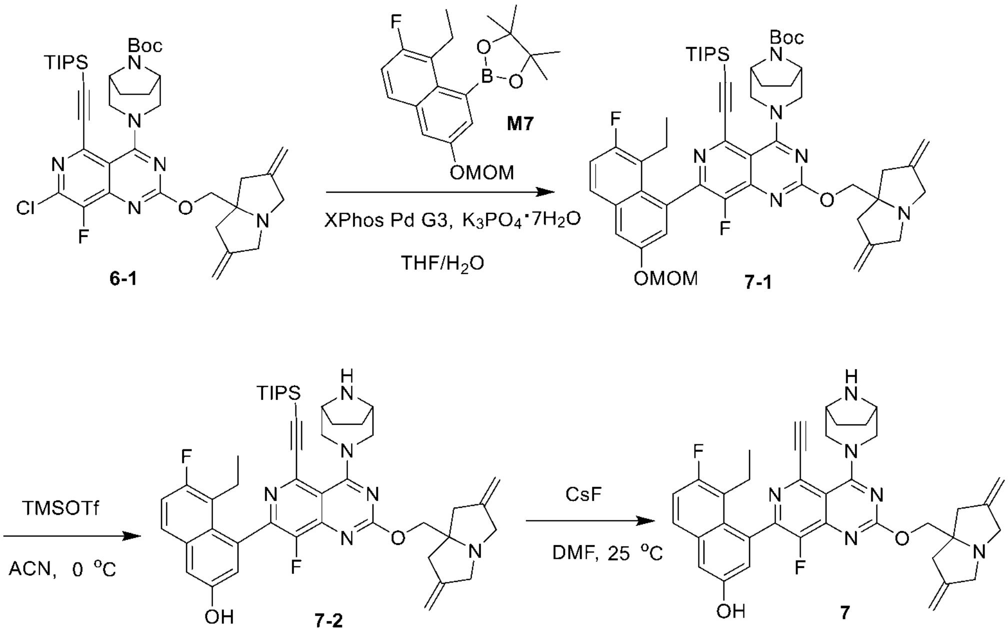


Step 1: Synthesis of Compound 7-1

Into a 50 mL two-necked flask were added compound **6-1** (80 mg, 0.11 mmol, 1.0 eq), **M7** (79 mg, 0.22 mmol, purchased from Shaoyuan Technology), Xphos Pd G4 (19 mg, 0.022 mmol), $K_3PO_4$-$7H_2O$ (93 mg, 0.28 mmol) and THF/$H_2O$ (3 mL/0.56 mL). The mixture was purged with nitrogen three times and stirred at room temperature 25°C for 1 h.

The addition of Xphos Pd G4 was repeated until the reaction of the raw material **6-1** was complete. The saturated ammonium chloride solution (15 mL) was added. The mixture was extracted with DCM (20 mL × 2). The combined organic phases were dried over anhydrous sodium sulfate and concentrated. The residue was purified by column chromatography using DCM/MeOH (v/v = 50/1) as eluent to obtain 83 mg of a yellow oil compound **7-1** with a yield of 82%. LC-MS (ESI, pos. ion) m/z: 468.4 $[M+2H]^{2+}$.

Step 2: Synthesis of Compound **7-2**

Into a 25 mL single-necked flask were added compound **7-1** (83 mg, 0.089 mmol) and acetonitrile (3 mL). The mixture was cooled to 0 °C and stirred for 5 minutes. TMSOTf (0.048 mL, 0.27 mmol) was added dropwise and stirred for 30 minutes, the reaction was stopped and quenched with saturated aqueous sodium bicarbonate solution (15 mL). The mixture was extracted with DCM (20 mL × 2). The combined organic phases were dried over anhydrous sodium sulfate and concentrated. The residue was purified by thin-layer chromatography using DCM/MeOH (v/v = 10/1) as eluent to obtain 37 mg of a yellow solid compound **7-2** with a yield of 53%, LC-MS (ESI, pos. ion) m/z: 791.4 $[M+H]^+$.

Step 3: Synthesis of Compound **7**

Into a 25 mL single-necked flask were added **7-2** (37 mg, 0.047 mmol), cesium fluoride (71 mg, 0.47 mmol) and DMF (0.4 mL). The mixture was purged with nitrogen three times and stirred at room temperature for 3 h. The reaction was stopped, and water (10 mL) was added, precipitating a solid. Filtration afforded the crude solid. The crude product was purified by thin-layer chromatography using DCM/MeOH (v/v = 9/1) as eluent to obtain 27 mg of a yellow solid compound **7** with a yield of 91%. LC-MS (ESI, pos. ion) m/z: 635.3 $[M+H]^+$; HRMS (ESI): 635.2973 $[M+H]^+$; $^1H$ NMR (400 MHz, $CD_3OD$) δ 7.69 (dd, *J* = 9.0, 5.9 Hz, 1H), 7.35 - 7.19 (m, 2H), 7.16 - 7.06 (m, 1H), 5.10 - 4.97 (m, 4H), 4.69 - 4.45 (m, 3H), 4.43 - 4.26 (m, 2H), 4.21 - 3.88 (m, 2H), 3.84 - 3.65 (m, 3H), 3.63 - 3.46 (m, 2H), 3.37 (s, 1H), 2.88 - 2.71 (m, 2H), 2.67 - 2.53 (m, 2H), 2.50 - 2.36 (m, 1H), 2.28 - 2.16 (m, 2H), 1.85 - 1.63 (m, 3H), 0.85 (t, *J*= 7.4 Hz, 3H). $^{19}F$ NMR (376 MHz, $CD_3OD$) δ -120.83 (1F), -138.10 (1F).

**Example 8: Synthesis of Compound 8**

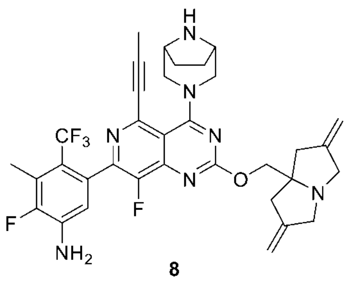

8

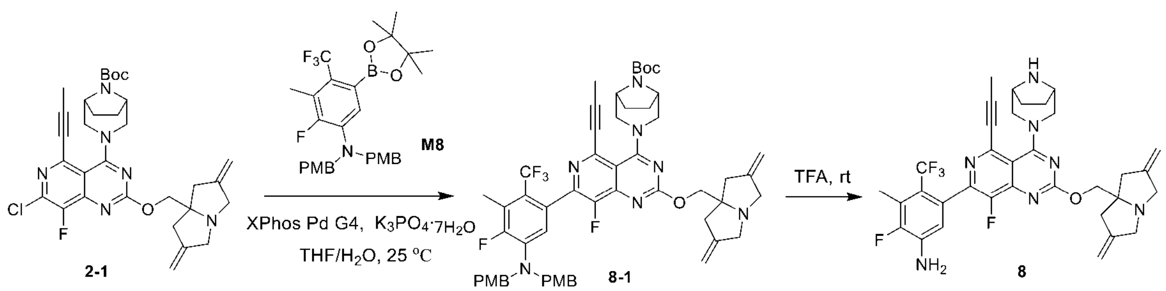


Step 1: Synthesis of Compound **8-1**

Into a 25 mL two-necked flask were added compound **2-1** (180 mg, 0.30 mmol, 1.0 eq), **M8** (0.25 g, 0.45 mmol), Xphos Pd G4 (52 mg, 0.060 mmol), $K_3PO_4 \cdot 7H_2O$ (305 mg, 0.90 mmol) and $THF/H_2O$ (6 mL/2 mL). The mixture was purged with nitrogen three times and stirred at room temperature 25°C for 7 h. The reaction was stopped and added saturated ammonium chloride solution (10 mL). The mixture was extracted with EA (20 mL × 2). The combined organic phases were

dried over anhydrous sodium sulfate and concentrated. The residue was purified by column chromatography using DCM/MeOH (v/v = 30/1) as eluent to obtain 230 mg of a brown solid compound **8-1** with a yield of 65%. LC-MS (ESI, pos. ion) m/z: 992.3 $[M+H]^+$.

Step 2: Synthesis of Compound **8**

Into a 25 mL single-necked flask were added compound **8-1** (230 mg, 0.23 mmol) and trifluoroacetic acid (20 mL), and the mixture was stirred at room temperature for 1 h. The reaction was stopped and dried, dissolved in DCM (10 mL), and washed with saturated sodium bicarbonate solution (20 mL). The organic phase was concentrated and dried, and then dried. The crude product was purified by thin-layer chromatography using DCM/MeOH (v/v = 15/1) as the eluent to obtain 73 mg of a yellow solid compound 8 with a yield of 48%. LC-MS (ESI, pos. ion) m/z: 652.3 $[M+H]^+$; $^1$H NMR (400 MHz, $CD_3OD$) δ 6.64 (d, *J* = 8.6 Hz, 1H), 5.08 - 5.00 (m, 4H), 4.99 - 4.93 (m, 2H), 4.40 - 4.26 (m, 2H), 3.99 - 3.63 (m, 5H), 3.63 - 3.44 (m, 3H), 3.38 - 3.34 (m, 2H), 2.86 - 2.71 (m, 2H), 2.64 - 2.55 (m, 2H), 2.41 (s, 3H), 2.21 (s, 3H), 1.80 - 1.54 (m, 4H). $^{19}$F NMR (376 MHz, $CD_3OD$) δ -54.95 (3F), -137.80 (1F), -141.73 (1F).

**Example 9: Synthesis of Compound 9**

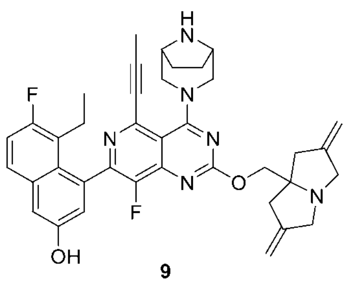

9

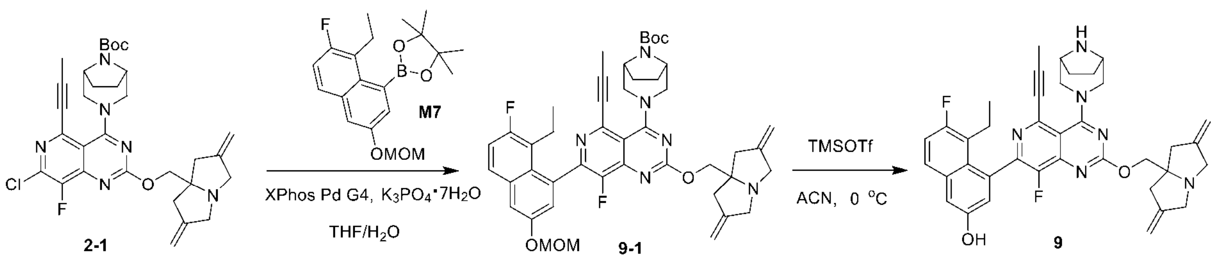


2-1 9-1 9

Step 1: Synthesis of Compound **9-1**

Into a 25 mL two-necked flask were added compound **2-1** (150 mg, 0.25 mmol, 1.0 eq), **M7** (180 mg, 0.50 mmol), Xphos Pd G4 (43 mg, 0.050 mmol, purchased from Shaoyuan Technology), $K_3PO_4$-$7H_2O$ (338 mg, 1 mmol) and THF/$H_2O$ (6 mL/2 mL). The mixture was purged with nitrogen three times and stirred at room temperature for 10 h. The reaction was stopped and added saturated ammonium chloride solution (10 mL). The mixture was extracted with EA (20 mL × 2). The combined organic phases were dried over anhydrous sodium sulfate and concentrated. The residue was purified by column chromatography using DCM/MeOH (v/v = 30/1) as eluent to obtain 145 mg of a reddish-brown solid compound **9-1** with a yield of 72%. LC-MS (ESI, pos. ion) m/z: 793.3 $[M+H]^+$.

Step 2: Synthesis of Compound **9**

Into a 25 mL single-necked flask were added compound **9-1** (145 mg, 0.18 mmol) and acetonitrile (3 mL). The mixture was cooled to -20 °C and stirred for 5 minutes. TMSOTf (0.098 mL, 0.54 mmol) was added dropwise, and the mixture was stirred for 60 minutes, the reaction was stopped and quenched with saturated aqueous sodium bicarbonate solution (15 mL). The mixture was extracted with DCM (20 mL × 2). The combined organic phases were dried over anhydrous sodium sulfate and concentrated. The residue was purified by thin-layer chromatography using DCM/MeOH (v/v = 10/1) as eluent to obtain 60 mg of a yellow solid compound **9** with a yield of 51%. LC-MS (ESI, pos. ion) m/z: 649.3 $[M+H]^+$; HRMS (ESI): 649.3098 $[M+H]^+$; $^1$H NMR (400 MHz, $CD_3OD$) δ 7.73 - 7.57 (m, 1H), 7.36 - 7.18 (m, 2H), 7.10 (s, 1H),

5.09 - 4.97 (m, 4H), 4.44 - 4.28 (m, 3H), 4.06 - 3.86 (m, 1H), 3.80 (d, *J* = 14.7 Hz, 3H), 3.69 - 3.52 (m, 3H), 3.41 - 3.34 (m, 2H), 2.87 - 2.71 (m, 2H), 2.66 - 2.53 (m, 2H), 2.51 - 2.36 (m, 1H), 2.29 - 2.11 (m, 4H), 1.86 - 1.48 (m, 4H), 0.84 *(t, J=* 7.3 Hz, 3H). $^{19}$F NMR (376 MHz, $CD_3OD$) δ -120.85 (1F), -139.75 (1F).

**Example 10: Synthesis of Compound 10**

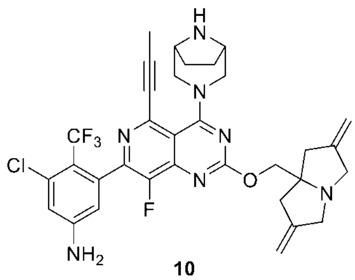

**10**

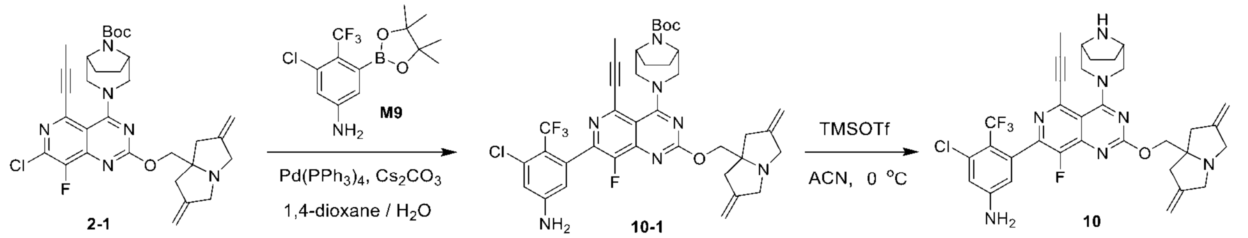


Step 1: Synthesis of Compound **10-1**

Into a 25 mL two-necked flask were added compound **2-1** (78 mg, 0.131 mmol), **M9** (64 mg, 0.2 mmol), $Pd(PPh_3)_4$ (30 mg, 0.026 mmol), cesium carbonate (130 mg, 0.4 mmol) and 1,4-dioxane/$H_2O$ (3 mL/0.8 mL). The mixture was purged with nitrogen three times and stirred at 100 °C for 6 h. The reaction was stopped and added saturated ammonium chloride solution (10 mL). The mixture was extracted with EA (20 mL × 2). The combined organic phases were dried over anhydrous sodium sulfate and concentrated. The residue was purified by thin-layer chromatography using DCM/MeOH (v/v = 40/1) as eluent to obtain 54 mg of a reddish-brown solid compound **10-1** with a yield of 55%. LC-MS (ESI, pos. ion) m/z: 754.2 $[M+H]^+$.

Step 2: Synthesis of Compound **10**

Into a 25 mL single-necked flask were added compound **10-1** (54 mg, 0.072 mmol) and acetonitrile (3 mL). The mixture was cooled to 0 °C and stirred for 5 minutes. TMSOTf (0.033 mL, 0.18 mmol) was added dropwise and stirred for 60 minutes, the reaction was stopped and quenched with saturated aqueous sodium bicarbonate solution (15 mL). The mixture was extracted with DCM (15 mL × 2). The combined organic phases were dried over anhydrous sodium sulfate and concentrated. The residue was purified by thin-layer chromatography using DCM/MeOH (v/v = 10/1) as eluent to obtain 31 mg of a yellow solid compound **10** with a yield of 66%, LC-MS (ESI, pos. ion) m/z: 654.2 $[M+H]^+$; $^1$H NMR (400 MHz, $CD_3OD$) δ 6.95 - 6.87 (m, 1H), 6.52 (s, 1H), 5.09 - 4.98 (m, 4H), 4.83 - 4.71 (m, 2H), 4.68 - 4.48 (m, 2H), 4.39 - 4.26 (m, 2H), 3.94 - 3.69 (m, 4H), 3.65 - 3.52 (m, 2H), 3.42 - 3.34 (m, 2H), 2.86 - 2.73 (m, 2H), 2.65 - 2.55 (m, 2H), 2.21 (s, 3H), 1.83 - 1.51 (m, 4H). $^{19}$F NMR (376 MHz, $CD_3OD$): δ -55.72 (3F), -141.83 (1F).

**Example 11: Synthesis of Compound 11**

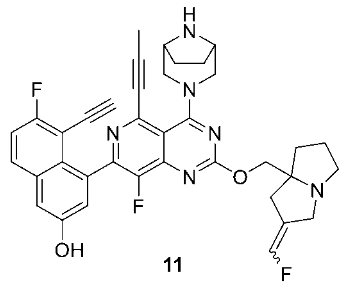

11

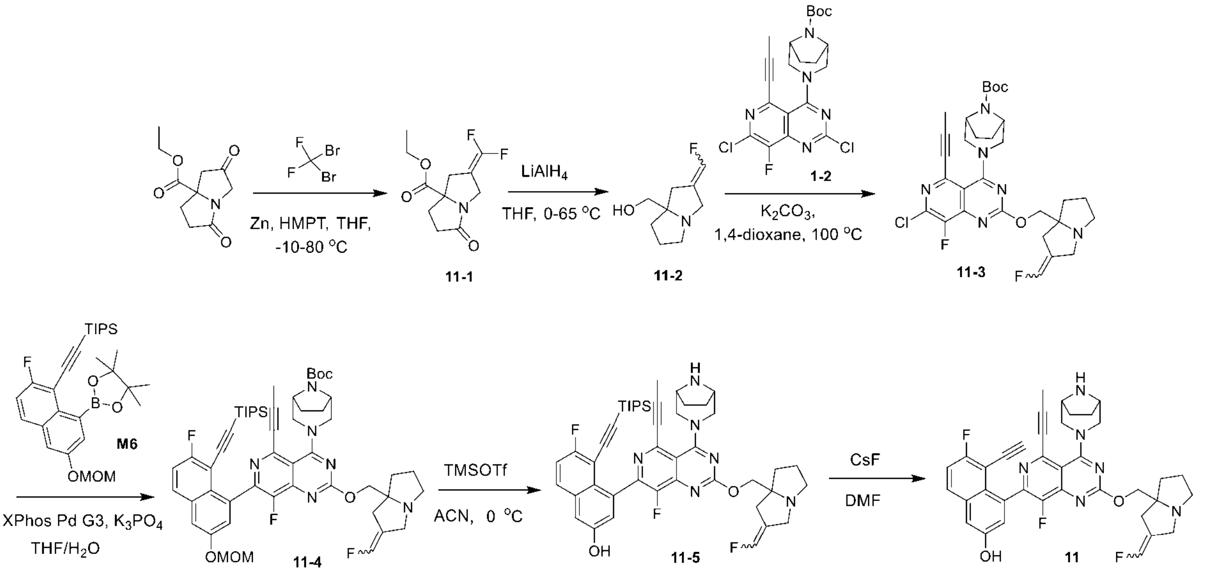


Step 1: Synthesis of Compound **11-1**

Into a 250 mL single-necked flask were added ethyl 2,5-dioxohexahydro-1*H*-pyrrolizine-7a-carboxylate (11 g, 52.08 mmol) and THF (100 mL) at room temperature. The temperature was lowered to -10 °C, and HMPT (34.0 g, 208.32 mmol) was added dropwise. After the addition was complete, the mixture was stirred for 5 minutes. Dibromodifluoromethane (54.64 g, 260.4 mmol) was introduced into the mixture. After stirring at -10 °C for 1.5 h, the mixture was transferred to room temperature for 60 min. Zinc powder (13.63 g, 208.32 mmol) was added, and the temperature was raised to 80 °C for 6 h. The reaction was stopped, cooled to room temperature, filtered through celite, and the filtrate was added with water (50 mL) and saturated sodium chloride (100 mL). The filtrate was extracted with EA (100 mL × 3). The layers were separated, and the organic phase was washed with saturated brine (100 mL × 2). The crude product was washed twice and evaporated to dryness. The crude product was purified by column chromatography using PE/EA (v/v = 1/1) as eluent to obtain 4.95 g of yellow oily compound **11-1** with a yield of 39%. LC-MS (ESI, pos. ion) m/z: 246.1 [M+H]$^+$.

Step 2: Synthesis of Compound **11-2**

Lithium aluminum tetrahydride (0.46 g, 12.24 mmol) was added to a 100 mL two-necked flask at room temperature. After the mixture was purged with nitrogen three times, the temperature was lowered to 0 °C, anhydrous THF (10 mL) was added, and **11-1** (1 g, 4.08 mmol, 10 mL of THF solution) was added dropwise. After the addition was complete, the temperature was heated to 65 °C for reaction. The reaction was stopped, the temperature was lowered to 0 °C, and water (0.5 mL) was slowly added. Then, 15% sodium hydroxide solution (0.5 mL) was slowly added dropwise, and then water (1.5 mL) was added. The mixture was dried over anhydrous sodium sulfate, filtered, and the filter cake was rinsed with DCM (50 mL). The combined filtrate was evaporated to dryness to obtain 0.65 g of yellow oily compound **11-2** with a yield of 93%. LC-MS (ESI, pos. ion) m/z: 172.1 [M+H]$^+$.

Step 3: Synthesis of Compound **11- 3**

**1-2** (1.77 g, 3.8 mmol), **11-2** (0.65 g, 3.80 mmol), potassium carbonate (1.58 g, 11.40 mmol) and 1,4-dioxane (16 mL) were added to a 250 mL single-necked flask at room temperature. The mixture was purged with nitrogen three times, and the temperature was heated to 100 °C and refluxed for 12 h. The reaction was stopped and filtered. The filter cake was washed with EA (60 mL). The combined filtrates were dried over anhydrous sodium sulfate, concentrated, and purified by

column chromatography using DCM/MeOH (v/v = 100/0-20/1) as the eluent to obtain 0.45 g of yellow-brown solid compound **11-3** with a yield of 20%. LC-MS (ESI, pos. ion) m/z: 601.3 $[M+H]^+$.

Step 4: Synthesis of Compound **11- 4**

Into a 50 mL single-necked flask were added compound **11-3** (270 mg, 0.45 mmol), **M6** (0.47 g, 0.90 mmol), $K_3PO_4 \cdot H_2O$ (450 mg, 1.35 mmol) and $THF/H_2O$ (7 mL/0.5 mL). The mixture was purged with nitrogen three times, and Xphos Pd G3 (114 mg, 0.135 mmol) was added portionwise. The mixture was stirred at 35 °C for 5 h. The reaction was stopped and to the mixturew was added saturated ammonium chloride solution (20 mL). The mixture was extracted with EA (30 mL × 2). The combined organic phases were dried over anhydrous sodium sulfate and concentrated. The residue was purified by column chromatography using DCM/EA (v/v = 1/1) as eluent to obtain 135 mg of a yellow solid compound **11-4** with a yield of 32%. LC-MS (ESI, pos. ion) m/z: 951.4 $[M+H]^+$.

Step 5: Synthesis of Compound **11-5**

Into a 50 mL single-necked flask were added compound **11-4** (135 mg, 0.14 mmol) and acetonitrile (3 mL). The mixture was cooled to 0 °C and stirred for 5 minutes. TMSOTf (0.16 mL, 0.70 mmol) was slowly added dropwise. After stirring for 3 h, the reaction was stopped and quenched with saturated aqueous sodium bicarbonate solution (20 mL). The mixture was extracted with EA (10 mL × 2). The combined organic phases were dried over anhydrous sodium sulfate and concentrated to obtain a yellow solid compound **11-5,** the yield was calculated as 100%, LC-MS (ESI, pos. ion) m/z: 807.3 $[M+H]^+$.

Step 6: Synthesis of Compound **11**

Into a 25 mL single-necked flask were added **11-5** (110 mg, 0.14 mmol), cesium fluoride (210 mg, 1.4 mmol) and DMF (1 mL). The mixture was purged with nitrogen three times and stirred at room temperature for 16 h. The reaction was stopped, and water (80 mL) was added, precipitating a solid. Filtration afforded the crude solid. The crude product was purified by thin-layer chromatography using DCM/MeOH (v/v = 8/1) as eluent to obtain 35 mg of an off-white solid compound **11** with a yield of 38%. LC-MS (ESI, pos. ion) m/z: 651.3 $[M+H]^+$; $^1H$ NMR (400 MHz, $CD_3OD$) δ 7.93 - 7.81 (m, 1H), 7.44 - 7.29 (m, 2H), 7.23 (s, 1H), 6.98 - 6.52 (m, 1H), 4.49 - 4.33 (m, 2H), 4.07 - 3.72 (m, 5H), 3.69 - 3.51 (m, 2H), 2.94 - 2.76 (m, 2H), 2.64 - 2.47 (m, 1H), 2.43 - 2.29 (m, 1H), 2.26 - 2.11 (m, 5H), 2.10 - 1.73 (m, 8H). $^{19}F$ NMR (376 MHz, $CD_3OD$): δ -111.50 (1F), -131.02 (1F), -140.03 (1F).

**Example 12: Synthesis of Compound 12-A and Compound 12-B**

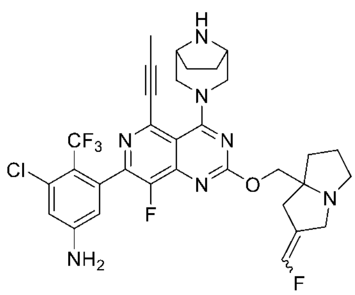

**12-A, 12-B**

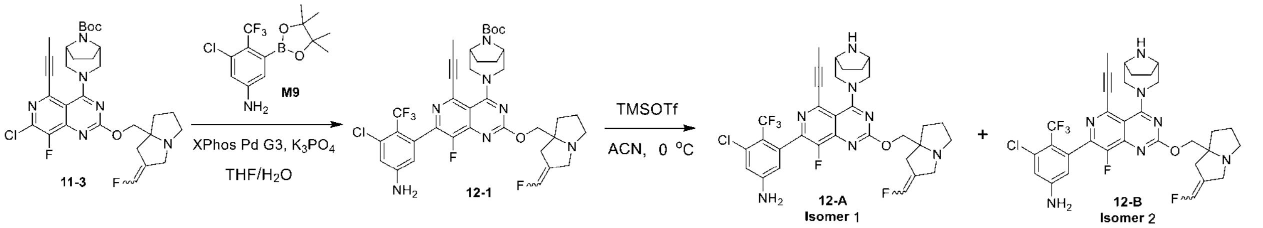

11-3
12-1
12-A Isomer 1
12-B Isomer 2

Step 1: Synthesis of Compound **12-1**

Into a 25 mL single-necked flask were added compound **11-3** (180 mg, 0.30 mmol, 1.0 eq), **M9** (140 mg, 0.45 mmol), potassium phosphate heptahydrate (410 mg, 1.2 mmol), and THF/$H_2O$ (4 mL/0.3 mL). The mixture was purged with nitrogen three times, and Xphos Pd G3 (76 mg, 0.090 mmol) was added portionwise. The mixture was stirred at 35 °C for 16 h under nitrogen protection. The reaction was stopped and saturated ammonium chloride solution (10 mL) was added. The mixture was extracted with EA (20 mL × 2). The organic phases were combined, dried over anhydrous sodium sulfate, concentrated, and purified by thin-layer chromatography using DCM/MeOH (v/v = 20/1) as the eluent to obtain 160 mg of a yellow solid compound **12-1** with a yield of 70%. LC-MS (ESI, pos. ion) m/z: 760.2 $[M+H]^+$.

Step 2: Synthesis of Compound **12**

Into a 25 mL single-necked flask were added compound **12-1** (160 mg, 0.21 mmol) and acetonitrile (3 mL). The mixture was cooled to 0 °C and stirred for 5 minutes. TMSOTf (0.23 mL, 1.05 mmol) was added dropwise and stirred for 1.5 h, the reaction was stopped and quenched with saturated aqueous sodium bicarbonate solution (15 mL). The mixture was extracted with DCM (15 mL × 2). The organic phases were combined and dried over anhydrous sodium sulfate, concentrated, and purified by thin-layer chromatography using DCM/MeOH (v/v = 8/1) as the eluent to obtain 20 mg of off-white solid compound **12-A** (the compound having low polarity showing on the TLC plate) and 17 mg of yellow solid compound **12-B** (the compound having high polarity showing on the TLC plate). The yield was 27%. LC-MS (ESI, pos. ion) m/z: 660.3 $[M+H]^+$. Compound **12-A :** $^1$H NMR (599 MHz, $CD_3OD$) δ 6.93 (s, 1H), 6.77 - 6.59 (m, 1H), 6.53 (s, 1H), 4.67 - 4.56 (m, 4H), 4.37 - 4.27 (m, 2H), 3.91 - 3.86 (m, 1H), 3.66 - 3.59 (m, 2H), 3.55 - 3.48 (m, 1H), 3.23 - 3.16 (m, 1H), 2.79 - 2.71 (m, 2H), 2.52 - 2.45 (m, 1H), 2.25 - 2.11 (m, 5H), 2.06 - 1.86 (m, 4H), 1.82 - 1.60 (m, 4H). $^{19}$F NMR (376 MHz, $CD_3OD$): δ -55.73 (3F), -131.93 (1F), -141.85 (1F). Compound **12-B:** $^1$H NMR (599 MHz, $CD_3OD$) δ 6.96 - 6.89 (m, 1H), 6.84 - 6.66 (m, 1H), 6.52 (s, 1H), 4.70 - 4.52 (m, 2H), 4.40 - 4.26 (m, 2H), 4.16 - 4.08 (m, 1H), 3.79 - 3.70 (m, 1H), 3.65 - 3.54 (m, 2H), 3.40 - 3.33 (m, 2H), 3.20 - 3.13 (m, 1H), 2.88 - 2.81 (m, 1H), 2.76 - 2.67 (m, 1H), 2.63 - 2.54 (m, 1H), 2.26 - 2.11 (m, 4H), 2.06 - 1.85 (m, 5H), 1.80 - 1.52 (m, 4H). $^{19}$F NMR (376 MHz, $CD_3OD$): δ -55.73 (3F), -133.28 (1F), -141.84 (1F).

**Example 13: Synthesis of Compound 13**

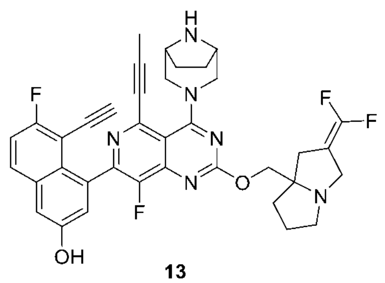

**13**

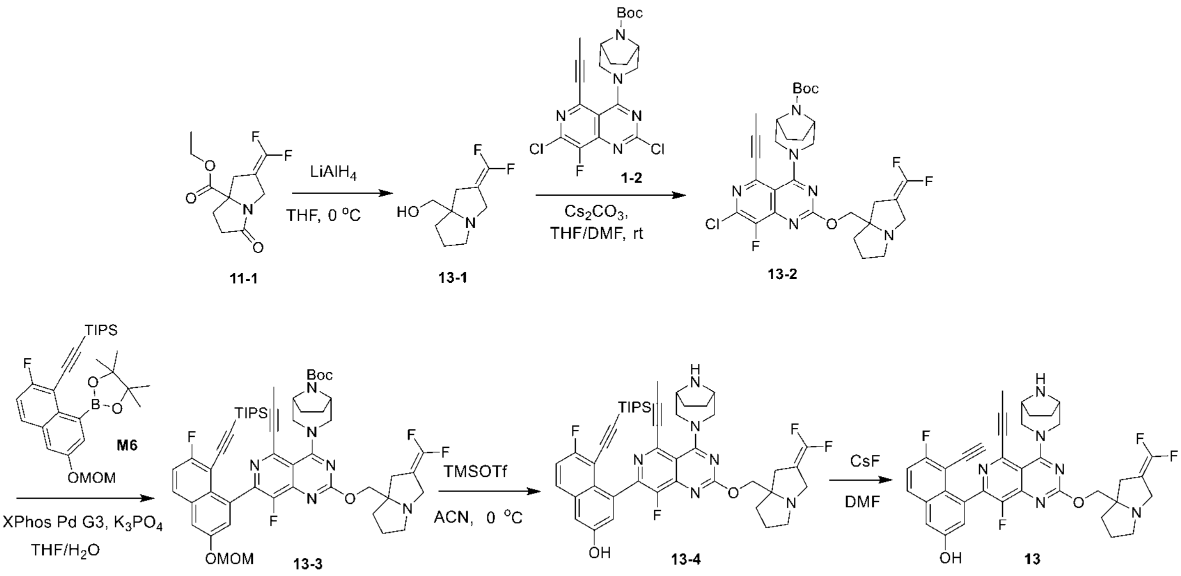


11-1 13-1 13-2 13-3 13-4 13

Step 1: Synthesis of Compound **13-1**

Lithium aluminum tetrahydride (1.24 g, 32.64 mmol) was added to a 100 mL two-necked flask at room temperature. After the mixture was purged with nitrogen three times, anhydrous THF (20 mL) was added, the temperature was lowered to 0 °C, and **11-1** (2 g, 8.16 mmol, 20 mL of THF solution) was added dropwise. After the addition was complete, the reaction was carried out at 0 °C for 6 h. The reaction was stopped, the temperature was lowered to 0 °C, and water (1.3 mL) was slowly added to the mixute. Then, 15% sodium hydroxide solution (1.3 mL) was slowly added dropwise, and then water (1.3 mL) was slowly added. A small amount of anhydrous sodium sulfate was added to dry the mixture, and the filter cake was rinsed with EA (20 mL). The filtrate was evaporated to dryness to obtain 1.4 g of yellow oily compound **13-1** with a yield of 45%. LC-MS (ESI, pos. ion) m/z: 190.0 $[M+H]^+$.

Step 2: Synthesis of Compound **13- 2**

Into a 100 mL single-necked flask were added **1-2** (1.0 g, 2.14 mmol), **13-1** (1.4 g, 3.70 mmol), cesium carbonate (3.49 g, 10.70 mmol), triethylamine (0.06 g, 0.54 mmol), anhydrous THF (5 mL), and anhydrous DMF (5 mL) at room temperature. The mixture was purged with nitrogen three times and the reaction was carried out at room temperature for 1.5 h. The reaction was stopped, and water (30 mL) and EA (30 mL) were added to the mixture, followed by adding saturated sodium chloride (20 mL). The organic phase was separated and washed with saturated sodium chloride (20 mL × 2). The organic phase was dried over anhydrous sodium sulfate, concentrated, and purified by column chromatography using DCM/EA (v/v = 1/1) as the eluent to obtain a yellow-white solid compound. The solid was slurried with EA (4 mL) for 10 minutes, and the solid was removed by filtration. The filtrate was spin-dried to obtain 0.15 g of brown solid compound **13-2** with a yield of 11%. LC-MS (ESI, pos. ion) m/z: 619.3 $[M+H]^+$.

Step 3: Synthesis of Compound **13- 3**

Into a 50 mL single-necked flask were added compound **11-3** (150 mg, 0.24 mmol), **M6** (0.25 g, 0.48 mmol), $K_3PO_4$-$7H_2O$ (320 mg, 0.96 mmol) and THF/$H_2O$ (4 mL/0.3 mL). The mixture was purged with nitrogen three times, and Xphos Pd G3 (61 mg, 0.072 mmol) was added portionwise. The mixture was stirred at 35 °C for 6 h. The reaction was stopped and to the mixture was added saturated ammonium chloride solution (10 mL). The mixture was extracted with EA (20 mL × 2). The combined organic phases were dried over anhydrous sodium sulfate and concentrated. The residue was purified by column chromatography using DCM/EA (v/v = 1/1) as eluent to obtain 100 mg of a tan solid compound **13-3** with a yield of 42%. LC-MS (ESI, pos. ion) m/z: 969.4 $[M+H]^+$.

Step 4: Synthesis of Compound **13-4**

Into a 50 mL single-necked flask were added compound **13-3** (100 mg, 0.10 mmol) and acetonitrile (2 mL). The mixture was cooled to 0 °C and stirred for 5 minutes. TMSOTf (0.11 mL, 0.50 mmol) was added dropwise. After stirring for 1.5 h, the reaction was stopped and quenched with saturated aqueous sodium bicarbonate solution (10 mL). The mixture

was extracted with EA (10 mL × 2). The combined organic phases were dried over anhydrous sodium sulfate and concentrated to obtain 140 mg of a yellow solid compound **13-4,** the yield was calculated as 100%, LC-MS (ESI, pos. ion) m/z: 825.3 [M+H]+.

Step 5: Synthesis of Compound **13**

Into a 25 mL single-necked flask were added **13-4** (85 mg, 0.10 mmol), cesium fluoride (91 mg, 0.60 mmol) and DMF (1 mL). The mixture was purged with nitrogen three times and stirred at room temperature for 16 h. The reaction was stopped, and water (80 mL) was added, precipitating a solid. Filtration afforded the crude solid. The crude product was purified by thin-layer chromatography using DCM/MeOH (v/v = 9/1) as eluent to obtain 10 mg of an off-white solid compound **11** with a yield of 13%. LC-MS (ESI, pos. ion) m/z: 669.2 [M+H]+; $^1$H NMR (400 MHz, MeOD) δ 7.92 - 7.83 (m, 1H), 7.39 - 7.31 (m, 2H), 7.26 - 7.19 (m, 1H), 4.42 - 4.27 (m, 2H), 3.89 - 3.74 (m, 2H), 3.71 - 3.55 (m, 3H), 3.50 - 3.41 (m, 1H), 3.30 (s, 1H), 3.21 - 3.13 (m, 1H), 2.87 - 2.67 (m, 2H), 2.60 - 2.47 (m, 1H), 2.22 (s, 3H), 2.16 - 1.87 (m, 5H), 1.86 - 1.56 (m, 5H). $^{19}$F NMR (376 MHz, $CD_3OD$): δ -92.80 (2F), -111.50 (1F), -140.14 (1F).

**Example 14: Synthesis of Compound 14**

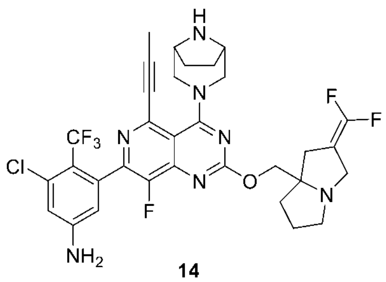

14

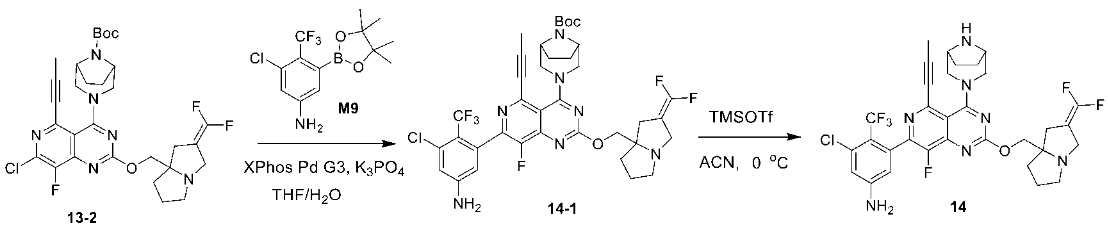


13-2 14-1 14

The synthesis of compound **14** was carried out according to the synthesis steps of compound **13,** except that **M9** was used to replace **M6,** and finally 12 mg of light yellow solid compound **14** was obtained. LC-MS (ESI, pos. ion) m/z: 678.3 [M+H]+.

**Example 15: Synthesis of Compound 15**

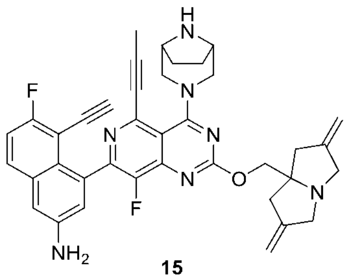

15

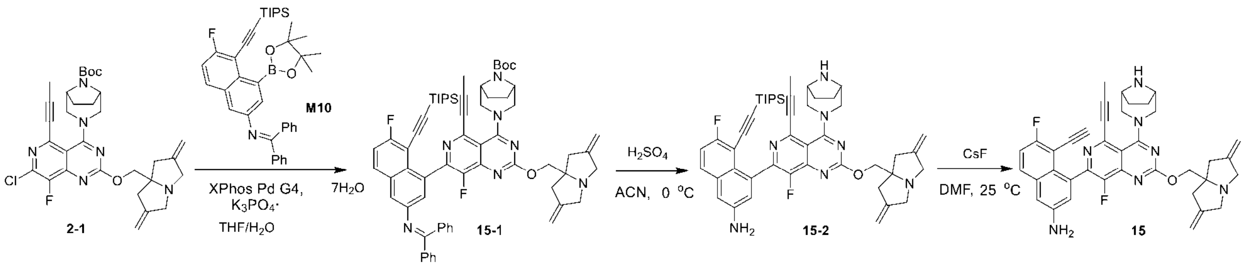


Step 1: Synthesis of Compound **15-1**

Into a 50 mL single-necked flask were added compound **2-1** (150 mg, 0.25 mmol), **M10** (190 mg, 0.30 mmol, synthesized according to the synthesis method of **001-5D** on pages 30-31 of patent WO2023138662), Xphos Pd G4 (22 mg, 0.025 mmol), $K_3PO_4\cdot 7H_2O$ (250 mg, 0.75 mmol) and THF/$H_2O$ (v/v = 6 mL/2 mL). The mixture was purged with nitrogen three times. The mixture was stirred for 16 h at room temperature. The reaction was added saturated ammonium chloride solution (20 mL). The mixture was extracted with EA (10 mL × 2). The combined organic phases were dried over anhydrous sodium sulfate and concentrated. The residue was purified by column chromatography using DCM/MeOH (v/v = 50/1-30/1) as eluent to obtain 60 mg of orange solid compound **15-1** with a yield of 22%. LC-MS (ESI, pos. ion) m/z: 1065.4 $[M+H]^+$.

Step 2: Synthesis of Compound **15- 2**

Into a 50 mL single-necked flask were added compound **15-1** (60 mg, 0.056 mmol) and acetonitrile (2 mL). The mixture was cooled to 0 °C and stirred for 5 minutes. Concentrated sulfuric acid (0.054 g, 0.56 mmol) was added dropwise. After stirring for 120 min, the reaction was stopped and quenched with saturated aqueous sodium bicarbonate solution (10 mL). The mixture was extracted with DCM (10 mL × 2). The combined organic phases were dried over anhydrous sodium sulfate and concentrated to obtain 51 mg of a yellow solid compound **15-2,** which was used directly in the next step. The yield was calculated as 100%, LC-MS (ESI, pos. ion) m/z: 800.3 $[M+H]^+$.

Step 3: Synthesis of Compound **15**

Into a 25 mL single-necked flask were added **15-2** (45 mg, 0.056 mmol), cesium fluoride (68 mg, 0.45 mmol) and DMF (1 mL). The mixture was purged with nitrogen three times and stirred at room temperature for 14 h. The reaction was stopped, and water (10 mL) was added, precipitating a solid. Filtration afforded the crude solid. The crude product was purified by thin-layer chromatography using DCM/MeOH (v/v = 12/1) as eluent to obtain 10 mg of a brown solid compound **15** with a yield of 28%. LC-MS (ESI, pos. ion) m/z: 644.4 $[M+H]^+$; $^1H$ NMR (400 MHz, $CD_3OD$) δ 7.83 - 7.68 (m, 1H), 7.33 - 7.08 (m, 3H), 5.12 - 4.98 (m, 4H), 4.64 - 4.52 (m, 6H), 4.38 (s, 2H), 3.88 - 3.77 (m, 2H), 3.74 - 3.64 (m, 2H), 3.43 - 3.35 (m, 2H), 3.25 (s, 1H), 2.89 - 2.75 (m, 2H), 2.67 - 2.53 (m, 2H), 2.22 (s, 3H), 2.12 - 1.82 (m, 4H). $^{19}F$ NMR (376 MHz, $CD_3OD$) δ -113.31 (1F), - 140.06(1F).

**Example 16: Synthesis of Compound 16**

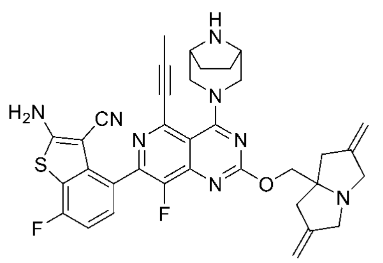

**16**

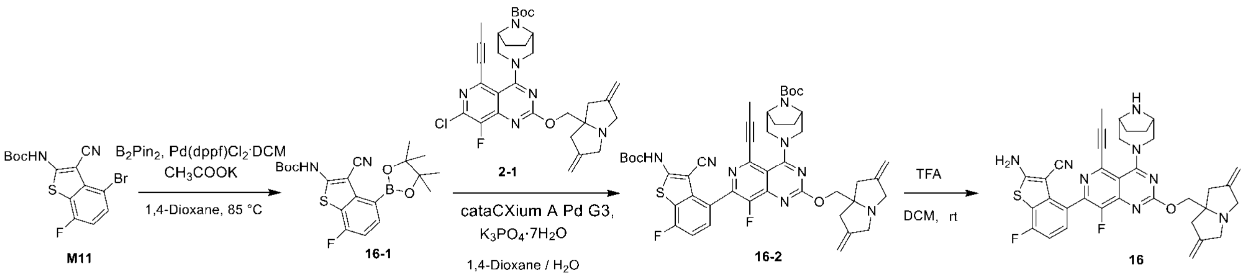


M11 16-1 2-1 16-2 16

Step 1: Synthesis of Compound **16-1**

Into a 100 mL single-necked flask were added **M11** (3 g, 8.08 mmol, purchased from Shaoyuan Technology), bis(pinacolato)diborane (3.08 g, 12.12 mmol), Pd(dppf)$Cl_2$· DCM (0.79 g, 0.97 mmol), potassium acetate (2.38 g, 24.24 mmol) and 1,4-dioxane (100 mL). After nitrogen substitution three times, the mixture was heated to 85 °C and stirred for 16 h to terminate the reaction. The reaction solution was filtered through celite pad and concentrated. The crude product was purified by column chromatography using PE/DCM (v/v = 10/1-2/1) as the eluent to obtain 2.83 g of a white solid compound **16-1** with a yield of 84%. LC-MS (ESI, pos. ion) m/z:417.2$[M+H]^+$.

Step 2: Synthesis of Compound **16-2**

Into a 50 mL single-necked flask were added compound **2-1** (110 mg, 0.18 mmol), 16-1 (110 mg, 0.27 mmol), potassium phosphate heptahydrate (180 mg, 0.54 mmol), 1,4-dioxane/$H_2O$ (4 mL/1 mL) and cataCXium A Pd G3 (13 mg, 0.018 mmol). The mixture was stirred at 60 °C for 14 h under nitrogen protection. The reaction was stopped and saturated ammonium chloride solution (10 mL) was added to the mixture. The mixture was extracted with EA (20 mL × 2). The organic phases were combined, dried over anhydrous sodium sulfate, concentrated, and purified by thin-layer chromatography using DCM/MeOH (v/v = 40/1) as the eluent to obtain 115 mg of yellow solid compound **16-2** with a yield of 73%. LC-MS (ESI, pos. ion) m/z: 851.3 $[M+H]^+$.

Step 3: Synthesis of Compound **16**

Into a 50 mL single-necked flask were added compound **16-2** (100 mg, 0.118 mmol) and DCM (1 mL), the reaction was cooled to 0 °C and stirred for 5 minutes, trifluoroacetic acid (1 mL) was slowly added dropwise, and stirred at room temperature for 4 h to stop the reaction. Saturated aqueous sodium bicarbonate solution (15 mL) was slowly added to quench the reaction, and the mixture was extracted with DCM/MeOH (15/1 mL). The organic phase was dried over anhydrous sodium sulfate, concentrated, and purified by thin-layer chromatography using DCM/MeOH (v/v = 9/1) as the eluent to obtain 10 mg of a gray solid compound **16** with a yield of 13%. LC-MS (ESI, pos. ion) m/z: 651.3 $[M+H]^+$; $^1H$ NMR (400 MHz, $CD_3OD$) δ 7.47 (dd, *J*= 8.4, 5.1 Hz, 1H), 7.06 (dd, *J*= 9.5, 8.4 Hz, 1H), 5.09 - 5.01 (m, 4H), 4.65 - 4.53 (m, 4H), 4.36 (s, 2H), 3.97 - 3.69 (m, 4H), 3.66 - 3.54 (m, 2H), 3.43 - 3.35 (m, 2H), 2.87 - 2.74 (m, 2H), 2.67 - 2.55 (m, 2H), 2.21 (s, 3H), 1.80 - 1.56 (m, 4H). $^{19}F$ NMR (376 MHz, $CD_3OD$): δ -118.00 (1F), -141.85 (1F).

**Example 17: Synthesis of Compound 17**

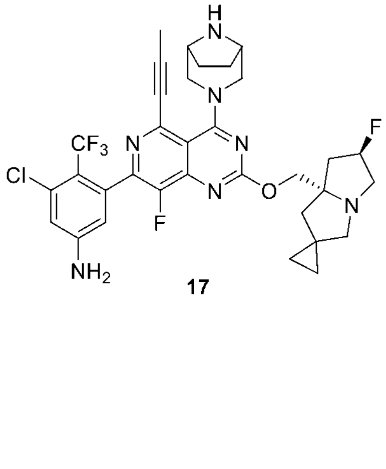

**17**

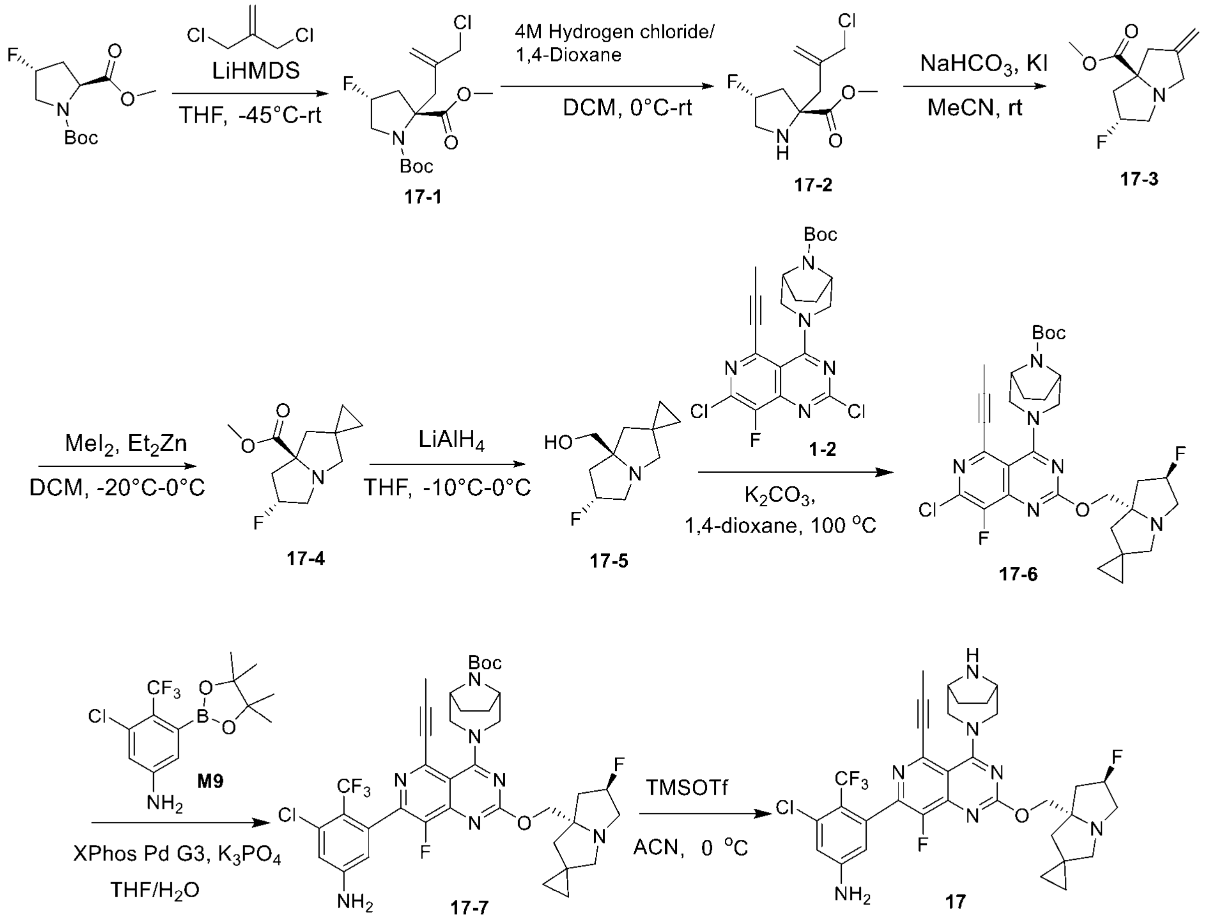


Step 1: Synthesis of Compound **17-1**

Into a 250 mL three-necked flask was added *N*-Boc-*trans*-4-fluoro-L-proline methyl ester (5.00 g, 20.2 mmol, Shanghai Bide Pharmatech). The mixture was cooled to -45 °C and stirred for 10 minutes, followed by slow dropwise addition of LiHMDS (30.33 mL, 30.33 mmol, 1 M in THF). The reaction mixture was maintained at -45 °C for 60 minutes, then a solution of 3-chloro-2-(chloromethyl)prop-1-ene (3.03 g, 24.26 mmol) in anhydrous THF (15 mL) was added dropwise slowly. Upon completion of addition, the mixture was warmed gradually to room temperature (about 1 h). Stirring was discontinued, and the reaction was quenched with saturated ammonium chloride solution (20 mL). The mixture was separated into layers, and the aqueous phase was extracted with ethyl acetate (50 mL × 2). The combined organic phases were dried over anhydrous sodium sulfate and concentrated. The residue was purified by column chromatography using PE/EA (v/v = 20/1-5/1) as eluent to obtain 6.06 g of a yellow oil compound **17-1** with a yield of 89%. LC-MS (ESI, pos. ion) m/z: 236.0 $[M\text{-}Boc+H+H]^+$.

Step 2: Synthesis of Compound **17-2**

Into a 250 mL single-necked flask were added compound **17-1** (6.06 g, 18.05 mmol) and DCM (100 mL). The mixture was cooled to 0 °C and stirred for 5 minutes. A solution of hydrogen chloride in dioxane (22.56 mL, 90.25 mmol, 4 M) was added dropwise. After completion of the addition, the reaction mixture was allowed to warm to room temperature and stirred for 14.5 h. Stirring was stopped, and the reaction solution was directly concentrated and drained to obtain **17-2,** which was directly used in the next step. The yield was calculated as 100%. LC-MS (ESI, pos. ion) m/z: 236.0 $[M+H]^+$.

Step 3: Synthesis of Compound **17-3**

Into a 250 mL single-necked flask were added **17-2** (4.25 g, 18.03 mmol), acetonitrile (50 mL), sodium bicarbonate (7.57 g, 90.15 mmol) and potassium iodide (0.30g, 1.80 mmol). The mixture was stirred at room temperture for 2 h. Stirring was stopped, and the reaction mixture was filtered, the filter cake was washed with acetonitrile (50 mL). The filtrate was concentrated, and the residue was purified by column chromatography using PE/EA (v/v = 4/1-3/2) as eluent to

obtain 3.00 g of a yellow oil compound **17-3** with a yield 83.5%. LC-MS (ESI, pos. ion) m/z: 200.1 $[M+H]^+$. $^1H$ NMR (400 MHz, $CDCl_3$) δ 5.29 - 5.12 (m, 1H), 4.95 (d, *J*= 13.7 Hz, 2H), 3.87 (d, *J*= 14.8 Hz, 1H), 3.75 (s, 3H), 3.59 - 3.40 (m, 1H), 3.27 - 3.16 (m, 1H), 2.98 - 2.86 (m, 2H), 2.85 - 2.73 (m, 1H), 2.57 - 2.48 (m, 1H), 2.21 - 1.93 (m, 1H).

Step 4: Synthesis of Compound **17-4**

Into a 100 mL single-necked flask was added compound **17-3** (400 mg, 2.01 mmol). The flask was purged with nitrogen three times. Anhydrous DCM (12 mL) was added, and the mixture was cooled to -20 °C and stirred for 10 minutes. Diethylzinc solution (10.05 mL, 10.05 mmol, 1 M in n-hexane) was added dropwise slowly., the mixture was stirring for 30 minutes. Diiodomethane (1.62 mL, 20.10 mmol) was slowly added dropwise, then the reaction mixture was transferred to 0 °C and stirred for 3 h. The reaction mixture was quenched with saturated aqueous ammonium chloride (20 mL). The aqueous phase was extracted with ethyl acetate (20 mL × 2). The combined organic layers were dried over anhydrous sodium sulfate, concentrated. The residue was purified by column chromatography using PE/EA (v/v = 7/3) as eluent to obtain 223 mg of a yellow oil compound **17-4** with a yield 52%. LC-MS (ESI, pos. ion) m/z: 214.4 $[M+H]^+$; $^1H$ NMR (400 MHz, $CDCl_3$) δ 5.37 - 5.16 (m, 1H), 3.77 (s, 3H), 3.68 - 3.52 (m, 1H), 3.19 (d, *J*= 10.6 Hz, 1H), 3.10 - 2.92 (m, 1H), 2.91 - 2.76 (m, 1H), 2.63 (d, *J= 10.6* Hz, 1H), 2.25 - 2.02 (m, 2H), 1.89 (d, *J* = 12.9 Hz, 1H), 0.66 - 0.42 (m, 4H).

Step 5: Synthesis of Compound **17-5**

Into a 100 mL two-necked flask was added $LiAlH_4$ (0.79 g, 2.08 mmol). The mixture was purged with nitrogen three times and cooled to -10 °C. THF (2 mL) was added and stirred for 5 min. Compound **17-4** (221 mg, 1.04 mmol) dissolved in THF (3 mL) was slowly added dropwise. After the addition was complete, the mixture was heated to 0 °C and stirred for 3.5 h. The mixture was quenched by slow addition of sodium sulfate decahydrate. The suspension was filtered through a celite pad, concentrated, and placed under high vacuum to obtain 162 mg of a light yellow oil compound **17-5** with a yield of 84%. LC-MS (ESI, pos. ion) m/z: 186.2 $[M+H]^+$.

Step 6: Synthesis of Compound **17- 6**

Into a 50 mL single-necked flask were added compound **1-2** (950 mg, 2.04 mmol), **17-5** (302.3 mg, 1.63 mmol), potassium carbonate (0.85 g, 6.12 mmol) and anhydrous 1,4-dioxane (15 mL). The mixture was purged with nitrogen three times, heated to 100 °C and stirred for 8 h. The reaction was stopped and cooled to room temperature. The reaction solution was concentrated, diluted with EA (20 mL), filtered, concentrated, and purified by column chromatography using DCM/MeOH (v/v = 50/1) as the eluent to obtain 160 mg of a red solid compound **17-6** with a yield of 13%. LC-MS (ESI, pos. ion) m/z: 615.3 $[M+H]^+$.

Step 7: Synthesis of Compound **17-7**

Into a 25 mL single-necked flask were added compound **17-6** (80 mg, 0.13 mmol), **M9** (63 mg, 0.20 mmol), Xphos Pd G3 (22 mg, 0.026 mmol), $K_3PO_4 \cdot 7H_2O$ (130 mg, 0.39 mmol) and THF/$H_2O$ (v/v = 2.5 mL/0.8 mL). The mixture was purged with nitrogen three times and stirred at room temperature for 1 h. Additional XPhos Pd G3 (22 mg, 0.026 mmol) was added, and stirring was continued at room temperature for 8 h. The reaction was quenched and diluted with DCM (15 mL). The mixture was washed with saturated ammonium chloride solution (15 mL) and extracted with DCM (15 mL × 2). The combined organic phases were dried over anhydrous sodium sulfate and concentrated. The residue was purified by thin-layer chromatography using DCM/MeOH (v/v = 40/1) as eluent to obtain 51 mg of a red solid compound **17-7** with a yield of 51%. LC-MS (ESI, pos. ion) m/z: 774.2 $[M+H]^+$.

Step 8: Synthesis of Compound **17**

Into a 25 mL single-necked flask were added compound **17-7** (51 mg, 0.066 mmol) and acetonitrile (2 mL). The mixture was cooled to 0 °C and stirred for 5 minutes. TMSOTf (0.024 mL, 0.13 mmol) was added dropwise and stirred for 60 minutes at 0 °C, the reaction was stopped and the mixture was quenched with saturated aqueous sodium bicarbonate solution (15 mL). The mixture was extracted with DCM (20 mL × 2). The combined organic phases were dried over anhydrous sodium sulfate, concentrated, and drained. The crude product was purified by thin-layer chromatography using DCM/MeOH (v/v = 10/1) as the eluent to obtain 28 mg of a yellow solid compound **17** with a yield of 63%. LC-MS (ESI, pos. ion) m/z: 674.2 $[M+H]^+$; HRMS (ESI): 674.2425 $[M+H]^+$; $^1H$ NMR (400 MHz, $CD_3OD$) δ 6.96 - 6.84 (m, 1H), 6.53 (s, 1H), 5.50 - 5.32 (m, 1H), 4.66 - 4.54 (m, 5H), 4.47 (s, 2H), 3.65 - 3.42 (m, 3H), 3.20 - 3.00 (m, 2H), 2.77 - 2.65 (m, 1H), 2.67 - 2.47 (m, 1H), 2.22 (s, 3H), 2.19 - 2.02 (m, 2H), 1.93 - 1.80 (m, 2H), 1.79 - 1.49 (m, 4H), 0.71 - 0.49 (m, 4H). $^{19}F$ NMR (376 MHz, $CD_3OD$) δ - 55.73 (3F), -141.87 (1F), -175.37 (1F).

**Example 18: Synthesis of Compound 18**

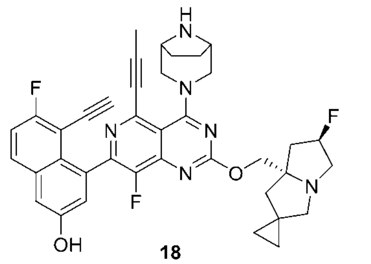

**18**

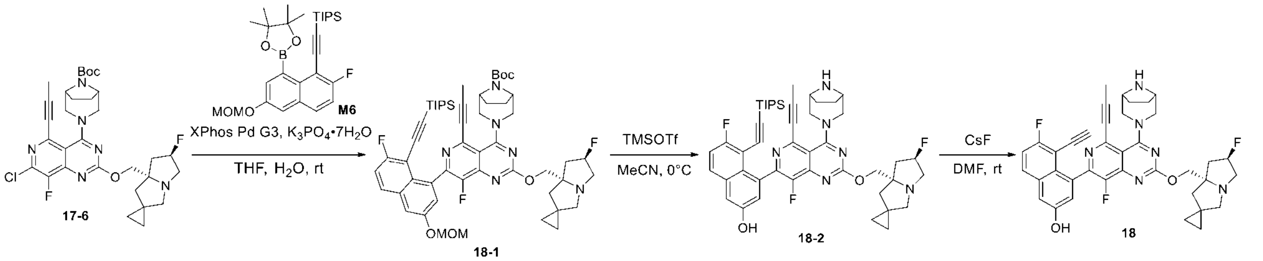


17-6 18-1 18-2 18

Step 1: Synthesis of Compound **18-1**

Into a 25 mL single-necked flask were added compound **17-6** (80 mg, 0.13 mmol), **M6** (0.13 g, 0.26 mmol), Xphos Pd G3 (22 mg, 0.026 mmol), $K_3PO_4$·$7H_2O$ (130 mg, 0.39 mmol) and THF/$H_2O$ (v/v = 2.5 mL/0.8 mL). The mixture was purged with nitrogen three times and stirred at room temperature for 1 h. Additional XPhos Pd G3 (0.2 equiv.) was added, and the resulting mixture was continued to stir at room temperature for 2 h. The reaction was stopped and the mixture diluted with DCM (15 mL). The mixture was washed with saturated ammonium chloride solution (15 mL) and extracted with DCM (15 mL × 2). The combined organic phases were dried over anhydrous sodium sulfate and concentrated. The residue was purified by thin-layer chromatography using DCM/MeOH (v/v = 50/1) as eluent to obtain 56 mg of a red oil compound **18-1** with a yield of 45%. LC-MS (ESI, pos. ion) m/z: 965.4 $[M+H]^+$.

Step 2: Synthesis of Compound **18-** 2

Into a 25 mL single-necked flask were added compound **18-1** (56 mg, 0.058 mmol) and acetonitrile (3 mL). The mixture was cooled to 0 °C and stirred for 5 min. TMSOTf (0.031 mL, 0.17 mmol) was added dropwise. After stirring at 0 °C for 2 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by thin-layer chromatography using DCM/MeOH (v/v = 9/1) as eluent to obtain 25 mg of a red solid compound **18-2** with a yield of 52%, LC-MS (ESI, pos. ion) m/z: 821.3 $[M+H]^+$.

Step 3: Synthesis of Compound **18**

Into a 25 mL single-necked flask were added compound **18-2** (25 mg, 0.076 mmol), cesium fluoride (36 mg, 0.24 mmol) and DMF (0.5 mL). The system was purged with nitrogen three times and stirred at room temperature for 13 h. The reaction was stopped, and water (10 mL) was added into the mixture, precipitating a solid. Filtration afforded the crude solid. The crude product was purified by thin-layer chromatography using DCM/MeOH (v/v = 8/1) as eluent to obtain 18 mg of a yellow solid compound **18** with a yield of 89%, LC-MS (ESI, pos. ion) m/z: 665.3 $[M+H]^+$; HRMS (ESI): 665.2824 $[M+H]^+$; $^1$H NMR (400 MHz, $CD_3OD$) δ 7.92 - 7.82 (m, 1H), 7.40 - 7.29 (m, 2H), 7.23 (s, 1H), 5.53 - 5.33 (m, 1H), 4.70 - 4.54 (m, 4H), 4.53 - 4.42 (m, 2H), 3.67 - 3.56 (m, 2H), 3.55 - 3.45 (m, 1H), 3.21 - 3.01 (m, 2H), 2.78 - 2.69 (m, 1H), 2.67 - 2.47 (m, 1H), 2.21 (s, 3H), 2.20 - 2.01 (m, 2H), 1.92 - 1.55 (m, 6H), 0.70 - 0.56 (m, 4H). $^{19}$F NMR (376 MHz, $CD_3OD$): δ -111.53 (1F), -140.24 (1F), -175.34 (1F).

Examples **19-24** (i.e., compounds **19-24)** were prepared with reference to the method of example 2 and the methodfor constructing the core in WO2023061463; Examples **25-37** and **Examples 40-42** (i.e., compounds **25-37, 40-42)** were prepared with reference to **M4** and the synthesis method of the right fragment in Example **11;** Examples **38** and **39** were prepared with reference to the synthesis method of Example **18;** it should be noted that, in the synthesis

process of Examples **19-42,** while reference to the synthesis method of the target compound , it also included a process in which technicians made appropriate or necessary adjustments to the synthesis method based on conventional technical knowledge in the field. The structures and characterization data of Examples **19-42** are shown in the following table.

| **Example Compound and Characterization Data** | **Example Compound and Characterization Data** |
|---|---|
| **Example 19**<br>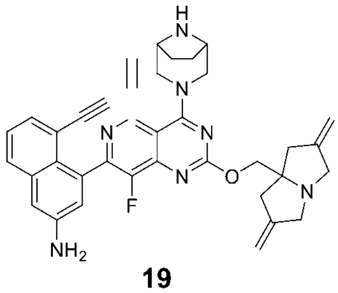<br>**19**<br>LC-MS (ESI, pos. ion) m/z:626.2 [M+H]$^+$. | **Example 20**<br>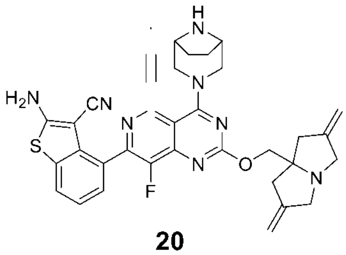<br>**20**<br>LC-MS (ESI, pos. ion) m/z:633.0 [M+H]$^+$. |
| **Example 21**<br>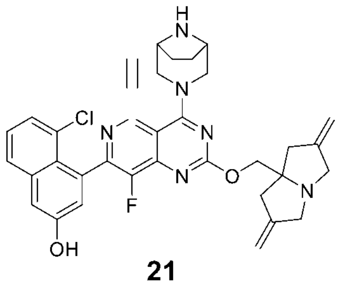<br>**21**<br>LC-MS (ESI, pos. ion) m/z:637.2 [M+H]$^+$. | **Example 22**<br>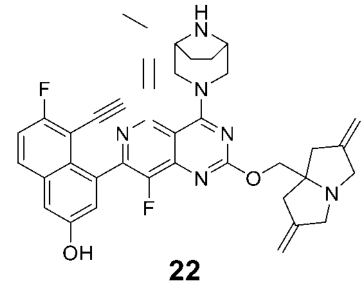<br>**22**<br>LC-MS (ESI, pos. ion) m/z:659.5 [M+H$^+$. |
| **Example 23**<br>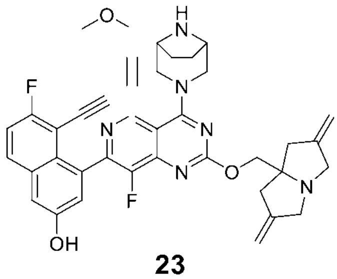<br>**23**<br>LC-MS (ESI, pos. ion) m/z:675.4 [M+H]$^+$. | **Example 24**<br>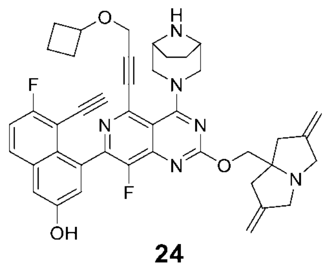<br>**24**<br>LC-MS (ESI, pos. ion) m/z:715.1 [M+H]$^+$. |
| **Example 25**<br>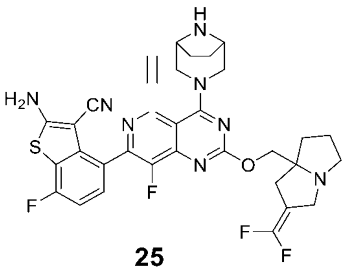<br>**25**<br>LC-MS (ESI, pos. ion) m/z:675.5 [M+H]$^+$. | **Example 26**<br>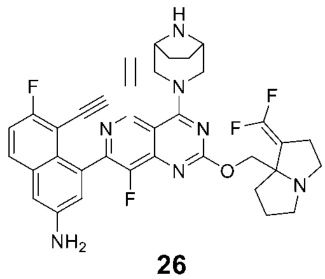<br>**26**<br>LC-MS (ESI, pos. ion) m/z: 668.2 [M+H]$^+$. |
| **Example 27** | **Example 28** |

(continued)

| Example Compound and Characterization Data | Example Compound and Characterization Data |
|---|---|
| 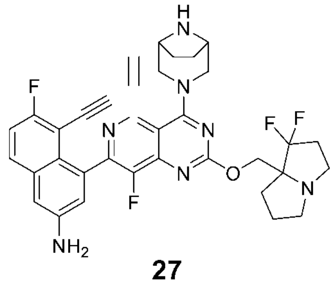<br>**27**<br>LC-MS (ESI, pos. ion) m/z: 656.2 [M+H]+. | 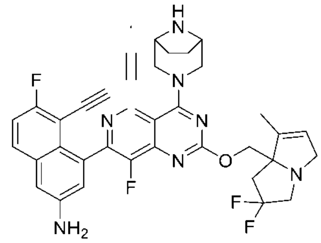<br>**28**<br>LC-MS (ESI, pos. ion) m/z: 668.5 [M+H]+. |
| **Example 29**<br>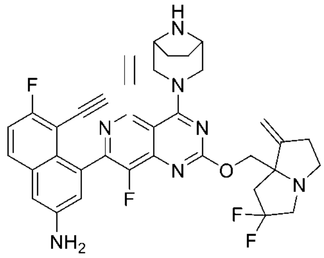<br>**29**<br>LC-MS (ESI, pos. ion) m/z: 668.5 [M+H]+. | **Example 30**<br>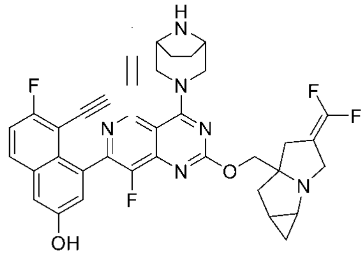<br>**30**<br>LC-MS (ESI, pos. ion) m/z: 681.0 [M+H]+. |
| **Example 31**<br>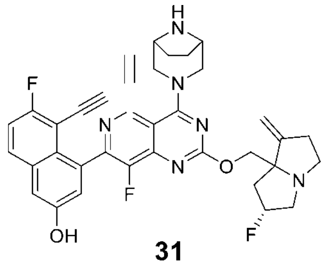<br>**31**<br>LC-MS (ESI, pos. ion) m/z: 651.3 [M+H]+. | **Example 32**<br>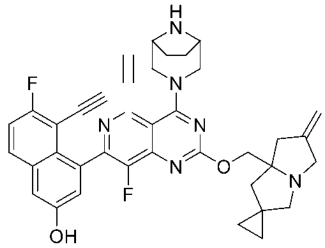<br>**32**<br>LC-MS (ESI, pos. ion) m/z: 659.1 [M+H]+. |
| **Example 33**<br>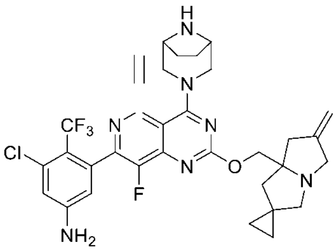<br>**33**<br>LC-MS (ESI, pos. ion) m/z: 668.3 [M+H]+. | **Example 34**<br>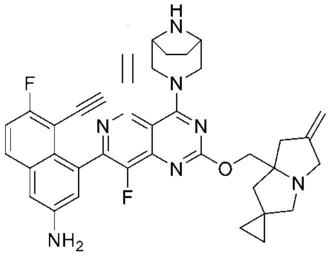<br>**34**<br>LC-MS (ESI, pos. ion) m/z: 658.2 [M+H]+. |
| **Example 35** | **Example 36** |

(continued)

| Example Compound and Characterization Data | Example Compound and Characterization Data |
|---|---|
| 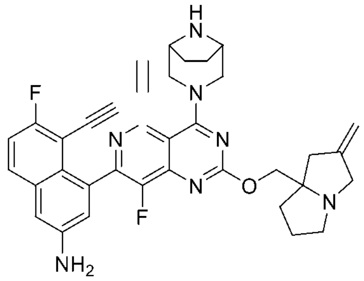 **35** LC-MS (ESI, pos. ion) m/z: 632.3 [M+H]$^+$. | 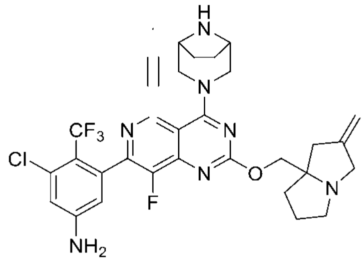 **36** LC-MS (ESI, pos. ion) m/z: 642.1 [M+H]$^+$. |
| **Example 37** 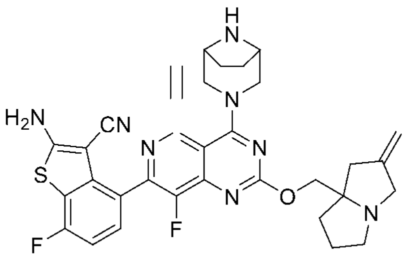 **37** LC-MS (ESI, pos. ion) m/z: 639.0 [M+H]$^+$. | **Example 38** 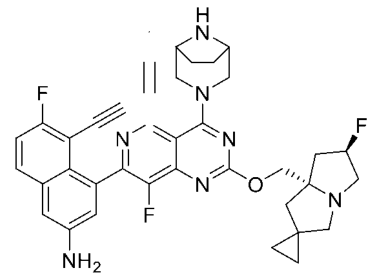 **38** LC-MS (ESI, pos. ion) m/z: 664.2 [M+H]$^+$. |
| **Example 39** 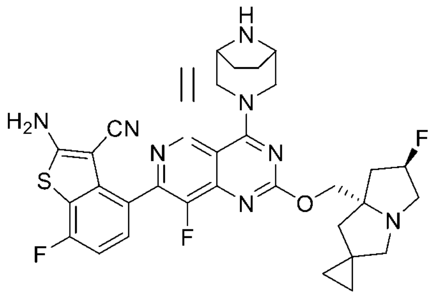 **39** LC-MS (ESI, pos. ion) m/z: 671.2 [M+H]$^+$. | **Example 40** 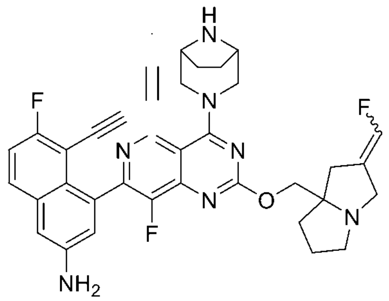 **40** LC-MS (ESI, pos. ion) m/z: 650.4 [M+H]$^+$. |
| **Example 41** 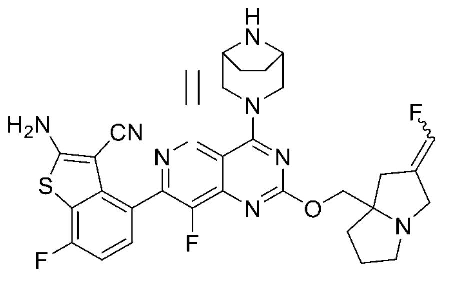 **41** LC-MS (ESI, pos. ion) m/z: 657.2 [M+H]$^+$. | **Example 42** 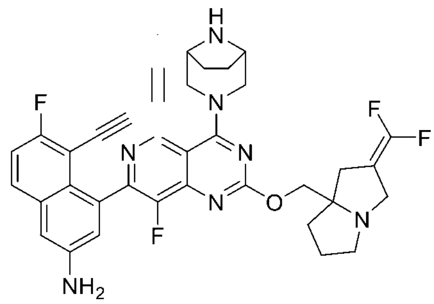 **42** LC-MS (ESI, pos. ion) m/z: 668.3 [M+H]$^+$. |

**Biological Activity Examples**

**1. KRAS G12D/cRAF Binding Assay**

Experimental steps:

(1) Compounds were prepared in DMSO and serially diluted 3-fold in DMSO.
(2) 0.1 μL of serially diluted compounds was added to a 384-well plate.
(3) 5 μL of a specific concentration of Tag2-KRASG12D&GTP was added to the 384-well plate and the mixture was centrifuged at 1000 rpm for 1 minute.
(4) 5 μL of a specific concentration of Tag1-cRAF was added to the 384-well plate and the mixture was centrifuged at 1000 rpm for 1 minute.
(5) The mixuture was incubated at 25 °C for 15 minutes.
(6) 10 μL of a mixture of anti-Tag1-Eu and anti-Tag2-XL665 was added to the 384-well plate.
(7) The mixture was centrifuged at 1000 rpm for 1 minute and incubated at 4 °C for 3 hours.
(8) The 665/615 nm ratio was read on a microplate reader.
(9) Data Analysis: GraphPad Prism 8.0 software was used to analyze the data and calculate the $IC_{50}$ values, with the logarithmic value of the compound concentration as the horizontal axis and the 665/615 nm ratio as the vertical axis.

Conclusion: The compounds of the present invention can effectively bind to KRAS G12D-GTP and inhibit the binding of KRAS to cRAF protein. Among them, the $IC_{50}$ of the compounds of the present invention against KRAS G12D-GTP/cRAFT is less than 50 nM; preferably, the $IC_{50}$ of the compounds of the present invention against KRAS G12D-GTP/cRAF is less than 20 nM.

**2. KRAS G12D/SOS1 Binding Assay**

Experimental steps:

(1) Compounds were prepared in DMSO and serially diluted 3-fold in DMSO.
(2) 0.1 μL of serially diluted compounds was added to a 384-well plate.
(3) 5 μL of a specific concentration of Tag2-KRASG12D&GTP was added to the 384-well plate and the mixture was centrifuged at 1000 rpm for 1 minute.
(4) 5 μL of a specific concentration of Tag1-SOS1 was added to the 384-well plate and the mixture was centrifuged at 1000 rpm for 1 minute.
(5) The mixture was incubated at 25 °C for 15 minutes.
(6) 10 μL of a mixture of anti-Tag1-$Tb^{3+}$ and anti-Tag2-XL665 was added to the 384-well plate.
(7) The mixture was centrifuged at 1000 rpm for 1 minute and incubated at 4 °C for 3 hours.
(8) The 665/615 nm ratio was read on a microplate reader.
(9) Data Analysis: GraphPad Prism 8.0 software was used to analyze the data and calculate the $IC_{50}$ values, with the logarithmic value of the compound concentration as the horizontal axis and the 665/615 nm ratio as the vertical axis.

Conclusion: The compounds of the present invention can effectively bind to KRAS G12D-GTP and inhibit the binding of KRAS to SOS1 protein. Among them, the $IC_{50}$ of the compounds of the present invention against KRAS G12D-GTP/SOS1 is less than 50 nM; preferably, the $IC_{50}$ of the compounds of the present invention against KRAS G12D-GTP/SOS1 is less than 20 nM.

**3. Determination of Inhibitory Effect on Intracellular pERK by In-Cell Western Assay**

Reagents Used

| Reagent | Source | Article No. |
|---|---|---|
| RPMI 1640 Medium | Gibco | A10491-01 |
| McCoy's 5A (modified) Medium | Invitrogen | 16600-108 |
| F-12K Medium | Gibco | 30-2004 |
| Fetal bovine serum (FBS) | Ausgenex | FBS500-S |

(continued)

| Reagent | Source | Article No. |
|---|---|---|
| DMEM Medium | Gibco | 11995065 |
| TrypLE™ Express Enzyme (1X), no phenol red | Gibco | 12604-021 |
| Penicillin-streptomycin | Gibco | 15140-122 |
| Blocking buffer: Odyssey Blocking Buffer (500mL) | LI-COR | 927-40000 |
| Secondary antibody: IRDye 800CW Goat anti-Rabbit IgG (H+L) (0.5 mg) | LI-COR | 926-32211 |
| Secondary antibody: IRDye 680RD Goat anti Mouse IgG (H+L) (0.5mg) | LI-COR | 926-68070 |
| Primary antibody: phospho-ERK Rabbit mAb | CST | 4370S |
| Primary antibody: GAPDH(D4C6R) Mouse mAb | CST | 97166S |
| Fixative solution: 8% fixative solution | Solarbio | P1112 |
| Dulbecco's Phosphate Buffered Saline (DPBS) | Gibco | 14190-144 |
| phosphate buffered saline(PBS) | Solarbio | P1010 |
| Tween-20 | Solarbio | T8220 |

Experimental steps:

(1) On the first day, the following cells were revived with the corresponding culture medium and incubated in a 5% $CO_2$ incubator at 37 °C until confluence:

| Cells | Medium | Cell number in 384-well plate |
|---|---|---|
| H358 | RPMI 1640 Medium+10% Fetal bovine serum+1% Penicillin-streptomycin | 5000~8000 |
| MKN1 | RPMI 1640 Medium+10% Fetal bovine serum+1% Penicillin-streptomycin | |
| HPAC | DMEM Medium+10% Fetal bovine serum+1% Penicillin-streptomycin | |
| A549 | F12K Medium+10% Fetal bovine serum+1% Penicillin-streptomycin | |
| PSN1 | RPMI 1640 Medium+10% Fetal bovine serum+1% Penicillin-streptomycin | |
| AGS | F-12K Medium+10% Fetal bovine serum+1% Penicillin-streptomycin | |
| HCT116 | McCoy's 5A (modified) Medium+10% +1% Penicillin-streptomycin | |
| NCI-H460 | RPMI 1640 Medium+10% Fetal bovine serum+1% Penicillin-streptomycin | |
| NCI-H727 | RPMI 1640 Medium+10% Fetal bovine serum+1% Penicillin-streptomycin | |

(2) On the third day, the culture medium was removed, the cells were rinsed once with DPBS, 2 mL of TrypLE™ Express Enzyme was added for digestion, and the cells were placed at room temperature until the cells detached.
(3) 5 mL of fresh culture medium was added to resuspend the cells to terminate the digestion, and the cells were gently pipetted and centrifuged at 1000 rpm for 5 minutes at room temperature.
(4) The supernatant was removed, and 5 mL of new culture medium was added to resuspend the cells and count them.
(5) The cell concentration was adjusted to an appropriate level using the corresponding cell culture medium, and the cells were plated in a 384-well plate and incubated in a 5% $CO_2$ incubator at 37 °C overnight.
(6) 200 nL of the prepared serial dilution compound (DMSO final concentration was 0.5%) was added and the cells were incubated in a 5% $CO_2$ incubator at 37 °C for 3 h (HPAC cell lines only needed to be incubated in the incubator for 1 h).
(7) The cells were fixed.
(8) The cells were washed twice with PBS.
(9) The blocking solution was added and the cells were incubated at room temperature for 1 h.
(10) The blocking solution was removed, and the primary antibody (phospho-ERK Rabbit mAb + GAPDH (D4C6R) Mouse mAb) was added and the cells were incubated at 4 °C overnight.

(11) The cells were washed twice with PBST (PBS + 0.05% Tween 20) for 2 minutes each time.
(12) PBST was removed and secondary antibody (IRDye 800CW Goat anti-Rabbit IgG (H+L) + IRDye 680RD Goat anti Mouse IgG (H+L)) was added at room temperature, protected from light.
(14) secondary antibody was dicareded and the mixture was wash twice with PBST, 2 minutes each time.
(15) The 384-well plate was inverted, centrifuged at 1000 rpm at room temperature for 1 minute, and was scanned with Odyssey CLx.
(16) Data analysis:

a) Assay stability was checked using DMSO and reference control data:

Relative signal value = pERK (signal value of 800 channel) / GAPDH (signal value of 700 channel) Inhibition rate % = 100-(Signal value of each sample-Relative signal value of quality control sample)/(Signal value of DMSO well-Relative signal value of quality control sample) × 100

b) Compound $IC_{50}$ nonlinear fitting:

$$Y=Bottom + (Top-Bottom) / (1+10^{\wedge} ((LogIC_{50}-X) \times HillSlope)).$$

Experimental conclusion: The compounds of the present invention can effectively inhibit the phosphorylation of ERK protein in the KRAS G12D mutant cell line AGS. The experimental test results of some compounds of the present invention are shown in Table A.

Table A Inhibitory effect of the compounds of the present invention on pERK in KRAS G12D mutant cell line AGS

| Compound No. | AGS, $IC_{50}$ (nM) |
|---|---|
| | KRAS p.G12D |
| Example 1 | 0.4 |
| Example 2 | 0.4 |
| Example 3 | 0.2 |

**4. Determination of Inhibitory Effect on Intracellular pERK by In-Cell Western Assay**

Reagents Used

| **Reagent** | **Source** | **Article No.** |
|---|---|---|
| RPMI 1640 Medium | Gibco | A10491-01 |
| Fetal bovine serum (FBS) | Gibco | 10091-141C |
| Trypsin | Gibco | 25200-072 |
| Penicillin-streptomycin | Gibco | 15140-122 |
| Blocking buffer: Odyssey Blocking Buffer (500mL) | LI-COR | 927-40000 |
| Secondary antibody: IRDye 800CW Goat anti-Rabbit IgG (H+L) (0.5 mg) | LI-COR | 926-32211 |
| Secondary antibody: IRDye 680RD Goat anti Mouse IgG (H+L) (0.5mg) | LI-COR | 926-68070 |
| Primary antibody: phospho-ERK Rabbit mAb | CST | 4370S |
| Primary antibody: α-Tubulin (DM1A) Mouse mAb | CST | 3873S |
| Fixative: 4% paraformaldehyde fixative | Beyotime | P0099 |
| Tris Buffered Saline (TBS-10X) | CST | 12498 |

Experimental steps:

(2) On the first day, the following cells were revived with the corresponding culture medium and incubated in a 5% $CO_2$ incubator at 37 °C until confluence:

| Cells | Medium | Cell number in 96-well plate |
|---|---|---|
| ASPC-1 | RPMI-1640+10% Fetal bovine serum+1% Penicillin-streptomycin | 50000/well |
| PK-59 | RPMI-1640+10% Fetal bovine serum+1% Penicillin-streptomycin | 50000/well |

(2) On the third day, the culture medium was removed, 2 mL of trypsin was added for digestion, and the cells were placed in an incubator until the cells detached.
(3) 5 mL of fresh culture medium was added to resuspend the cells to terminate the digestion, and the cells were gently pipetted and centrifuged at 1000 rpm for 5 minutes at room temperature.
(4) The supernatant was removed, and 5 mL of new culture medium was added to resuspend the cells and count them.
(5) The cells were adjusted to the appropriate cell concentration with complete culture medium and plated in a 96-well plate. 90 μL of cells were added to each well, the cells were incubated in a 5% $CO_2$ incubator at 37 °C overnight.
(6) 10 μL of the prepared serial dilution compound (DMSO final concentration was 0.5%) was added and the cells were incubated in a 5% $CO_2$ incubator at 37 °C for 2 h.
(7) The supernatant was discarded, and 200 μL of 4% paraformaldehyde fixative was added to fix the cells.
(8) The cells were washed three times with TBST.
(9) The blocking solution was added and the cells were incubated at room temperature for 1 h.
(10) The blocking solution was removed, and the primary antibody (phospho-ERK Rabbit mAb+α-Tubulin (DM1A) Mouse mAb) was added and the cells were incubated at 4 °C overnight.
(11) The cells were washed three times with 1× PBST for 5 minutes each time.
(12) PBST was removed and secondary antibody (IRDye 800CW Goat anti-Rabbit IgG (H+L) + IRDye 680RD Goat anti Mouse IgG (H+L)) was added at room temperature, the cells was protected from light.
(14) Secondary antibody was discarded and the cell was washed twice with PBST, 5 minutes each time.
(15) The 96-well plate was inverted, centrifuge at 1000 rpm at room temperature for 1 minute, and the 96-well plate was scan with Odyssey CLx.
(16) Data analysis:

c) Assay stability was checked using DMSO and reference control data:

Relative signal value = pERK (signal value of 800 channel) / GAPDH (signal value of 700 channel) Inhibition rate % = 100-(Signal value of each sample-Relative signal value of quality control product)/(Signal value of DMSO well-Relative signal value of quality control product) × 100

d) Compound $IC_{50}$ nonlinear fitting:

$$Y=Bottom + (Top-Bottom) / (1+10\^{} ((LogIC_{50}-X) \times HillSlope)).$$

Conclusion: The compounds of the present invention can effectively inhibit the phosphorylation of ERK protein in the KRAS G12D mutant cell line ASPC-1. The experimental test results of some compounds of the present invention are shown in Table B.

Table B Inhibitory effect of the compounds of the present invention on pERK in KRAS G12D mutant cell line

| Compound No. | ASPC-1, $IC_{50}$ (nM) |
|---|---|
| | KRAS p.G12D |
| Example 2 | 0.86 |

5. 3D-CTG method was used to detect the effect of compounds on the proliferation inhibition of KRAS G12D mutant cells:

Reagents Used

| Reagent | Source | Article No. |
|---|---|---|
| RPMI 1640 Medium | Gibco | A10491-01 |
| Fetal bovine serum (FBS) | Gibco | 10091-141C |
| Trypsin | Gibco | 25200-072 |
| Penicillin-streptomycin | Gibco | 15140-122 |
| CellTiter-Glo® 3D Cell Viability Assay | Promega | 9682 |

Experimental steps:

(1) On the first day, the following cells were revived with the corresponding culture medium and incubated in a 5% $CO_2$ incubator at 37 °C until confluence:

| Cells | Medium | Cell number in 96-well plate |
|---|---|---|
| ASPC-1 | RPMI-1640+10% Fetal bovine serum+1% Penicillin-streptomycin | 3000/well |
| PK-59 | RPMI-1640+10% Fetal bovine serum+1% Penicillin-streptomycin | 3000/well |
| Panc040.03 | RPMI-1640+10% Fetal bovine serum+1% Penicillin-streptomycin | 3000/well |

(2) On the second day, the cells were rinsed once with PBS, preheated trypsin was added, and the cells were placed in a 37 °C 5% $CO_2$ incubator for digestion for 3~5 minutes. The digestion was terminated by adding an appropriate amount of complete culture medium (containing 10% fetal bovine serum), and the cells were transferred to a centrifuge tube and centrifuged at 1000 rpm for 5 minutes.
(3) The cells were resuspended in complete culture medium, counted, and adjusted to the appropriate cell concentration with complete culture medium.
(4) 90 μl/well of cell suspension was added to a 96-well plate and incubated in a 5% $CO_2$ incubator at 37 °C overnight.
(5) Each well was added with 10 μl of the prepared 10× serial dilution compound (final DMSO concentration was 0.5%) and incubated in a 37°C 5% $CO_2$ incubator.
(6) After the 7th day of incubation, 50 μl of 3D CTG detection reagent was added to each well and the cells were shaken at 350 rpm for 20 minutes.
(7) Signal values were read using an enzyme meter.
(8) Data analysis:
IC50 values were fitted using the nonlinear regression equation in Graphpad software.

$$Y=Bottom + (Top-Bottom)/(1+10^{\wedge}((LogIC50-X)*HillSlope))$$

X: Log value of compound concentration
Y: Inhibition rate (% inh)

Conclusion: The compounds of the present invention have good inhibitory activity against the proliferation of KRAS-G12D mutant cell lines. See Table C for the activity of some compounds of the present invention against KRAS-G12D mutant cell lines.

Table C Inhibitory effect of the compounds of the present invention on cell proliferation of KRAS G12D mutant cell lines

| Compound No. | ASPC-1, $IC_{50}$ (nM) | PK-59, $IC_{50}$ (nM) | Panc04.03, $IC_{50}$ (nM) |
|---|---|---|---|
| | KRAS G12D | KRAS G12D | KRAS G12D |
| Example 2 | 0.42 | 0.0002 | 0.85 |

**6. 3D-CTG method was used to detect the effect of compounds on the proliferation inhibition of GP2D, HPAC cells:**

Experimental steps:

(1) GP2D and HPAC cells were revived in DMEM medium containing 10% fetal bovine serum and used for experiments after the cells were recovered.
(2) On the first day, the cells were rinsed once with PBS, preheated trypsin was added, and the cells were placed in a 37 °C 5% $CO_2$ incubator for 3-5 minutes. The digestion was terminated by adding an appropriate amount of complete culture medium (containing 10% fetal bovine serum), and the cells were transferred to a centrifuge tube and centrifuged at 1000 rpm for 5 minutes.
(3) The cells were resuspended in complete culture medium, counted, and adjusted to the appropriate cell concentration with complete culture medium.
(4) Using the ECHO 655 ultrasonic liquid handling system, 0.2 μl of the prepared 200× serial dilution compound (final DMSO concentration of 0.5%) was added to a 384-well plate. 40 μl of the cell suspension counted in step (3) was then added to each well and the plate was incubated in a 5% $CO_2$ incubator at 37 °C.
(6) After the 8th day of incubation, 3D CTG detection reagent was added to each well.
(7) Signal values were read using Envision.
(8) Data analysis:

a) DMSO and culture medium were used to test the stability of the experimental method:

H = DMSO well
L = Culture medium well

H, CV% (DMSO well) = 100 × (SD value of DMSO well / average value of DMSO well)

L, CV% (corresponding culture medium well) = 100 × (SD value of culture medium well / average value of corresponding culture medium well)

Z' = 1-3 × (SD value of DMSO well + SD value of culture medium well)/(average value of DMSO well - average value of corresponding culture medium well)

Inhibition rate, % = (average value of DMSO well - average value of sample well)/(average value of DMSO well - average value of corresponding culture medium well) × 100

b) IC50 values were fitted using the nonlinear regression equation in XLfit 5.5.0 software.

Y=Bottom + (Top-Bottom)/(1+10^((LogIC50-X)*HillSlope))

X: Log value of compound concentration
Y: Inhibition rate (% inh)

Conclusion: The $IC_{50}$ of the compounds of the present invention on the inhibition of GP2D, HPAC cell proliferation is less than 1 μM, and most of the compounds have an $IC_{50}$ value of less than 100 nM; among them, a large part of the

compounds have an $IC_{50}$ value of less than 10 nM. Therefore, the compounds of the present invention have good inhibitory effects on the proliferation of GP2D, HPAC cells. The partial inhibitory activities of some compounds of the present invention are shown in Table D-1 and Table D-2.

Table D-1 Inhibitory effect of the compounds of the present invention on cell proliferation of KRAS G12D mutant cell lines GP2D

| Compound No. | GP2D, $IC_{50}$ (nM) |
|---|---|
| | KRAS G12D |
| Example 2 | 0.17 |
| Example 5 | 0.07 |
| Example 6 | 1.6 |
| Example 9 | 0.19 |
| Example 10 | 12.36 |
| Example 11 | 0.03 |
| Example 12-A | 3.49 |
| Example 12-B | 3.19 |
| Example 17 | 3.76 |
| Example 18 | 0.04 |

Table D-2 Inhibitory effect of the compounds of the present invention on cell proliferation of KRAS G12D mutant cell lines HPAC

| Compound No. | GP2D, $IC_{50}$ (nM) |
|---|---|
| | KRAS G12D |
| Example 2 | 0.77 |

7. Pharmacokinetic evaluation of the compounds of the present invention by intravenous injection or oral gavage in mice, rats, and dogs

The inventors conducted a pharmacokinetic evaluation of the compounds of this invention in mice, rats, and dogs. Wherein, detailed animal information is provided in Table 2.

**Table 2: Animal Information Table**

| **Strain** | **Grade** | **Sex** | **Weight** | **Age** | **Source** |
|---|---|---|---|---|---|
| BALB/c mice | SPF | male | 18- 30 g | 6-8 weeks | Hunan SJA Laboratory Animal Co., Ltd |
| SD rats | SPF | male | 180- 350 g | 6-11 weeks | Hunan SJA Laboratory Animal Co., Ltd |
| Beagle dogs | Ordinary | male | 7~12 kg | 6-12 months | Beijing Marshall Biotechnology Co., Ltd. |
| Macaca fascicularis | Ordinary | male | 2.5~6.0 kg | 3-6 years old | Guangzhou Huazhen Biosciences Co., Ltd. |

Test method

The compounds of the present invention were administered to test animals in the form of 5% DMSO + 30% PEG400 + 65% saline, 10% DMSO + 10% Kolliphor HS15 + 80% Saline, 10% DMSO + 89% (25% SBE-B-CD) + (2% HCl), 20% PEG400 + 80% sterile water for injection, or 10% DMA + 10% HS15 + 30% PEG400 + 50% sterile water for injection. The animals were fasted for 12 hours before administration and had free access to water. For the intravenous administration group, the dose was 1 mg/kg. Venous blood samples (approximately 0.15 mL) were collected at the following time points after administration: 0.083, 0.25, 0.5, 1.0, 2.0, 4.0, 6.0, 8.0 and 24 h (dogs), 0.083, 0.25, 0.5, 1.0, 2.0, 5.0, 7.0 and 24 h (mice and rats) or 0.083, 0.25, 0.5, 1.0, 2.0, 6.0, 8.0 and 24 h (monkeys). EDTA-$K_2$ was pre-added to the blood collection tube as an anticoagulant. Blood samples were centrifuged at 12,000 rpm for 2 minutes, and plasma was

collected and stored at -20°C or -70°C. For the oral gavage administration group, the dose was 1 mg/kg (mice, dogs and monkeys) or 5 mg/kg (rats). Venous blood samples (approximately 0.15 mL) were collected at the following time points after administration: 0.25, 0.5, 1.0, 2.0, 4.0, 6.0, 8.0 and 24 h (dogs), 0.25, 0.5, 1.0, 2.0, 5.0, 7.0 and 24 h (mice and rats) or 0.25, 0.5, 1.0, 2.0, 6.0, 8.0 and 24 h (monkeys). EDTA-$K_2$ was pre-added to the blood collection tube as an anticoagulant. Blood samples were centrifuged at 12,000 rpm for 2 minutes, and plasma was collected and stored at -20°C or - 70°C.

The collected plasma samples were processed (frozen plasma was thawed at room temperature and vortexed for 15 seconds to mix thoroughly. 10-20 μL of plasma was added to 120-150 μL of acetonitrile solution containing the internal standard and the mixture vortexed for 5 minutes to mix thoroughly. The sample was centrifuged at 4,000 rpm for 5 minutes. 100 μL of the supernatant was added to 120-150 μL of methanol/water (v/v = 1/1) and mixed). The concentrations of the compounds in the plasma were analyzed by LC-MS/MS.

The analysis results show that the compound of the present invention has good pharmacokinetic properties in mice, rats and dogs, indicating that the compound of the present invention has better drugability and better clinical application prospects.

Reference throughout this specification to "one embodiment," "some embodiments," "certain implementations," "example," "specific example," or "some examples" means that a particular feature, structure, material, or characteristic described in connection with the embodiment or example is included in at least one embodiment or example of the present disclosure. In this specification, the schematic expressions of the above terms do not necessarily refer to the same embodiment or example. Furthermore, the particular features, structures, materials, or characteristics may be combined in any suitable manner in one or more embodiments or examples. In addition, those skilled in the art can integrate and combine different embodiments, implementations, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure, the scope of the invention is defined by the claims and their equivalents.

## Claims

**1.** A compound having Formula (I) or a stereoisomer, a tautomer, an N-oxide, a solvate, a metabolite, a pharmaceutically acceptable salt or a prodrug thereof,

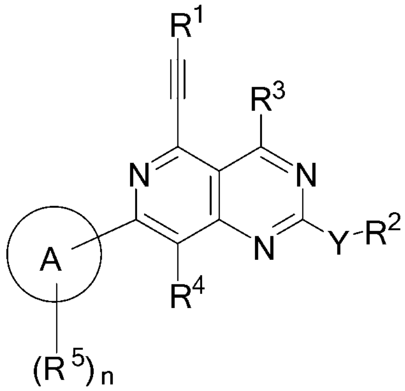

(I),

wherein,

$R^1$ is -H, -D, -CN, $-NH_2$, -C(=O)H, -C(=O)OH, $-C(=O)OR^{6a}$, $-C(=O)NR^6R^7$, $-NR^6C(=O)R^7$, - $NR^{6a}C(=O)NR^6R^7$, $-C(=O)R^{6a}$, $-NR^6S(=O)_2R^7$, $-S(=O)_2NR^6R^7$, $-NR^{6a}S(=O)_2NR^6R^7$, $-NR^6R^7$, $-C_{1-6}$ alkyl-$NR^6C(=O)R^7$, $-C_{1-6}$ alkyl-$NR^6R^7$, $-C_{1-6}$ alkyl-$C(=O)OR^6$, $-C_{1-6}$ alkyl-$C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl or 3-12 membered heterocyclyl; wherein each of $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl and 3-12

membered heterocyclyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$ and $C_{1-6}$ alkyl;
Y is bond, O or S;
$R^2$ is

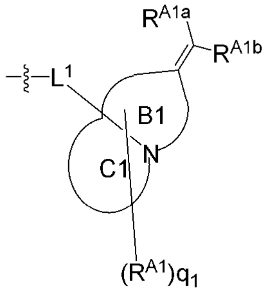
,
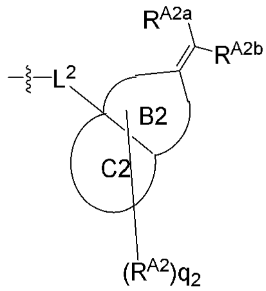
,
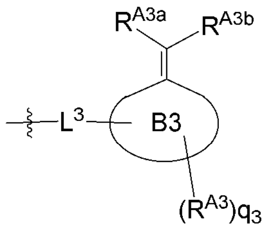
,

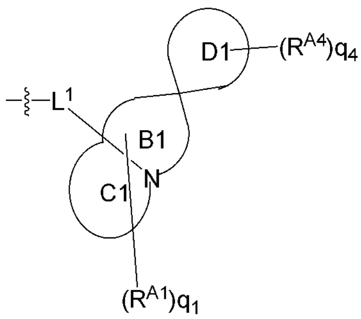
,
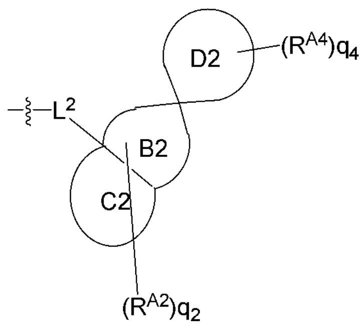
or
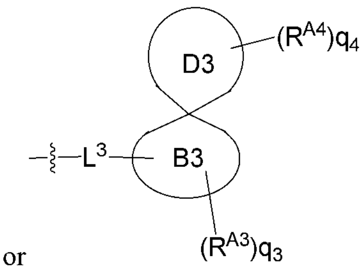
;

each $L^1$, $L^2$ and $L^3$ is independently $C_{1-6}$ alkylene or $C_{1-6}$ heteroalkylene, wherein each of the $C_{1-6}$ alkylene and $C_{1-6}$ heteroalkylene is independently optionally substituted with 1, 2, 3 or 4 $R^a$;
$R^a$ is -D, -OH, -F, -Cl, -Br, -I, -CN, $-NH_2$, -C(=O)H, -C(=O)OH, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{3-6}$ cycloalkyl or 3-6 membered heterocyclyl; or two $R^a$ attached to the same carbon atom together form $C_{3-6}$ cycloalkyl;
each Ring B1 and ring C1 is independently 3-7 membered monocyclic heterocyclyl;
each Ring B2, ring B3 and ring C2 is independently $C_{3-7}$ monocyclic cycloalkyl or 3-7 membered monocyclic heterocyclyl;
each of Ring D1, ring D2 and ring D3 is independently $C_{3-7}$ monocyclic cycloalkyl or 3-7 membered monocyclic heterocyclyl;
each $R^{A1}$, $R^{A2}$, $R^{A3}$ and $R^{A4}$ is independently -H, -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl;
or, two $R^{A1}$ attached to the same carbon atom together form

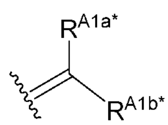
;

or, two $R^{A1}$ together with the same carbon atom to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{8a}$; or, two $R^{A1}$ attached to two adjacent atoms together with the two adjacent atoms to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{8a*}$;
or, two $R^{A2}$ attached to the same carbon atom together form

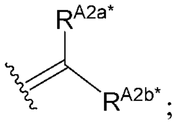

or, two $R^{A2}$ together with the same carbon atom to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{9a}$; or, two $R^{A2}$ attached to two adjacent atoms together with the two adjacent atoms to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{9a*}$;
or, two $R^{A3}$ attached to the same carbon atom together form

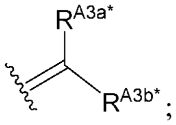

or, two $R^{A3}$ together with the same carbon atom to which they are attached, form $C_{3-6}$ cycloalkyl or 3-6 membered heterocyclyl, wherein each of the $C_{3-6}$ cycloalkyl and 3-6 membered heterocyclyl is independently optionally substituted with 1, 2, 3 or 4 $R^{10a}$; or, two $R^{A3}$ attached to two adjacent atoms together with the two adjacent atoms to which they are attached, form $C_{3-6}$ cycloalkyl or a 3-6 membered heterocyclyl, wherein each of the $C_{3-6}$ cycloalkyl and 3-6 membered heterocyclyl is independently optionally substituted with 1, 2, 3 or 4 $R^{10a*}$;
each of $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, - D, -F, -Cl, -Br, -I, -CN, -$C_{1-6}$ alkyl-$NR^6C(=O)R^7$, -$C_{1-6}$ alkyl-$NR^6R^7$, -$C_{1-6}$ alkyl-$C(=O)OR^6$, -$C_{1-6}$ alkyl-$C(=O)NR^6R^7$, $C_{1-6}$ alkyl, $C_{1-6}$ haloalkyl, $C_{1-6}$ cyanoalkyl, $C_{1-6}$ hydroxyalkyl, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{6-12}$ aryl, 5-12 membered heteroaryl, $C_{3-12}$ cycloalkyl or 3-12 membered heterocyclyl;
each $R^{8a}$, $R^{8a*}$, $R^{9a}$, $R^{9a*}$, $R^{10a}$ and $R^{10a*}$ is independently -D, -OH, -F, -Cl, -Br, -I, -CN, -$NH_2$, -C(=O)OH, - C(=O)$NH_2$, oxo, $C_{1-6}$ alkyl, $C_{1-6}$ haloalkyl, $C_{1-6}$ alkoxy, $C_{1-6}$ hydroxyalkyl, $C_{6-10}$ aryl, 5-10 membered heteroaryl, -C(=O)O$C_{1-6}$ alkyl, -C(=O)NH$C_{1-6}$ alkyl or -C(=O)N($C_{1-6}$ alkyl)$_2$;
$R^3$ is

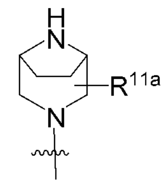 , 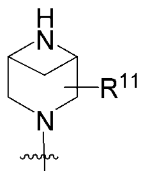 , 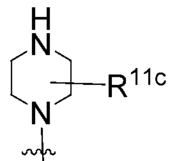 or 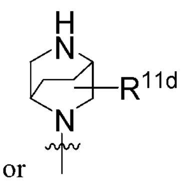 ;

each of $R^{11a}$, $R^{11b}$, $R^{11c}$ and $R^{11d}$ is independently -H, -D, -OH, -SH, -F, -Cl, -Br, -I, -CN, -C(=O)H, - C(=O)$OR^{6a}$, -C(=O)$NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ haloalkoxy or 5-6 membered heteroaryl;
$R^4$ is -H, -D, -OH, -SH, -F, -Cl, -Br, -I, -CN, methyl, ethyl, n-propyl, i-propyl, n-butyl or $C_{1-4}$ haloalkyl;
Ring A is $C_{6-12}$ aryl or 5-12 membered heteroaryl;
each $R^5$ is independently -D, -OH, -F, -Cl, -Br, -I, -CN, -SH, -$CH_2C(=O)NR^{6b}R^{7b}$, -C(=O)$R^{6c}$, -C(=O)$OR^{6c}$, -C(=O)$NR^{6b}R^{7b}$, -$NR^{6b}C(=O)R^{7b}$, -$NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein each of the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl is independently optionally substituted with 1, 2, 3 or 4 substituents selected from -D, -OH, -F, -Cl, -Br, -I, -CN, -$NH_2$, -C(=O)H, -C(=O)OH, $C_{1-6}$ alkyl, $C_{1-6}$ alkoxy, $C_{3-6}$ cycloalkyl and 3-6 membered heterocyclyl;
each $R^6$, $R^7$, $R^{6b}$, $R^{6b}$, $R^{6c}$, $R^{7b}$, $R^{6d}$ and $R^{7d}$ is independently -H, -D or $C_{1-6}$ alkyl, wherein the $C_{1-6}$ alkyl is independently optionally substituted with 1, 2, 3 or 4 substituents selected from -D, -OH, -F, -Cl, -Br, -I, -CN, - C(=O)H, -C(=O)OH, -$NH_2$, $C_{1-6}$ alkoxy, $C_{6-12}$ aryl, $C_{3-6}$ cycloalkyl and 3-6 membered heterocyclyl;
n is 0, 1, 2, 3, 4, 5, 6 or 7; and

each $q_1$, $q_2$ and $q_3$ is independently 0, 1, 2, 3, 4, 5 or 6;
each $q_4$ is independently 0, 1, 2, 3 or 4;
wherein, the compound of formula (I) is not

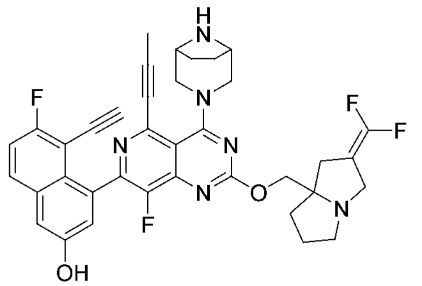

and

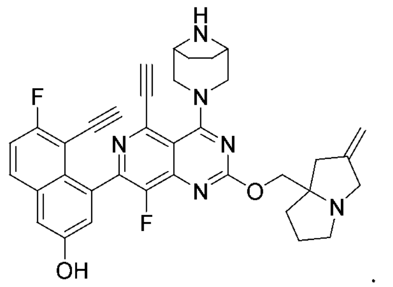

.

**2.** The compound of claim 1, wherein, $R^1$ is -H, -D, -CN, $-NH_2$, -C(=O)H, -C(=O)OH, $-C(=O)OR^{6a}$, - $C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, - $NR^{6a}S(=O)_2NR^6R^7$, $-NR^6R^7$, $-C_{1-4}$ alkyl-$NR^6C(=O)R^7$, $-C_{1-4}$ alkyl-$NR^6R^7$, $-C_{1-4}$ alkyl-$C(=O)OR^6$, $-C_{1-4}$ alkyl-$C(=O)NR^6R^7$, $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl or 3-6 membered heterocyclyl; wherein each of the $C_{1-4}$ alkyl, $C_{1-4}$ cyanoalkyl, $C_{1-4}$ hydroxyalkyl, $C_{1-4}$ haloalkyl, $C_{1-4}$ alkoxy, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-$C_{1-4}$ alkyl)-O-$C_{1-4}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl and 3-6 membered heterocyclyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$ and $C_{1-4}$ alkyl.

**3.** The compound of any one of claims 1-2, wherein, $R^1$ is -H, -D, -CN, $-NH_2$, -C(=O)H, -C(=O)OH, - $C(=O)OR^{6a}$, $-C(=O)NR^6R^7$, $-NR^6C(=O)R^7$, $-NR^{6a}C(=O)NR^6R^7$, $-C(=O)R^{6a}$, $-NR^6S(=O)_2R^7$, $-S(=O)_2NR^6R^7$, - $NR^{6a}S(=O)_2NR^6R^7$, $-NR^6R^7$, $-CH_2NR^6C(=O)R^7$, $-CH_2NR^6R^7$, $-(CH_2)_2NR^6R^7$, $-CH_2C(=O)OR^6$, - $(CH_2)_2C(=O)OR^6$, $-(CH_2)_3C(=O)OR^6$, $-CH_2C(=O)NR^6R^7$, $-(CH_2)_2C(=O)NR^6R^7$, $-(CH_2)_3C(=O)NR^6R^7$, $-CH_3$, - $CH_2CH_3$, $-(CH_2)_2CH_3$, $-(CH_2)_3CH_3$, $-C(CH_3)_3$, $-CH(CH_3)_2$, $-CH_2CH(CH_3)_2$, $-CH_2CN$, $-(CH_2)_2CN$, $-(CH_2)_3CN$, - $CH(CH_3)CN$, $-C(CH_3)_2CN$, $-CH_2OH$, $-(CH_2)_2OH$, $-(CH_2)_3OH$, $-CH(OH)CH_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCH_3$, $-OCH_2CH_3$, $-O(CH_2)_2CH_3$, $-O(CH_2)_3CH_3$, - $OC(CH_3)_3$, $-OCH(CH_3)_2$, $-CH_2OCH_3$, $-(CH_2)_2OCH_3$, $-(CH_2)_2OCH_2CH_3$, $-CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, - $(CH_2)_2$-cyclohexyl, $-CH_2$-cyclopropyl, $-CH_2$-cyclobutyl, $-(CH_2)_2$-cyclobutyl, $-CH_2$-cyclopentyl, - $(CH_2)_2$cyclopentyl, $-CH_2$-azetidinyl, $-CH_2$-pyrrolidinyl, $-CH_2$-piperidyl, $-CH_2$-morpholinyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl,$-CH_2O$-spiro[2.3]hexyl, - $CH_2O$-spiro[3.3]heptanyl, $-CH_2SH$, $-(CH_2)_2SH$, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl or pyrrolidinyl; wherein each of the $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$,

$-CH_2CN$, - $(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, $-CH(OH)CH_3$, - $(CH_2)_2CHF_2$, $-(CH_2)_2CF(CF_3)_2$, $-CHF_2$, $-CH_2F$, $-(CH_2)_2F$, $-(CH_2)_2Cl$, $-CH_2CF_3$, $-OCH_3$, $-OCH_2CH_3$, - $O(CH_2)_2CH_3$, $-O(CH_2)_3CH_3$, $-OC(CH_3)_3$, $-OCH(CH_3)_2$, $-CH_2OCH_3$, $-(CH_2)_2OCH_3$, $-(CH_2)_2OCH_2CH_3$, - $CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, - $(CH_2)_2$-cyclopentyl, $-(CH_2)_3$-cyclopentyl, $-(CH_2)_2$-cyclohexyl, $-CH_2$-cyclopropyl, $-CH_2$-cyclobutyl, $-(CH_2)_2$-cyclobutyl, $-CH_2$-cyclopentyl, $-(CH_2)_2$cyclopentyl, $-CH_2$-azetidinyl, $-CH_2$-pyrrolidinyl, $-CH_2$-piperidyl, $-CH_2$-morpholinyl, $-CH_2$-phenyl, $-CH_2$-imidazolyl, $-CH_2$-pyrazolyl, $-CH_2O$-cyclopropyl, $-CH_2O$-cyclobutyl, - $(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, $-CH_2O$-spiro[2.3]hexyl, $-CH_2O$-spiro[3.3]heptanyl, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl and pyrrolidinyl is independently optionally substituted with 1, 2, 3 or 4 substituents selected from D, -OH, -F, -Cl, -Br, -I, CN, $-C(=O)OR^{6e}$, $-NR^{6e}R^{7e}$, $-C(=O)NR^{6e}R^{7e}$, $-NR^{6e}C(=O)R^{7e}$, methyl, ethyl, n-propyl, isopropyl, n-butyl, isobutyl and tert-butyl;

**4.** The compound of any one of claims 1-3, wherein,

each $R^{A1}$, $R^{A2}$, $R^{A3}$ and $R^{A4}$ is independently -H, -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl or 3-6 membered heterocyclyl; or, $R^{A1}$, $R^{A2}$, $R^{A3}$ and $R^{A4}$ are each independently -H, -D, -OH, -F, - Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $-CH_3$, $-CH_2CH_3$, $-(CH_2)_2CH_3$, $-(CH_2)_3CH_3$, $-C(CH_3)_3$, $-CH(CH_3)_2$, $-CH_2CH(CH_3)_2$, $-OCH_3$, $-OCH_2CH_3$, $-OCH_2CH_3$, $-OC(CH_3)_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-OCF_3$, $-OCHF_2$, $-CH_2$-phenyl, $-CH_2$-pyridyl, $-CH_2$-pyrrolidinyl, $-CH_2$-piperidinyl, $-CH_2$-cyclopropyl, $-CH_2$-cyclopentyl, cyclopropyl, cyclobutyl, cyclopentyl, cyclohexyl, oxiranyl, oxetanyl, tetrahydropyranyl, morpholinyl, azetidinyl or pyrrolidinyl;
or, two $R^{A1}$ attached to the same carbon atom together form

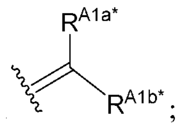;

or, two $R^{A1}$ together with the same carbon atom to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{8a}$; or, two $R^{A1}$ attached to two adjacent atoms together with the two adjacent atoms to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{8a*}$;
or, two $R^{A2}$ attached to the same carbon atom together form

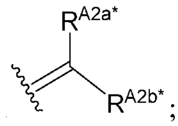;

or, two $R^{A2}$ together with the same carbon atom to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{9a}$; or, two $R^{A2}$ attached to two adjacent atoms together with the two adjacent atoms to which they are attached, form

cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{9a*}$;
or, two $R^{A3}$ attached to the same carbon atom together form

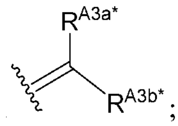;

or, two $R^{A3}$ together with the same carbon atom to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{10a}$; or, two $R^{A3}$ attached to two adjacent atoms together with the two adjacent atoms to which they are attached, form cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein each of the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl is independently optionally substituted with 1, 2, 3 or 4 $R^{10a*}$;
each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$C_{1-4}$ alkyl-$NR^6C(=O)R^7$, -$C_{1-4}$ alkyl-$NR^6R^7$, -$C_{1-4}$ alkyl-$C(=O)OR^6$, -$C_{1-4}$ alkyl-$C(=O)NR^6R^7$, $C_{1-4}$ alkyl, $C_{1-4}$ haloalkyl, $C_{1-4}$ cyanoalkyl, $C_{1-4}$ hydroxyalkyl, $C_{1-4}$ alkoxy $C_{1-4}$ alkyl, ($C_{3-6}$ cycloalkyl)-$C_{1-4}$ alkyl, (3-6 membered heterocyclyl)-$C_{1-4}$ alkyl, ($C_{3-6}$ cycloalkyl)-O-$C_{1-4}$ alkyl, (3-6 membered heterocyclyl)-O-$C_{1-4}$ alkyl, phenyl, 5-6 membered heteroaryl, $C_{3-6}$ cycloalkyl or 3-6 membered heterocyclyl;
each $R^{8a}$, $R^{8a*}$, $R^{9a}$, $R^{9a*}$, $R^{10a}$ and $R^{10a*}$ is independently -D, -OH, -F, -Cl, -Br, -I, -CN, -$NH_2$, -C(=O)OH, - C(=O) $NH_2$, oxo, $C_{1-4}$ alkyl, $C_{1-4}$ haloalkyl, $C_{1-4}$ alkoxy, $C_{1-4}$ hydroxyalkyl, phenyl, 5-6 membered heteroaryl, - C(=O) $OC_{1-4}$ alkyl, -C(=O)$NHC_{1-4}$ alkyl or -C(=O)N($C_{1-4}$ alkyl)$_2$.

**5.** The compound of any one of claims 1-4, wherein,

each $R^{A1a}$, $R^{A1b}$, $R^{A2a}$, $R^{A2b}$, $R^{A3a}$, $R^{A3b}$, $R^{A1a*}$, $R^{A1b*}$, $R^{A2a*}$, $R^{A2b*}$, $R^{A3a*}$ and $R^{A3b*}$ is independently -H, -D, -F, -Cl, -Br, -I, -CN, -$CH_2NR^6C(=O)R^7$, -$CH_2NR^6R^7$, -$CH_2C(=O)OR^6$, -$(CH_2)_2C(=O)OR^6$, -$(CH_2)_3C(=O)OR^6$, - $CH_2C(=O)NR^6R^7$, $(CH_2)_2C(=O)NR^6R^7$, -$(CH_2)_3C(=O)NR^6R^7$, -$CH_3$, -$CH_2CH_3$, -$(CH_2)_2CH_3$, -$(CH_2)_3CH_3$, - $C(CH_3)_3$, -$CH(CH_3)_2$, -$CH_2CH(CH_3)_2$, -$(CH_2)_2CHF_2$, -$(CH_2)_2CF(CF_3)_2$, -$CF_3$, -$CHF_2$, -$CH_2F$, -$(CH_2)_2F$, - $(CH_2)_2Cl$, -$CH_2CF_3$, -$CH_2CN$, -$(CH_2)_2CN$, -$(CH_2)_3CN$, -$CH(CH_3)CN$, -$C(CH_3)_2CN$, -$CH_2OH$, -$(CH_2)_2OH$, - $(CH_2)_3OH$, -$CH(OH)CH_3$, -$CH_2OCH_3$, -$(CH_2)_2OCH_3$, -$(CH_2)_2OCH_2CH_3$, -$CH_2OCH_2CH_3$, -$CH_2OC(CH_3)_3$, - $CH_2$-cyclopropyl, -$CH_2$-cyclobutyl, -$CH_2$-cyclopentyl, -$CH_2$-cyclohexyl, -$(CH_2)_2$-cyclopentyl, -$(CH_2)_3$-cyclopentyl, -$(CH_2)_2$-cyclohexyl, -$CH_2$-cyclopentyl, -$CH_2$-azetidinyl, -$CH_2$-oxetanyl, -$CH_2$-tetrahydrofuranyl, - $CH_2$-morpholinyl, -$CH_2O$-cyclopropyl, -$CH_2O$-cyclobutyl, -$(CH_2)_2O$-cyclobutyl, -$CH_2O$-cyclopentyl, - $(CH_2)_2O$-cyclopentyl, -$CH_2O$-azetidinyl, -$CH_2O$-oxetanyl, -$CH_2O$-tetrahydrofuranyl, -$CH_2O$-morpholinyl, phenyl, pyridinyl, pyrimidinyl, cyclopropyl, cyclobutyl, cyclopentyl, cyclohexyl, oxiranyl, oxetanyl, tetrahydropyranyl, morpholinyl, azetidinyl or pyrrolidinyl;
each $R^{8a}$, $R^{8a*}$, $R^{9a}$, $R^{9a*}$, $R^{10a}$ and $R^{10a*}$ is independently-D, -OH, -F, -Cl, -Br, -I, -CN, -$NH_2$, -C(=O)OH, - C(=O) $NH_2$, oxo, -$CH_3$, -$CH_2CH_3$, -$(CH_2)_2CH_3$, -$(CH_2)_3CH_3$, -$C(CH_3)_3$, -$CH(CH_3)_2$, -$CH_2CH(CH_3)_2$, - $(CH_2)_2CHF_2$, -$(CH_2)_2CF(CF_3)_2$, -$CF_3$, -$CHF_2$, -$CH_2F$, -$(CH_2)_2F$, -$(CH_2)_2Cl$, -$CH_2CF_3$, -$OCH_3$, -$OCH_2CH_3$, - $OCH_2CH_3$, -$OC(CH_3)_3$, -$CH_2OH$, -$(CH_2)_2OH$, -$(CH_2)_3OH$, phenyl, pyridinyl, pyrimidinyl, -$C(=O)OCH_3$, - $C(=O)NHCH_3$ or -$C(=O)N(CH_3)_2$.

**6.** The compound of any one of claims 1-5, wherein, ring A is one of the following sub-structures,

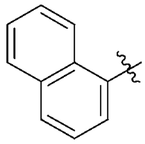, 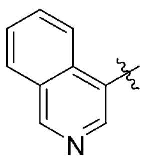, 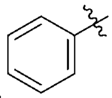, 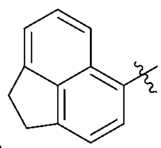, 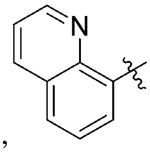, 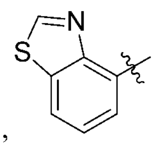, 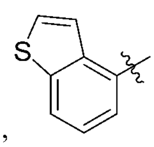, 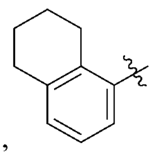,

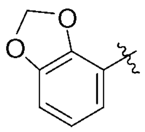, 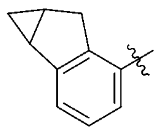, 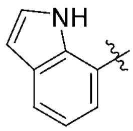, 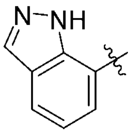, 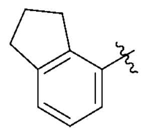, 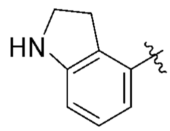, 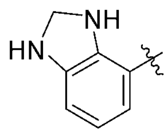,

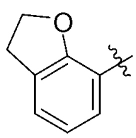, 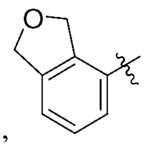, 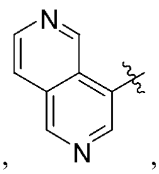, 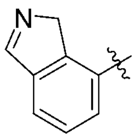, 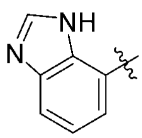, 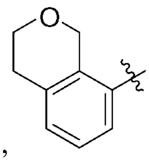, 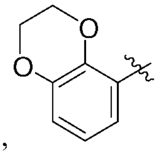 or 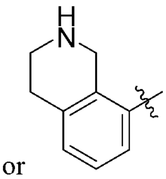;

each $R^5$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-12 membered aryl, 5-12 membered heteroaryl, $C_{3-6}$ cycloalkyl or 3-6 membered heterocyclyl, wherein each of the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-10 membered heteroaryl, $C_{3-6}$ cycloalkyl and 3-6 membered heterocyclyl is independently optionally substituted with 1, 2, 3 or 4 substituents selected from -D, - OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl and 3-6 membered heterocyclyl.

**7.** The compound of any one of claims 1-6, wherein,
each $R^5$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, $-C\equiv CH$, $-C\equiv CCH_3$, $-CH_2C\equiv CH$, $-C\equiv CCH_2OH$, $- C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $- (CH_2)_3OH$, $-CH(OH)CH_3$, $-(CH_2)_2F$, $-CH_2CHF_2$, $-CF_3$, $-CH_2CF_3$, $-CHF_2$, $-CH_2F$, $-(CH_2)_2Cl$, -CH=CHF, - CH=CHCl, $-CH=CHCH_2F$, $-C\equiv CCH_2F$, $-C\equiv C(CH_2)_2F$, $-C\equiv CF$, $-OCF_3$, $-OCHF_2$, $-OCH_2CHF_2$, $-OCH_2CF_3$, $- OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein each of the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $- SCH_2CH_3$, $-CH=CH_2$, $-CH=CHCH_3$, $-CH_2CH=CH_2$, $-C\equiv CH$, $-C\equiv CCH_3$, $-CH_2C\equiv CH$, $-C\equiv CCH_2OH$, $- C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$,$-(CH_2)_3OH$, $-CH(OH)CH_3$, $-(CH_2)_2F$, $-CH_2CHF_2$, $-CH_2CF_3$, $-CHF_2$, $-CH_2F$, $-(CH_2)_2Cl$, -CH=CHF, -CH=CHCl, $- CH=CHCH_2F$, $-C=CCH_2F$, $-C\equiv C(CH_2)_2F$, $-OCHF_2$, $-OCH_2CHF_2$, $-OCH_2CF_3$, $-OCHClCHCl_2$, $-OCH_2CH_2F$, $- SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl is independently optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, n-propoxy, i-propoxy, cyclopropyl, cyclobutyl, cyclopentyl, cyclohexyl, pyrrolidinyl, oxazolidinyl, tetrahydrofuranyl, piperidyl and piperazinyl.

**8.** The compound of any one of claims 1-7, wherein,
each $R^6$, $R^7$, $R^{6a}$, $R^{6b}$, $R^{6c}$, $R^{7b}$, $R^{6d}$, $R^{7d}$, $R^{6e}$ and $R^{7e}$ is independently -H, -D, methyl, ethyl, n-propyl, isopropyl, n-butyl, isobutyl or tert-butyl, wherein each of the methyl, ethyl, n-propyl, isopropyl, n-butyl, isobutyl or tert-butyl is independently optionally substituted with 1, 2, 3 or 4 substituents selected from -D, -OH, -F, -Cl, -Br, -I, -CN, C(=O)H, -C(=O)OH, $-NH_2$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl.

**9.** The compound of any one of claims 1-8, wherein,
$R^2$ is

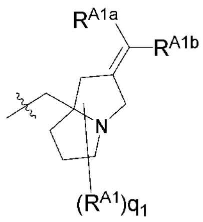, 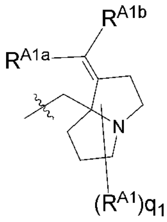, 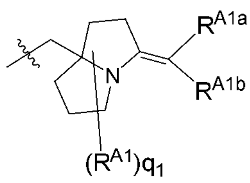, 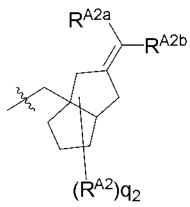, 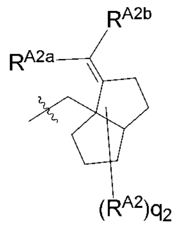

or

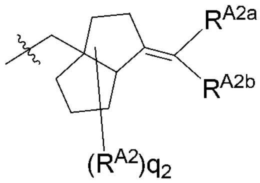.

**10.** The compound of any one of claims 1-8, wherein,
$R^2$ is

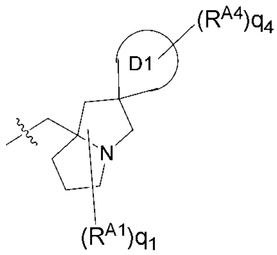, 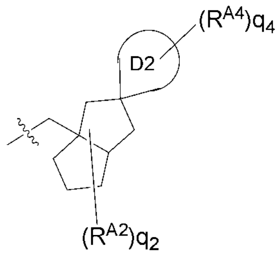 or 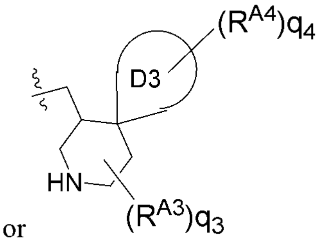,

wherein, each of ring D1, ring D2 and ring D3 is independently cyclopropyl, cyclobutyl, cyclopentyl, cyclohexyl, oxiranyl, oxetanyl, azetidinyl, pyrrolidinyl, oxazolidinyl, tetrahydrofuranyl, piperidinyl and piperazinyl.

**11.** The compound of any one of claims 1-9, wherein,
$R^2$ is

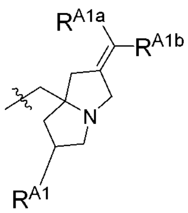, 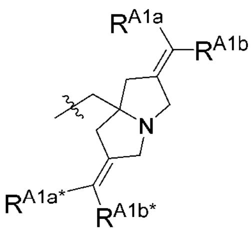, 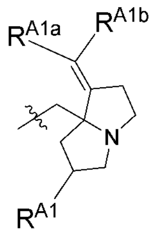, 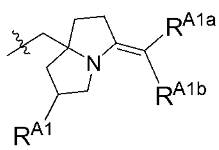, 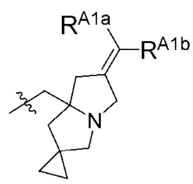,

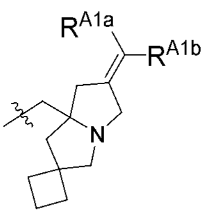, 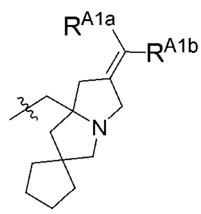, 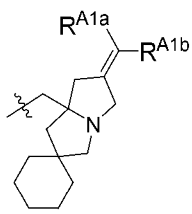, 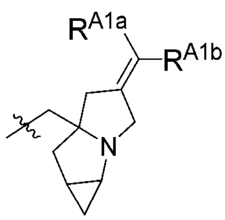, 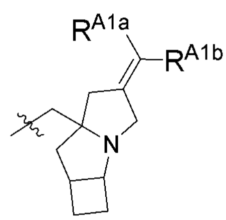,

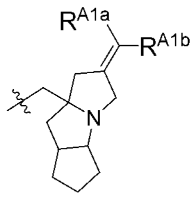, 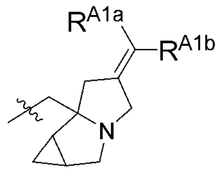, 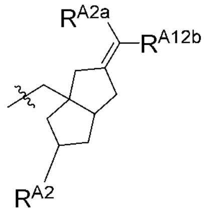, 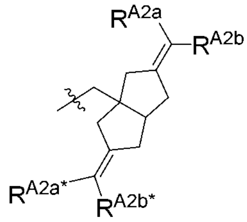, 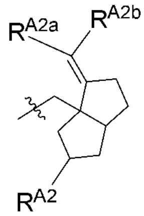,

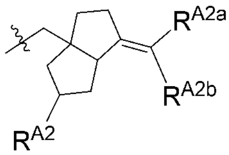, 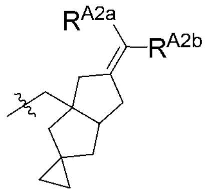 or 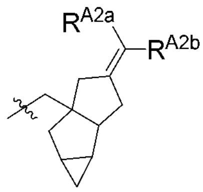.

**12.** The compound of any one of claims 1-11 having Formula (I-1a), (I-1b) or (I-1c), or a stereoisomer, a tautomer, an N-oxide, a solvate, a metabolite, a pharmaceutically acceptable salt or a prodrug thereof,

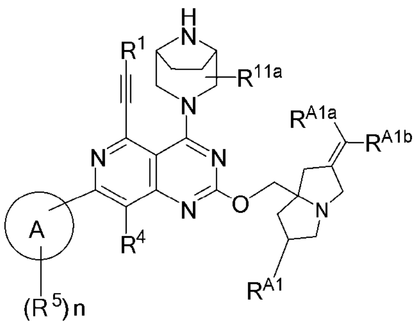

(I-1a),

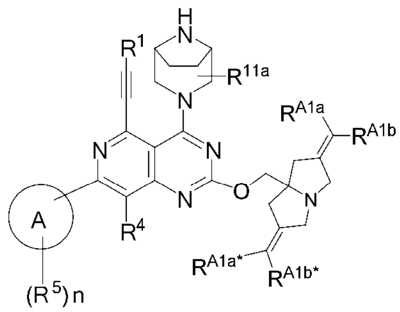

(I-1b)

or

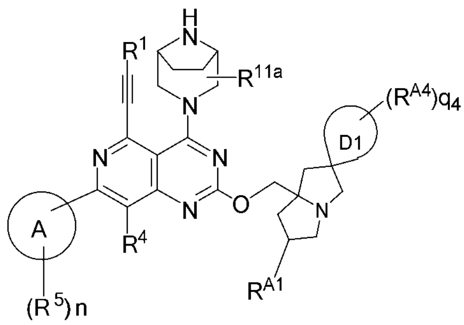

(I-1c),

wherein, the $R^1$, $R^4$, $R^5$, $R^{A1}$, $R^{A1a}$, $R^{A1b}$, $R^{A1a*}$, $R^{A1b*}$, $R^{11a}$, $R^{A4}$, $q_4$ and n are each as defined in any one of claims 1-11.

**13.** A compound having the following structure, or a stereoisomer, tautomer, N-oxide, solvate, metabolite, pharmaceutically acceptable salt, or prodrug thereof,

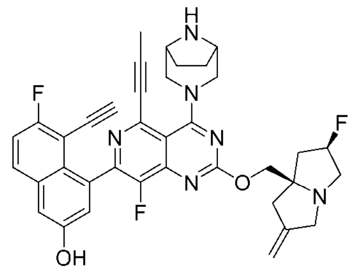
1,
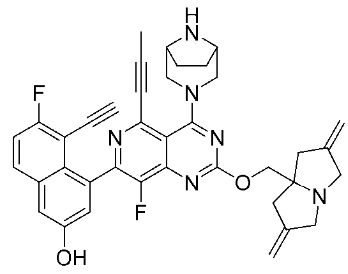
2,
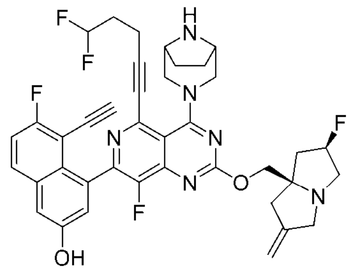
3,

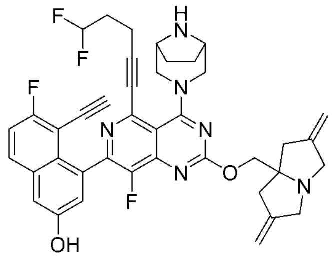
4,
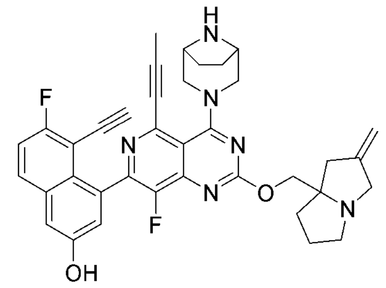
5,
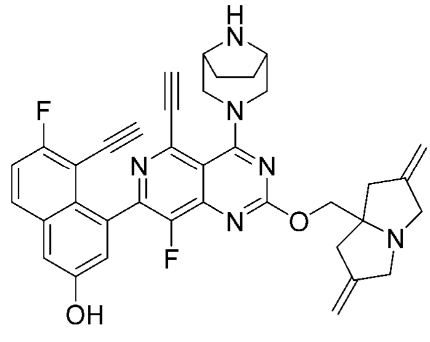

6,
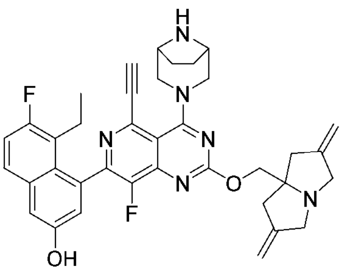
7,
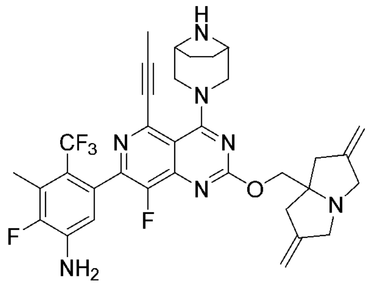
8,
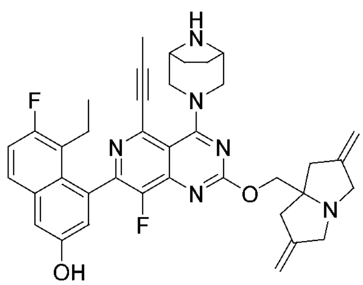
9,

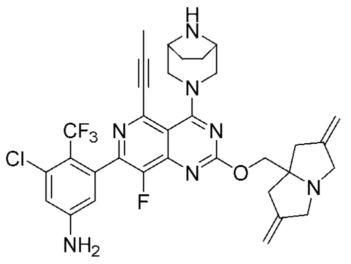
10,
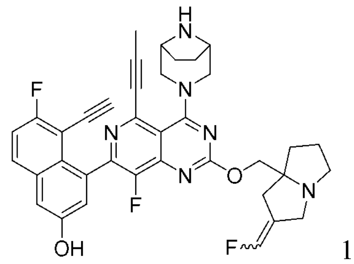
11,
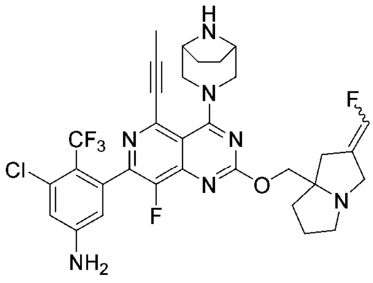
12,

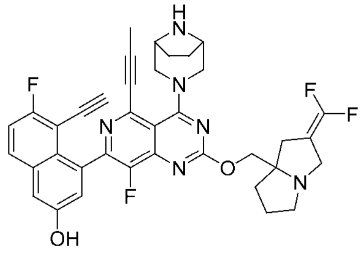
13,
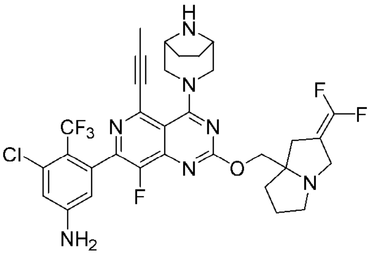
14,
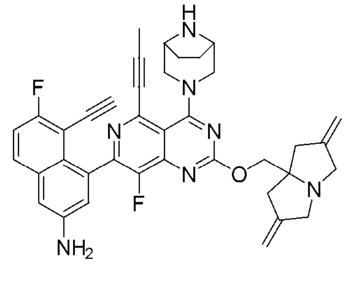
15,

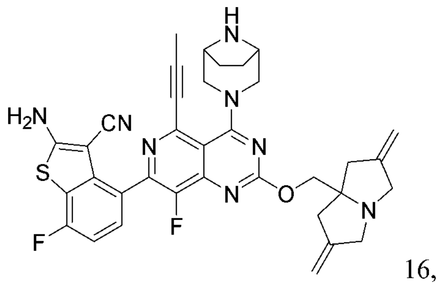 16, 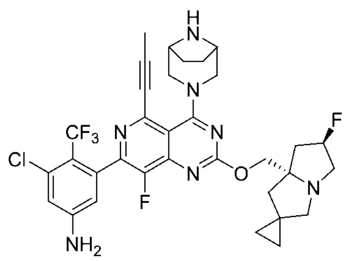 17, 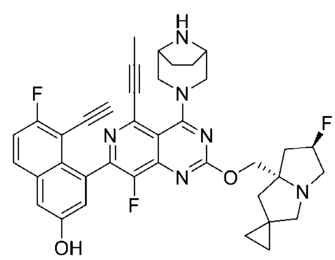 18,

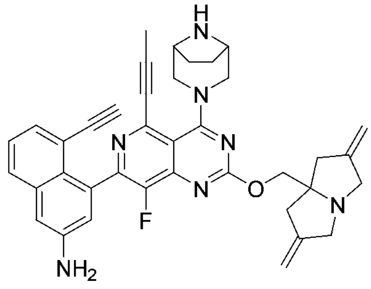 19, 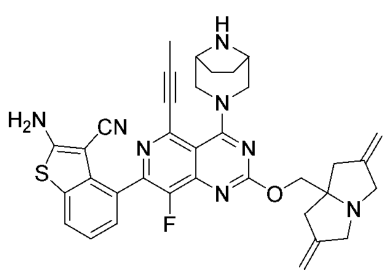 20, 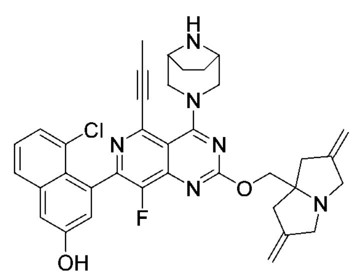

21, 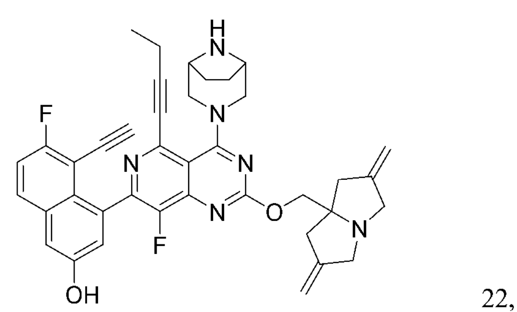 22, 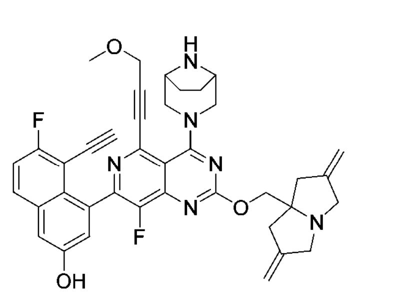 23,

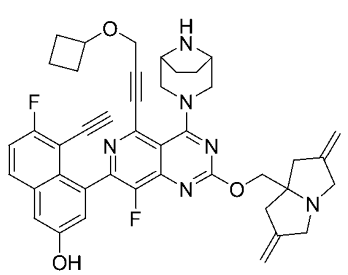 24, 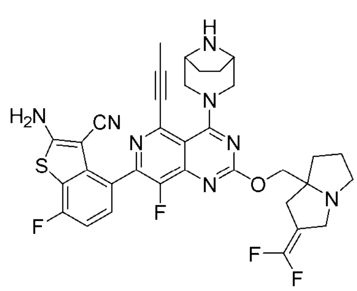 25, 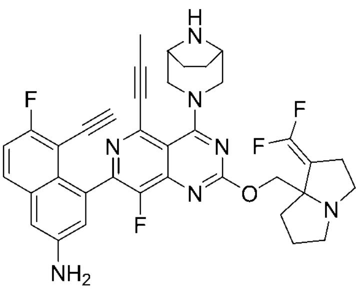 26,

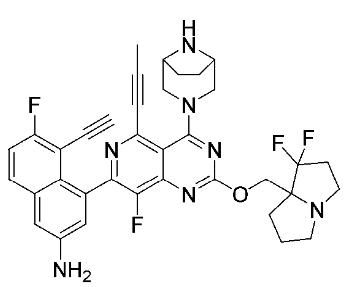 27, 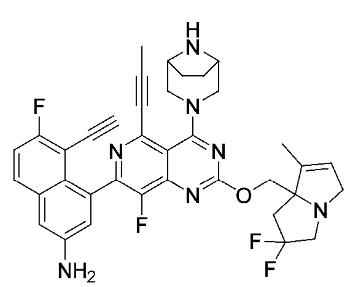 28, 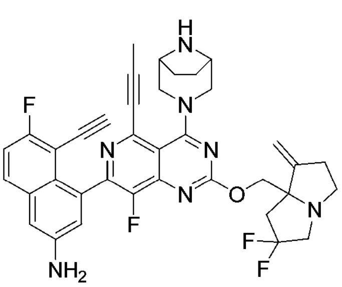 29,

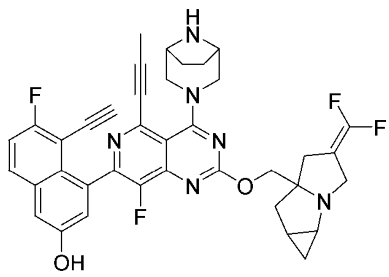 30, 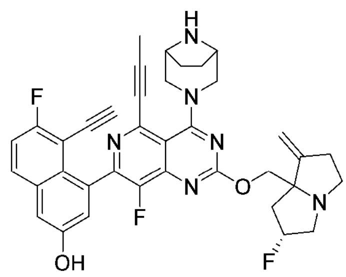 31, 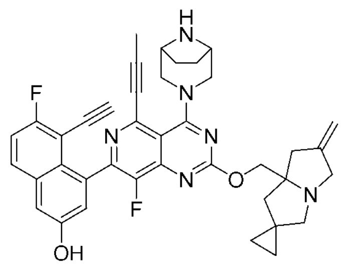 32,

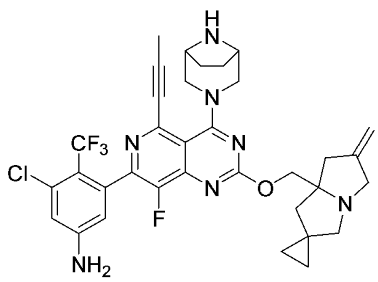 33, 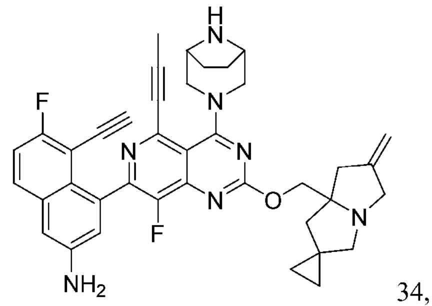 34, 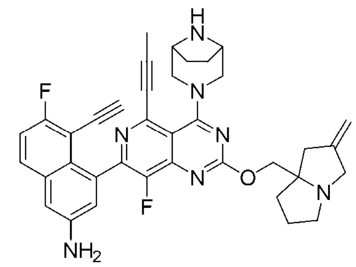

35, 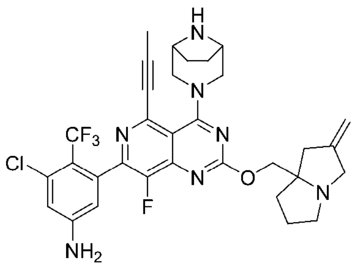 36, 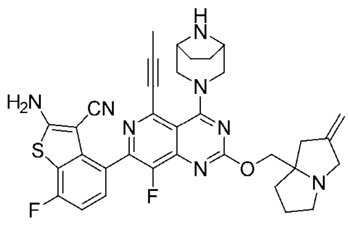 37, 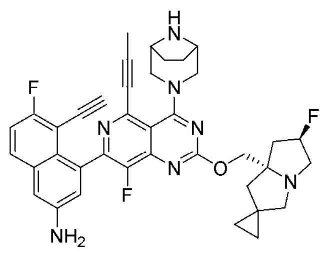 38,

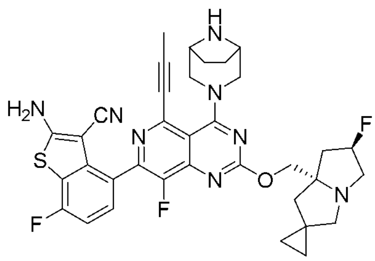 39, 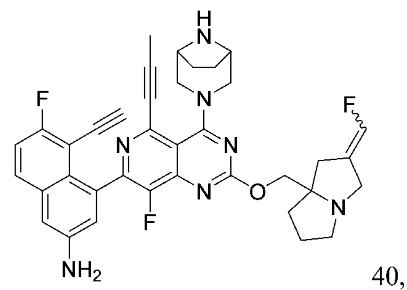 40, 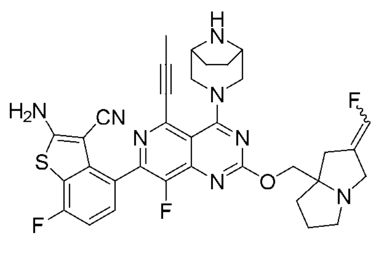

41 or 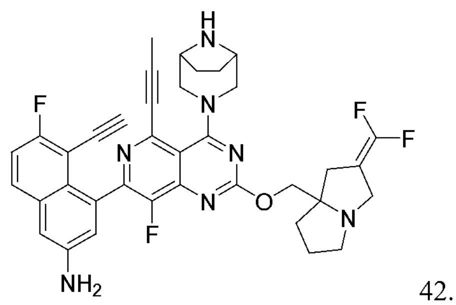 42.

**14.** A pharmaceutical composition comprising the compound of any one of claims 1 to 13; optionally, the pharmaceutical composition further comprises a pharmaceutically acceptable adjuvant.

**15.** Use of the compound of any one of claims 1-13 or the pharmaceutical composition of claim 14 in the preparation of a medicament for preventing, treating or alleviating a KRAS G12D-related disease; wherein the KRAS G12D-related

disease is cancer; optionally, the cancer is non-small cell lung cancer, small cell lung cancer, colorectal cancer, rectal cancer, colon cancer, small intestinal cancer, pancreatic cancer, uterine cancer, gastric cancer, esophageal cancer, prostate cancer, ovarian cancer, breast cancer, leukemia, melanoma, lymphoma or neuroma.

## INTERNATIONAL SEARCH REPORT

International application No.
**PCT/CN2024/102179**

### A. CLASSIFICATION OF SUBJECT MATTER

C07D 487/08(2006.01)i; C07D 519/00(2006.01)i; A61P 35/00(2006.01)i; A61P 35/02(2006.01)i; A61K 31/517(2006.01)i; A61K 31/519(2006.01)i

According to International Patent Classification (IPC) or to both national classification and IPC

### B. FIELDS SEARCHED

Minimum documentation searched (classification system followed by classification symbols)

IPC: C07D,A61K,A61P

Documentation searched other than minimum documentation to the extent that such documents are included in the fields searched

Electronic data base consulted during the international search (name of data base and, where practicable, search terms used)

CNTXT, ENTXTC, CNKI, CAPLUS(STN), REGISTRY (STN), MARPAT (STN): KRAS G12D, RAS, 抑制剂, 嘧啶并吡啶, 酪氨酸激酶, 胰腺癌, inhibitors, pyrimidinopyridines, tyrosine kinases, pancreatic cancer, 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| PX | WO 2024061333 A1 (GAN & LEE PHARM CO., LTD.) 28 March 2024 (2024-03-28) description, page 2, paragraph 2 to page 49, paragraph 2, for specific inhibitor compounds , see description, page 27, row 5, the third item, and page 30, row 4, the second item, and page 36, row 1, the third item, and embodiments | 1-15 |
| PX | CN 117800975 A (TYLIGAND BIOSCIENCE SHANGHAI LTD.) 02 April 2024 (2024-04-02) description, paragraphs 0080-00398, and embodiments 332, 334, 338, and 408 | 1-15 |
| PX | WO 2024041606 A1 (TYLIGAND BIOSCIENCE SHANGHAI LTD.) 29 February 2024 (2024-02-29) description, page 12, paragraph 4 to page 62, paragraph 3, and embodiments | 1-15 |
| PX | WO 2023138583 A1 (SHANGHAI APEIRON THERAPEUTICS CO., LTD.) 27 July 2023 (2023-07-27) description, page 2, paragraph 1 to page 52, paragraph 1, and embodiments | 1-15 |
| X | WO 2022184178 A1 (JACOBIO PHARMACEUTICALS CO., LTD.) 09 September 2022 (2022-09-09) description, page 1, paragraph 4 to page 40, paragraph 3, embodiments, and table 10 | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2024** | **26 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/102179

C. DOCUMENTS CONSIDERED TO BE RELEVANT

| Category* | Citation of document, with indication, where appropriate, of the relevant passages | Relevant to claim No. |
|---|---|---|
| X | CN 115968286 A (INVENTISBIO (SHANGHAI) CO., LTD. et al.) 14 April 2023 (2023-04-14)<br>description, paragraphs 0008-0496, and embodiments | 1-15 |
| X | WO 2023020523 A1 (JACOBIO PHARMACEUTICALS CO., LTD.) 23 February 2023 (2023-02-23)<br>description, page 2, line 30 to page 18, line 17, and embodiments | 1-15 |
| A | CN 115974896 A (GUANGDONG HEC PHARMACEUTICAL CO., LTD.) 18 April 2023 (2023-04-18)<br>embodiment | 1-15 |
| A | WO 2022105859 A1 (JACOBIO PHARMACEUTICALS CO., LTD.) 27 May 2022 (2022-05-27)<br>entire description | 1-15 |
| A | CN 115836055 A (INVENTISBIO (SHANGHAI) CO., LTD.) 21 March 2023 (2023-03-21)<br>entire description | 1-15 |

Form PCT/ISA/210 (second sheet) (July 2022)

**INTERNATIONAL SEARCH REPORT**
**Information on patent family members**

International application No.
**PCT/CN2024/102179**

| Patent document cited in search report | Publication date (day/month/year) | Patent family member(s) | Publication date (day/month/year) |
|---|---|---|---|
| WO 2024061333 A1 | 28 March 2024 | None | |
| CN 117800975 A | 02 April 2024 | WO 2024067714 A1 | 04 April 2024 |
| WO 2024041606 A1 | 29 February 2024 | None | |
| WO 2023138583 A1 | 27 July 2023 | TW 202330536 A | 01 August 2023 |
| | | TWI 841200 B | 01 May 2024 |
| WO 2022184178 A1 | 09 September 2022 | None | |
| CN 115968286 A | 14 April 2023 | EP 4204412 A1 | 05 July 2023 |
| | | WO 2022042630 A1 | 03 March 2022 |
| | | JP 2023540228 A | 22 September 2023 |
| | | US 2023357233 A1 | 09 November 2023 |
| WO 2023020523 A1 | 23 February 2023 | None | |
| CN 115974896 A | 18 April 2023 | WO 2023061463 A1 | 20 April 2023 |
| | | EP 4417606 A1 | 21 August 2024 |
| WO 2022105859 A1 | 27 May 2022 | US 2024059710 A1 | 22 February 2024 |
| CN 115836055 A | 21 March 2023 | US 2023242544 A1 | 03 August 2023 |
| | | WO 2022002102 A1 | 06 January 2022 |
| | | JP 2023531269 A | 21 July 2023 |
| | | EP 4175947 A1 | 10 May 2023 |
| | | EP 4175947 A4 | 03 July 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20211041671 A
- WO 2023061463 A
- WO 2023138662 A


### Non-patent literature cited in the description


- *Nature*, 2013, vol. 503, 548-551
- McGraw-Hill Dictionary of Chemical Terms. McGraw-Hill Book Company, 1984
- **ELIEL, E.** ; **WILEN, S.** Stereochemistry of Organic Compounds. John Wiley & Sons, Inc., 1994
- **S. M. BERGE et al.** *J. Pharmaceutical Sciences*, 1977, vol. 66, 1-19
- Remington: The Science and Practice of Pharmacy. Lippincott Williams& Wilkins, 2005
- Encyclopedia of Pharmaceutical Technology. Marcel Dekker, 1988